# EUROPEAN PATENT SPECIFICATION

(11) **EP 2 115 003 B1**
(45) Date of publication and mention of the grant of the patent: **06.06.2012**
(21) Application number: 07862940.9
(22) Date of filing: 14.12.2007
(51) Int. Cl.: C07K 16/22, A61K 39/395, A61P 35/00

(54) **HUMAN ANTIBODIES TO HUMAN DELTA LIKE LIGAND 4**
HUMANE ANTIKÖRPER GEGEN HUMAN DELTA LIKE-LIGANDEN 4
ANTICORPS HUMAINS AU LIGAND DELTA-4 HUMAIN

(30) Priority: 14.12.2006 US 874922 P; 07.05.2007 US 916415 P; 05.11.2007 US 985323 P
(43) Date of publication of application: 11.11.2009
(60) Divisional application: 11190612.9
(73) Proprietor: Regeneron Pharmaceuticals, Inc., Tarrytown, NY 10591 (US)
(72) Inventor: PAPADOPOULOS, Nicholas J., Lagrangeville, NY 12540 (US); MARTIN, Joel H., Putnam Valley, NY 10579 (US); SMITH, Eric, New York, NY 10016 (US); NOGUERA-TROISE, Irene, Bay Shore, NY 11706 (US); THURSTON, Gavin, White Plains, NY 10603 (US)
(74) Representative: Bentham, Andrew
(86) International application number: PCT/US2007/025653
(87) International publication number: WO 2008/076379

(56) References cited:
- WO-A-2008/060705
- US-A1- 2006 134 121
- LAURET E ET AL: "Membrane-bound Delta-4 Notch ligand reduces the proliferative activity of primitive human hematopoietic CD34+CD38low cells while maintaining their LTC-IC potential" 20040401; 20040400, vol. 18, no. 4, 1 April 2004 (2004-04-01), pages 788-797, XP002421237
- WILLIAMS CASSIN KIMMEL ET AL: "Up-regulation of the Notch ligand Delta-like 4 inhibits VEGF-induced endothelial cell function" BLOOD, W.B.SAUNDERS COMPANY, ORLANDO, FL, vol. 107, no. 3, 1 February 2006 (2006-02-01), pages 931-939, XP002457722 ISSN: 0006-4971
- HAINAUD PATRICIA ET AL: "The role of the vascular endothelial growth factor-Delta-like 4 ligand/Notch4-ephrin B2 cascade in tumor vessel remodeling and endothelial cell functions." CANCER RESEARCH 1 SEP 2006, vol. 66, no. 17, 1 September 2006 (2006-09-01), pages 8501-8510, XP002484535 ISSN: 1538-7445
- DANDO JONATHAN S ET AL: "Notch/Delta4 interaction in human embryonic liver CD34+ CD38- cells: positive influence on BFU-E production and LTC-IC potential maintenance." STEM CELLS (DAYTON, OHIO) APR 2005, vol. 23, no. 4, April 2005 (2005-04), pages 550-560, XP002484536 ISSN: 1066-5099
- SUGIMOTO ET AL: "Delta-4 Notch ligand promotes erythroid differentiation of human umbilical cord blood CD34<+> cells" EXPERIMENTAL HEMATOLOGY, NEW YORK, NY, US, vol. 34, no. 4, 1 April 2006 (2006-04-01), pages 424-432, XP005353748 ISSN: 0301-472X
- DORSCH MARION ET AL: "Ectopic expression of Delta4 impairs hematopoietic development and leads to lymphoproliferative disease." BLOOD 15 SEP 2002, vol. 100, no. 6, 15 September 2002 (2002-09-15), pages 2046-2055, XP002484537 ISSN: 0006-4971
- LIU ZHAO-JUN ET AL: "Inhibition of endothelial cell proliferation by Notch1 signaling is mediated by repressing MAPK and PI3K/Akt pathways and requires MAML1." THE FASEB JOURNAL : OFFICIAL PUBLICATION OF THE FEDERATION OF AMERICAN SOCIETIES FOR EXPERIMENTAL BIOLOGY MAY 2006, vol. 20, no. 7, May 2006 (2006-05), pages E201-E210, XP002484538 ISSN: 1530-6860
- NOGUERA-TROISE IRENE ET AL: "Blockade of Dll4 inhibits tumour growth by promoting non-productive angiogenesis" NATURE, NATURE PUBLISHING GROUP, LONDON, vol. 444, no. 7122, 21 December 2006 (2006-12-21), pages 1032-1037, XP002452162 ISSN: 0028-0836

### SEQUENCE LISTING

<110> Regeneron Pharmaceuticals, Inc.
<120> Human Antibodies to Human Delta Like Ligand 4
<130> 6040A-WO
<140> To be assigned
   <141> 2007-12-14
<150> 60/874,922
   <151> 2006-12-14
<150> 60/916,415
   <151> 2007-05-07
<150> 60/985,323
   <151> 2007-11-05
<160> 957
<170> FastSEQ for Windows Version 4.0
<210> 1
   <211> 2058
   <212> DNA
   <213> Homo sapiens
<400> 1
<210> 2
   <211> 685
   <212> PRT
   <213> Homo sapiens
<400> 2
<210> 3
   <211> 358
   <212> DNA
   <213> Artificial Sequence
<220>
   <223> Synthetic
<400> 3
   gaggtgcaac tggtggagtc tgggggaggc ttggtccagc ctggggggtc cctgagactc 60
   tcctgtgcag cctctggatt cacctttagt acctattgga tgaactgggt ccgccaggct 120
   ccagggaagg ggctggagtg ggtggccaac ataaaccaag atggaagtga gaaatactat 180
   gtggactctg tgaagggccg aatcaccatc tccagagaca acgccaagaa ctcactgtat 240
   ctgcaaatga acagcctgag agtcgaggac acggctgtat attactgtgc gagaaaatgg 300
   aacaactgga acccggagga gaactggggc cagggaaccc tggtcaccgt ctcctcag   358
<210> 4
   <211> 119
   <212> PRT
   <213> Artificial Sequence
<220>
   <223> Synthetic
<400> 4
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Thr Tyr
               20                  25                  30
   Trp Met Asn Trp Val Arg Gln Ala Pro Gly Lys Gly Leu Glu Trp Val
           35                  40                  45
   Ala Asn Ile Asn Gln Asp Gly Ser Glu Lys Tyr Tyr Val Asp Ser Val
       50                  55                  60
   Lys Gly Arg Ile Thr Ile Ser Arg Asp Asn Ala Lys Asn Ser Leu Tyr
   65                  70                  75                  80
   Leu Gln Met Asn Ser Leu Arg Val Glu Asp Thr Ala Val Tyr Tyr Cys
                   85                  90                  95
   Ala Arg Lys Trp Asn Asn Trp Asn Pro Glu Glu Asn Trp Gly Gln Gly
               100                 105                 110
   Thr Leu Val Thr Val Ser Ser
           115
<210> 5
   <211> 24
   <212> DNA
   <213> Artificial Sequence
<220>
   <223> Synthetic
<400> 5
   ggattcacct ttagtaccta ttgg 24
<210> 6
   <211> 8
   <212> PRT
   <213> Artificial Sequence
<220>
   <223> Synthetic
<400> 6
   Gly Phe Thr Phe Ser Thr Tyr Trp
    1               5
<210> 7
   <211> 24
   <212> DNA
   <213> Artificial Sequence
<220>
   <223> Synthetic
<400> 7
   ataaaccaag atggaagtga gaaa 24
<210> 8
   <211> 8
   <212> PRT
   <213> Artificial Sequence
<220>
   <223> Synthetic
<400> 8
   Ile Asn Gln Asp Gly Ser Glu Lys
   1 5
<210> 9
   <211> 36
   <212> DNA
   <213> Artificial Sequence
<220>
   <223> Synthetic
<400> 9
   gcgagaaaat ggaacaactg gaacccggag gagaac 36
<210> 10
   <211> 12
   <212> PRT
   <213> Artificial Sequence
<220>
   <223> Synthetic
<400> 10
   Ala Arg Lys Trp Asn Asn Trp Asn Pro Glu Glu Asn
   1 5 10
<210> 11
   <211> 337
   <212> DNA
   <213> Artificial Sequence
<220>
   <223> Synthetic
<400> 11
   gatattgtga tgactcagtc tccactctcc ctgcccgtca cccctggaga gccggcctcc 60
   atctcctgca ggtctagtca gagcctcctg cataatagtg gatacaactt tttggattgg 120
   tacctgcaga agccagggca gtctccacaa ctcctgatct atttgcgttc taatcgggcc 180
   tccggggtcc ctgacaggtt cagtggcagt ggatcaggca cagattttac actgaagatc 240
   agaagagtgg aggctgagga tgttgggatt tattactgca tgcaagctct acacactcct 300
   tacacttttg gccaggggac caaggtggag atcaaac 337
<210> 12
   <211> 112
   <212> PRT
   <213> Artificial Sequence
<220>
   <223> Synthetic
<400> 12
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Asn
               20                  25                  30
   Ser Gly Tyr Asn Phe Leu Asp Trp Tyr Leu Gln Lys Pro Gly Gln Ser
           35                  40                  45
   Pro Gln Leu Leu Ile Tyr Leu Arg Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Arg Arg Val Glu Ala Glu Asp Val Gly Ile Tyr Tyr Cys Met Gln Ala
                   85                  90                  95
   Leu His Thr Pro Tyr Thr Phe Gly Gln Gly Thr Lys Val Glu Ile Lys
               100                 105                 110
<210> 13
   <211> 33
   <212> DNA
   <213> Artificial Sequence
<220>
   <223> Synthetic
<400> 13
   cagagcctcc tgcataatag tggatacaac ttt 33
<210> 14
   <211> 11
   <212> PRT
   <213> Artificial Sequence
<220>
   <223> Synthetic
<400> 14
   Gln Ser Leu Leu His Asn Ser Gly Tyr Asn Phe
   1               5                   10
<210> 15
   <211> 9
   <212> DNA
   <213> Artificial Sequence
<220>
   <223> Synthetic
<400> 15
   ttgcgttct 9
<210> 16
   <211> 3
   <212> PRT
   <213> Artificial Sequence
<220>
   <223> Synthetic
<400> 16
   Leu Arg Ser
   1
<210> 17
   <211> 27
   <212> DNA
   <213> Artificial Sequence
<220>
   <223> Synthetic
<400> 17
   atgcaagctc tacacactcc ttacact 27
<210> 18
   <211> 9
   <212> PRT
   <213> Artificial Sequence
<220>
   <223> Synthetic
<400> 18
   Met Gln Ala Leu His Thr Pro Tyr Thr
   1 5
<210> 19
   <211> 376
   <212> DNA
   <213> Artificial Sequence
<220>
   <223> Synthetic
<400> 19
   gaggtgcagc tggtggagtc tgggggaggc ttggtccagc ctggggggtc cctgagactc 60
   tcctgtgtag cctctggatt cacctttagt agctattgga tgacctgggt ccgccaggct 120
   ccagggaagg ggctggagtg ggtggccaac ataaaacaag atggaagtga gaaatactat 180
   gtggactctg tgaagggccg attcaccgtc tccagagaca acgccaagaa ctcagtgtat 240
   ctgcaaatga gcagcctgag agccgaggac acggctgtgt attactgtgc gagagattgg 300
   aactatggcc ccgattacta ctactaccac ggtttggacg tctggggcca agggaccacg 360
   gtcaccgtct cctcag 376
<210> 20
   <211> 125
   <212> PRT
   <213> Artificial Sequence
<220>
   <223> Synthetic
<400> 20
   Glu Val Gln Leu Val Glu Ser Gly Gly Gly Leu Val Gln Pro Gly Gly
    1               5                  10                  15
   Ser Leu Arg Leu Ser Cys Val Ala Ser Gly Phe Thr Phe Ser Ser Tyr
               20                  25                  30
   Trp Met Thr Trp Val Arg Gln Ala Pro Gly Lys Gly Leu Glu Trp Val
           35                  40                  45
   Ala Asn Ile Lys Gln Asp Gly Ser Glu Lys Tyr Tyr Val Asp Ser Val
       50                  55                  60
   Lys Gly Arg Phe Thr Val Ser Arg Asp Asn Ala Lys Asn Ser Val Tyr
   65                  70                  75                  80
   Leu Gln Met Ser Ser Leu Arg Ala Glu Asp Thr Ala Val Tyr Tyr Cys
                   85                  90                  95
   Ala Arg Asp Trp Asn Tyr Gly Pro Asp Tyr Tyr Tyr Tyr His Gly Leu
               100                 105                 110
   Asp Val Trp Gly Gln Gly Thr Thr Val Thr Val Ser Ser
           115                 120                 125
<210> 21
   <211> 24
   <212> DNA
   <213> Artificial Sequence
<220>
   <223> Synthetic
<400> 21
   ggattcacct ttagtagcta ttgg 24
<210> 22
   <211> 8
   <212> PRT
   <213> Artificial Sequence
<220>
   <223> Synthetic
<400> 22
   Gly Phe Thr Phe Ser Ser Tyr Trp
    1               5
<210> 23
   <211> 24
   <212> DNA
   <213> Artificial Sequence
<220>
   <223> Synthetic
<400> 23
   ataaaacaag atggaagtga gaaa 24
<210> 24
   <211> 8
   <212> PRT
   <213> Artificial Sequence
<220>
   <223> Synthetic
<400> 24
   Ile Lys Gln Asp Gly Ser Glu Lys
   1 5
<210> 25
   <211> 54
   <212> DNA
   <213> Artificial Sequence
<220>
   <223> Synthetic
<400> 25
   gcgagagatt ggaactatgg ccccgattac tactactacc acggtttgga cgtc 54
<210> 26
   <211> 18
   <212> PRT
   <213> Artificial Sequence
<220>
   <223> Synthetic
<400> 26
   Ala Arg Asp Trp Asn Tyr Gly Pro Asp Tyr Tyr Tyr Tyr His Gly Leu
   1 5 10 15
   Asp Val
<210> 27
   <211> 322
   <212> DNA
   <213> Artificial Sequence
<220>
   <223> Synthetic
<400> 27
   gacctccaga tgacccagtc tccgtcctcc ctgtctgtat ctgtaggaga cagagtcacc 60
   atcacttgcc gggcaagtca gggcattaga aatgatttag gctggtttca gcagaaacca 120
   gggaaagccc ctaagcgcct gatctatgct gcatccagtt tgcaaagtgg ggtcccatca 180
   aggttcagcg gcagtggatc tgggacagaa ttcactctca caatcagcag cctgctgcct 240
   gaagattttg caacttatta ctgtctacag cataatactt acccgtacac ttttggccag 300
   gggaccaagc tggagatcaa ac 322
<210> 28
   <211> 107
   <212> PRT
   <213> Artificial Sequence
<220>
   <223> Synthetic
<400> 28
   Asp Leu Gln Met Thr Gln Ser Pro Ser Ser Leu Ser Val Ser Val Gly
    1               5                  10                  15
   Asp Arg Val Thr Ile Thr Cys Arg Ala Ser Gln Gly Ile Arg Asn Asp
               20                  25                  30
   Leu Gly Trp Phe Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Leu Pro
   65                  70                  75                  80
   Glu Asp Phe Ala Thr Tyr Tyr Cys Leu Gln His Asn Thr Tyr Pro Tyr
                   85                  90                  95
   Thr Phe Gly Gln Gly Thr Lys Leu Glu Ile Lys
               100                 105
<210> 29
   <211> 18
   <212> DNA
   <213> Artificial Sequence
<220>
   <223> Synthetic
<400> 29
   cagggcatta gaaatgat 18
<210> 30
   <211> 6
   <212> PRT
   <213> Artificial Sequence
<220>
   <223> Synthetic
<400> 30
   Gln Gly Ile Arg Asn Asp
    1               5
<210> 31
   <211> 9
   <212> DNA
   <213> Artificial Sequence
<220>
   <223> Synthetic
<400> 31
   gctgcatcc 9
<210> 32
   <211> 3
   <212> PRT
   <213> Artificial Sequence
<220>
   <223> Synthetic
<400> 32
   Ala Ala Ser
   1
<210> 33
   <211> 27
   <212> DNA
   <213> Artificial Sequence
<220>
   <223> Synthetic
<400> 33
   ctacagcata atacttaccc gtacact 27
<210> 34
   <211> 9
   <212> PRT
   <213> Artificial Sequence
<220>
   <223> Synthetic
<400> 34
   Leu Gln His Asn Thr Tyr Pro Tyr Thr
   1 5
<210> 35
   <211> 361
   <212> DNA
   <213> Artificial Sequence
<220>
   <223> Synthetic
<400> 35
   caggggcagt tggtggagtc tgggggaggc gtggtccagc ctgggaggtc cctgagactc 60
   tcctgtgaag catctggatt cagtttcaga agttatggca tgcactgggt ccgccaggct 120
   ccaggcaggg gactggagtg gatggcagtt atttggtacg atggcagtaa gacatactat 180
   acagagtccg tgacgggccg attcaccatc tccagagaca attccaagaa cacgctatat 240
   ctgcaaatga acagcctgag agccgaggac acggctgttt attactgtgc gagcggtttt 300
   tcagtgcctg ccacgatcct tgacaactgg ggccagggaa ccctggtctc cgtctcctca 360
   g 361
<210> 36
   <211> 120
   <212> PRT
   <213> Artificial Sequence
<220>
   <223> Synthetic
<400> 36
   Gln Gly Gln Leu Val Glu Ser Gly Gly Gly Val Val Gln Pro Gly Arg
   1 5 10 15
   Ser Leu Arg Leu Ser Cys Glu Ala Ser Gly Phe Ser Phe Arg Ser Tyr
   20 25 30
   Gly Met His Trp Val Arg Gln Ala Pro Gly Arg Gly Leu Glu Trp Met
   35 40 45
   Ala Val Ile Trp Tyr Asp Gly Ser Lys Thr Tyr Tyr Thr Glu Ser Val
   50 55 60
   Thr Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Ser Gly Phe Ser Val Pro Ala Thr Ile Leu Asp Asn Trp Gly Gln
   100 105 110
   Gly Thr Leu Val Ser Val Ser Ser
   115 120
<210> 37
   <211> 24
   <212> DNA
   <213> Artificial Sequence
<220>
   <223> Synthetic
<400> 37
   ggattcagtt tcagaagtta tggc 24
<210> 38
   <211> 8
   <212> PRT
   <213> Artificial Sequence
<220>
   <223> Synthetic
<400> 38
   Gly Phe Ser Phe Arg Ser Tyr Gly
   1 5
<210> 39
   <211> 24
   <212> DNA
   <213> Artificial Sequence
<220>
   <223> Synthetic
<400> 39
   atttggtacg atggcagtaa gaca 24
<210> 40
   <211> 8
   <212> PRT
   <213> Artificial Sequence
<220>
   <223> Synthetic
<400> 40
   Ile Trp Tyr Asp Gly Ser Lys Thr
   1 5
<210> 41
   <211> 39
   <212> DNA
   <213> Artificial Sequence
<220>
   <223> Synthetic
<400> 41
   gcgagcggtt tttcagtgcc tgccacgatc cttgacaac 39
<210> 42
   <211> 13
   <212> PRT
   <213> Artificial Sequence
<220>
   <223> Synthetic
<400> 42
   Ala Ser Gly Phe Ser Val Pro Ala Thr Ile Leu Asp Asn
   1 5 10
<210> 43
   <211> 322
   <212> DNA
   <213> Artificial Sequence
<220>
   <223> Synthetic
<400> 43
   gacatccaga tgacccagtc tccatcctcc ctgtctgcat ctgtaggaga cagagtcacc 60
   atcacttgcc gggcaagtca gggcattaga aatgatttag gctggtttca gcagaaacca 120
   gggaaagccc ctaaccgcct gatctatgga gcatccagtt tggaaggtgg ggtcccatca 180
   aggttcagcg gcagtggatc tgggacagat ttcactctca caatcagcag cctgcagcca 240
   gaagattttg caacttatta ctgtctacag cataattctt acccgtggac gttcggccaa 300
   gggaccaagg tggaaatcaa ac 322
<210> 44
   <211> 107
   <212> PRT
   <213> Artificial Sequence
<220>
   <223> Synthetic
<400> 44
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Asp
               20                  25                  30
   Leu Gly Trp Phe Gln Gln Lys Pro Gly Lys Ala Pro Asn Arg Leu Ile
           35                  40                  45
   Tyr Gly Ala Ser Ser Leu Glu Gly Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Trp
                   85                  90                  95
   Thr Phe Gly Gln Gly Thr Lys Val Glu Ile Lys
               100                 105
<210> 45
   <211> 18
   <212> DNA
   <213> Artificial Sequence
<220>
   <223> Synthetic
<400> 45
   cagggcatta gaaatgat 18
<210> 46
   <211> 6
   <212> PRT
   <213> Artificial Sequence
<220>
   <223> Synthetic
<400> 46
   Gln Gly Ile Arg Asn Asp
    1               5
<210> 47
   <211> 9
   <212> DNA
   <213> Artificial Sequence
<220>
   <223> Synthetic
<400> 47
   ggagcatcc 9
<210> 48
   <211> 3
   <212> PRT
   <213> Artificial Sequence
<220>
   <223> Synthetic
<400> 48
   Gly Ala Ser
   1
<210> 49
   <211> 27
   <212> DNA
   <213> Artificial Sequence
<220>
   <223> Synthetic
<400> 49
   ctacagcata attcttaccc gtggacg 27
<210> 50
   <211> 9
   <212> PRT
   <213> Artificial Sequence
<220>
   <223> Synthetic
<400> 50
   Leu Gln His Asn Ser Tyr Pro Trp Thr
   1 5
<210> 51
   <211> 367
   <212> DNA
   <213> Artificial Sequence
<220>
   <223> Synthetic
<400> 51
   caggtgcagc tggtggagtc tgggggaggc gtagtccagc ctgggaggtc cctgagactc 60
   tcctgtgcag cgtctggatt caccttcagt agctatggca tgcactgggt ccgccaggct 120
   ccaggcaagg ggctggagtg ggtggcagtt atatggtatg atggaaataa taaatactat 180
   atagactccg tgaagggccg attcaccatc tccagagaca attccaagaa cacgctgtat 240
   ctgcaaatga acagcctgag agccgaggac acggctgtgt attactgtgc gagagaccgt 300
   ggatatagtg gctacgaggg atacttcgat ctctggggcc gtggcaccct ggtcactgtc 360
   tcctcag 367
<210> 52
   <211> 122
   <212> PRT
   <213> Artificial Sequence
<220>
   <223> Synthetic
<400> 52
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Trp Tyr Asp Gly Asn Asn Lys Tyr Tyr Ile Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp Arg Gly Tyr Ser Gly Tyr Glu Gly Tyr Phe Asp Leu Trp
   100 105 110
   Gly Arg Gly Thr Leu Val Thr Val Ser Ser
   115 120
<210> 53
   <211> 24
   <212> DNA
   <213> Artificial Sequence
<220>
   <223> Synthetic
<400> 53
   ggattcacct tcagtagcta tggc 24
<210> 54
   <211> 8
   <212> PRT
   <213> Artificial Sequence
<220>
   <223> Synthetic
<400> 54
   Gly Phe Thr Phe Ser Ser Tyr Gly
   1 5
<210> 55
   <211> 24
   <212> DNA
   <213> Artificial Sequence
<220>
   <223> Synthetic
<400> 55
   atatggtatg atggaaataa taaa 24
<210> 56
   <211> 8
   <212> PRT
   <213> Artificial Sequence
<220>
   <223> Synthetic
<400> 56
   Ile Trp Tyr Asp Gly Asn Asn Lys
   1 5
<210> 57
   <211> 45
   <212> DNA
   <213> Artificial Sequence
<220>
   <223> Synthetic
<400> 57
   gcgagagacc gtggatatag tggctacgag ggatacttcg atctc 45
<210> 58
   <211> 15
   <212> PRT
   <213> Artificial Sequence
<220>
   <223> Synthetic
<400> 58
   Ala Arg Asp Arg Gly Tyr Ser Gly Tyr Glu Gly Tyr Phe Asp Leu
   1 5 10 15
<210> 59
   <211> 337
   <212> DNA
   <213> Artificial Sequence
<220>
   <223> Synthetic
<400> 59
   gacatcgtga tgacccagtc tccagactcc ctggctgtgt ctctgggcga gagggccacc 60
   ctcaactgta agtccagcca gagtgtttta tacagctcca acaataagaa ctacttagct 120
   tggtaccagc agaaaccagg acagcctcct aagctgctca tttactgggc atctacccgg 180
   gaatccgggg tccctgaccg attcagtggc agcgggtctg ggacagattt cactctcacc 240
   atcagcagcc tgcaggctga agatgtggca gtttatttct gtcagcaata ttatactact 300
   tggacgttcg gccaagggac caaggtggaa atcaaac 337
<210> 60
   <211> 112
   <212> PRT
   <213> Artificial Sequence
<220>
   <223> Synthetic
<400> 60
   Asp Ile Val Met Thr Gln Ser Pro Asp Ser Leu Ala Val Ser Leu Gly
   1 5 10 15
   Glu Arg Ala Thr Leu Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Ser Leu Gln Ala Glu Asp Val Ala Val Tyr Phe Cys Gln Gln
   85 90 95
   Tyr Tyr Thr Thr Trp Thr Phe Gly Gln Gly Thr Lys Val Glu Ile Lys
   100 105 110
<210> 61
   <211> 36
   <212> DNA
   <213> Artificial Sequence
<220>
   <223> Synthetic
<400> 61
   cagagtgttt tatacagctc caacaataag aactac 36
<210> 62
   <211> 12
   <212> PRT
   <213> Artificial Sequence
<220>
   <223> Synthetic
<400> 62
   Gln Ser Val Leu Tyr Ser Ser Asn Asn Lys Asn Tyr
   1 5 10
<210> 63
   <211> 9
   <212> DNA
   <213> Artificial Sequence
<220>
   <223> Synthetic
<400> 63
   tgggcatct 9
<210> 64
   <211> 3
   <212> PRT
   <213> Artificial Sequence
<220>
   <223> Synthetic
<400> 64
   Trp Ala Ser
   1
<210> 65
   <211> 24
   <212> DNA
   <213> Artificial Sequence
<220>
   <223> Synthetic
<400> 65
   cagcaatatt atactacttg gacg 24
<210> 66
   <211> 8
   <212> PRT
   <213> Artificial Sequence
<220>
   <223> Synthetic
<400> 66
   Gln Gln Tyr Tyr Thr Thr Trp Thr
   1 5
<210> 67
   <211> 385
   <212> DNA
   <213> Artificial Sequence
<220>
   <223> Synthetic
<400> 67
   caggtgcagt tggtggagtc tgggggaggc gtggtccagc ctgggaggtc cctgagactc 60
   tcctgtgcag cctctgcatc caccttcagt aggcatggca tgcactgggt ccgccaggct 120
   ccaggcaagg gactggagtg ggtggcagtt atatcatatg atggaaataa taaatactat 180
   gtagactccg tgaagggccg attcaccatc tccagagaca attccaagaa cacgctgtat 240
   ctgcaaatga atagcctgag aactgacgac acggctgtgt attattgttc gaaagagtta 300
   gtaggtatta ctggaaacct ggtctactac tactactacg gaatggacgt ctggggccaa 360
   gggaccacgg tcaccgtctc ctcag 385
<210> 68
   <211> 128
   <212> PRT
   <213> Artificial Sequence
<220>
   <223> Synthetic
<400> 68
   Gln Val Gln Leu Val Glu Ser Gly Gly Gly Val Val Gln Pro Gly Arg
   1 5 10 15
   Ser Leu Arg Leu Ser Cys Ala Ala Ser Ala Ser Thr Phe Ser Arg His
   20 25 30
   Gly Met His Trp Val Arg Gln Ala Pro Gly Lys Gly Leu Glu Trp Val
   35 40 45
   Ala Val Ile Ser Tyr Asp Gly Asn Asn Lys Tyr Tyr Val Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Thr Asp Asp Thr Ala Val Tyr Tyr Cys
   85 90 95
   Ser Lys Glu Leu Val Gly Ile Thr Gly Asn Leu Val Tyr Tyr Tyr Tyr
   100 105 110
   Tyr Gly Met Asp Val Trp Gly Gln Gly Thr Thr Val Thr Val Ser Ser
   115 120 125
<210> 69
   <211> 24
   <212> DNA
   <213> Artificial Sequence
<220>
   <223> Synthetic
<400> 69
   gcatccacct tcagtaggca tggc 24
<210> 70
   <211> 8
   <212> PRT
   <213> Artificial Sequence
<220>
   <223> Synthetic
<400> 70
   Ala Ser Thr Phe Ser Arg His Gly
   1 5
<210> 71
   <211> 24
   <212> DNA
   <213> Artificial Sequence
<220>
   <223> Synthetic
<400> 71
   atatcatatg atggaaataa taaa 24
<210> 72
   <211> 8
   <212> PRT
   <213> Artificial Sequence
<220>
   <223> Synthetic
<400> 72
   Ile Ser Tyr Asp Gly Asn Asn Lys
   1 5
<210> 73
   <211> 63
   <212> DNA
   <213> Artificial Sequence
<220>
   <223> Synthetic
<400> 73
   tcgaaagagt tagtaggtat tactggaaac ctggtctact actactacta cggaatggac 60
   gtc 63
<210> 74
   <211> 21
   <212> PRT
   <213> Artificial Sequence
<220>
   <223> Synthetic
<400> 74
   Ser Lys Glu Leu Val Gly Ile Thr Gly Asn Leu Val Tyr Tyr Tyr Tyr
   1 5 10 15
   Tyr Gly Met Asp Val
   20
<210> 75
   <211> 325
   <212> DNA
   <213> Artificial Sequence
<220>
   <223> Synthetic
<400> 75
   gaaattgtgt tgacgcagtc tccaggcacc ctgtctttgt ctccagggga aagagccacc 60
   ctctcctgca gggccagtca gactattaac agcagctact taggctggta ccagcagaaa 120
   cctggccagg ctcccagact cctcatctat ggtgcatcca acagggccac tggcatccca 180
   gacagtttca gtggcagtgg gtctgggaca gacttcactc tcaccatcag cagactggag 240
   cctgaagatt ttgcagtgta ttactgtcaa cattataaca actcacctta cacttttggc 300
   caggggacca agctggagat caaac 325
<210> 76
   <211> 108
   <212> PRT
   <213> Artificial Sequence
<220>
   <223> Synthetic
<400> 76
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Thr Ile Asn Ser Ser
   20 25 30
   Tyr Leu Gly Trp Tyr Gln Gln Lys Pro Gly Gln Ala Pro Arg Leu Leu
   35 40 45
   Ile Tyr Gly Ala Ser Asn Arg Ala Thr Gly Ile Pro Asp Ser Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln His Tyr Asn Asn Ser Pro
   85 90 95
   Tyr Thr Phe Gly Gln Gly Thr Lys Leu Glu Ile Lys
   100 105
<210> 77
   <211> 21
   <212> DNA
   <213> Artificial Sequence
<220>
   <223> Synthetic
<400> 77
   cagactatta acagcagcta c 21
<210> 78
   <211> 7
   <212> PRT
   <213> Artificial Sequence
<220>
   <223> Synthetic
<400> 78
   Gln Thr Ile Asn Ser Ser Tyr
   1 5
<210> 79
   <211> 9
   <212> DNA
   <213> Artificial Sequence
<220>
   <223> Synthetic
<400> 79
   ggtgcatcc 9
<210> 80
   <211> 3
   <212> PRT
   <213> Artificial Sequence
<220>
   <223> Synthetic
<400> 80
   Gly Ala Ser
   1
<210> 81
   <211> 27
   <212> DNA
   <213> Artificial Sequence
<220>
   <223> Synthetic
<400> 81
   caacattata acaactcacc ttacact 27
<210> 82
   <211> 9
   <212> PRT
   <213> Artificial Sequence
<220>
   <223> Synthetic

<400> 82
   Gln His Tyr Asn Asn Ser Pro Tyr Thr
   1 5
<210> 83
   <211> 361
   <212> DNA
   <213> Artificial Sequence
<220>
   <223> Synthetic
<400> 83
   caggtgcagc tgcaggagtc gggcccagga ctggtgaagc cttcacagac cctgtccctc 60
   acctgcactg tctctggtgg ctccataagc agtggtggtt actactggag ctggatccgc 120
   cactacccag ggaagggcct ggagtggatt ggctacgtcc attacagtgg gaacacccac 180
   tacaatacgt ccctcaagag gcgacttacc atatcaatag acacgtctaa gagccaattc 240
   tccctggatc tgagctctgt gactgccgcg gacacggccg tgtattactg tgcgagagcc 300
   ccccgtggat accattactt tgcctactgg ggccagggaa ccctggtcac cgtctcctca 360
   g 361
<210> 84
   <211> 120
   <212> PRT
   <213> Artificial Sequence
<220>
   <223> Synthetic
<400> 84
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg His Tyr Pro Gly Lys Gly Leu Glu
           35                  40                  45
   Trp Ile Gly Tyr Val His Tyr Ser Gly Asn Thr His Tyr Asn Thr Ser
       50                  55                  60
   Leu Lys Arg Arg Leu Thr Ile Ser Ile Asp Thr Ser Lys Ser Gln Phe
   65                  70                  75                  80
   Ser Leu Asp Leu Ser Ser Val Thr Ala Ala Asp Thr Ala Val Tyr Tyr
                   85                  90                  95
   Cys Ala Arg Ala Pro Arg Gly Tyr His Tyr Phe Ala Tyr Trp Gly Gln
               100                 105                 110
   Gly Thr Leu Val Thr Val Ser Ser
           115                 120
<210> 85
   <211> 30
   <212> DNA
   <213> Artificial Sequence
<220>
   <223> Synthetic
<400> 85
   ggtggctcca taagcagtgg tggttactac 30
<210> 86
   <211> 10
   <212> PRT
   <213> Artificial Sequence
<220>
   <223> Synthetic
<400> 86
   Gly Gly Ser Ile Ser Ser Gly Gly Tyr Tyr
   1               5                   10
<210> 87
   <211> 21
   <212> DNA
   <213> Artificial Sequence
<220>
   <223> Synthetic
<400> 87
   gtccattaca gtgggaacac c 21
<210> 88
   <211> 7
   <212> PRT
   <213> Artificial Sequence
<220>
   <223> Synthetic
<400> 88
   Val His Tyr Ser Gly Asn Thr
   1 5
<210> 89
   <211> 36
   <212> DNA
   <213> Artificial Sequence
<220>
   <223> Synthetic
<400> 89
   gcgagagccc cccgtggata ccattacttt gcctac 36
<210> 90
   <211> 12
   <212> PRT
   <213> Artificial Sequence
<220>
   <223> Synthetic
<400> 90
   Ala Arg Ala Pro Arg Gly Tyr His Tyr Phe Ala Tyr
   1 5 10
<210> 91
   <211> 325
   <212> DNA
   <213> Artificial Sequence
<220>
   <223> Synthetic
<400> 91
   gaaattgggt tgacgcagtc tccaggcacc ctgtctttgt ctccagggga aagagccacc 60
   ctctcctgca gggccagtca gagtattagc agcaggtact tagcctggta ccagcagaaa 120
   cctggccagg ctcccaggct cctcatcttt ggtgcatcca gcagggccac tggcatacca 180
   gacaggttca gtggcagtgg gtctgggaca gacttcactc tcaccatcag cagactggag 240
   cctgaagatt ttgcagtgta ttactgtcag cagtatggta gctcaccgct cactttcggc 300
   ggagggacca aggtggagat caaac 325
<210> 92
   <211> 108
   <212> PRT
   <213> Artificial Sequence
<220>
   <223> Synthetic
<400> 92
   Glu Ile Gly Leu Thr Gln Ser Pro Gly Thr Leu Ser Leu Ser Pro Gly
   1 5 10 15
   Glu Arg Ala Thr Leu Ser Cys Arg Ala Ser Gln Ser Ile Ser Ser Arg
   20 25 30
   Tyr Leu Ala Trp Tyr Gln Gln Lys Pro Gly Gln Ala Pro Arg Leu Leu
   35 40 45
   Ile Phe Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Pro
   85 90 95
   Leu Thr Phe Gly Gly Gly Thr Lys Val Glu Ile Lys
   100 105
<210> 93
   <211> 21
   <212> DNA
   <213> Artificial Sequence
<220>
   <223> Synthetic
<400> 93
   cagagtatta gcagcaggta c 21
<210> 94
   <211> 7
   <212> PRT
   <213> Artificial Sequence
<220>
   <223> Synthetic
<400> 94
   Gln Ser Ile Ser Ser Arg Tyr
   1 5
<210> 95
   <211> 9
   <212> DNA
   <213> Artificial Sequence
<220>
   <223> Synthetic
<400> 95
   ggtgcatcc 9
<210> 96
   <211> 3
   <212> PRT
   <213> Artificial Sequence
<220>
   <223> Synthetic
<400> 96
   Gly Ala Ser
   1
<210> 97
   <211> 27
   <212> DNA
   <213> Artificial Sequence
<220>
   <223> Synthetic
<400> 97
   cagcagtatg gtagctcacc gctcact 27
<210> 98
   <211> 9
   <212> PRT
   <213> Artificial Sequence
<220>
   <223> Synthetic
<400> 98
   Gln Gln Tyr Gly Ser Ser Pro Leu Thr
   1 5
<210> 99
   <211> 361
   <212> DNA
   <213> Artificial Sequence
<220>
   <223> Synthetic
<400> 99
   caggtgcagc tgcaggagtc gggcccagga ctggtgaagc cttcacagac cctgtccctc 60
   acctgcactg tctctggtgg ctccatcagc agtagtggtt actactggag ttggatccgc 120
   cagcacccag ggaagggcct ggagtggatt gggtacgtcc attacagtgg gaacacccac 180
   tacagcccgt ccctcaagag tcgacttacc atatcagttg acacgtctaa gaaccagttc 240
   tccctgaagc tgagctctgt gactgccgcg gacacggccg tgtattactg tgcgagagcc 300
   ccccgtggat accattactt tgcctactgg ggccagggaa ccctggtcac cgtctcctca 360
   g 361
<210> 100
   <211> 120
   <212> PRT
   <213> Artificial Sequence
<220>
   <223> Synthetic
<400> 100
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Ser Ser Ser
   20 25 30
   Gly Tyr Tyr Trp Ser Trp Ile Arg Gln His Pro Gly Lys Gly Leu Glu
   35 40 45
   Trp Ile Gly Tyr Val His Tyr Ser Gly Asn Thr His Tyr Ser Pro Ser
   50 55 60
   Leu Lys Ser Arg Leu Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Ala Pro Arg Gly Tyr His Tyr Phe Ala Tyr Trp Gly Gln
   100 105 110
   Gly Thr Leu Val Thr Val Ser Ser
   115 120
<210> 101
   <211> 30
   <212> DNA
   <213> Artificial Sequence
<220>
   <223> Synthetic
<400> 101
   ggtggctcca tcagcagtag tggttactac 30
<210> 102
   <211> 10
   <212> PRT
   <213> Artificial Sequence
<220>
   <223> Synthetic
<400> 102
   Gly Gly Ser Ile Ser Ser Ser Gly Tyr Tyr
   1 5 10
<210> 103
   <211> 21
   <212> DNA
   <213> Artificial Sequence
<220>
   <223> Synthetic
<400> 103
   gtccattaca gtgggaacac c 21
<210> 104
   <211> 7
   <212> PRT
   <213> Artificial Sequence
<220>
   <223> Synthetic
<400> 104
   Val His Tyr Ser Gly Asn Thr
   1               5
<210> 105
   <211> 36
   <212> DNA
   <213> Artificial Sequence
<220>
   <223> Synthetic
<400> 105
   gcgagagccc cccgtggata ccattacttt gcctac 36
<210> 106
   <211> 12
   <212> PRT
   <213> Artificial Sequence
<220>
   <223> Synthetic
<400> 106
   Ala Arg Ala Pro Arg Gly Tyr His Tyr Phe Ala Tyr
   1               5                   10
<210> 107
   <211> 325
   <212> DNA
   <213> Artificial Sequence
<220>
   <223> Synthetic
<400> 107
   aaaattgtgt tgacgcagtc tccaggcacc ctgtctttgt ctccagggga gagagccacc 60
   ctctcctgca gggccagtca gagtattagc agcagctact tagcctggta ccagcagaaa 120
   cctggccagg ctcccaggct cctcatcttt ggtgcatcca gcagggccac tggcatccca 180
   gacaggttca gtggcagtgg gtctgggcca gacttcactc tcaccattag gagactggag 240
   cctgaagatt ttgcggtgta ttactgtcag cagtatggta gttcaccgct cactttcggc 300
   ggagggacca aggtggagat caaac                                       325
<210> 108
   <211> 108
   <212> PRT
   <213> Artificial Sequence
<220>
   <223> Synthetic
<400> 108
   Lys Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Ile Ser Ser Ser
               20                  25                  30
   Tyr Leu Ala Trp Tyr Gln Gln Lys Pro Gly Gln Ala Pro Arg Leu Leu
           35                  40                  45
   Ile Phe Gly Ala Ser Ser Arg Ala Thr Gly Ile Pro Asp Arg Phe Ser
       50                  55                  60
   Gly Ser Gly Ser Gly Pro Asp Phe Thr Leu Thr Ile Arg Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gly Gly Thr Lys Val Glu Ile Lys
               100                 105
<210> 109
   <211> 21
   <212> DNA
   <213> Artificial Sequence
<220>
   <223> Synthetic
<400> 109
   cagagtatta gcagcagcta c 21
<210> 110
   <211> 7
   <212> PRT
   <213> Artificial Sequence
<220> <223> Synthetic
<400> 110
   Gln Ser Ile Ser Ser Ser Tyr
   1               5
<210> 111
   <211> 9
   <212> DNA
   <213> Artificial Sequence
<220>
   <223> Synthetic
<400> 111
   ggtgcatcc 9
<210> 112
   <211> 3
   <212> PRT
   <213> Artificial Sequence
<220>
   <223> Synthetic
<400> 112
   Gly Ala Ser
   1
<210> 113
   <211> 27
   <212> DNA
   <213> Artificial Sequence
<220>
   <223> Synthetic
<400> 113
   cagcagtatg gtagttcacc gctcact 27
<210> 114
   <211> 9
   <212> PRT
   <213> Artificial Sequence
<220>
   <223> Synthetic
<400> 114
   Gln Gln Tyr Gly Ser Ser Pro Leu Thr
   1 5
<210> 115
   <211> 358
   <212> DNA
   <213> Artificial Sequence
<220>
   <223> Synthetic
<400> 115
   caggttcagt tggtgcagtc tggagctgag gtgaagaagc ctggggcctc agtgaaggtc 60
   tcctgcaagg cttctggtta caccttttcc acctatggta tcagctgggt gcgacaggcc 120
   cctggacaag ggcttgagtg gatgggatgg atcagcgctt acgacaataa cgcggactat 180
   gcacagaact tccaggccag agtcaccatg accacagaca catccacgac cacagcctac 240
   atggagctga ggagcctgag atctgacgac acggccgtgt attactgtgc gaggtatagc 300
   tggaactttc actggttcga cccctggggc cagggaaccc tggtcaccgt ctcctcag 358
<210> 116
   <211> 119
   <212> PRT
   <213> Artificial Sequence
<220>
   <223> Synthetic
<400> 116
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Ser Thr Tyr
               20                  25                  30
   Gly Ile Ser Trp Val Arg Gln Ala Pro Gly Gln Gly Leu Glu Trp Met
           35                  40                  45
   Gly Trp Ile Ser Ala Tyr Asp Asn Asn Ala Asp Tyr Ala Gln Asn Phe
       50                  55                  60
   Gln Ala Arg Val Thr Met Thr Thr Asp Thr Ser Thr Thr Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Tyr Ser Trp Asn Phe His Trp Phe Asp Pro Trp Gly Gln Gly
               100                 105                 110
   Thr Leu Val Thr Val Ser Ser
           115
<210> 117
   <211> 24
   <212> DNA
   <213> Artificial Sequence
<220>
   <223> Synthetic
<400> 117
   ggttacacct tttccaccta tggt 24
<210> 118
   <211> 8
   <212> PRT
   <213> Artificial Sequence
<220>
   <223> Synthetic
<400> 118
   Gly Tyr Thr Phe Ser Thr Tyr Gly
   1               5
<210> 119
   <211> 24
   <212> DNA
   <213> Artificial Sequence
<220>
   <223> Synthetic
<400> 119
   atcagcgctt acgacaataa cgcg 24
<210> 120
   <211> 8
   <212> PRT
   <213> Artificial Sequence
<220>
   <223> Synthetic
<400> 120
   Ile Ser Ala Tyr Asp Asn Asn Ala
   1 5
<210> 121
   <211> 36
   <212> DNA
   <213> Artificial Sequence
<220>
   <223> Synthetic
<400> 121
   gcgaggtata gctggaactt tcactggttc gacccc 36
<210> 122
   <211> 12
   <212> PRT
   <213> Artificial Sequence
<220>
   <223> Synthetic
<400> 122
   Ala Arg Tyr Ser Trp Asn Phe His Trp Phe Asp Pro
   1 5 10
<210> 123
   <211> 325
<212> DNA
   <213> Artificial Sequence
<220>
   <223> Synthetic
<400> 123
   gaaattgtgt tgacgcagtc tccaggcacc ctgtctttgt ctccagggga aagagccacc 60
   ctctcctgca gggccagtca gagtgttagc agtacctact tagcctggta ccagcagcaa 120
   cctggccagg ctcccaggct cctcatctat ggtgcatcca gcagggccac tggcatccca 180
   gacaggttca gtggcagtgg gtctgggaca gacttcactc tcaccatcag cagactggag 240
   cctgaagatt ttgcagtgta ttactgtcag cagtatggta actcaccgtg gacgttcggc 300
   caagggacca aggtggaaat caaac                                       325
<210> 124
   <211> 108
   <212> PRT
   <213> Artificial Sequence
<220>
   <223> Synthetic
<400> 124
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Thr
               20                  25                  30
   Tyr Leu Ala Trp Tyr Gln Gln Gln Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Asn Ser Pro
                   85                  90                  95
   Trp Thr Phe Gly Gln Gly Thr Lys Val Glu Ile Lys
               100                 105
<210> 125
   <211> 21
   <212> DNA
   <213> Artificial Sequence
<220>
   <223> Synthetic
<400> 125
   cagagtgtta gcagtaccta c 21
<210> 126
   <211> 7
   <212> PRT
   <213> Artificial Sequence
<220>
   <223> Synthetic
<400> 126
   Gln Ser Val Ser Ser Thr Tyr
   1               5
<210> 127
   <211> 9
   <212> DNA
   <213> Artificial Sequence
<220>
   <223> Synthetic
<400> 127
   ggtgcatcc 9
<210> 128
   <211> 3
   <212> PRT
   <213> Artificial Sequence
<220>
   <223> Synthetic
<400> 128
   Gly Ala Ser
   1
<210> 129
   <211> 27
   <212> DNA
   <213> Artificial Sequence
<220>
   <223> Synthetic
<400> 129
   cagcagtatg gtaactcacc gtggacg 27
<210> 130
   <211> 9
   <212> PRT
   <213> Artificial Sequence
<220>
   <223> Synthetic
<400> 130
   Gln Gln Tyr Gly Asn Ser Pro Trp Thr
   1 5
<210> 131
   <211> 358
   <212> DNA
   <213> Artificial Sequence
<220>
   <223> Synthetic
<400> 131
   caggttcacc tggtgcagtc tggagctgag gtgaagaagc ctggggcctc agtgaaggtc 60
   tcctgcaggg cttctggtta cacctttacc aactatggta tcacctgggt gcgacaggcc 120
   cctggacaag ggcttgagtg gatgggatgg atcagcgctt acagtggtaa cacagactat 180
   gcacagaagt tccaggccag aatcaccatg accacagaca catccacgac cacagcctac 240
   atggaactga ggagcctgac atctgacgac acggccgtgt attactgtgc gaggtatagc 300
   tggaactttc actggttcga cccctggggc cagggaaccc tggtcaccgt ctcctcag 358
<210> 132
   <211> 119
   <212> PRT
   <213> Artificial Sequence
<220>
   <223> Synthetic
<400> 132
   Gln Val His Leu Val Gln Ser Gly Ala Glu Val Lys Lys Pro Gly Ala
   1 5 10 15
   Ser Val Lys Val Ser Cys Arg Ala Ser Gly Tyr Thr Phe Thr Asn Tyr
   20 25 30
   Gly Ile Thr Trp Val Arg Gln Ala Pro Gly Gln Gly Leu Glu Trp Met
   35 40 45
   Gly Trp Ile Ser Ala Tyr Ser Gly Asn Thr Asp Tyr Ala Gln Lys Phe
   50 55 60
   Gln Ala Arg Ile Thr Met Thr Thr Asp Thr Ser Thr Thr Thr Ala Tyr
   65 70 75 80
   Met Glu Leu Arg Ser Leu Thr Ser Asp Asp Thr Ala Val Tyr Tyr Cys
   85 90 95
   Ala Arg Tyr Ser Trp Asn Phe His Trp Phe Asp Pro Trp Gly Gln Gly
   100 105 110
   Thr Leu Val Thr Val Ser Ser
   115
<210> 133
   <211> 24
   <212> DNA
   <213> Artificial Sequence
<220>
   <223> Synthetic
<400> 133
   ggttacacct ttaccaacta tggt 24
<210> 134
   <211> 8
   <212> PRT
   <213> Artificial Sequence
<220>
   <223> Synthetic
<400> 134
   Gly Tyr Thr Phe Thr Asn Tyr Gly
   1 5
<210> 135
   <211> 24
   <212> DNA
   <213> Artificial Sequence
<220>
   <223> Synthetic
<400> 135
   atcagcgctt acagtggtaa caca 24
<210> 136
   <211> 8
   <212> PRT
   <213> Artificial Sequence
<220>
   <223> Synthetic
<400> 136
   Ile Ser Ala Tyr Ser Gly Asn Thr
   1               5
<210> 137
   <211> 36
   <212> DNA
   <213> Artificial Sequence
<220>
   <223> Synthetic
<400> 137
   gcgaggtata gctggaactt tcactggttc gacccc 36
<210> 138
   <211> 12
   <212> PRT
   <213> Artificial Sequence
<220>
   <223> Synthetic
<400> 138
   Ala Arg Tyr Ser Trp Asn Phe His Trp Phe Asp Pro
   1               5                   10
<210> 139
   <211> 325
   <212> DNA
   <213> Artificial Sequence
<220>
   <223> Synthetic
<400> 139
   gaaattgtgt tgacgcagtc tccaggcacc ctgtctttgt ctccagggga cagagtcacc 60
   ctctcctgca gggccagtca gagtgttagt agcagctact tagcctggta ccagcagaaa 120
   cctggccagg ctcccaggct cctcatctat ggtgcatcca gcagggccac tggcatccca 180
   gacaggttca gtggcagtgg gtctgggaca gacttcactc tcaccatcag cagtctggag 240
   cctgaagatt ttgcagtgta ttactgtcag cagtgtggtg gctcaccgtg gacgttcggc 300
   caagggacca gggtggagat caaac 325
<210> 140
   <211> 108
   <212> PRT
   <213> Artificial Sequence
<220>
   <223> Synthetic
<400> 140
   Glu Ile Val Leu Thr Gln Ser Pro Gly Thr Leu Ser Leu Ser Pro Gly
   1 5 10 15
   Asp Arg Val Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Ser Leu Glu
   65 70 75 80
   Pro Glu Asp Phe Ala Val Tyr Tyr Cys Gln Gln Cys Gly Gly Ser Pro
   85 90 95
   Trp Thr Phe Gly Gln Gly Thr Arg Val Glu Ile Lys
   100 105
<210> 141
   <211> 21
   <212> DNA
   <213> Artificial Sequence
<220>
   <223> Synthetic
<400> 141
   cagagtgtta gtagcagcta c 21
<210> 142
   <211> 7
   <212> PRT
   <213> Artificial Sequence
<220>
   <223> Synthetic
<400> 142
   Gln Ser Val Ser Ser Ser Tyr
   1 5
<210> 143
   <211> 9
   <212> DNA
   <213> Artificial Sequence
<220>
   <223> Synthetic
<400> 143
   ggtgcatcc 9
<210> 144
   <211> 3
   <212> PRT
   <213> Artificial Sequence
<220>
   <223> Synthetic
<400> 144
   Gly Ala Ser
   1
<210> 145
   <211> 27
   <212> DNA
   <213> Artificial Sequence
<220>
   <223> Synthetic
<400> 145
   cagcagtgtg gtggctcacc gtggacg 27
<210> 146
   <211> 9
   <212> PRT
   <213> Artificial Sequence
<220>
   <223> Synthetic
<400> 146
   Gln Gln Cys Gly Gly Ser Pro Trp Thr
   1 5
<210> 147
   <211> 358
   <212> DNA
   <213> Artificial Sequence
<220>
   <223> Synthetic
<400> 147
   caggttcaac tggtgcagtc tgggcctgag gtgaagaagc ctggggcctc agtgaaggtc 60
   gcctgcaagg cttctggtta cacctttacc cactatggtt tcacctgggt gcgacaggcc 120
   cctggacaag ggcttgagtg gatgggatgg atcagcgctt acagtggtca tacagactat 180
   gcacggaagt tccaggccag agtcaccatg accacagaca cattcacgac cacagcctac 240
   atggaactga ggagcctgag atctgacgac acggccgttt attactgtgc gagttatagc 300
   tggaactttc actggttcga cccctggggc cagggaaccc tggtcaccgt ctcctcag 358
<210> 148
   <211> 119
   <212> PRT
   <213> Artificial Sequence
<220>
   <223> Synthetic
<400> 148
   Gln Val Gln Leu Val Gln Ser Gly Pro Glu Val Lys Lys Pro Gly Ala
    1               5                  10                  15
   Ser Val Lys Val Ala Cys Lys Ala Ser Gly Tyr Thr Phe Thr His Tyr
               20                  25                  30
   Gly Phe Thr Trp Val Arg Gln Ala Pro Gly Gln Gly Leu Glu Trp Met
           35                  40                  45
   Gly Trp Ile Ser Ala Tyr Ser Gly His Thr Asp Tyr Ala Arg Lys Phe
       50                  55                  60
   Gln Ala Arg Val Thr Met Thr Thr Asp Thr Phe Thr Thr Thr Ala Tyr
   65                  70                  75                  80
   Met Glu Leu Arg Ser Leu Arg Ser Asp Asp Thr Ala Val Tyr Tyr Cys
                   85                  90                  95
   Ala Ser Tyr Ser Trp Asn Phe His Trp Phe Asp Pro Trp Gly Gln Gly
               100                 105                 110
   Thr Leu Val Thr Val Ser Ser
           115
<210> 149
   <211> 24
   <212> DNA
   <213> Artificial Sequence
<220>
   <223> Synthetic
<400> 149
   ggttacacct ttacccacta tggt 24
<210> 150
   <211> 8
   <212> PRT
   <213> Artificial Sequence
<220>
   <223> Synthetic
<400> 150
   Gly Tyr Thr Phe Thr His Tyr Gly
    1               5
<210> 151
   <211> 24
   <212> DNA
   <213> Artificial Sequence
<220>
   <223> Synthetic
<400> 151
   atcagcgctt acagtggtca taca 24
<210> 152
   <211> 8
   <212> PRT
   <213> Artificial Sequence
<220>
   <223> Synthetic
<400> 152
   Ile Ser Ala Tyr Ser Gly His Thr
   1 5
<210> 153
   <211> 36
   <212> DNA
   <213> Artificial Sequence
<220>
   <223> Synthetic
<400> 153
   gcgagttata gctggaactt tcactggttc gacccc 36
<210> 154
   <211> 12
   <212> PRT
   <213> Artificial Sequence
<220>
   <223> Synthetic
<400> 154
   Ala Ser Tyr Ser Trp Asn Phe His Trp Phe Asp Pro
   1 5 10
<210> 155
   <211> 325
   <212> DNA
   <213> Artificial Sequence
<220>
   <223> Synthetic
<400> 155
   caaattgtgt tgacgcagtc tccaggcacc ctgtctttgt ctccagggga aagagccacc 60
   ctctcctgca gggccagtca gagtgttagt accacctact tagcctggta ccagcagaaa 120
   cctggccagg ctcccagtct cctcatctat ggtacatcca ccagggccac tggcatccca 180
   gacaggttca gtggcagtgg gtctgggact gacttcactc tcaccatcag cagactggag 240
   cctgaagatt ttgcagtgta ttactgtcag cagtgtggtg gctcaccgtg gacgttcggc 300
   caagggacca aggtgaaaat caaac 325
<210> 156
   <211> 108
   <212> PRT
   <213> Artificial Sequence
<220>
   <223> Synthetic
<400> 156
   Gln Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Thr Thr
   20 25 30
   Tyr Leu Ala Trp Tyr Gln Gln Lys Pro Gly Gln Ala Pro Ser Leu Leu
   35 40 45
   Ile Tyr Gly Thr Ser Thr Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Cys Gly Gly Ser Pro
   85 90 95
   Trp Thr Phe Gly Gln Gly Thr Lys Val Lys Ile Lys
   100 105
<210> 157
   <211> 21
   <212> DNA
   <213> Artificial Sequence
<220>
   <223> Synthetic
<400> 157
   cagagtgtta gtaccaccta c 21
<210> 158
   <211> 7
   <212> PRT
   <213> Artificial Sequence
<220>
   <223> Synthetic
<400> 158
   Gln Ser Val Ser Thr Thr Tyr
   1 5
<210> 159
   <211> 9
   <212> DNA
   <213> Artificial Sequence
<220>
   <223> Synthetic
<400> 159
   ggtacatcc 9
<210> 160
   <211> 3
   <212> PRT
   <213> Artificial Sequence
<220>
   <223> Synthetic
<400> 160
   Gly Thr Ser
   1
<210> 161
   <211> 27
   <212> DNA
   <213> Artificial Sequence
<220>
   <223> Synthetic
<400> 161
   cagcagtgtg gtggctcacc gtggacg 27
<210> 162
   <211> 9
   <212> PRT
   <213> Artificial Sequence
<220>
   <223> Synthetic
<400> 162
   Gln Gln Cys Gly Gly Ser Pro Trp Thr
   1 5
<210> 163
   <211> 367
   <212> DNA
   <213> Artificial Sequence
<220>
   <223> Synthetic
<400> 163
   caggtgcagt tggtgcagtc tggggctgag gtgaagaagc ctggggccgc tgtgaaggtc 60
   tcctgcaagg cttctggata cacgttcacc agttatgata tcaactgggt gcgacaggcc 120
   actggacaag ggcttgagtg gatgggatgg ataaacccta acagtggtaa cacaggctat 180
   gcacagaagt ttcagggcag agtcaccttg accaggaaca cctccataag cacagcctac 240
   atggaactga gcagcctgag atctgaggac acggccgttt attactgtgc gagagaggga 300
   tattgtggtg gtgattgcta tgcttttgat atctggggcc aagggacaat ggtcaccgtc 360
   tcttcag 367
<210> 164
   <211> 122
   <212> PRT
   <213> Artificial Sequence
<220>
   <223> Synthetic
<400> 164
   Gln Val Gln Leu Val Gln Ser Gly Ala Glu Val Lys Lys Pro Gly Ala
   1               5                   10                  15
   Ala Val Lys Val Ser Cys Lys Ala Ser Gly Tyr Thr Phe Thr Ser Tyr
               20                  25                  30
   Asp Ile Asn Trp Val Arg Gln Ala Thr Gly Gln Gly Leu Glu Trp Met
           35                  40                  45
   Gly Trp Ile Asn Pro Asn Ser Gly Asn Thr Gly Tyr Ala Gln Lys Phe
       50                  55                  60
   Gln Gly Arg Val Thr Leu Thr Arg Asn Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Glu Gly Tyr Cys Gly Gly Asp Cys Tyr Ala Phe Asp Ile Trp
               100                 105                 110
   Gly Gln Gly Thr Met Val Thr Val Ser Ser
           115                 120
<210> 165
   <211> 24
   <212> DNA
   <213> Artificial Sequence
<220>
   <223> Synthetic
<400> 165
   ggatacacgt tcaccagtta tgat 24
<210> 166
   <211> 8
   <212> PRT
   <213> Artificial Sequence
<220>
   <223> Synthetic
<400> 166
   Gly Tyr Thr Phe Thr Ser Tyr Asp
   1               5
<210> 167
   <211> 24
   <212> DNA
   <213> Artificial Sequence
<220>
   <223> Synthetic
<400> 167
   ataaacccta acagtggtaa caca 24
<210> 168
   <211> 8
   <212> PRT
   <213> Artificial Sequence
<220>
   <223> Synthetic
<400> 168
   Ile Asn Pro Asn Ser Gly Asn Thr
   1 5
<210> 169
   <211> 45
   <212> DNA
   <213> Artificial Sequence
<220>
   <223> Synthetic
<400> 169
   gcgagagagg gatattgtgg tggtgattgc tatgcttttg atatc 45
<210> 170
   <211> 15
   <212> PRT
   <213> Artificial Sequence
<220>
   <223> Synthetic
<400> 170
   Ala Arg Glu Gly Tyr Cys Gly Gly Asp Cys Tyr Ala Phe Asp Ile
   1 5 10 15
<210> 171
   <211> 325
   <212> DNA
   <213> Artificial Sequence
<220>
   <223> Synthetic
<400> 171
   gaaattgtgt tgacacaatc tccaggcacc ctgtctttgt ctccagggga aagagccacc 60
   ctctcctgca gggccagtca gagtgttagc agcagctact tagcctggta ccagcagaaa 120
   cctggccagg ctcccaggct cctcatcttt ggtgcatcca gcagggccac tggcatccca 180
   gacaggttca gtggcagtgg gtctgggaca gacttcactc tcaccatcag cagactggag 240
   cctgaagatt ttgcagtgta ttactgtcag cagtatggta gctcaccgct cactttcggc 300
   ggagggacca aggtggagat caaac 325
<210> 172
   <211> 108
   <212> PRT
   <213> Artificial Sequence
<220>
   <223> Synthetic
<400> 172
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Phe Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Pro
   85 90 95
   Leu Thr Phe Gly Gly Gly Thr Lys Val Glu Ile Lys
   100 105

<210> 173
   <211> 21
   <212> DNA
   <213> Artificial Sequence
<220>
   <223> Synthetic
<400> 173
   cagagtgtta gcagcagcta c 21
<210> 174
   <211> 7
   <212> PRT
   <213> Artificial Sequence
<220>
   <223> Synthetic
<400> 174
   Gln Ser Val Ser Ser Ser Tyr
   1 5
<210> 175
   <211> 9
   <212> DNA
   <213> Artificial Sequence
<220>
   <223> Synthetic
<400> 175
   ggtgcatcc 9
<210> 176
   <211> 3
   <212> PRT
   <213> Artificial Sequence
<220>
   <223> Synthetic
<400> 176
   Gly Ala Ser
   1
<210> 177
   <211> 27
   <212> DNA
   <213> Artificial Sequence
<220>
   <223> Synthetic
<400> 177
   cagcagtatg gtagctcacc gctcact 27
<210> 178
   <211> 9
   <212> PRT
   <213> Artificial Sequence
<220>
   <223> Synthetic
<400> 178
   Gln Gln Tyr Gly Ser Ser Pro Leu Thr
   1 5
<210> 179
   <211> 361
   <212> DNA
   <213> Artificial Sequence
<220>
   <223> Synthetic
<400> 179
   cagctgcagc tgcaggagtc gggcccagga ctggtgaagc cttcggagac cctgtccctc 60
   acctgcactg tctctggtgg ctccatcagc agtagtagtt actactgggg ctggatccgc 120
   cagcccccag ggaaggggct ggagtggatt gggagtatct attatagtgg gagcacctac 180
   tacaacccgt ccctcaagag tcgagtcacc atatccgtag acacgtccaa gaaccagttc 240
   tccctgaagc tgagctctgt gaccgccgca gacacggctg tgtattactg tgcggcaaac 300
   tgggacgacg ccttcttctt tgactactgg ggccagggaa ccctggtcac cgtctcctca 360
   g 361
<210> 180
   <211> 120
   <212> PRT
   <213> Artificial Sequence
<220>
   <223> Synthetic
<400> 180
   Gln Leu Gln Leu Gln Glu Ser Gly Pro Gly Leu Val Lys Pro Ser Glu
   1 5 10 15
   Thr Leu Ser Leu Thr Cys Thr Val Ser Gly Gly Ser Ile Ser Ser Ser
   20 25 30
   Ser Tyr Tyr Trp Gly Trp Ile Arg Gln Pro Pro Gly Lys Gly Leu Glu
   35 40 45
   Trp Ile Gly Ser Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Ala Asn Trp Asp Asp Ala Phe Phe Phe Asp Tyr Trp Gly Gln
   100 105 110
   Gly Thr Leu Val Thr Val Ser Ser
   115 120
<210> 181
   <211> 30
   <212> DNA
   <213> Artificial Sequence
<220>
   <223> Synthetic
<400> 181
   ggtggctcca tcagcagtag tagttactac 30
<210> 182
   <211> 10
   <212> PRT
   <213> Artificial Sequence
<220>
   <223> Synthetic
<400> 182
   Gly Gly Ser Ile Ser Ser Ser Ser Tyr Tyr
   1 5 10
<210> 183
   <211> 21
   <212> DNA
   <213> Artificial Sequence
<220>
   <223> Synthetic
<400> 183
   atctattata gtgggagcac c 21
<210> 184
   <211> 7
   <212> PRT
   <213> Artificial Sequence
<220>
   <223> Synthetic
<400> 184
   Ile Tyr Tyr Ser Gly Ser Thr
   1 5
<210> 185
   <211> 36
   <212> DNA
   <213> Artificial Sequence
<220>
   <223> Synthetic
<400> 185
   gcggcaaact gggacgacgc cttcttcttt gactac 36
<210> 186
   <211> 12
   <212> PRT
   <213> Artificial Sequence
<220>
   <223> Synthetic
<400> 186
   Ala Ala Asn Trp Asp Asp Ala Phe Phe Phe Asp Tyr
   1 5 10
<210> 187
   <211> 322
   <212> DNA
   <213> Artificial Sequence
<220>
   <223> Synthetic
<400> 187
   gacatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tataatagtt attcgtacac ttttggccag 300
   gggaccaagc tggagatcaa ac 322
<210> 188
   <211> 107
   <212> PRT
   <213> Artificial Sequence
<220>
   <223> Synthetic
<400> 188
   Asp Ile Gln Met Thr Gln Ser Pro Ser Thr Leu Ser Ala Ser Val Gly
    1               5                   10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Ser Tyr Ser Tyr
                   85                  90                  95
   Thr Phe Gly Gln Gly Thr Lys Leu Glu Ile Lys
               100                 105
<210> 189
   <211> 18
   <212> DNA
   <213> Artificial Sequence
<220>
   <223> Synthetic
<400> 189
   cagagtatta gtagctgg 18
<210> 190
   <211> 6
   <212> PRT
   <213> Artificial Sequence
<220>
   <223> Synthetic
<400> 190
   Gln Ser Ile Ser Ser Trp
    1               5
<210> 191
   <211> 9
   <212> DNA
   <213> Artificial Sequence
<220>
   <223> Synthetic
<400> 191
   aaggcgtct 9
<210> 192
   <211> 3
   <212> PRT
   <213> Artificial Sequence
<220>
   <223> Synthetic
<400> 192
   Lys Ala Ser
   1
<210> 193
   <211> 27
   <212> DNA
   <213> Artificial Sequence
<220>
   <223> Synthetic
<400> 193
   caacagtata atagttattc gtacact 27
<210> 194
   <211> 9
   <212> PRT
   <213> Artificial Sequence
<220>
   <223> Synthetic
<400> 194
   Gln Gln Tyr Asn Ser Tyr Ser Tyr Thr
   1 5
<210> 195
   <211> 352
   <212> DNA
   <213> Artificial Sequence
<220>
   <223> Synthetic
<400> 195
   caggtgcagc tggtgcagtc tggggctgag gtgaagaagc ctggggcctc agtgaaggtc 60
   tcctgcaagg cttctggata caccttcacc ggctactata ttcactgggt acgacaggcc 120
   cctggacaag gccttgagtg gatgggatgg atcaacccta acagtggtgg cacaaactat 180
   gcacagaagt ttcagggcag ggtcaccatg accagggaca cgtccatcac cacagcctac 240
   atggagctga gcaggctgat atctgacgac acggccgtgt attactgtgc gagaggaccc 300
   tgggatttct ttgactactg gggccaggga accctggtca ccgtctcctc ag 352
<210> 196
   <211> 117
   <212> PRT
   <213> Artificial Sequence
<220>
   <223> Synthetic
<400> 196
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Ile His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Thr Thr Ala Tyr
   65 70 75 80
   Met Glu Leu Ser Arg Leu Ile Ser Asp Asp Thr Ala Val Tyr Tyr Cys
   85 90 95
   Ala Arg Gly Pro Trp Asp Phe Phe Asp Tyr Trp Gly Gln Gly Thr Leu
   100 105 110
   Val Thr Val Ser Ser
   115
<210> 197
   <211> 24
   <212> DNA
   <213> Artificial Sequence
<220>
   <223> Synthetic
<400> 197
   ggatacacct tcaccggcta ctat 24
<210> 198
   <211> 8
   <212> PRT
   <213> Artificial Sequence
<220>
   <223> Synthetic
<400> 198
   Gly Tyr Thr Phe Thr Gly Tyr Tyr
   1 5
<210> 199
   <211> 24
   <212> DNA
   <213> Artificial Sequence
<220>
   <223> Synthetic
<400> 199
   atcaacccta acagtggtgg caca 24
<210> 200
   <211> 8
   <212> PRT
   <213> Artificial Sequence
<220>
   <223> Synthetic
<400> 200
   Ile Asn Pro Asn Ser Gly Gly Thr
   1 5
<210> 201
   <211> 30
   <212> DNA
   <213> Artificial Sequence
<220>
   <223> Synthetic
<400> 201
   gcgagaggac cctgggattt ctttgactac 30
<210> 202
   <211> 10
   <212> PRT
   <213> Artificial Sequence
<220>
   <223> Synthetic
<400> 202
   Ala Arg Gly Pro Trp Asp Phe Phe Asp Tyr
   1 5 10
<210> 203
   <211> 340
   <212> DNA
   <213> Artificial Sequence
<220>
   <223> Synthetic
<400> 203
   gacatcgtga tgacccagtc tccagactcc ctggctgtgt ctctgggcga gagggccacc 60
   atcaactgca agtccagcca gagtgtttta tacagctcca acaataagaa ctacttagct 120
   tggtaccagc agaaaccagg acagcctcct aagctgctca tttactgggc atctacccgg 180
   gaatccgggg tccctgaccg attcagtggc agcgggtctg ggacagattt cactctcacc 240
   atcagcagcc tgcaggctga agatgtggca gtttattact gtcagcaata ttatagtact 300
   ccgtacactt ttggccaggg gaccaagctg gagatcaaac 340
<210> 204
   <211> 113
   <212> PRT
   <213> Artificial Sequence
<220>
   <223> Synthetic
<400> 204
   Asp Ile Val Met Thr Gln Ser Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Gln
   85 90 95
   Tyr Tyr Ser Thr Pro Tyr Thr Phe Gly Gln Gly Thr Lys Leu Glu Ile
   100 105 110
   Lys
<210> 205
   <211> 36
   <212> DNA
   <213> Artificial Sequence
<220>
   <223> Synthetic
<400> 205
   cagagtgttt tatacagctc caacaataag aactac 36
<210> 206
   <211> 12
   <212> PRT
   <213> Artificial Sequence
<220>
   <223> Synthetic
<400> 206
   Gln Ser Val Leu Tyr Ser Ser Asn Asn Lys Asn Tyr
   1 5 10
<210> 207
   <211> 9
   <212> DNA
   <213> Artificial Sequence
<220>
   <223> Synthetic
<400> 207
   tgggcatct 9
<210> 208
   <211> 3
   <212> PRT
   <213> Artificial Sequence
<220>
   <223> Synthetic
<400> 208
   Trp Ala Ser
   1

<210> 209
   <211> 27
   <212> DNA
   <213> Artificial Sequence
<220>
   <223> Synthetic
<400> 209
   cagcaatatt atagtactcc gtacact 27
<210> 210
   <211> 9
   <212> PRT
   <213> Artificial Sequence
<220>
   <223> Synthetic
<400> 210
   Gln Gln Tyr Tyr Ser Thr Pro Tyr Thr
   1 5
<210> 211
   <211> 352
   <212> DNA
   <213> Artificial Sequence
<220>
   <223> Synthetic
<400> 211
   caggtgcagc tggtgcagtc tggggctgag gtgaagaagc ctggggcctc agtgaaggtc 60
   tcctgcaagg cttctggata caccttcacc ggctactata ttcactgggt acgacaggcc 120
   cctggacaag gccttgagtg gatgggatgg atcaacccta acagtggtgg cacaaactat 180
   gcacagaagt ttcagggcag ggtcaccatg accagggaca cgtccatcag cacagcctac 240
   atggagctga gcaggctgag atctgacgac acggccgtgt attactgtgc gagaggaccc 300
   tgggatttct ttgactactg gggccaggga accctggtca ccgtctcctc ag 352
<210> 212
   <211> 117
   <212> PRT
   <213> Artificial Sequence
<220>
   <223> Synthetic
<400> 212
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Ile His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Gly Pro Trp Asp Phe Phe Asp Tyr Trp Gly Gln Gly Thr Leu
               100                 105                 110
   Val Thr Val Ser Ser
           115
<210> 213
   <211> 24
   <212> DNA
   <213> Artificial Sequence
<220>
   <223> Synthetic
<400> 213
   ggatacacct tcaccggcta ctat 24
<210> 214
   <211> 8
   <212> PRT
   <213> Artificial Sequence
<220>
   <223> Synthetic
<400> 214
   Gly Tyr Thr Phe Thr Gly Tyr Tyr
   1               5
<210> 215
   <211> 24
   <212> DNA
   <213> Artificial Sequence
<220>
   <223> Synthetic
<400> 215
   atcaacccta acagtggtgg caca 24
<210> 216
   <211> 8
   <212> PRT
   <213> Artificial Sequence
<220>
   <223> Synthetic
<400> 216
   Ile Asn Pro Asn Ser Gly Gly Thr
   1 5
<210> 217
   <211> 30
   <212> DNA
   <213> Artificial Sequence
<220>
   <223> Synthetic
<400> 217
   gcgagaggac cctgggattt ctttgactac 30
<210> 218
   <211> 10
   <212> PRT
   <213> Artificial Sequence
<220>
   <223> Synthetic
<400> 218
   Ala Arg Gly Pro Trp Asp Phe Phe Asp Tyr
   1 5 10
<210> 219
   <211> 340
   <212> DNA
   <213> Artificial Sequence
<220>
   <223> Synthetic
<400> 219
   gacatcgtga tgacccagtc tccagactcc ctggctgtgt ctctgggcga gagggccacc 60
   atcaactgca agtccagcca gagtgtttta tacagctcca acaataagaa ctacttagct 120
   tggtaccagc agaaaccagg acagcctcct aagctgctca tttactgggc atctacccgg 180
   gaatccgggg tccctgaccg attcagtggc agcgggtctg ggacagattt cactctcacc 240
   atcagcagcc tgcaggctga agatgtggca gtttattact gtcagcaata ttatagtact 300
   ccgtacactt ttggccaggg gaccaagctg gagatcaaac 340
<210> 220
   <211> 113
   <212> PRT
   <213> Artificial Sequence
<220>
   <223> Synthetic
<400> 220
   Asp Ile Val Met Thr Gln Ser Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Gln
   85 90 95
   Tyr Tyr Ser Thr Pro Tyr Thr Phe Gly Gln Gly Thr Lys Leu Glu Ile
   100 105 110
   Lys
<210> 221
   <211> 36
   <212> DNA
   <213> Artificial Sequence
<220>
   <223> Synthetic
<400> 221
   cagagtgttt tatacagctc caacaataag aactac 36
<210> 222
   <211> 12
   <212> PRT
   <213> Artificial Sequence
<220>
   <223> Synthetic
<400> 222
   Gln Ser Val Leu Tyr Ser Ser Asn Asn Lys Asn Tyr
   1 5 10
<210> 223
   <211> 9
   <212> DNA
   <213> Artificial Sequence
<220>
   <223> Synthetic
<400> 223
   tgggcatct 9
<210> 224
   <211> 3
   <212> PRT
   <213> Artificial Sequence
<220>
   <223> Synthetic
<400> 224
   Trp Ala Ser
   1
<210> 225
   <211> 27
   <212> DNA
   <213> Artificial Sequence
<220>
   <223> Synthetic
<400> 225
   cagcaatatt atagtactcc gtacact 27
<210> 226
   <211> 9
   <212> PRT
   <213> Artificial Sequence
<220>
   <223> Synthetic
<400> 226
   Gln Gln Tyr Tyr Ser Thr Pro Tyr Thr
   1 5
<210> 227
   <211> 361
   <212> DNA
   <213> Artificial Sequence
<220>
   <223> Synthetic
<400> 227
   caggtgcagt tgcaggagtc gggcccagga ctggtgaagc cttcacagac cctgtccctc 60
   acctgcactg tctctggtgg ctccatcggc agtggtggtt actactggag ctggatccgc 120
   cagcacccag ggaagggcct ggagtggatt gggtacgtcc attacagtgg gaacacccac 180
   tacaacccgt ccctcaagag tcgactttcc atatcaatag acacgtctaa gatccagttc 240
   tccctgaagc tgagctctgt gactgccgcg gacacggccg tgtattactg tgcgagagcc 300
   ccccgtggat accattactt tgcctactgg ggccagggaa ccctggtcac cgtctcctca 360
   g 361
<210> 228
   <211> 120
   <212> PRT
   <213> Artificial Sequence
<220>
   <223> Synthetic
<400> 228
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Gly Ser Gly
   20 25 30
   Gly Tyr Tyr Trp Ser Trp Ile Arg Gln His Pro Gly Lys Gly Leu Glu
   35 40 45
   Trp Ile Gly Tyr Val His Tyr Ser Gly Asn Thr His Tyr Asn Pro Ser
   50 55 60
   Leu Lys Ser Arg Leu Ser Ile Ser Ile Asp Thr Ser Lys Ile Gln Phe
   65 70 75 80
   Ser Leu Lys Leu Ser Ser Val Thr Ala Ala Asp Thr Ala Val Tyr Tyr
   85 90 95
   Cys Ala Arg Ala Pro Arg Gly Tyr His Tyr Phe Ala Tyr Trp Gly Gln
   100 105 110
   Gly Thr Leu Val Thr Val Ser Ser
   115 120
<210> 229
   <211> 30
   <212> DNA
   <213> Artificial Sequence
<220>
   <223> Synthetic
<400> 229
   ggtggctcca tcggcagtgg tggttactac 30
<210> 230
   <211> 10
   <212> PRT
   <213> Artificial Sequence
<220>
   <223> Synthetic
<400> 230
   Gly Gly Ser Ile Gly Ser Gly Gly Tyr Tyr
   1 5 10
<210> 231
   <211> 21
   <212> DNA
   <213> Artificial Sequence
<220>
   <223> Synthetic
<400> 231
   gtccattaca gtgggaacac c 21
<210> 232
   <211> 7
   <212> PRT
   <213> Artificial Sequence
<220>
   <223> Synthetic
<400> 232
   Val His Tyr Ser Gly Asn Thr
   1 5
<210> 233
   <211> 36
   <212> DNA
   <213> Artificial Sequence
<220>
   <223> Synthetic
<400> 233
   gcgagagccc cccgtggata ccattacttt gcctac 36
<210> 234
   <211> 12
   <212> PRT
   <213> Artificial Sequence
<220>
   <223> Synthetic
<400> 234
   Ala Arg Ala Pro Arg Gly Tyr His Tyr Phe Ala Tyr
   1 5 10
<210> 235
   <211> 325
   <212> DNA
   <213> Artificial Sequence
<220>
   <223> Synthetic
<400> 235
   gaaattgtgt tgacacaatc tccaggcacc ctgtctttgt ctccagggga aagagccacc 60
   ctctcctgca gggccagtca gagtgttagc agcagctact tagcctggta ccagcagaaa 120
   cctggccagg ctcccaggct cctcatcttt ggtgcatcca gcagggccac tggcatccca 180
   gacaggttca gtggcagtgg gtctgggaca gacttcactc tcaccatcag cagactggag 240
   cctgaagatt ttgcagtgta ttactgtcag cagtatggta gctcaccgct cactttcggc 300
   ggagggacca aggtggagat caaac 325
<210> 236
   <211> 108
   <212> PRT
   <213> Artificial Sequence
<220>
   <223> Synthetic
<400> 236
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Phe Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gly Gly Thr Lys Val Glu Ile Lys
               100                 105
<210> 237
   <211> 21
   <212> DNA
   <213> Artificial Sequence
<220>
   <223> Synthetic
<400> 237
   cagagtgtta gcagcagcta c 21
<210> 238
   <211> 7
   <212> PRT
   <213> Artificial Sequence
<220>
   <223> Synthetic
<400> 238
   Gln Ser Val Ser Ser Ser Tyr
   1               5
<210> 239
   <211> 9
   <212> DNA
   <213> Artificial Sequence
<220>
   <223> Synthetic
<400> 239
   ggtgcatcc 9
<210> 240
   <211> 3
   <212> PRT
   <213> Artificial Sequence
<220>
   <223> Synthetic
<400> 240
   Gly Ala Ser
   1
<210> 241
   <211> 27
   <212> DNA
   <213> Artificial Sequence
<220>
   <223> Synthetic
<400> 241
   cagcagtatg gtagctcacc gctcact 27
<210> 242
   <211> 9
   <212> PRT
   <213> Artificial Sequence
<220>
   <223> Synthetic
<400> 242
   Gln Gln Tyr Gly Ser Ser Pro Leu Thr
   1 5
<210> 243
   <211> 361
   <212> DNA
   <213> Artificial Sequence
<220>
   <223> Synthetic
<400> 243
   caggtgcagc tgcaggagtc gggcccagga ctggtgaagc cttcacagac cctgtccctc 60
   acctgcactg tctctggtgg ctccatcagc agtggtggtt actactggag ctggatccgc 120
   cagtacccag ggaagggcct ggagtggatt ggttacgtcc attacagtgg gagcacccac 180
   tacaacccgt ccctcaagag tcgacttacc atatcaatag acacgtctaa gagccagttc 240
   tccctgaagc tgagctctgt gactgccgcg gacacggccg tgtattactg tgcgagagcc 300
   ccccgtggat accattactt tgcctactgg ggccagggaa ccctggtcac cgtctcctca 360
   g 361
<210> 244
   <211> 120
   <212> PRT
   <213> Artificial Sequence
<220>
   <223> Synthetic
<400> 244
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Ser Ser Gly
   20 25 30
   Gly Tyr Tyr Trp Ser Trp Ile Arg Gln Tyr Pro Gly Lys Gly Leu Glu
   35 40 45
   Trp Ile Gly Tyr Val His Tyr Ser Gly Ser Thr His Tyr Asn Pro Ser
   50 55 60
   Leu Lys Ser Arg Leu Thr Ile Ser Ile Asp Thr Ser Lys Ser Gln Phe
   65 70 75 80
   Ser Leu Lys Leu Ser Ser Val Thr Ala Ala Asp Thr Ala Val Tyr Tyr
   85 90 95
   Cys Ala Arg Ala Pro Arg Gly Tyr His Tyr Phe Ala Tyr Trp Gly Gln
   100 105 110
   Gly Thr Leu Val Thr Val Ser Ser
   115 120
<210> 245
   <211> 30
   <212> DNA
   <213> Artificial Sequence
<220>
   <223> Synthetic
<400> 245
   ggtggctcca tcagcagtgg tggttactac 30
<210> 246
   <211> 10
   <212> PRT
   <213> Artificial Sequence
<220>
   <223> Synthetic
<400> 246
   Gly Gly Ser Ile Ser Ser Gly Gly Tyr Tyr
   1 5 10
<210> 247
   <211> 21
   <212> DNA
   <213> Artificial Sequence
<220>
   <223> Synthetic
<400> 247
   gtccattaca gtgggagcac c 21
<210> 248
   <211> 7
   <212> PRT
   <213> Artificial Sequence
<220>
   <223> Synthetic
<400> 248
   Val His Tyr Ser Gly Ser Thr
   1 5
<210> 249
   <211> 36
   <212> DNA
   <213> Artificial Sequence
<220>
   <223> Synthetic
<400> 249
   gcgagagccc cccgtggata ccattacttt gcctac 36
<210> 250
   <211> 12
   <212> PRT
   <213> Artificial Sequence
<220>
   <223> Synthetic
<400> 250
   Ala Arg Ala Pro Arg Gly Tyr His Tyr Phe Ala Tyr
   1 5 10
<210> 251
   <211> 325
   <212> DNA
   <213> Artificial Sequence
<220>
   <223> Synthetic
<400> 251
   gaaattgtgt tgacgcagtc tccaggcacc ctgtctttgt ctccagggga aagagccacc 60
   ctctcctgca gggccagtca gagtgttagc agcaggtact tagcctggta ccagcagaaa 120
   cctggccagg ctcccaggct cctcatcttt ggtgcatcca gcagggccac tggcatacca 180
   gacaggttca gtggcagtgg gtctgggaca gacttcactc tcaccatcag cagactggag 240
   cctgaagatt ttgcagtgta ttactgtcag cagtatggta gctcaccgct cactttcggc 300
   ggagggacca aggtggagat caaac 325
<210> 252
   <211> 108
   <212> PRT
   <213> Artificial Sequence
<220>
   <223> Synthetic
<400> 252
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Arg
   20 25 30
   Tyr Leu Ala Trp Tyr Gln Gln Lys Pro Gly Gln Ala Pro Arg Leu Leu
   35 40 45
   Ile Phe Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Pro
   85 90 95
   Leu Thr Phe Gly Gly Gly Thr Lys Val Glu Ile Lys
   100 105
<210> 253
   <211> 21
   <212> DNA
   <213> Artificial Sequence
<220>
   <223> Synthetic
<400> 253
   cagagtgtta gcagcaggta c 21
<210> 254
   <211> 7
   <212> PRT
   <213> Artificial Sequence
<220>
   <223> Synthetic
<400> 254
   Gln Ser Val Ser Ser Arg Tyr
   1 5
<210> 255
   <211> 9
   <212> DNA
   <213> Artificial Sequence
<220>
   <223> Synthetic
<400> 255
   ggtgcatcc 9
<210> 256
   <211> 3
   <212> PRT
   <213> Artificial Sequence
<220>
   <223> Synthetic
<400> 256
   Gly Ala Ser
   1
<210> 257
   <211> 27
   <212> DNA
   <213> Artificial Sequence
<220>
   <223> Synthetic
<400> 257
   cagcagtatg gtagctcacc gctcact 27
<210> 258
   <211> 9
   <212> PRT
   <213> Artificial Sequence
<220>
   <223> Synthetic
<400> 258
   Gln Gln Tyr Gly Ser Ser Pro Leu Thr
   1 5
<210> 259
   <211> 355
   <212> DNA
   <213> Artificial Sequence
<220>
   <223> Synthetic
<400> 259
   caggtgcagc tgcaggagtc gggcccagga ctggtgaagc cttcacagac cctgtccctc 60
   acctgcactg tctctggtgg ctccatcagc agtggtggtt actactggag ctggatccgc 120
   cagcacccag ggaagggcct ggagtggatt gggtacatct attacagtgg gagcacctac 180
   tacaacccgt ccctcaagag tcgaattatc atatcagtag acacgtctaa gaaccagttc 240
   tccctgaagc tgagctctgt gactgccgcg gacacggccg tgtattactg tgcgagagaa 300
   ggggctatgg tttttgacta ctggggccag ggaaccctgg tcaccgtctc ctcag 355
<210> 260
   <211> 118
   <212> PRT
   <213> Artificial Sequence
<220>
   <223> Synthetic
<400> 260
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Ile Ile Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Gly Ala Met Val Phe Asp Tyr Trp Gly Gln Gly Thr
               100                 105                 110
   Leu Val Thr Val Ser Ser
           115
<210> 261
   <211> 30
   <212> DNA
   <213> Artificial Sequence
<220>
   <223> Synthetic
<400> 261
   ggtggctcca tcagcagtgg tggttactac 30
<210> 262
   <211> 10
   <212> PRT
   <213> Artificial Sequence
<220>
   <223> Synthetic
<400> 262
   Gly Gly Ser Ile Ser Ser Gly Gly Tyr Tyr
   1               5                   10
<210> 263
   <211> 21
   <212> DNA
   <213> Artificial Sequence
<220>
   <223> Synthetic

<400> 263
   atctattaca gtgggagcac c 21
<210> 264
   <211> 7
   <212> PRT
   <213> Artificial Sequence
<220>
   <223> Synthetic
<400> 264
   Ile Tyr Tyr Ser Gly Ser Thr
   1 5
<210> 265
   <211> 30
   <212> DNA
   <213> Artificial Sequence
<220>
   <223> Synthetic
<400> 265
   gcgagagaag gggctatggt ttttgactac 30
<210> 266
   <211> 10
   <212> PRT
   <213> Artificial Sequence
<220>
   <223> Synthetic
<400> 266
   Ala Arg Glu Gly Ala Met Val Phe Asp Tyr
   1 5 10
<210> 267
   <211> 322
   <212> DNA
   <213> Artificial Sequence
<220>
   <223> Synthetic
<400> 267
   gacatccagt tgacccagtc tccatccttc ctgtctgcat ctgtaggaga cagagtcacc 60
   atcacttgct gggccagtca gggcattagc agttatttag cctggtatca gcaaaaacca 120
   gggaaagccc ctaaactcct gatttatgct gcatccgctt tgcaaagtgg ggtcccatca 180
   aggttcagcg gcagtggatc tgggacagaa ttcactctca ccatcagcag cctgcagcct 240
   gaagattttg caacttatta ctgtcaacag cttaatagtt acccgttcac ttttggccag 300
   gggaccaagc tggagatcaa ac 322
<210> 268
   <211> 107
   <212> PRT
   <213> Artificial Sequence
<220>
   <223> Synthetic
<400> 268
   Asp Ile Gln Leu Thr Gln Ser Pro Ser Phe Leu Ser Ala Ser Val Gly
   1 5 10 15
   Asp Arg Val Thr Ile Thr Cys Trp Ala Ser Gln Gly Ile Ser Ser Tyr
   20 25 30
   Leu Ala Trp Tyr Gln Gln Lys Pro Gly Lys Ala Pro Lys Leu Leu Ile
   35 40 45
   Tyr Ala Ala Ser Ala Leu Gln Ser Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Glu Asp Phe Ala Thr Tyr Tyr Cys Gln Gln Leu Asn Ser Tyr Pro Phe
   85 90 95
   Thr Phe Gly Gln Gly Thr Lys Leu Glu Ile Lys
   100 105
<210> 269
   <211> 18
   <212> DNA
   <213> Artificial Sequence
<220>
   <223> Synthetic
<400> 269
   cagggcatta gcagttat 18
<210> 270
   <211> 6
   <212> PRT
   <213> Artificial Sequence
<220>
   <223> Synthetic
<400> 270
   Gln Gly Ile Ser Ser Tyr
   1 5
<210> 271
   <211> 9
   <212> DNA
   <213> Artificial Sequence
<220>
   <223> Synthetic
<400> 271
   gctgcatcc 9
<210> 272
   <211> 3
   <212> PRT
   <213> Artificial Sequence
<220>
   <223> Synthetic
<400> 272
   Ala Ala Ser
   1
<210> 273
   <211> 27
   <212> DNA
   <213> Artificial Sequence
<220>
   <223> Synthetic
<400> 273
   caacagctta atagttaccc gttcact 27
<210> 274
   <211> 9
   <212> PRT
   <213> Artificial Sequence
<220>
   <223> Synthetic
<400> 274
   Gln Gln Leu Asn Ser Tyr Pro Phe Thr
   1 5
<210> 275
   <211> 355
   <212> DNA
   <213> Artificial Sequence
<220>
   <223> Synthetic
<400> 275
   caggtgcagc tgcaggagtc gggcccagga ctggtgaagc cttcacagac cctgtccctc 60
   acctgcactg tctctggtgg ctccatcagc agtggtggtt actactggag ctggatccgc 120
   cagcacccag ggaagggcct ggagtggatt gggtacatct attacagtgg gagcacctac 180
   tacaacccgt ccctcaagag tcgaattatc atatcagtag acacgtctaa gaaccagttc 240
   tccctgaagc tgagctctgt gactgccgcg gacacggccg tgtattactg tgcgagagaa 300
   ggggctatgg tttttgacta ctggggccag ggaaccctgg tcaccgtctc ctcag 355
<210> 276
   <211> 118
   <212> PRT
   <213> Artificial Sequence
<220>
   <223> Synthetic
<400> 276
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Ser Ser Gly
   20 25 30
   Gly Tyr Tyr Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Ile Ile Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Gly Ala Met Val Phe Asp Tyr Trp Gly Gln Gly Thr
   100 105 110
   Leu Val Thr Val Ser Ser
   115
<210> 277
   <211> 30
   <212> DNA
   <213> Artificial Sequence
<220>
   <223> Synthetic
<400> 277
   ggtggctcca tcagcagtgg tggttactac 30
<210> 278
   <211> 10
   <212> PRT
   <213> Artificial Sequence
<220>
   <223> Synthetic
<400> 278
   Gly Gly Ser Ile Ser Ser Gly Gly Tyr Tyr
   1 5 10
<210> 279
   <211> 21
   <212> DNA
   <213> Artificial Sequence
<220>
   <223> Synthetic
<400> 279
   atctattaca gtgggagcac c 21
<210> 280
   <211> 7
   <212> PRT
   <213> Artificial Sequence
<220>
   <223> Synthetic
<400> 280
   Ile Tyr Tyr Ser Gly Ser Thr
   1 5
<210> 281
   <211> 30
   <212> DNA
   <213> Artificial Sequence
<220>
   <223> Synthetic
<400> 281
   gcgagagaag gggctatggt ttttgactac 30
<210> 282
   <211> 10
   <212> PRT
   <213> Artificial Sequence
<220>
   <223> Synthetic
<400> 282
   Ala Arg Glu Gly Ala Met Val Phe Asp Tyr
   1 5 10
<210> 283
   <211> 322
   <212> DNA
   <213> Artificial Sequence
<220>
   <223> Synthetic
<400> 283
   gacatccagt tgacccagtc tccatccttc ctgtctgcat ctgtaggaga cagagtcacc 60
   atcacttgct gggccagtca gggcattagc agttatttag cctggtatca gcaaaaacca 120
   gggaaagccc ctaaactcct gatttatgct gcatccgctt tgcaaagtgg ggtcccatca 180
   aggttcagcg gcagtggatc tgggacagaa ttcactctca ccatcagcag cctgcagcct 240
   gaagattttg caacttatta ctgtcaacag cttaatagtt acccgttcac ttttggccag 300
   gggaccaagc tggagatcaa ac 322
<210> 284
   <211> 107
   <212> PRT
   <213> Artificial Sequence
<220>
   <223> Synthetic
<400> 284
   Asp Ile Gln Leu Thr Gln Ser Pro Ser Phe Leu Ser Ala Ser Val Gly
   1               5                   10                  15
   Asp Arg Val Thr Ile Thr Cys Trp Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ala Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Phe
                   85                  90                  95
   Thr Phe Gly Gln Gly Thr Lys Leu Glu Ile Lys
               100                 105
<210> 285
   <211> 18
   <212> DNA
   <213> Artificial Sequence
<220>
   <223> Synthetic
<400> 285
   cagggcatta gcagttat 18
<210> 286
   <211> 6
   <212> PRT
   <213> Artificial Sequence
<220>
   <223> Synthetic
<400> 286
   Gln Gly Ile Ser Ser Tyr
   1               5
<210> 287
   <211> 9
   <212> DNA
   <213> Artificial Sequence
<220>
   <223> Synthetic
<400> 287
   gctgcatcc 9
<210> 288
   <211> 3
   <212> PRT
   <213> Artificial Sequence
<220>
   <223> Synthetic
<400> 288
   Ala Ala Ser
   1
<210> 289
   <211> 27
   <212> DNA
   <213> Artificial Sequence
<220>
   <223> Synthetic
<400> 289
   caacagctta atagttaccc gttcact 27
<210> 290
   <211> 9
   <212> PRT
   <213> Artificial Sequence
<220>
   <223> Synthetic
<400> 290
   Gln Gln Leu Asn Ser Tyr Pro Phe Thr
   1 5
<210> 291
   <211> 355
   <212> DNA
   <213> Artificial Sequence
<220>
   <223> Synthetic
<400> 291
   caggtgcagc tgcaggagtc gggcccagga ctggtgaagc cttcacagac cctgtccctc 60
   acctgcactg tctctggtgg ctccatcagc agtggtggtt actactggac ctggatccgc 120
   cagcacccag ggaagggcct ggagtggatt gggtacatct attacagtgg gagcacctac 180
   tacaacccgt ccctcaagag tcgagttatc atatcagtag acacgtctaa gaaccagttc 240
   tccctgaagc tgagctctgt gactgccgcg gacacggccg tgtattactg tgcgagagaa 300
   ggggctatgg tttttgacta ctggggccag ggaaccctgg tcaccgtctc ctcag 355
<210> 292
   <211> 118
   <212> PRT
   <213> Artificial Sequence
<220>
   <223> Synthetic
<400> 292
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Ser Ser Gly
   20 25 30
   Gly Tyr Tyr Trp Thr Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Ile Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Gly Ala Met Val Phe Asp Tyr Trp Gly Gln Gly Thr
   100 105 110
   Leu Val Thr Val Ser Ser
   115
<210> 293
   <211> 30
   <212> DNA
   <213> Artificial Sequence
<220>
   <223> Synthetic
<400> 293
   ggtggctcca tcagcagtgg tggttactac 30
<210> 294
   <211> 10
   <212> PRT
   <213> Artificial Sequence
<220>
   <223> Synthetic
<400> 294
   Gly Gly Ser Ile Ser Ser Gly Gly Tyr Tyr
   1 5 10
<210> 295
   <211> 21
   <212> DNA
   <213> Artificial Sequence
<220>
   <223> Synthetic
<400> 295
   atctattaca gtgggagcac c 21
<210> 296
   <211> 7
   <212> PRT
   <213> Artificial Sequence
<220>
   <223> Synthetic
<400> 296
   Ile Tyr Tyr Ser Gly Ser Thr
   1 5
<210> 297
   <211> 30
   <212> DNA
   <213> Artificial Sequence
<220>
   <223> Synthetic
<400> 297
   gcgagagaag gggctatggt ttttgactac 30
<210> 298
   <211> 10
   <212> PRT
   <213> Artificial Sequence
<220>
   <223> Synthetic
<400> 298
   Ala Arg Glu Gly Ala Met Val Phe Asp Tyr
   1 5 10
<210> 299
   <211> 322
   <212> DNA
   <213> Artificial Sequence
<220>
   <223> Synthetic
<400> 299
   gacatccagt tgacccagtc tccatccttc ctgtctgcat ctgtaggaga cagagtcacc 60
   atcacttgct gggccagtca gggcattagc agttatttag cctggtatca gcaaaaacca 120
   gggaaagccc ctaagctcct gatctatgct gcatccgctt tgcaaagtgg ggtcccatca 180
   aggttcagcg gcagtggatc tgggacagaa ttcactctca caatcagcag cctgcagcct 240
   gaagattttg caacttatta ctgtcaacag cttaatagtt acccgttcac ttttggccag 300
   gggaccaagc tggagatcaa ac 322
<210> 300
   <211> 107
   <212> PRT
   <213> Artificial Sequence
<220>
   <223> Synthetic
<400> 300
   Asp Ile Gln Leu Thr Gln Ser Pro Ser Phe Leu Ser Ala Ser Val Gly
   1               5                   10                  15
   Asp Arg Val Thr Ile Thr Cys Trp Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ala Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Phe
                   85                  90                  95
   Thr Phe Gly Gln Gly Thr Lys Leu Glu Ile Lys
               100                 105
<210> 301
   <211> 18
   <212> DNA
   <213> Artificial Sequence
<220>
   <223> Synthetic
<400> 301
   cagggcatta gcagttat 18
<210> 302
   <211> 6
   <212> PRT
   <213> Artificial Sequence
<220>
   <223> Synthetic
<400> 302
   Gln Gly Ile Ser Ser Tyr
   1               5
<210> 303
   <211> 9
   <212> DNA
   <213> Artificial Sequence
<220>
   <223> Synthetic
<400> 303
   gctgcatcc 9
<210> 304
   <211> 3
   <212> PRT
   <213> Artificial Sequence
<220>
   <223> Synthetic
<400> 304
   Ala Ala Ser
   1
<210> 305
   <211> 27
   <212> DNA
   <213> Artificial Sequence
<220>
   <223> Synthetic
<400> 305
   caacagctta atagttaccc gttcact 27
<210> 306
   <211> 9
   <212> PRT
   <213> Artificial Sequence
<220>
   <223> Synthetic
<400> 306
   Gln Gln Leu Asn Ser Tyr Pro Phe Thr
   1 5
<210> 307
   <211> 355
   <212> DNA
   <213> Artificial Sequence
<220>
   <223> Synthetic
<400> 307
   caggtgcagc tgcaggagtc gggcccagga ctggtgaagc cttcacagac cctgtccctc 60
   acctgcactg tctctggtgg ctccatcagc agtggtggtt actactggac ctggatccgc 120
   cagtacccag ggaagggcct ggagtggatt gggtacatct attacagtgg gagcacctac 180
   tacaacccgt ccctcaagag tcgagttatc atatcagtag acacgtctaa gaaccagttc 240
   tccctgaggc tgagctctgt gactgccgcg gacacggccg tgtattactg tgcgagagaa 300
   ggggctatgg tttttgacta ctggggccag ggaaccctgg tcaccgtctc ctcag 355
<210> 308
   <211> 118
   <212> PRT
   <213> Artificial Sequence
<220>
   <223> Synthetic
<400> 308
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Ser Ser Gly
   20 25 30
   Gly Tyr Tyr Trp Thr Trp Ile Arg Gln Tyr Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Ile Ile Ser Val Asp Thr Ser Lys Asn Gln Phe
   65 70 75 80
   Ser Leu Arg Leu Ser Ser Val Thr Ala Ala Asp Thr Ala Val Tyr Tyr
   85 90 95
   Cys Ala Arg Glu Gly Ala Met Val Phe Asp Tyr Trp Gly Gln Gly Thr
   100 105 110
   Leu Val Thr Val Ser Ser
   115
<210> 309
   <211> 30
   <212> DNA
   <213> Artificial Sequence
<220>
   <223> Synthetic
<400> 309
   ggtggctcca tcagcagtgg tggttactac 30
<210> 310
   <211> 10
   <212> PRT
   <213> Artificial Sequence
<220>
   <223> Synthetic
<400> 310
   Gly Gly Ser Ile Ser Ser Gly Gly Tyr Tyr
   1 5 10
<210> 311
   <211> 21
   <212> DNA
   <213> Artificial Sequence
<220>
   <223> Synthetic
<400> 311
   atctattaca gtgggagcac c 21
<210> 312
   <211> 7
   <212> PRT
   <213> Artificial Sequence
<220>
   <223> Synthetic
<400> 312
   Ile Tyr Tyr Ser Gly Ser Thr
   1 5
<210> 313
   <211> 30
   <212> DNA
   <213> Artificial Sequence
<220>
   <223> Synthetic
<400> 313
   gcgagagaag gggctatggt ttttgactac 30
<210> 314
   <211> 10
   <212> PRT
   <213> Artificial Sequence
<220>
   <223> Synthetic
<400> 314
   Ala Arg Glu Gly Ala Met Val Phe Asp Tyr
   1 5 10
<210> 315
   <211> 322
   <212> DNA
   <213> Artificial Sequence
<220>
   <223> Synthetic
<400> 315
   gacatccagt tgacccagtc tccatccttc ctgtctgcat ctgtaggaga cagagtcacc 60
   atcacttgct gggccagtca gggcattagc agttatttag cctggtatca gcaaaaacca 120
   gggaaagccc ctaagctcct gatctatgct gcatccgctt tgcaaagtgg ggtcccatca 180
   aggttcagcg gcagtggatc tgggacagaa ttcactctca caatcagcag cctgcagcct 240
   gaagattttg caacttatta ctgtcaacag cttaatagtt acccgttcac ttttggccag 300
   gggaccaagc tggagatcaa ac 322
<210> 316
   <211> 107
   <212> PRT
   <213> Artificial Sequence
<220>
   <223> Synthetic
<400> 316
   Asp Ile Gln Leu Thr Gln Ser Pro Ser Phe Leu Ser Ala Ser Val Gly
   1               5                   10                  15
   Asp Arg Val Thr Ile Thr Cys Trp Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ala Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Phe
                   85                  90                  95
   Thr Phe Gly Gln Gly Thr Lys Leu Glu Ile Lys
               100                 105
<210> 317
   <211> 18
   <212> DNA
   <213> Artificial Sequence
<220>
   <223> Synthetic
<400> 317
   cagggcatta gcagttat 18
<210> 318
   <211> 6
   <212> PRT
   <213> Artificial Sequence
<220>
   <223> Synthetic
<400> 318
   Gln Gly Ile Ser Ser Tyr
   1               5
<210> 319
   <211> 9
   <212> DNA
   <213> Artificial Sequence
<220>
   <223> Synthetic
<400> 319
   gctgcatcc 9
<210> 320
   <211> 3
   <212> PRT
   <213> Artificial Sequence
<220>
   <223> Synthetic
<400> 320
   Ala Ala Ser
   1
<210> 321
   <211> 27
   <212> DNA
   <213> Artificial Sequence
<220>
   <223> Synthetic
<400> 321
   caacagctta atagttaccc gttcact 27
<210> 322
   <211> 9
   <212> PRT
   <213> Artificial Sequence
<220>
   <223> Synthetic
<400> 322
   Gln Gln Leu Asn Ser Tyr Pro Phe Thr
   1 5
<210> 323
   <211> 358
   <212> DNA
   <213> Artificial Sequence
<220>
   <223> Synthetic
<400> 323
   caggttcacc tggtgcagtc tggagctgag gtgaagaagc ctggggcctc agtgaaggtc 60
   tcctgcaggg cttctggtta cacctttacc aactatggta tcacctgggt gcgacaggcc 120
   cctggacaag ggcttgagtg gatgggatgg atcagcgcta acagtggtaa cacagactct 180
   gcacagaagt tccaggccag agtcaccatg accacagaca catccacgac cacagcctac 240
   atggaactga ggagcctgag atctgacgac acggccgtgt attattgtgc gacgtatagt 300
   tggaactttc actggttcga cccctggggc cagggaaccc tggtcaccgt ctcctcag   358
<210> 324
   <211> 119
   <212> PRT
   <213> Artificial Sequence
<220>
   <223> Synthetic
<400> 324
   Gln Val His Leu Val Gln Ser Gly Ala Glu Val Lys Lys Pro Gly Ala
   1 5 10 15
   Ser Val Lys Val Ser Cys Arg Ala Ser Gly Tyr Thr Phe Thr Asn Tyr
   20 25 30
   Gly Ile Thr Trp Val Arg Gln Ala Pro Gly Gln Gly Leu Glu Trp Met
   35 40 45
   Gly Trp Ile Ser Ala Asn Ser Gly Asn Thr Asp Ser Ala Gln Lys Phe
   50 55 60
   Gln Ala Arg Val Thr Met Thr Thr Asp Thr Ser Thr Thr Thr Ala Tyr
   65 70 75 80
   Met Glu Leu Arg Ser Leu Arg Ser Asp Asp Thr Ala Val Tyr Tyr Cys
   85 90 95
   Ala Thr Tyr Ser Trp Asn Phe His Trp Phe Asp Pro Trp Gly Gln Gly
   100 105 110
   Thr Leu Val Thr Val Ser Ser
   115
<210> 325
   <211> 24
   <212> DNA
   <213> Artificial Sequence
<220>
   <223> Synthetic
<400> 325
   ggttacacct ttaccaacta tggt 24
<210> 326
   <211> 8
   <212> PRT
   <213> Artificial Sequence
<220>
   <223> Synthetic
<400> 326
   Gly Tyr Thr Phe Thr Asn Tyr Gly
   1 5
<210> 327
   <211> 24
   <212> DNA
   <213> Artificial Sequence
<220>
   <223> Synthetic
<400> 327
   atcagcgcta acagtggtaa caca 24
<210> 328
   <211> 8
   <212> PRT
   <213> Artificial Sequence
<220>
   <223> Synthetic
<400> 328
   Ile Ser Ala Asn Ser Gly Asn Thr
   1 5
<210> 329
   <211> 36
   <212> DNA
   <213> Artificial Sequence
<220>
   <223> Synthetic
<400> 329
   gcgacgtata gttggaactt tcactggttc gacccc 36
<210> 330
   <211> 12
   <212> PRT
   <213> Artificial Sequence
<220>
   <223> Synthetic
<400> 330
   Ala Thr Tyr Ser Trp Asn Phe His Trp Phe Asp Pro
   1 5 10
<210> 331
   <211> 325
   <212> DNA
   <213> Artificial Sequence
<220>
   <223> Synthetic
<400> 331
   gaaattgtgt tgacgcagtc tccaggcact ctgtctttgt ctccagggga aagagtcacc 60
   ctctcctgca gggccagtca gagtgtcagt accaactact taacctggta ccagcagaaa 120
   cctggccagg ctcccaggct cctcatctat ggtgcatcca gcagggccac tggcatccca 180
   gacaggttca gtggcagtgg gtctgggaca gacttcattc tcaccatcag aagtctggag 240
   cctgaagatt ttgcagtgta ttactgtcag cagtgtggtg gctcaccgtg gacgttcggc 300
   caagggacca gggtggaaat caaac 325
<210> 332
   <211> 108
   <212> PRT
   <213> Artificial Sequence
<220>
   <223> Synthetic
<400> 332
   Glu Ile Val Leu Thr Gln Ser Pro Gly Thr Leu Ser Leu Ser Pro Gly
   1               5                   10                  15
   Glu Arg Val Thr Leu Ser Cys Arg Ala Ser Gln Ser Val Ser Thr Asn
               20                  25                  30
   Tyr Leu Thr Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Ile Leu Thr Ile Arg Ser Leu Glu
   65                  70                  75                  80
   Pro Glu Asp Phe Ala Val Tyr Tyr Cys Gln Gln Cys Gly Gly Ser Pro
                   85                  90                  95
   Trp Thr Phe Gly Gln Gly Thr Arg Val Glu Ile Lys
               100                 105
<210> 333
   <211> 21
   <212> DNA
   <213> Artificial Sequence
<220>
   <223> Synthetic
<400> 333
   cagagtgtca gtaccaacta c 21
<210> 334
   <211> 7
   <212> PRT
   <213> Artificial Sequence
<220>
   <223> Synthetic
<400> 334
   Gln Ser Val Ser Thr Asn Tyr
   1               5
<210> 335
   <211> 9
   <212> DNA
   <213> Artificial Sequence
<220>
   <223> Synthetic
<400> 335
   ggtgcatcc 9
<210> 336
   <211> 3
   <212> PRT
   <213> Artificial Sequence
<220>
   <223> Synthetic
<400> 336
   Gly Ala Ser
   1
<210> 337
   <211> 27
   <212> DNA
   <213> Artificial Sequence
<220>
   <223> Synthetic
<400> 337
   cagcagtgtg gtggctcacc gtggacg 27
<210> 338
   <211> 9
   <212> PRT
   <213> Artificial Sequence
<220>
   <223> Synthetic
<400> 338
   Gln Gln Cys Gly Gly Ser Pro Trp Thr
   1 5
<210> 339
   <211> 355
   <212> DNA
   <213> Artificial Sequence
<220>
   <223> Synthetic
<400> 339
   caggtgcagc tggtggagtc tgggggaggc gtggtccagc ctgggaggtc tctgagactc 60
   tcctgtgcag cgtctggatt caccttcagt agctatggca tgcactgggt ccgccaggct 120
   ccaggcaagg gactggagtg ggtggcagtt atatggtatg atggaagtaa taaatattat 180
   ggagactccg tgaagggccg attcaccatc tccagagaca attccaagaa cacgctgtat 240
   ctgcaaatga acagcctgag agccgaggac acggctgtat attactgtgc gagagatgga 300
   gtagacggtg cttttgatat ttggggccaa gggacaacgg tcaccgtctc ttcag 355
<210> 340
   <211> 118
   <212> PRT
   <213> Artificial Sequence
<220>
   <223> Synthetic
<400> 340
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Trp Tyr Asp Gly Ser Asn Lys Tyr Tyr Gly Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp Gly Val Asp Gly Ala Phe Asp Ile Trp Gly Gln Gly Thr
   100 105 110
   Thr Val Thr Val Ser Ser
   115
<210> 341
   <211> 24
   <212> DNA
   <213> Artificial Sequence
<220>
   <223> Synthetic
<400> 341
   ggattcacct tcagtagcta tggc 24
<210> 342
   <211> 8
   <212> PRT
   <213> Artificial Sequence
<220>
   <223> Synthetic
<400> 342
   Gly Phe Thr Phe Ser Ser Tyr Gly
   1 5
<210> 343
   <211> 24
   <212> DNA
   <213> Artificial Sequence
<220>
   <223> Synthetic
<400> 343
   atatggtatg atggaagtaa taaa 24
<210> 344
   <211> 8
   <212> PRT
   <213> Artificial Sequence
<220>
   <223> Synthetic
<400> 344
   Ile Trp Tyr Asp Gly Ser Asn Lys
   1 5
<210> 345
   <211> 33
   <212> DNA
   <213> Artificial Sequence
<220>
   <223> Synthetic
<400> 345
   gcgagagatg gagtagacgg tgcttttgat att 33
<210> 346
   <211> 11
   <212> PRT
   <213> Artificial Sequence
<220>
   <223> Synthetic
<400> 346
   Ala Arg Asp Gly Val Asp Gly Ala Phe Asp Ile
   1 5 10
<210> 347
   <211> 322
   <212> DNA
   <213> Artificial Sequence
<220>
   <223> Synthetic
<400> 347
   gccatccaga tgacccagtc tccatcctcc ctgtctgcat ctgtaggaga cagagtcacc 60
   atcacttgcc gggcaagtca ggacattaga aatgatttag gctggtatca gcagaaacca 120
   gggaaagccc ctaaactcct gatctatgct gcatccagtt tacaaagtgg ggtcccatca 180
   aggttcagcg gcagtggatc tggcacagat ttcactctca ccatcagcag actgcagcct 240
   gaagattttg caacttatta ctgtcaacaa gattacaatt acctgtatac ttttggccag 300
   gggaccaacc tggagatcaa ac 322
<210> 348
   <211> 107
   <212> PRT
   <213> Artificial Sequence
<220>
   <223> Synthetic
<400> 348
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Arg Asn Asp
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Arg Leu Gln Pro
   65                  70                  75                  80
   Glu Asp Phe Ala Thr Tyr Tyr Cys Gln Gln Asp Tyr Asn Tyr Leu Tyr
                   85                  90                  95
   Thr Phe Gly Gln Gly Thr Asn Leu Glu Ile Lys
               100                 105
<210> 349
   <211> 18
   <212> DNA
   <213> Artificial Sequence
<220>
   <223> Synthetic
<400> 349
   caggacatta gaaatgat 18
<210> 350
   <211> 6
   <212> PRT
   <213> Artificial Sequence
<220>
   <223> Synthetic
<400> 350
   Gln Asp Ile Arg Asn Asp
    1               5
<210> 351
   <211> 9
   <212> DNA
   <213> Artificial Sequence
<220>
   <223> Synthetic
<400> 351
   gctgcatcc 9
<210> 352
   <211> 3
   <212> PRT
   <213> Artificial Sequence
<220>
   <223> Synthetic
<400> 352
   Ala Ala Ser
   1
<210> 353
   <211> 27
   <212> DNA
   <213> Artificial Sequence
<220>
   <223> Synthetic
<400> 353
   caacaagatt acaattacct gtatact 27
<210> 354
   <211> 9
   <212> PRT
   <213> Artificial Sequence
<220>
   <223> Synthetic
<400> 354
   Gln Gln Asp Tyr Asn Tyr Leu Tyr Thr
   1 5
<210> 355
   <211> 364
   <212> DNA
   <213> Artificial Sequence
<220>
   <223> Synthetic

<400> 355
   gaggtgcagt tggtggagtc ggggggaggc ttggtccagc ctggggggtc cctgagactc 60
   tcctgtgaag cctctggatt cacctttgat aactattata tgacctgggt ccgccagact 120
   ccagggaagg ggctggagtg ggtggccaac ataaaggaag atggaaatga tagatactat 180
   gtggactctg tgaagggccg cttcaccatc tccagagaca acgccaagca gtcactgttt 240
   ctacaaatga acagtctgag agccgaggac acggctgttt attactgtgc gagagaattt 300
   tggagtggcc ctcactacgg tttggacgtc tggggccaag ggaccacggt caccgtctcc 360
   tcag 364
<210> 356
   <211> 121
   <212> PRT
   <213> Artificial Sequence
<220>
   <223> Synthetic
<400> 356
   Glu Val Gln Leu Val Glu Ser Gly Gly Gly Leu Val Gln Pro Gly Gly
   1 5 10 15
   Ser Leu Arg Leu Ser Cys Glu Ala Ser Gly Phe Thr Phe Asp Asn Tyr
   20 25 30
   Tyr Met Thr Trp Val Arg Gln Thr Pro Gly Lys Gly Leu Glu Trp Val
   35 40 45
   Ala Asn Ile Lys Glu Asp Gly Asn Asp Arg Tyr Tyr Val Asp Ser Val
   50 55 60
   Lys Gly Arg Phe Thr Ile Ser Arg Asp Asn Ala Lys Gln Ser Leu Phe
   65 70 75 80
   Leu Gln Met Asn Ser Leu Arg Ala Glu Asp Thr Ala Val Tyr Tyr Cys
   85 90 95
   Ala Arg Glu Phe Trp Ser Gly Pro His Tyr Gly Leu Asp Val Trp Gly
   100 105 110
   Gln Gly Thr Thr Val Thr Val Ser Ser
   115 120
<210> 357
   <211> 24
   <212> DNA
   <213> Artificial Sequence
<220>
   <223> Synthetic
<400> 357
   ggattcacct ttgataacta ttat 24
<210> 358
   <211> 8
   <212> PRT
   <213> Artificial Sequence
<220>
   <223> Synthetic
<400> 358
   Gly Phe Thr Phe Asp Asn Tyr Tyr
   1 5
<210> 359
   <211> 24
   <212> DNA
   <213> Artificial Sequence
<220>
   <223> Synthetic
<400> 359
   ataaaggaag atggaaatga taga 24
<210> 360
   <211> 8
   <212> PRT
   <213> Artificial Sequence
<220>
   <223> Synthetic
<400> 360
   Ile Lys Glu Asp Gly Asn Asp Arg
   1 5
<210> 361
   <211> 42
   <212> DNA
   <213> Artificial Sequence
<220>
   <223> Synthetic
<400> 361
   gcgagagaat tttggagtgg ccctcactac ggtttggacg tc 42
<210> 362
   <211> 14
   <212> PRT
   <213> Artificial Sequence
<220>
   <223> Synthetic
<400> 362
   Ala Arg Glu Phe Trp Ser Gly Pro His Tyr Gly Leu Asp Val
   1 5 10
<210> 363
   <211> 322
   <212> DNA
   <213> Artificial Sequence
<220>
   <223> Synthetic
<400> 363
   gccctccaga tgacccagtc tccatcctcc ctgtctgcac ctgtaggcga cagagtcacc 60
   atcacttgcc gggcaagtca ggacgttaga aataatttag gctggtatca gcagaaacca 120
   gggaatgccc ctaaattcct gatctatgct gcatccagtt tacaaagtgg aatcccatca 180
   aggttcagcg gcagtggatc tggctcagat ttcactctca ccatcagcag cctgcagcct 240
   gaagattttg caacttatta ctgtctacaa gattacaatt accctccgac gttcggccag 300
   gggaccaagg tggaaatcaa gc 322
<210> 364
   <211> 107
   <212> PRT
   <213> Artificial Sequence
<220>
   <223> Synthetic
<400> 364
   Ala Leu Gln Met Thr Gln Ser Pro Ser Ser Leu Ser Ala Pro Val Gly
   1 5 10 15
   Asp Arg Val Thr Ile Thr Cys Arg Ala Ser Gln Asp Val Arg Asn Asn
   20 25 30
   Leu Gly Trp Tyr Gln Gln Lys Pro Gly Asn Ala Pro Lys Phe Leu Ile
   35 40 45
   Tyr Ala Ala Ser Ser Leu Gln Ser Gly Ile Pro Ser Arg Phe Ser Gly
   50 55 60
   Ser Gly Ser Gly Ser Asp Phe Thr Leu Thr Ile Ser Ser Leu Gln Pro
   65 70 75 80
   Glu Asp Phe Ala Thr Tyr Tyr Cys Leu Gln Asp Tyr Asn Tyr Pro Pro
   85 90 95
   Thr Phe Gly Gln Gly Thr Lys Val Glu Ile Lys
   100 105
<210> 365
   <211> 18
   <212> DNA
   <213> Artificial Sequence
<220>
   <223> Synthetic
<400> 365
   caggacgtta gaaataat 18
<210> 366
   <211> 6
   <212> PRT
   <213> Artificial Sequence
<220>
   <223> Synthetic
<400> 366
   Gln Asp Val Arg Asn Asn
   1 5

<210> 367
   <211> 9
   <212> DNA
   <213> Artificial Sequence
<220>
   <223> Synthetic
<400> 367
   gctgcatcc 9
<210> 368
   <211> 3
   <212> PRT
   <213> Artificial Sequence
<220>
   <223> Synthetic
<400> 368
   Ala Ala Ser
   1
<210> 369
   <211> 27
   <212> DNA
   <213> Artificial Sequence
<220>
   <223> Synthetic
<400> 369
   ctacaagatt acaattaccc tccgacg 27
<210> 370
   <211> 9
   <212> PRT
   <213> Artificial Sequence
<220>
   <223> Synthetic
<400> 370
   Leu Gln Asp Tyr Asn Tyr Pro Pro Thr
   1 5
<210> 371
   <211> 370
   <212> DNA
   <213> Artificial Sequence
<220>
   <223> Synthetic
<400> 371
   caggtgcagc tggtggagtc tgggggaggc gtggtccagc ctgggaggtc cctgagactc 60
   tcctgtgcag cgtctggatt caccttcagt agttatggca tgcactgggt ccgccaggct 120
   ccaggcaagg ggctggagtg ggtggcagtt ttatggtatg atggaagtaa taaaaactat 180
   gtagactccg tgaagggccg attcaccatc tcaagagaca attccaagaa cacgctgtat 240
   ctgcaaatga acagcctgag agccgaggac acggctgtgt attactgtgc gagagatcac 300
   gattttagga gtggttatga ggggtggttc gacccctggg gccagggaac cttggtcacc 360
   gtctcctcag 370
<210> 372
   <211> 123
   <212> PRT
   <213> Artificial Sequence
<220>
   <223> Synthetic
<400> 372
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Leu Trp Tyr Asp Gly Ser Asn Lys Asn Tyr Val Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp His Asp Phe Arg Ser Gly Tyr Glu Gly Trp Phe Asp Pro
   100 105 110
   Trp Gly Gln Gly Thr Leu Val Thr Val Ser Ser
   115 120
<210> 373
   <211> 24
   <212> DNA
   <213> Artificial Sequence
<220>
   <223> Synthetic
<400> 373
   ggattcacct tcagtagtta tggc 24
<210> 374
   <211> 8
   <212> PRT
   <213> Artificial Sequence
<220>
   <223> Synthetic
<400> 374
   Gly Phe Thr Phe Ser Ser Tyr Gly
   1 5
<210> 375
   <211> 24
   <212> DNA
   <213> Artificial Sequence
<220>
   <223> Synthetic
<400> 375
   ttatggtatg atggaagtaa taaa 24
<210> 376
   <211> 8
   <212> PRT
   <213> Artificial Sequence
<220>
   <223> Synthetic
<400> 376
   Leu Trp Tyr Asp Gly Ser Asn Lys
   1 5
<210> 377
   <211> 48
   <212> DNA
   <213> Artificial Sequence
<220>
   <223> Synthetic
<400> 377
   gcgagagatc acgattttag gagtggttat gaggggtggt tcgacccc 48
<210> 378
   <211> 16
   <212> PRT
   <213> Artificial Sequence
<220>
   <223> Synthetic
<400> 378
   Ala Arg Asp His Asp Phe Arg Ser Gly Tyr Glu Gly Trp Phe Asp Pro
   1 5 10 15
<210> 379
   <211> 322
   <212> DNA
   <213> Artificial Sequence
<220>
   <223> Synthetic
<400> 379
   gaaattgtgt tgacacagtc tccagccacc ctgtctttgt ctccagggga aagagccacc 60
   ctctcctgca gggccagtca gagtgttcgc agctacttag cctggtacca acagaaacct 120
   ggccaggctc ccaggctcct catctatgat gcatccaaca gggccactgg catcccagcc 180
   aggttcagtg gcagtgggtc tgggacagac ttcactctca ccatcagcag cctagagcct 240
   gaagattttg cagtttatta ctgtcagcac cgtagcaact ggcctcccac tttcggcgga 300
   gggaccgagg tggaggtcag ac 322
<210> 380
   <211> 107
   <212> PRT
   <213> Artificial Sequence
<220>
   <223> Synthetic
<400> 380
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Arg Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln His Arg Ser Asn Trp Pro Pro
                   85                  90                  95
   Thr Phe Gly Gly Gly Thr Glu Val Glu Val Arg
               100                 105
<210> 381
   <211> 18
   <212> DNA
   <213> Artificial Sequence
<220>
   <223> Synthetic
<400> 381 18
   cagagtgttc gcagctac 18
<210> 382
   <211> 6
   <212> PRT
   <213> Artificial Sequence
<220>
   <223> Synthetic
<400> 382
   Gln Ser Val Arg Ser Tyr
    1               5
<210> 383
   <211> 9
   <212> DNA
   <213> Artificial Sequence
<220>
   <223> Synthetic
<400> 383
   gatgcatcc 9
<210> 384
   <211> 3
   <212> PRT
   <213> Artificial Sequence
<220>
   <223> Synthetic
<400> 384
   Asp Ala Ser
   1
<210> 385
   <211> 27
   <212> DNA
   <213> Artificial Sequence
<220>
   <223> Synthetic
<400> 385
   cagcaccgta gcaactggcc tcccact 27
<210> 386
   <211> 9
   <212> PRT
   <213> Artificial Sequence
<220>
   <223> Synthetic
<400> 386
   Gln His Arg Ser Asn Trp Pro Pro Thr
   1 5
<210> 387
   <211> 16
   <212> DNA
   <213> Artificial Sequence
<220>
   <223> Synthetic
<400> 387
   taccggctcc cgatgg 16
<210> 388
   <211> 19
   <212> DNA
   <213> Artificial Sequence
<220>
   <223> Synthetic
<400> 388
   ggcggctaag gtgtttgga 19
<210> 389
   <211> 22
   <212> DNA
   <213> Artificial Sequence
<220>
   <223> Synthetic
<400> 389
   ttgggaatga ggaaagcaaa ct 22
<210> 390
   <211> 18
   <212> DNA
   <213> Artificial Sequence
<220>
   <223> Synthetic
<400> 390
   ctgaagtacc ggaggaga 18
<210> 391
   <211> 20
   <212> DNA
   <213> Artificial Sequence
<220>
   <223> Synthetic
<400> 391
   atcacgctgg tggtcctctt 20
<210> 392
   <211> 16
   <212> DNA
   <213> Artificial Sequence
<220>
   <223> Synthetic
<400> 392
   gctgcggcga gtgctt 16
<210> 393
   <211> 23
   <212> DNA
   <213> Artificial Sequence
<220>
   <223> Synthetic
<400> 393
   tggcaaatgc tggacccaac aca 23
<210> 394
   <211> 19
   <212> DNA
   <213> Artificial Sequence
<220>
   <223> Synthetic
<400> 394
   gggtcctggc atcttgtcc 19
<210> 395
   <211> 22
   <212> DNA
   <213> Artificial Sequence
<220>
   <223> Synthetic
<400> 395
   gcagatgaaa aactgggaac ca 22
<210> 396
   <211> 372
   <212> DNA
   <213> Artificial Sequence
<220>
   <223> Synthetic
<400> 396
   caggtgcagc tggtgcagtc aggtccagga ctggtgaagc cctcgcagaa cctctcactc 60
   acctgtgcca tctccggaga cagtgtctct agtgatagtg ctgcttggaa ctggatcagg 120
   cagtccccat cgagaggcct tgagtggctg ggaaggacat actacaggtc caagtggtat 180
   aatgattatg cagtatctgt gaaaagtcga ataaccttca acccagatac atccaagaac 240
   cacatctccc tgcagctgaa ctctgtgact cccgaggaca cggctatcta ttactgtgca 300
   agagaggggg ataattggaa ttacggctgg ctcgacccct ggggccaggg aaccacggtc 360
   accgtctcct ca                                                     372
<210> 397
   <211> 124
   <212> PRT
   <213> Artificial Sequence
<220>
   <223> Synthetic
<400> 397
   Gln Val Gln Leu Val Gln Ser Gly Pro Gly Leu Val Lys Pro Ser Gln
   1               5                   10                  15
   Asn Leu Ser Leu Thr Cys Ala Ile Ser Gly Asp Ser Val Ser Ser Asp
               20                  25                  30
   Ser Ala Ala Trp Asn Trp Ile Arg Gln Ser Pro Ser Arg Gly Leu Glu
           35                  40                  45
   Trp Leu Gly Arg Thr Tyr Tyr Arg Ser Lys Trp Tyr Asn Asp Tyr Ala
       50                  55                  60
   Val Ser Val Lys Ser Arg Ile Thr Phe Asn Pro Asp Thr Ser Lys Asn
   65                  70                  75                  80
   His Ile Ser Leu Gln Leu Asn Ser Val Thr Pro Glu Asp Thr Ala Ile
                   85                  90                  95
   Tyr Tyr Cys Ala Arg Glu Gly Asp Asn Trp Asn Tyr Gly Trp Leu Asp
               100                 105                 110
   Pro Trp Gly Gln Gly Thr Thr Val Thr Val Ser Ser
           115                 120
<210> 398
   <211> 30
   <212> DNA
   <213> Artificial Sequence
<220>
   <223> Synthetic
<400> 398
   ggagacagtg tctctagtga tagtgctgct 30
<210> 399
   <211> 10
   <212> PRT
   <213> Artificial Sequence
<220>
   <223> Synthetic
<400> 399
   Gly Asp Ser Val Ser Ser Asp Ser Ala Ala
   1 5 10
<210> 400
   <211> 27
   <212> DNA
   <213> Artificial Sequence
<220>
   <223> Synthetic
<400> 400
   acatactaca ggtccaagtg gtataat 27
<210> 401
   <211> 9
   <212> PRT
   <213> Artificial Sequence
<220>
   <223> Synthetic
<400> 401
   Thr Tyr Tyr Arg Ser Lys Trp Tyr Asn
   1 5
<210> 402
   <211> 42
   <212> DNA
   <213> Artificial Sequence
<220>
   <223> Synthetic
<400> 402
   gcaagagagg gggataattg gaattacggc tggctcgacc cc 42
<210> 403
   <211> 14
   <212> PRT
   <213> Artificial Sequence
<220>
   <223> Synthetic
<400> 403
   Ala Arg Glu Gly Asp Asn Trp Asn Tyr Gly Trp Leu Asp Pro
    1               5                   10
<210> 404
   <211> 336
   <212> DNA
   <213> Artificial Sequence
<220>
   <223> Synthetic
<400> 404
   gacatccagt tgacccagtc tccactctcc ctgcccgtca cccctggaga gccggcctcc 60
   atctcctgca ggtctagtca gagcctcctt cttagtaatg gatacaacta tttggattgg 120
   tacctgcaga agccagggca gtctccacaa ctcctgatct atttggtttc tagtcgggcc 180
   tccggggtcc ctgacaggtt cagtggcagt ggatccggca cagattttac actgaaaatc 240
   agcagagtgg aggctgagga ttttggaatt tattattgta tgcaagctct acaaactccg 300
   tacacttttg gccgggggac caaggtggaa atcaaa                           336
<210> 405
   <211> 112
   <212> PRT
   <213> Artificial Sequence
<220>
   <223> Synthetic
<400> 405
   Asp Ile Gln Leu Thr Gln Ser Pro Leu Ser Leu Pro Val Thr Pro Gly
    1               5                   10                  15
   Glu Pro Ala Ser Ile Ser Cys Arg Ser Ser Gln Ser Leu Leu Leu Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Val Ser Ser Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Phe Gly Ile Tyr Tyr Cys Met Gln Ala
                   85                  90                  95
   Leu Gln Thr Pro Tyr Thr Phe Gly Arg Gly Thr Lys Val Glu Ile Lys
               100                 105                 110
<210> 406
   <211> 33
   <212> DNA
   <213> Artificial Sequence
<220>
   <223> Synthetic
<400> 406
   cagagcctcc ttcttagtaa tggatacaac tat 33
<210> 407
   <211> 11
   <212> PRT
   <213> Artificial Sequence
<220>
   <223> Synthetic
<400> 407
   Gln Ser Leu Leu Leu Ser Asn Gly Tyr Asn Tyr
   1 5 10
<210> 408
   <211> 9
   <212> DNA
   <213> Artificial Sequence
<220>
   <223> Synthetic
<400> 408
   ttggtttct 9
<210> 409
   <211> 3
   <212> PRT
   <213> Artificial Sequence
<220>
   <223> Synthetic
<400> 409
   Leu Val Ser
   1
<210> 410
   <211> 27
   <212> DNA
   <213> Artificial Sequence
<220>
   <223> Synthetic
<400> 410
   atgcaagctc tacaaactcc gtacact 27
<210> 411
   <211> 9
   <212> PRT
   <213> Artificial Sequence
<220>
   <223> Synthetic
<400> 411
   Met Gln Ala Leu Gln Thr Pro Tyr Thr
    1               5
<210> 412
   <211> 360
   <212> DNA
   <213> Artificial Sequence
<220>
   <223> Synthetic
<400> 412
   caggtgcagc tgcaggagtc gggcccagga ctggtgaagc cttcacagac cctgtccctc 60
   acctgcactg tctctggtgg ctccgtcagc agtggtaatt actactggag ctgggtccgc 120
   caacacccag ggaagggcct ggagtggttt ggttacatca aaaacagtgg gggcacctac 180
   tacaacccgt ccctcaagag tcgaattacc atatcagtag acacgtctaa gaaccacttc 240
   tccctgaggc tgagctctat gacggccgcg gacacggccg tgtattactg tgcgagagct 300
   ggttcgggga gtcactactt tgactactgg ggccagggaa ccctggtcac cgtctcctca 360
<210> 413
   <211> 120
   <212> PRT
   <213> Artificial Sequence
<220>
   <223> Synthetic
<400> 413
   Gln Val Gln Leu Gln Glu Ser Gly Pro Gly Leu Val Lys Pro Ser Gln
    1               5                  10                  15
   Thr Leu Ser Leu Thr Cys Thr Val Ser Gly Gly Ser Val Ser Ser Gly
               20                  25                  30
   Asn Tyr Tyr Trp Ser Trp Val Arg Gln His Pro Gly Lys Gly Leu Glu
           35                  40                  45
   Trp Phe Gly Tyr Ile Lys Asn Ser Gly Gly Thr Tyr Tyr Asn Pro Ser
       50                  55                  60
   Leu Lys Ser Arg Ile Thr Ile Ser Val Asp Thr Ser Lys Asn His Phe
   65                  70                  75                  80
   Ser Leu Arg Leu Ser Ser Met Thr Ala Ala Asp Thr Ala Val Tyr Tyr
                   85                  90                  95
   Cys Ala Arg Ala Gly Ser Gly Ser His Tyr Phe Asp Tyr Trp Gly Gln
               100                 105                 110
   Gly Thr Leu Val Thr Val Ser Ser
           115                 120
<210> 414
   <211> 30
   <212> DNA
   <213> Artificial Sequence
<220>
   <223> Synthetic
<400> 414
   ggtggctccg tcagcagtgg taattactac 30
<210> 415
   <211> 10
   <212> PRT
   <213> Artificial Sequence
<220>
   <223> Synthetic
<400> 415
   Gly Gly Ser Val Ser Ser Gly Asn Tyr Tyr
   1 5 10
<210> 416
   <211> 21
   <212> DNA
   <213> Artificial Sequence
<220>
   <223> Synthetic
<400> 416
   atcaaaaaca gtgggggcac c 21
<210> 417
   <211> 7
   <212> PRT
   <213> Artificial Sequence
<220>
   <223> Synthetic
<400> 417
   Ile Lys Asn Ser Gly Gly Thr
   1 5
<210> 418
   <211> 36
   <212> DNA
   <213> Artificial Sequence
<220>
   <223> Synthetic
<400> 418
   gcgagagctg gttcggggag tcactacttt gactac 36
<210> 419
   <211> 12
   <212> PRT
   <213> Artificial Sequence
<220>
   <223> Synthetic
<400> 419
   Ala Arg Ala Gly Ser Gly Ser His Tyr Phe Asp Tyr
   1 5 10
<210> 420
   <211> 324
   <212> DNA
   <213> Artificial Sequence
<220>
   <223> Synthetic
<400> 420
   gacatccagt tgacccagtc tccaggcacc ctgtctttgt ctccagggga aagagccacc 60
   ctctcctgca gggccagtca gagtgttagc agcaactact tagcctggta ccagcagaaa 120
   cctggccagg ctcccaggct cctcatctat ggtgcatcca gcagggccac tggcatccca 180
   gacaggttca gtggcagtgg gtctgggaca gacttcactc tcaccatcag cagactggag 240
   cctgaagatt gtgcagtgta ttactgtcag cagtacggtt actcaccgat caccttcggc 300
   caagggacca agctggagat caaa 324
<210> 421
   <211> 108
   <212> PRT
   <213> Artificial Sequence
<220>
   <223> Synthetic
<400> 421
   Asp Ile Gln Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Asn
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Cys Ala Val Tyr Tyr Cys Gln Gln Tyr Gly Tyr Ser Pro
   85 90 95
   Ile Thr Phe Gly Gln Gly Thr Lys Leu Glu Ile Lys
   100 105
<210> 422
   <211> 21
   <212> DNA
   <213> Artificial Sequence
<220>
   <223> Synthetic
<400> 422
   cagagtgtta gcagcaacta c 21
<210> 423
   <211> 7
   <212> PRT
   <213> Artificial Sequence
<220>
   <223> Synthetic
<400> 423
   Gln Ser Val Ser Ser Asn Tyr
   1 5

<210> 424
   <211> 9
   <212> DNA
   <213> Artificial Sequence
<220>
   <223> Synthetic
<400> 424
   ggtgcatcc 9
<210> 425
   <211> 3
   <212> PRT
   <213> Artificial Sequence
<220>
   <223> Synthetic
<400> 425
   Gly Ala Ser
   1
<210> 426
   <211> 27
   <212> DNA
   <213> Artificial Sequence
<220>
   <223> Synthetic
<400> 426
   cagcagtacg gttactcacc gatcacc 27
<210> 427
   <211> 9
   <212> PRT
   <213> Artificial Sequence
<220>
   <223> Synthetic
<400> 427
   Gln Gln Tyr Gly Tyr Ser Pro Ile Thr
   1 5
<210> 428
   <211> 369
   <212> DNA
   <213> Artificial Sequence
<220>
   <223> Synthetic
<400> 428
   gaggtgcagc tggtgcagtc tgggggaggc gtggtccagc ctgggaggtc cctgagactc 60
   tcctgtgcag cgtctggatt caccttcagt agttatggca tgcactgggt ccgccaggct 120
   ccaggcaagg ggctggagtg ggtgtcattt ttatggtatg atggaactaa taaaaactat 180
   gtagagtccg tgaagggccg attcaccatc tcaagagaca attccaagaa tatgctgtat 240
   ctggaaatga acagcctgag agccgaggac acggctgtgt attactgtgc gagagatcac 300
   gattttagga gtggttatga ggggtggttc gacccctggg gccagggaac cctggtcacc 360
   gtctcctca                                                         369
<210> 429
   <211> 123
   <212> PRT
   <213> Artificial Sequence
<220>
   <223> Synthetic
<400> 429
   Glu Val Gln Leu Val Gln Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ser Phe Leu Trp Tyr Asp Gly Thr Asn Lys Asn Tyr Val Glu Ser Val
       50                  55                  60
   Lys Gly Arg Phe Thr Ile Ser Arg Asp Asn Ser Lys Asn Met Leu Tyr
   65                  70                  75                  80
   Leu Glu Met Asn Ser Leu Arg Ala Glu Asp Thr Ala Val Tyr Tyr Cys
                   85                  90                  95
   Ala Arg Asp His Asp Phe Arg Ser Gly Tyr Glu Gly Trp Phe Asp Pro
               100                 105                 110
   Trp Gly Gln Gly Thr Leu Val Thr Val Ser Ser
           115                 120
<210> 430
   <211> 24
   <212> DNA
   <213> Artificial Sequence
<220>
   <223> Synthetic
<400> 430
   ggattcacct tcagtagtta tggc 24
<210> 431
   <211> 8
   <212> PRT
   <213> Artificial Sequence
<220>
   <223> Synthetic
<400> 431
   Gly Phe Thr Phe Ser Ser Tyr Gly
   1 5
<210> 432
   <211> 24
   <212> DNA
   <213> Artificial Sequence
<220>
   <223> Synthetic
<400> 432
   ttatggtatg atggaactaa taaa 24
<210> 433
   <211> 8
   <212> PRT
   <213> Artificial Sequence
<220>
   <223> Synthetic
<400> 433
   Leu Trp Tyr Asp Gly Thr Asn Lys
   1 5
<210> 434
   <211> 48
   <212> DNA
   <213> Artificial Sequence
<220>
   <223> Synthetic
<400> 434
   gcgagagatc acgattttag gagtggttat gaggggtggt tcgacccc 48
<210> 435
   <211> 16
   <212> PRT
   <213> Artificial Sequence
<220>
   <223> Synthetic
<400> 435
   Ala Arg Asp His Asp Phe Arg Ser Gly Tyr Glu Gly Trp Phe Asp Pro
    1               5                  10                  15
<210> 436
   <211> 321
   <212> DNA
   <213> Artificial Sequence
<220>
   <223> Synthetic
<400> 436
   gaaatagtga tgacacagtc tccagccacc ctgtctttgt ctccagggga aagagccacc 60
   ctctcctgca gggccagtca gagtgttagc agctacttag cctggtacca acagaaacct 120
   ggccaggctc ccaggctcct catctatgat gcatccaaca gggccactgg catcccagcc 180
   aggttcagtg gcagtgggtc tgggacagac ttcactctca ccatcagcag cctagagcct 240
   gaagattttg cagtttatta ctgtcaacac cgtagcaact ggcctcccac tttcggcgga 300
   gggaccaagg tggaaatcaa a                                           321
<210> 437
   <211> 107
   <212> PRT
   <213> Artificial Sequence
<220>
   <223> Synthetic
<400> 437
   Glu Ile Val Met Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln His Arg Ser Asn Trp Pro Pro
                   85                  90                  95
   Thr Phe Gly Gly Gly Thr Lys Val Glu Ile Lys
               100                 105
<210> 438
   <211> 18
   <212> DNA
   <213> Artificial Sequence
<220>
   <223> Synthetic
<400> 438
   cagagtgtta gcagctac 18
<210> 439
   <211> 6
   <212> PRT
   <213> Artificial Sequence
<220>
   <223> Synthetic
<400> 439
   Gln Ser Val Ser Ser Tyr
   1 5
<210> 440
   <211> 9
   <212> DNA
   <213> Artificial Sequence
<220>
   <223> Synthetic
<400> 440
   gatgcatcc 9
<210> 441
   <211> 3
   <212> PRT
   <213> Artificial Sequence
<220>
   <223> Synthetic
<400> 441
   Asp Ala Ser
   1
<210> 442
   <211> 27
   <212> DNA
   <213> Artificial Sequence
<220>
   <223> Synthetic
<400> 442
   caacaccgta gcaactggcc tcccact 27
<210> 443
   <211> 9
   <212> PRT
   <213> Artificial Sequence
<220>
   <223> Synthetic
<400> 443
   Gln His Arg Ser Asn Trp Pro Pro Thr
   1               5
<210> 444
   <211> 366
   <212> DNA
   <213> Artificial Sequence
<220>
   <223> Synthetic
<400> 444
   caggtacagc tgcagcagtc gggtccagga ctgctgaaac cttcacagac cctgtccctc 60
   acctgctctg tctctggtgg ctccatcagc agtggtaatt actactggac ctggatccgc 120
   cagcacccag ggaagggcct ggagtggatt gggtacatca agaacagtgg aagcgcctac 180
   tacaatccgt ccctcaagag tcgacttacc atgtcaatag acacgtctca gaaccacttc 240
   tccttgattt tgacttctgt gactgccgcg gacacggcct tatattactg tgcgagagat 300
   gaaaatatag cagttcgtca tgcttttgat atctggggcc aagggacatc ggtcaccgtc 360
   tcctca 366
<210> 445
   <211> 122
   <212> PRT
   <213> Artificial Sequence
<220>
   <223> Synthetic
<400> 445
   Gln Val Gln Leu Gln Gln Ser Gly Pro Gly Leu Leu Lys Pro Ser Gln
   1               5                   10                  15
   Thr Leu Ser Leu Thr Cys Ser Val Ser Gly Gly Ser Ile Ser Ser Gly
               20                  25                  30
   Asn Tyr Tyr Trp Thr Trp Ile Arg Gln His Pro Gly Lys Gly Leu Glu
           35                  40                  45
   Trp Ile Gly Tyr Ile Lys Asn Ser Gly Ser Ala Tyr Tyr Asn Pro Ser
       50                  55                  60
   Leu Lys Ser Arg Leu Thr Met Ser Ile Asp Thr Ser Gln Asn His Phe
   65                  70                  75                  80
   Ser Leu Ile Leu Thr Ser Val Thr Ala Ala Asp Thr Ala Leu Tyr Tyr
                   85                  90                  95
   Cys Ala Arg Asp Glu Asn Ile Ala Val Arg His Ala Phe Asp Ile Trp
               100                 105                 110
   Gly Gln Gly Thr Ser Val Thr Val Ser Ser
           115                 120
<210> 446
   <211> 30
   <212> DNA
   <213> Artificial Sequence
<220>
   <223> Synthetic
<400> 446
   ggtggctcca tcagcagtgg taattactac 30
<210> 447
   <211> 10
   <212> PRT
   <213> Artificial Sequence
<220>
   <223> Synthetic

<400> 447
   Gly Gly Ser Ile Ser Ser Gly Asn Tyr Tyr
   1 5 10
<210> 448
   <211> 21
   <212> DNA
   <213> Artificial Sequence
<220>
   <223> Synthetic
<400> 448
   atcaagaaca gtggaagcgc c 21
<210> 449
   <211> 7
   <212> PRT
   <213> Artificial Sequence
<220>
   <223> Synthetic
<400> 449
   Ile Lys Asn Ser Gly Ser Ala
   1 5
<210> 450
   <211> 42
   <212> DNA
   <213> Artificial Sequence
<220>
   <223> Synthetic
<400> 450
   gcgagagatg aaaatatagc agttcgtcat gcttttgata tc 42
<210> 451
   <211> 14
   <212> PRT
   <213> Artificial Sequence
<220>
   <223> Synthetic
<400> 451
   Ala Arg Asp Glu Asn Ile Ala Val Arg His Ala Phe Asp Ile
   1               5                   10
<210> 452
   <211> 324
   <212> DNA
   <213> Artificial Sequence
<220>
   <223> Synthetic
<400> 452
   gaaatagtgt tgacacagtc tccaggcgcc ctgtctttgt ctccaggaga aagagccacc 60
   ctctcctgta gggccagtcg gactgttagc agcagctact tagcctggta ccaacagaaa 120
   cctggccagg ctcccaggct cctcatctat ggtacatcca gccgggccac tggcatccca 180
   gacaggttca gtggcagtgg gtctgggaca gacttcactc tcaccatcac cagactggag 240
   cctgaagatt ttgcaatata ttactgtcag cagtctggtt actcacctct cactttcggc 300
   ggagggacca aggtggaaat caaa 324
<210> 453
   <211> 108
   <212> PRT
   <213> Artificial Sequence
<220>
   <223> Synthetic
<400> 453
   Glu Ile Val Leu Thr Gln Ser Pro Gly Ala Leu Ser Leu Ser Pro Gly
   1               5                   10                  15
   Glu Arg Ala Thr Leu Ser Cys Arg Ala Ser Arg Thr Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Thr Ser Ser Arg Ala Thr Gly Ile Pro Asp Arg Phe Ser
       50                  55                  60
   Gly Ser Gly Ser Gly Thr Asp Phe Thr Leu Thr Ile Thr Arg Leu Glu
   65                  70                  75                  80
   Pro Glu Asp Phe Ala Ile Tyr Tyr Cys Gln Gln Ser Gly Tyr Ser Pro
                   85                  90                  95
   Leu Thr Phe Gly Gly Gly Thr Lys Val Glu Ile Lys
               100                 105
<210> 454
   <211> 21
   <212> DNA
   <213> Artificial Sequence
<220>
   <223> Synthetic
<400> 454
   cggactgtta gcagcagcta c 21
<210> 455
   <211> 7
   <212> PRT
   <213> Artificial Sequence
<220>
   <223> Synthetic
<400> 455
   Arg Thr Val Ser Ser Ser Tyr
   1 5
<210> 456
   <211> 9
   <212> DNA
   <213> Artificial Sequence
<220>
   <223> Synthetic
<400> 456
   ggtacatcc 9
<210> 457
   <211> 3
   <212> PRT
   <213> Artificial Sequence
<220>
   <223> Synthetic
<400> 457
   Gly Thr Ser
   1
<210> 458
   <211> 27
   <212> DNA
   <213> Artificial Sequence
<220>
   <223> Synthetic
<400> 458
   cagcagtctg gttactcacc tctcact 27
<210> 459
   <211> 9
   <212> PRT
   <213> Artificial Sequence
<220>
   <223> Synthetic
<400> 459
   Gln Gln Ser Gly Tyr Ser Pro Leu Thr
    1               5
<210> 460
   <211> 357
   <212> DNA
   <213> Artificial Sequence
<220>
   <223> Synthetic
<400> 460
   caggtgcagc tgcaggagtc gggcccagga ctggtgaagc cttcacagac cctgtccctc 60
   acctgcactg tctctgatga ctccatcaac aatgttgaat cctactggac ctggatccgc 120
   caacacccag ggaagggcct ggagtggatt ggatacatca aatacactgg gggcatccac 180
   tataacccgt ccctcaagag tcgactcgcc atatcagtgg acacgtcaaa gaaccagttc 240
   tccctgaaaa tgaactctgt gactgccgcg gacacggcca aatattactg tgcgagagca 300
   cgtggaagtc atacttttga tgtctggggc caggggacaa cggtcaccgt ctcctca    357
<210> 461
   <211> 119
   <212> PRT
   <213> Artificial Sequence
<220>
   <223> Synthetic
<400> 461
   Gln Val Gln Leu Gln Glu Ser Gly Pro Gly Leu Val Lys Pro Ser Gln
   1               5                   10                  15
   Thr Leu Ser Leu Thr Cys Thr Val Ser Asp Asp Ser Ile Asn Asn Val
               20                  25                  30
   Glu Ser Tyr Trp Thr Trp Ile Arg Gln His Pro Gly Lys Gly Leu Glu
           35                  40                  45
   Trp Ile Gly Tyr Ile Lys Tyr Thr Gly Gly Ile His Tyr Asn Pro Ser
       50                  55                  60
   Leu Lys Ser Arg Leu Ala Ile Ser Val Asp Thr Ser Lys Asn Gln Phe
   65                  70                  75                  80
   Ser Leu Lys Met Asn Ser Val Thr Ala Ala Asp Thr Ala Lys Tyr Tyr
                   85                  90                  95
   Cys Ala Arg Ala Arg Gly Ser His Thr Phe Asp Val Trp Gly Gln Gly
               100                 105                 110
   Thr Thr Val Thr Val Ser Ser
           115
<210> 462
   <211> 30
   <212> DNA
   <213> Artificial Sequence
<220>
   <223> Synthetic
<400> 462
   gatgactcca tcaacaatgt tgaatcctac 30
<210> 463
   <211> 10
   <212> PRT
   <213> Artificial Sequence
<220>
   <223> Synthetic
<400> 463
   Asp Asp Ser Ile Asn Asn Val Glu Ser Tyr
   1 5 10
<210> 464
   <211> 21
   <212> DNA
   <213> Artificial Sequence
<220>
   <223> Synthetic
<400> 464
   atcaaataca ctgggggcat c 21
<210> 465
   <211> 7
   <212> PRT
   <213> Artificial Sequence
<220>
   <223> Synthetic
<400> 465
   Ile Lys Tyr Thr Gly Gly Ile
   1 5
<210> 466
   <211> 33
   <212> DNA
   <213> Artificial Sequence
<220>
   <223> Synthetic
<400> 466
   gcgagagcac gtggaagtca tacttttgat gtc 33
<210> 467
   <211> 11
   <212> PRT
   <213> Artificial Sequence
<220>
   <223> Synthetic
<400> 467
   Ala Arg Ala Arg Gly Ser His Thr Phe Asp Val
   1 5 10
<210> 468
   <211> 324
   <212> DNA
   <213> Artificial Sequence
<220>
   <223> Synthetic
<400> 468
   gccatccggt tgacccagtc tccaggcacc ctgtcttggt ctccagggga aagagccacc 60
   ctctcctgca gggccagtca gagtgttagc agtaactact tagcctggta ccagcagaaa 120
   cctggccagg ctccccgact cctcatttat ggtgcatcca gcagggtcgc tggcatccca 180
   gacaggttca gtggcagtgg gtctgggaca gacttcactc tcaccatcag cagactggag 240
   cctgaagatt ttgcactgta ttattgtcag caatatagta ggtcaccgat caccttcggc 300
   caagggacca agctggagat caaa 324
<210> 469
   <211> 108
   <212> PRT
   <213> Artificial Sequence
<220>
   <223> Synthetic
<400> 469
   Ala Ile Arg Leu Thr Gln Ser Pro Gly Thr Leu Ser Trp Ser Pro Gly
   1 5 10 15
   Glu Arg Ala Thr Leu Ser Cys Arg Ala Ser Gln Ser Val Ser Ser Asn
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Val Ala Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Leu Tyr Tyr Cys Gln Gln Tyr Ser Arg Ser Pro
   85 90 95
   Ile Thr Phe Gly Gln Gly Thr Lys Leu Glu Ile Lys
   100 105
<210> 470
   <211> 21
   <212> DNA
   <213> Artificial Sequence
<220>
   <223> Synthetic
<400> 470
   cagagtgtta gcagtaacta c 21
<210> 471
   <211> 7
   <212> PRT
   <213> Artificial Sequence
<220>
   <223> Synthetic
<400> 471
   Gln Ser Val Ser Ser Asn Tyr
   1 5
<210> 472
   <211> 9
   <212> DNA
   <213> Artificial Sequence
<220>
   <223> Synthetic
<400> 472
   ggtgcatcc 9
<210> 473
   <211> 3
   <212> PRT
   <213> Artificial Sequence
<220>
   <223> Synthetic
<400> 473
   Gly Ala Ser
   1
<210> 474
   <211> 27
   <212> DNA
   <213> Artificial Sequence
<220>
   <223> Synthetic
<400> 474
   cagcaatata gtaggtcacc gatcacc 27
<210> 475
   <211> 9
   <212> PRT
   <213> Artificial Sequence
<220>
   <223> Synthetic
<400> 475
   Gln Gln Tyr Ser Arg Ser Pro Ile Thr
    1               5
<210> 476
   <211> 357
   <212> DNA
   <213> Artificial Sequence
<220>
   <223> Synthetic
<400> 476
   caggtgcagc tgcaggagtc gggcccagga ctggtgaagc cttcacagac cctgtccctc 60
   acctgcactg tctctggtgg ctccatcaac agtgttactt actactggac ctggatccgc 120
   cagcacccag ggaggggcct agagtggatt gggtacatca aattcagtgg gagcacctac 180
   tacaacccgt ccctcaaggg tcgagtcacc atatcagtgg acacgtctaa gaaccaattc 240
   tcccttaaaa ttaactctgt gactgccgcg gacacggccg tgttttactg tgcgagagct 300
   tctggaagtc atacttttga tatctggggc caagggacaa tggtcaccgt ctcctca    357
<210> 477
   <211> 119
   <212> PRT
   <213> Artificial Sequence
<220>
   <223> Synthetic
<400> 477
   Gln Val Gln Leu Gln Glu Ser Gly Pro Gly Leu Val Lys Pro Ser Gln
    1               5                  10                  15
   Thr Leu Ser Leu Thr Cys Thr Val Ser Gly Gly Ser Ile Asn Ser Val
               20                  25                  30
   Thr Tyr Tyr Trp Thr Trp Ile Arg Gln His Pro Gly Arg Gly Leu Glu
           35                  40                  45
   Trp Ile Gly Tyr Ile Lys Phe Ser Gly Ser Thr Tyr Tyr Asn Pro Ser
       50                  55                  60
   Leu Lys Gly Arg Val Thr Ile Ser Val Asp Thr Ser Lys Asn Gln Phe
   65                  70                  75                  80
   Ser Leu Lys Ile Asn Ser Val Thr Ala Ala Asp Thr Ala Val Phe Tyr
                   85                  90                  95
   Cys Ala Arg Ala Ser Gly Ser His Thr Phe Asp Ile Trp Gly Gln Gly
               100                 105                 110
   Thr Met Val Thr Val Ser Ser
           115
<210> 478
   <211> 30
   <212> DNA
   <213> Artificial Sequence
<220>
   <223> Synthetic
<400> 478
   ggtggctcca tcaacagtgt tacttactac 30
<210> 479
   <211> 10
   <212> PRT
   <213> Artificial Sequence
<220>
   <223> Synthetic
<400> 479
   Gly Gly Ser Ile Asn Ser Val Thr Tyr Tyr
   1 5 10
<210> 480
   <211> 21
   <212> DNA
   <213> Artificial Sequence
<220>
   <223> Synthetic
<400> 480
   atcaaattca gtgggagcac c 21
<210> 481
   <211> 7
   <212> PRT
   <213> Artificial Sequence
<220>
   <223> Synthetic
<400> 481
   Ile Lys Phe Ser Gly Ser Thr
   1 5
<210> 482
   <211> 33
   <212> DNA
   <213> Artificial Sequence
<220>
   <223> Synthetic
<400> 482
   gcgagagctt ctggaagtca tacttttgat atc 33
<210> 483
   <211> 11
   <212> PRT
   <213> Artificial Sequence
<220>
   <223> Synthetic
<400> 483
   Ala Arg Ala Ser Gly Ser His Thr Phe Asp Ile
   1 5 10
<210> 484
   <211> 321
   <212> DNA
   <213> Artificial Sequence
<220>
   <223> Synthetic
<400> 484
   gccatccgga tgacccagtc tccagccacc ctgtctttgt ctccagggga aagagccacc 60
   ctctcctgct gggccagtca gagtattagc ggctattttg cctggtatca acagaaacct 120
   ggccaggctc ccaggctcct catctatgat acatcctaca gggccactga cgtcccagcc 180
   aggttcagtg gcagtgggtc tgggacagac ttcactctca ccatcaacaa cctagagcct 240
   gaagattttg cagtttatta ctgtcagcag cgtagcgact ggccgctcag cttcggcgga 300
   gggaccaaac tggagatcaa a 321
<210> 485
   <211> 107
   <212> PRT
   <213> Artificial Sequence
<220>
   <223> Synthetic
<400> 485
   Ala Ile Arg Met Thr Gln Ser Pro Ala Thr Leu Ser Leu Ser Pro Gly
   1 5 10 15
   Glu Arg Ala Thr Leu Ser Cys Trp Ala Ser Gln Ser Ile Ser Gly Tyr
   20 25 30
   Phe Ala Trp Tyr Gln Gln Lys Pro Gly Gln Ala Pro Arg Leu Leu Ile
   35 40 45
   Tyr Asp Thr Ser Tyr Arg Ala Thr Asp Val Pro Ala Arg Phe Ser Gly
   50 55 60
   Ser Gly Ser Gly Thr Asp Phe Thr Leu Thr Ile Asn Asn Leu Glu Pro
   65 70 75 80
   Glu Asp Phe Ala Val Tyr Tyr Cys Gln Gln Arg Ser Asp Trp Pro Leu
   85 90 95
   Ser Phe Gly Gly Gly Thr Lys Leu Glu Ile Lys
   100 105
<210> 486
   <211> 18
   <212> DNA
   <213> Artificial Sequence
<220>
   <223> Synthetic
<400> 486
   cagagtatta gcggctat 18
<210> 487
   <211> 6
   <212> PRT
   <213> Artificial Sequence
<220>
   <223> Synthetic
<400> 487
   Gln Ser Ile Ser Gly Tyr
   1 5
<210> 488
   <211> 9
   <212> DNA
   <213> Artificial Sequence
<220>
   <223> Synthetic
<400> 488
   gatacatcc 9
<210> 489
   <211> 3
   <212> PRT
   <213> Artificial Sequence
<220>
   <223> Synthetic
<400> 489
   Asp Thr Ser
   1
<210> 490
   <211> 27
   <212> DNA
   <213> Artificial Sequence
<220>
   <223> Synthetic
<400> 490
   cagcagcgta gcgactggcc gctcagc 27
<210> 491
   <211> 9
   <212> PRT
   <213> Artificial Sequence
<220>
   <223> Synthetic
<400> 491
   Gln Gln Arg Ser Asp Trp Pro Leu Ser
    1               5
<210> 492
   <211> 357
   <212> DNA
   <213> Artificial Sequence
<220>
   <223> Synthetic
<400> 492
   caggtgcagc tgcaggagtc gggcccagga ctggtgaagc cttcacagac cctgtccctc 60
   acctgcactg tctctggtgg ctccatcaac agtgttactt actactggac ctggatccgc 120
   cagcacccag ggaggggcct agagtggatt gggtacatca aattcagtgg gagcacctac 180
   tacaacccgt ccctcaaggg tcgagtcacc atatcagtgg acacgtctaa gaaccaattc 240
   tcccttaaaa ttaactctgt gactgccgcg gacacggccg tgttttactg tgcgagagct 300
   tctggaagtc atacttttga tatctggggc caagggacaa tggtcaccgt ctcctca    357
<210> 493
   <211> 119
   <212> PRT
   <213> Artificial Sequence
<220>
   <223> Synthetic
<400> 493
   Gln Val Gln Leu Gln Glu Ser Gly Pro Gly Leu Val Lys Pro Ser Gln
    1               5                  10                  15
   Thr Leu Ser Leu Thr Cys Thr Val Ser Gly Gly Ser Ile Asn Ser Val
               20                  25                  30
   Thr Tyr Tyr Trp Thr Trp Ile Arg Gln His Pro Gly Arg Gly Leu Glu
           35                  40                  45
   Trp Ile Gly Tyr Ile Lys Phe Ser Gly Ser Thr Tyr Tyr Asn Pro Ser
       50                  55                  60
   Leu Lys Gly Arg Val Thr Ile Ser Val Asp Thr Ser Lys Asn Gln Phe
   65                  70                  75                  80
   Ser Leu Lys Ile Asn Ser Val Thr Ala Ala Asp Thr Ala Val Phe Tyr
                   85                  90                  95
   Cys Ala Arg Ala Ser Gly Ser His Thr Phe Asp Ile Trp Gly Gln Gly
               100                 105                 110
   Thr Met Val Thr Val Ser Ser
           115
<210> 494
   <211> 30
   <212> DNA
   <213> Artificial Sequence
<220>
   <223> Synthetic
<400> 494
   ggtggctcca tcaacagtgt tacttactac 30
<210> 495
   <211> 10
   <212> PRT
   <213> Artificial Sequence
<220>
   <223> Synthetic
<400> 495
   Gly Gly Ser Ile Asn Ser Val Thr Tyr Tyr
   1 5 10
<210> 496
   <211> 21
   <212> DNA
   <213> Artificial Sequence
<220>
   <223> Synthetic
<400> 496
   atcaaattca gtgggagcac c 21
<210> 497
   <211> 7
   <212> PRT
   <213> Artificial Sequence
<220>
   <223> Synthetic
<400> 497
   Ile Lys Phe Ser Gly Ser Thr
   1 5
<210> 498
   <211> 33
   <212> DNA
   <213> Artificial Sequence
<220>
   <223> Synthetic
<400> 498
   gcgagagctt ctggaagtca tacttttgat atc 33
<210> 499
   <211> 11
   <212> PRT
   <213> Artificial Sequence
<220>
   <223> Synthetic
<400> 499
   Ala Arg Ala Ser Gly Ser His Thr Phe Asp Ile
   1 5 10
<210> 500
   <211> 324
   <212> DNA
   <213> Artificial Sequence
<220>
   <223> Synthetic
<400> 500
   gatattgtga tgacccagtc tccaggcacc ctgtctttgt ctccagggga aagagccacc 60
   ctctcctgca gggccagtca gagtgttagc aacagctact tagcctggta ccagcagaaa 120
   cctggccagg ctcccaggct cctcatctct ggtgcgtcca gcagggtcac tggcatccca 180
   gacaggttca gtggcagtgg gtctgggaca gacttcactc tcaccatcag cagactggag 240
   cctgaagatt ttggaatgta ttactgtcag cagtatagta ggtcaccgat caccttcggc 300
   caagggacca aggtggaaat caaa 324
<210> 501
   <211> 108
   <212> PRT
   <213> Artificial Sequence
<220>
   <223> Synthetic
<400> 501
   Asp Ile Val Met Thr Gln Ser Pro Gly Thr Leu Ser Leu Ser Pro Gly
   1 5 10 15
   Glu Arg Ala Thr Leu Ser Cys Arg Ala Ser Gln Ser Val Ser Asn Ser
   20 25 30
   Tyr Leu Ala Trp Tyr Gln Gln Lys Pro Gly Gln Ala Pro Arg Leu Leu
   35 40 45
   Ile Ser Gly Ala Ser Ser Arg Val Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Gly Met Tyr Tyr Cys Gln Gln Tyr Ser Arg Ser Pro
   85 90 95
   Ile Thr Phe Gly Gln Gly Thr Lys Val Glu Ile Lys
   100 105
<210> 502
   <211> 21
   <212> DNA
   <213> Artificial Sequence
<220>
   <223> Synthetic
<400> 502
   cagagtgtta gcaacagcta c 21
<210> 503
   <211> 7
   <212> PRT
   <213> Artificial Sequence
<220>
   <223> Synthetic
<400> 503
   Gln Ser Val Ser Asn Ser Tyr
   1 5
<210> 504
   <211> 9
   <212> DNA
   <213> Artificial Sequence
<220>
   <223> Synthetic
<400> 504
   ggtgcgtcc 9
<210> 505
   <211> 3
   <212> PRT
   <213> Artificial Sequence
<220>
   <223> Synthetic
<400> 505
   Gly Ala Ser
   1
<210> 506
   <211> 27
   <212> DNA
   <213> Artificial Sequence
<220>
   <223> Synthetic
<400> 506
   cagcagtata gtaggtcacc gatcacc 27
<210> 507
   <211> 9
   <212> PRT
   <213> Artificial Sequence
<220>
   <223> Synthetic
<400> 507
   Gln Gln Tyr Ser Arg Ser Pro Ile Thr
   1 5
<210> 508
   <211> 357
   <212> DNA
   <213> Artificial Sequence
<220>
   <223> Synthetic
<400> 508
   gaggtgcagc tggtgcagtc tggagctgag gtgaagaagc ctggggcctc agtgaaggtc 60
   tcctgcaagg cttctggtta ctcctttacc agctttggtt tcagctgggt gcgacaggcc 120
   cctggacaag gacttgagtg gctgggatgg atcagcgctt acagtggtga cacagactat 180
   gcacagaagt tccagggcag agtcaccctg accactgaca catccacgac cactgcctac 240
   atggagctga ggagcctgag atctgacgac acggccgtct attactgtgc gcgatataac 300
   tggaacctcc actggttcga cccctggggc cagggaaccc tggtcaccgt ctcctca 357
<210> 509
   <211> 119
   <212> PRT
   <213> Artificial Sequence
<220>
   <223> Synthetic
<400> 509
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Ser Phe Thr Ser Phe
   20 25 30
   Gly Phe Ser Trp Val Arg Gln Ala Pro Gly Gln Gly Leu Glu Trp Leu
   35 40 45
   Gly Trp Ile Ser Ala Tyr Ser Gly Asp Thr Asp Tyr Ala Gln Lys Phe
   50 55 60
   Gln Gly Arg Val Thr Leu Thr Thr Asp Thr Ser Thr Thr Thr Ala Tyr
   65 70 75 80
   Met Glu Leu Arg Ser Leu Arg Ser Asp Asp Thr Ala Val Tyr Tyr Cys
   85 90 95
   Ala Arg Tyr Asn Trp Asn Leu His Trp Phe Asp Pro Trp Gly Gln Gly
   100 105 110
   Thr Leu Val Thr Val Ser Ser
   115
<210> 510
   <211> 24
   <212> DNA
   <213> Artificial Sequence
<220>
   <223> Synthetic
<400> 510
   ggttactcct ttaccagctt tggt 24
<210> 511
   <211> 8
   <212> PRT
   <213> Artificial Sequence
<220>
   <223> Synthetic
<400> 511
   Gly Tyr Ser Phe Thr Ser Phe Gly
   1 5
<210> 512
   <211> 24
   <212> DNA
   <213> Artificial Sequence
<220>
   <223> Synthetic
<400> 512
   atcagcgctt acagtggtga caca 24
<210> 513
   <211> 8
   <212> PRT
   <213> Artificial Sequence
<220>
   <223> Synthetic
<400> 513
   Ile Ser Ala Tyr Ser Gly Asp Thr
   1 5
<210> 514
   <211> 36
   <212> DNA
   <213> Artificial Sequence
<220>
   <223> Synthetic
<400> 514
   gcgcgatata actggaacct ccactggttc gacccc 36
<210> 515
   <211> 12
   <212> PRT
   <213> Artificial Sequence
<220>
   <223> Synthetic
<400> 515
   Ala Arg Tyr Asn Trp Asn Leu His Trp Phe Asp Pro
   1 5 10
<210> 516
   <211> 324
   <212> DNA
   <213> Artificial Sequence
<220>
   <223> Synthetic
<400> 516
   gacatccgga tgacccagtc tccaggcatc ctgtctttgt ctccagggga aagagccacc 60
   ctctcctgca gggccagtca gaatattaaa agcaactact tagcctggta ccagcagaaa 120
   cctggccagg ctcccaggct cctcatcttt ggtacatcca acagggccac tgccatttca 180
   gacaggttca gtggcagtgg gtctgggaca gacttccttt tcaccatcag cagactggag 240
   cctgaagatt ttgcagtgta ttactgtcag cagtatggta actcaccgtg gacgttcggc 300
   caagggacca aagtggatat caaa 324
<210> 517
   <211> 108
   <212> PRT
   <213> Artificial Sequence
<220>
   <223> Synthetic
<400> 517
   Asp Ile Arg Met Thr Gln Ser Pro Gly Ile Leu Ser Leu Ser Pro Gly
   1 5 10 15
   Glu Arg Ala Thr Leu Ser Cys Arg Ala Ser Gln Asn Ile Lys Ser Asn
   20 25 30
   Tyr Leu Ala Trp Tyr Gln Gln Lys Pro Gly Gln Ala Pro Arg Leu Leu
   35 40 45
   Ile Phe Gly Thr Ser Asn Arg Ala Thr Ala Ile Ser Asp Arg Phe Ser
   50 55 60
   Gly Ser Gly Ser Gly Thr Asp Phe Leu Phe Thr Ile Ser Arg Leu Glu
   65 70 75 80
   Pro Glu Asp Phe Ala Val Tyr Tyr Cys Gln Gln Tyr Gly Asn Ser Pro
   85 90 95
   Trp Thr Phe Gly Gln Gly Thr Lys Val Asp Ile Lys
   100 105
<210> 518
   <211> 21
   <212> DNA
   <213> Artificial Sequence
<220>
   <223> Synthetic
<400> 518
   cagaatatta aaagcaacta c 21
<210> 519
   <211> 7
   <212> PRT
   <213> Artificial Sequence
<220>
   <223> Synthetic
<400> 519
   Gln Asn Ile Lys Ser Asn Tyr
   1 5
<210> 520
   <211> 9
   <212> DNA
   <213> Artificial Sequence
<220>
   <223> Synthetic
<400> 520
   ggtacatcc 9
<210> 521
   <211> 3
   <212> PRT
   <213> Artificial Sequence
<220>
   <223> Synthetic
<400> 521
   Gly Thr Ser
   1
<210> 522
   <211> 27
   <212> DNA
   <213> Artificial Sequence
<220>
   <223> Synthetic
<400> 522
   cagcagtatg gtaactcacc gtggacg 27
<210> 523
   <211> 9
   <212> PRT
   <213> Artificial Sequence
<220>
   <223> Synthetic
<400> 523
   Gln Gln Tyr Gly Asn Ser Pro Trp Thr
   1 5
<210> 524
   <211> 357
   <212> DNA
   <213> Artificial Sequence
<220>
   <223> Synthetic
<400> 524
   caggtgcagc tgcaggagtc gggcccagga ctggtgaagc cttcacagac cctgtccctc 60
   acctgcactg tctctgatgg ctccatcaac agtgttgaat cctactggac ctggatccgc 120
   cagcacccag ggaagggcct ggagtggatt ggatacatca aatacactgg gggcatccac 180
   tataacccgt ccctcaagag tcgacttgcc atatcagtgg acacgtcaaa gaaccagttc 240
   tccctgaaaa tgagctctgt gactgccgcg gacacggccg tgtattactg tgcgagagca 300
   cgtggaagtc atacttttga tgtctggggc caggggacaa tggtcaccgt ctcttca    357
<210> 525
   <211> 119
   <212> PRT
   <213> Artificial Sequence
<220>
   <223> Synthetic
<400> 525
   Gln Val Gln Leu Gln Glu Ser Gly Pro Gly Leu Val Lys Pro Ser Gln
   1               5                   10                  15
   Thr Leu Ser Leu Thr Cys Thr Val Ser Asp Gly Ser Ile Asn Ser Val
               20                  25                  30
   Glu Ser Tyr Trp Thr Trp Ile Arg Gln His Pro Gly Lys Gly Leu Glu
           35                  40                  45
   Trp Ile Gly Tyr Ile Lys Tyr Thr Gly Gly Ile His Tyr Asn Pro Ser
       50                  55                  60
   Leu Lys Ser Arg Leu Ala Ile Ser Val Asp Thr Ser Lys Asn Gln Phe
   65                  70                  75                  80
   Ser Leu Lys Met Ser Ser Val Thr Ala Ala Asp Thr Ala Val Tyr Tyr
                   85                  90                  95
   Cys Ala Arg Ala Arg Gly Ser His Thr Phe Asp Val Trp Gly Gln Gly
               100                 105                 110
   Thr Met Val Thr Val Ser Ser
           115
<210> 526
   <211> 30
   <212> DNA
   <213> Artificial Sequence
<220>
   <223> Synthetic
<400> 526
   gatggctcca tcaacagtgt tgaatcctac 30
<210> 527
   <211> 10
   <212> PRT
   <213> Artificial Sequence
<220>
   <223> Synthetic
<400> 527
   Asp Gly Ser Ile Asn Ser Val Glu Ser Tyr
   1 5 10
<210> 528
   <211> 21
   <212> DNA
   <213> Artificial Sequence
<220>
   <223> Synthetic
<400> 528
   atcaaataca ctgggggcat c 21
<210> 529
   <211> 7
   <212> PRT
   <213> Artificial Sequence
<220>
   <223> Synthetic
<400> 529
   Ile Lys Tyr Thr Gly Gly Ile
   1 5
<210> 530
   <211> 33
   <212> DNA
   <213> Artificial Sequence
<220>
   <223> Synthetic
<400> 530
   gcgagagcac gtggaagtca tacttttgat gtc 33
<210> 531
   <211> 11
   <212> PRT
   <213> Artificial Sequence
<220>
   <223> Synthetic
<400> 531
   Ala Arg Ala Arg Gly Ser His Thr Phe Asp Val
   1 5 10
<210> 532
   <211> 324
   <212> DNA
   <213> Artificial Sequence
<220>
   <223> Synthetic
<400> 532
   gaaattgtgc tgactcagtc tccaggcacc ctgtcttggt ctccagggga aagagccacc 60
   ctctcctgca gggccagtca gagtattagc agtaactact tagcctggta ccagcagaaa 120
   cctggccagg ctcccagact cctcatttat ggtgcatcca gcagggtcac tggcatccca 180
   gacaggttca gtggcagtgg gtctgggaca gacttcactc tcaccatcag cagactggag 240
   cctgaagatt ttgcactgta ttattgtcag cagtatagta ggtcaccgat caccttcggc 300
   caagggacca aagtggatat caaa                                         324
<210> 533
   <211> 108
   <212> PRT
   <213> Artificial Sequence
<220>
   <223> Synthetic
<400> 533
   Glu Ile Val Leu Thr Gln Ser Pro Gly Thr Leu Ser Trp Ser Pro Gly
    1               5                  10                  15
   Glu Arg Ala Thr Leu Ser Cys Arg Ala Ser Gln Ser Ile Ser Ser Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Val Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Leu Tyr Tyr Cys Gln Gln Tyr Ser Arg Ser Pro
                   85                  90                  95
   Ile Thr Phe Gly Gln Gly Thr Lys Val Asp Ile Lys
               100                 105
<210> 534
   <211> 21
   <212> DNA
   <213> Artificial Sequence
<220>
   <223> Synthetic
<400> 534
   cagagtatta gcagtaacta c 21
<210> 535
   <211> 7
   <212> PRT
   <213> Artificial Sequence
<220>
   <223> Synthetic
<400> 535
   Gln Ser Ile Ser Ser Asn Tyr
   1 5
<210> 536
   <211> 9
   <212> DNA
   <213> Artificial Sequence
<220>
   <223> Synthetic
<400> 536
   ggtgcatcc 9
<210> 537
   <211> 3
   <212> PRT
   <213> Artificial Sequence
<220>
   <223> Synthetic
<400> 537
   Gly Ala Ser
   1
<210> 538
   <211> 27
   <212> DNA
   <213> Artificial Sequence
<220>
   <223> Synthetic
<400> 538
   cagcagtata gtaggtcacc gatcacc 27
<210> 539
   <211> 9
   <212> PRT
   <213> Artificial Sequence
<220>
   <223> Synthetic

<400> 539
   Gln Gln Tyr Ser Arg Ser Pro Ile Thr
   1 5
<210> 540
   <211> 360
   <212> DNA
   <213> Artificial Sequence
<220>
   <223> Synthetic
<400> 540
   caggtgcagc tgcaggagtc gggcccagga ctggtgaagc cttcacagac cctgtccctc 60
   acctgcactg tctctggtgg ctccgtcagc agtggtaatt actactggag ctggatccgc 120
   cagcacccag ggaagggcct ggagtggttt gggtacatca aaaacagtgg gggcacctac 180
   tacaacccgt ccctcaagag tcgaattacc atatcagtag acacgtctaa gaaccacttc 240
   tccctgaggc tgagctctat gacggccgcg gacacggccg tgtattactg tgcgagagct 300
   ggttcgggga gtcactactt tgactactgg ggccagggaa ccctggtcac cgtctcctca 360
<210> 541
   <211> 120
   <212> PRT
   <213> Artificial Sequence
<220>
   <223> Synthetic
<400> 541
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Val Ser Ser Gly
               20                  25                  30
   Asn Tyr Tyr Trp Ser Trp Ile Arg Gln His Pro Gly Lys Gly Leu Glu
           35                  40                  45
   Trp Phe Gly Tyr Ile Lys Asn Ser Gly Gly Thr Tyr Tyr Asn Pro Ser
       50                  55                  60
   Leu Lys Ser Arg Ile Thr Ile Ser Val Asp Thr Ser Lys Asn His Phe
   65                  70                  75                  80
   Ser Leu Arg Leu Ser Ser Met Thr Ala Ala Asp Thr Ala Val Tyr Tyr
                   85                  90                  95
   Cys Ala Arg Ala Gly Ser Gly Ser His Tyr Phe Asp Tyr Trp Gly Gln
               100                 105                 110
   Gly Thr Leu Val Thr Val Ser Ser
           115                 120
<210> 542
   <211> 30
   <212> DNA
   <213> Artificial Sequence
<220>
   <223> Synthetic
<400> 542
   ggtggctccg tcagcagtgg taattactac 30
<210> 543
   <211> 10
   <212> PRT
   <213> Artificial Sequence
<220>
   <223> Synthetic
<400> 543
   Gly Gly Ser Val Ser Ser Gly Asn Tyr Tyr
   1 5 10
<210> 544
   <211> 21
   <212> DNA
   <213> Artificial Sequence
<220>
   <223> Synthetic
<400> 544
   atcaaaaaca gtgggggcac c 21
<210> 545
   <211> 7
   <212> PRT
   <213> Artificial Sequence
<220>
   <223> Synthetic
<400> 545
   Ile Lys Asn Ser Gly Gly Thr
   1 5
<210> 546
   <211> 36
   <212> DNA
   <213> Artificial Sequence
<220>
   <223> Synthetic
<400> 546
   gcgagagctg gttcggggag tcactacttt gactac 36
<210> 547
   <211> 12
   <212> PRT
   <213> Artificial Sequence
<220>
   <223> Synthetic
<400> 547
   Ala Arg Ala Gly Ser Gly Ser His Tyr Phe Asp Tyr
   1 5 10
<210> 548
   <211> 324
   <212> DNA
   <213> Artificial Sequence
<220>
   <223> Synthetic
<400> 548
   gaaacgacac tcacgcagtc tccaggcacc ctgtctttgt ctccagggga aagagccacc 60
   ctctcctgca gggccagtca gagtgttagc agcagctact tagcctggta ccagcagaaa 120
   cctggccagg ctcccaggct cctcatctat ggtgcatcca gcagggccac tggcatccca 180
   gacaggctca gtggcagtgg gtctgggaca gacttcactc tcaccatcag cagactggag 240
   cctgaagatt gtgcagtgta ttactgtcag cagtatggtt actcaccgat caccttcggc 300
   caagggacca aggtggaaat caaa                                        324
<210> 549
   <211> 108
   <212> PRT
   <213> Artificial Sequence
<220>
   <223> Synthetic
<400> 549
   Glu Thr Thr Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Leu Ser
       50                  55                  60
   Gly Ser Gly Ser Gly Thr Asp Phe Thr Leu Thr Ile Ser Arg Leu Glu
   65                  70                  75                  80
   Pro Glu Asp Cys Ala Val Tyr Tyr Cys Gln Gln Tyr Gly Tyr Ser Pro
                   85                  90                  95
   Ile Thr Phe Gly Gln Gly Thr Lys Val Glu Ile Lys
               100                 105
<210> 550
   <211> 21
   <212> DNA
   <213> Artificial Sequence
<220>
   <223> Synthetic
<400> 550
   cagagtgtta gcagcagcta c 21
<210> 551
   <211> 7
   <212> PRT
   <213> Artificial Sequence
<220>
   <223> Synthetic
<400> 551
   Gln Ser Val Ser Ser Ser Tyr
   1               5
<210> 552
   <211> 9
   <212> DNA
   <213> Artificial Sequence
<220>
   <223> Synthetic
<400> 552
   ggtgcatcc 9
<210> 553
   <211> 3
   <212> PRT
   <213> Artificial Sequence
<220>
   <223> Synthetic
<400> 553
   Gly Ala Ser
   1
<210> 554
   <211> 27
   <212> DNA
   <213> Artificial Sequence
<220>
   <223> Synthetic
<400> 554
   cagcagtatg gttactcacc gatcacc 27
<210> 555
   <211> 9
   <212> PRT
   <213> Artificial Sequence
<220>
   <223> Synthetic
<400> 555
   Gln Gln Tyr Gly Tyr Ser Pro Ile Thr
   1               5
<210> 556
   <211> 369
   <212> DNA
   <213> Artificial Sequence
<220>
   <223> Synthetic
<400> 556
   gaagtgcagc tggtgcagtc tgggggaggc gtggtccagc ctgggaggtc cctgagactc 60
   tcctgtacag cgtctggatt caccttcagt agctatgcca tgtactgggt ccgccaggct 120
   ccaggcaagg ggctggagtg ggtggcagtt atatggtatg atggaagtaa taaaaactat 180
   gcagactccg tgaagggccg attcaccatc tccagagaca attccaagaa cacactgtat 240
   ctgcaagtga acagcctgag agccgaggac acggctgtgt attactgtgc gagagatcac 300
   gattttttga gtggttatga ggggtggttc gacccctggg gccagggaac cctggtcacc 360
   gtctcctca                                                         369
<210> 557
   <211> 123
   <212> PRT
   <213> Artificial Sequence
<220>
   <223> Synthetic
<400> 557
   Glu Val Gln Leu Val Gln Ser Gly Gly Gly Val Val Gln Pro Gly Arg
   1               5                   10                  15
   Ser Leu Arg Leu Ser Cys Thr Ala Ser Gly Phe Thr Phe Ser Ser Tyr
               20                  25                  30
   Ala Met Tyr Trp Val Arg Gln Ala Pro Gly Lys Gly Leu Glu Trp Val
           35                  40                  45
   Ala Val Ile Trp Tyr Asp Gly Ser Asn Lys Asn Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Val Asn Ser Leu Arg Ala Glu Asp Thr Ala Val Tyr Tyr Cys
                   85                  90                  95
   Ala Arg Asp His Asp Phe Leu Ser Gly Tyr Glu Gly Trp Phe Asp Pro
               100                 105                 110
   Trp Gly Gln Gly Thr Leu Val Thr Val Ser Ser
           115                 120
<210> 558
   <211> 24
   <212> DNA
   <213> Artificial Sequence
<220>
   <223> Synthetic
<400> 558
   ggattcacct tcagtagcta tgcc 24
<210> 559
   <211> 8
   <212> PRT
   <213> Artificial Sequence
<220>
   <223> Synthetic
<400> 559
   Gly Phe Thr Phe Ser Ser Tyr Ala
   1 5
<210> 560
   <211> 24
   <212> DNA
   <213> Artificial Sequence
<220>
   <223> Synthetic
<400> 560
   atatggtatg atggaagtaa taaa 24
<210> 561
   <211> 8
   <212> PRT
   <213> Artificial Sequence
<220>
   <223> Synthetic
<400> 561
   Ile Trp Tyr Asp Gly Ser Asn Lys
   1 5
<210> 562
   <211> 48
   <212> DNA
   <213> Artificial Sequence
<220>
   <223> Synthetic
<400> 562
   gcgagagatc acgatttttt gagtggttat gaggggtggt tcgacccc 48
<210> 563
   <211> 16
   <212> PRT
   <213> Artificial Sequence
<220>
   <223> Synthetic
<400> 563
   Ala Arg Asp His Asp Phe Leu Ser Gly Tyr Glu Gly Trp Phe Asp Pro
   1 5 10 15
<210> 564
   <211> 321
   <212> DNA
   <213> Artificial Sequence
<220>
   <223> Synthetic
<400> 564
   gaaattgtgc tgactcagtc tccagccacc ctgtctttgt ctccagggga aagagccacc 60
   ctctcctgca gggccagtca gagtgttagt agctacttag cctggtacca acagaaacct 120
   ggccaggctc ccaggctcct catctatgat gcatccaaca gggccactgg catcccagcc 180
   aggttcagtg gcagtgggtc tgggacagac ttcactctca ccatcagtag cctagagcct 240
   gaagattttg cagtttatta ctgtcagcaa cgtagcaact ggcctcccac tttcggcgga 300
   gggaccaaag tggatatcaa a                                           321
<210> 565
   <211> 107
   <212> PRT
   <213> Artificial Sequence
<220>
   <223> Synthetic
<400> 565
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Pro
                   85                  90                  95
   Thr Phe Gly Gly Gly Thr Lys Val Asp Ile Lys
               100                 105
<210> 566
   <211> 18
   <212> DNA
   <213> Artificial Sequence
<220>
   <223> Synthetic
<400> 566
   cagagtgtta gtagctac 18
<210> 567
   <211> 6
   <212> PRT
   <213> Artificial Sequence
<220>
   <223> Synthetic
<400> 567
   Gln Ser Val Ser Ser Tyr
   1 5
<210> 568
   <211> 9
   <212> DNA
   <213> Artificial Sequence
<220>
   <223> Synthetic
<400> 568
   gatgcatcc 9
<210> 569
   <211> 3
   <212> PRT
   <213> Artificial Sequence
<220>
   <223> Synthetic
<400> 569
   Asp Ala Ser
   1
<210> 570
   <211> 27
   <212> DNA
   <213> Artificial Sequence
<220>
   <223> Synthetic
<400> 570
   cagcaacgta gcaactggcc tcccact 27
<210> 571
   <211> 9
   <212> PRT
   <213> Artificial Sequence
<220>
   <223> Synthetic
<400> 571
   Gln Gln Arg Ser Asn Trp Pro Pro Thr
   1 5
<210> 572
   <211> 375
   <212> DNA
   <213> Artificial Sequence
<220>
   <223> Synthetic
<400> 572
   gaggtgcagc tggtgcagtc tggagctgaa gtgaagaacc ctggggcctc agttagggtc 60
   tcctgcaagg cttctggtta cacctttacc acctatggta tcacctgggt gcgacagggc 120
   cctggacaag ggcttgagtg gatgggatgg atcagcgctt tcaatggtga cacaaacttt 180
   gcacagaacc tccagaacag agtcaccctg accacagaca catccactag cacagcctat 240
   atggaactga ggagcctgag atctgacgac acggccgttt attactgtgc gagaggggga 300
   ggagctcgtc cggggaactt cttcttctac ggtatggacg tctggggcca ggggaccacg 360
   gtcaccgtct cctca                                                  375
<210> 573
   <211> 125
   <212> PRT
   <213> Artificial Sequence
<220>
   <223> Synthetic
<400> 573
   Glu Val Gln Leu Val Gln Ser Gly Ala Glu Val Lys Asn Pro Gly Ala
    1               5                  10                  15
   Ser Val Arg Val Ser Cys Lys Ala Ser Gly Tyr Thr Phe Thr Thr Tyr
               20                  25                  30
   Gly Ile Thr Trp Val Arg Gln Gly Pro Gly Gln Gly Leu Glu Trp Met
           35                  40                  45
   Gly Trp Ile Ser Ala Phe Asn Gly Asp Thr Asn Phe Ala Gln Asn Leu
       50                  55                  60
   Gln Asn Arg Val Thr Leu Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Gly Gly Ala Arg Pro Gly Asn Phe Phe Phe Tyr Gly Met
               100                 105                 110
   Asp Val Trp Gly Gln Gly Thr Thr Val Thr Val Ser Ser
           115                 120                 125
<210> 574
   <211> 24
   <212> DNA
   <213> Artificial Sequence
<220>
   <223> Synthetic
<400> 574
   ggttacacct ttaccaccta tggt 24
<210> 575
   <211> 8
   <212> PRT
   <213> Artificial Sequence
<220>
   <223> Synthetic
<400> 575
   Gly Tyr Thr Phe Thr Thr Tyr Gly
   1 5
<210> 576
   <211> 24
   <212> DNA
   <213> Artificial Sequence
<220>
   <223> Synthetic
<400> 576
   atcagcgctt tcaatggtga caca 24
<210> 577
   <211> 8
   <212> PRT
   <213> Artificial Sequence
<220>
   <223> Synthetic
<400> 577
   Ile Ser Ala Phe Asn Gly Asp Thr
   1 5
<210> 578
   <211> 54
   <212> DNA
   <213> Artificial Sequence
<220>
   <223> Synthetic
<400> 578
   gcgagagggg gaggagctcg tccggggaac ttcttcttct acggtatgga cgtc 54
<210> 579
   <211> 18
   <212> PRT
   <213> Artificial Sequence
<220>
   <223> Synthetic
<400> 579
   Ala Arg Gly Gly Gly Ala Arg Pro Gly Asn Phe Phe Phe Tyr Gly Met
   1 5 10 15
   Asp Val
<210> 580
   <211> 321
   <212> DNA
   <213> Artificial Sequence
<220>
   <223> Synthetic
<400> 580
   gatgttgtga tgactcagtc tccagccacc ctgtctttgt ctccagggga aagagccacc 60
   ctctcctgta gggccagtca gagttttgcc agctacttag cctggtacca acagaaacct 120
   ggccaggctc ccaggctcct catctatgat acctcctaca gggccactgg cgtcccagcc 180
   aggttcagtg gcagtgggtc tgggacagac ttcactctca acatcagcaa cctggagcct 240
   gaagattttg cagtttatta ctgtcagcaa cgtggcaact ggccgctcac tttcggcgga 300
   gggaccaagg tggaaatcaa a                                           321
<210> 581
   <211> 107
   <212> PRT
   <213> Artificial Sequence
<220>
   <223> Synthetic
<400> 581
   Asp Val Val Met Thr Gln Ser Pro Ala Thr Leu Ser Leu Ser Pro Gly
    1               5                  10                  15
   Glu Arg Ala Thr Leu Ser Cys Arg Ala Ser Gln Ser Phe Ala Ser Tyr
               20                  25                  30
   Leu Ala Trp Tyr Gln Gln Lys Pro Gly Gln Ala Pro Arg Leu Leu Ile
           35                  40                  45
   Tyr Asp Thr Ser Tyr Arg Ala Thr Gly Val Pro Ala Arg Phe Ser Gly
       50                  55                  60
   Ser Gly Ser Gly Thr Asp Phe Thr Leu Asn Ile Ser Asn Leu Glu Pro
   65                  70                  75                  80
   Glu Asp Phe Ala Val Tyr Tyr Cys Gln Gln Arg Gly Asn Trp Pro Leu
                   85                  90                  95
   Thr Phe Gly Gly Gly Thr Lys Val Glu Ile Lys
               100                 105
<210> 582
   <211> 18
   <212> DNA
   <213> Artificial Sequence
<220>
   <223> Synthetic
<400> 582
   cagagttttg ccagctac 18
<210> 583
   <211> 6
   <212> PRT
   <213> Artificial Sequence
<220>
   <223> Synthetic
<400> 583
   Gln Ser Phe Ala Ser Tyr
   1 5
<210> 584
   <211> 9
   <212> DNA
   <213> Artificial Sequence
<220>
   <223> Synthetic
<400> 584
   gatacctcc 9
<210> 585
   <211> 3
   <212> PRT
   <213> Artificial Sequence
<220>
   <223> Synthetic
<400> 585
   Asp Thr Ser
   1
<210> 586
   <211> 27
   <212> DNA
   <213> Artificial Sequence
<220>
   <223> Synthetic
<400> 586
   cagcaacgtg gcaactggcc gctcact 27
<210> 587
   <211> 9
   <212> PRT
   <213> Artificial Sequence
<220>
   <223> Synthetic
<400> 587
   Gln Gln Arg Gly Asn Trp Pro Leu Thr
   1 5
<210> 588
   <211> 357
   <212> DNA
   <213> Artificial Sequence
<220>
   <223> Synthetic
<400> 588
   caggtgcagc tggtgcagtc tggaactgag gtgaagaagc ctggggcctc agtgaaggtc 60
   tcctgcaagg cttctggtta cacctttagc tacaatggtg tcacttgggt acgacaggcc 120
   cctggacaag ggcttgagtg gatgggatgg atcagcgctt acgatggtaa cacagactat 180
   gcacagaagt tccaagacag aatcaccatg accacagaca catccacgag tacagcctac 240
   atggaactga ggagccttag atctgacgac acggccgtct attactgtgc gaggtatagt 300
   tggaacaacc actggttcga cccctggggc cagggaaccc tggtcaccgt ctcctca    357
<210> 589
   <211> 119
   <212> PRT
   <213> Artificial Sequence
<220>
   <223> Synthetic
<400> 589
   Gln Val Gln Leu Val Gln Ser Gly Thr Glu Val Lys Lys Pro Gly Ala
    1               5                  10                  15
   Ser Val Lys Val Ser Cys Lys Ala Ser Gly Tyr Thr Phe Ser Tyr Asn
               20                  25                  30
   Gly Val Thr Trp Val Arg Gln Ala Pro Gly Gln Gly Leu Glu Trp Met
           35                  40                  45
   Gly Trp Ile Ser Ala Tyr Asp Gly Asn Thr Asp Tyr Ala Gln Lys Phe
       50                  55                  60
   Gln Asp Arg Ile Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Tyr Ser Trp Asn Asn His Trp Phe Asp Pro Trp Gly Gln Gly
               100                 105                 110
   Thr Leu Val Thr Val Ser Ser
           115
<210> 590
   <211> 24
   <212> DNA
   <213> Artificial Sequence
<220>
   <223> Synthetic
<400> 590
   ggttacacct ttagctacaa tggt 24
<210> 591
   <211> 8
   <212> PRT
   <213> Artificial Sequence
<220>
   <223> Synthetic
<400> 591
   Gly Tyr Thr Phe Ser Tyr Asn Gly
    1               5
<210> 592
   <211> 24
   <212> DNA
   <213> Artificial Sequence
<220>
   <223> Synthetic
<400> 592
   atcagcgctt acgatggtaa caca 24
<210> 593
   <211> 8
   <212> PRT
   <213> Artificial Sequence
<220>
   <223> Synthetic
<400> 593
   Ile Ser Ala Tyr Asp Gly Asn Thr
   1 5
<210> 594
   <211> 36
   <212> DNA
   <213> Artificial Sequence
<220>
   <223> Synthetic
<400> 594
   gcgaggtata gttggaacaa ccactggttc gacccc 36
<210> 595
   <211> 12
   <212> PRT
   <213> Artificial Sequence
<220>
   <223> Synthetic
<400> 595
   Ala Arg Tyr Ser Trp Asn Asn His Trp Phe Asp Pro
   1 5 10
<210> 596
   <211> 324
   <212> DNA
   <213> Artificial Sequence
<220>
   <223> Synthetic
<400> 596
   gatattgtga tgactcagtc tccaggcacc ctgtctttgt ctccaggaga cggggccacc 60
   ctctcctgca gggccagtca gagtgtttcc ggcagctact tagcctggta ccagcagaaa 120
   cctggccagg ctcccaggct cctcatttat ggtgcatcca acagggccac tggcatccca 180
   gacaggttca ctggcagtgg gtctgggaca gacttcactc tcaccatcag cagactggag 240
   cctgaagatt ttgcagtgta tttctgtcag cagagtgctt tctcaccgtg gacgttcggc 300
   caagggacca agctggagat caaa                                        324

<210> 597
   <211> 108
   <212> PRT
   <213> Artificial Sequence
<220>
   <223> Synthetic
<400> 597
   Asp Ile Val Met Thr Gln Ser Pro Gly Thr Leu Ser Leu Ser Pro Gly
    1               5                  10                  15
   Asp Gly Ala Thr Leu Ser Cys Arg Ala Ser Gln Ser Val Ser Gly Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Asn Arg Ala Thr Gly Ile Pro Asp Arg Phe Thr
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Phe Cys Gln Gln Ser Ala Phe Ser Pro
                   85                  90                  95
   Trp Thr Phe Gly Gln Gly Thr Lys Leu Glu Ile Lys
               100                 105
<210> 598
   <211> 21
   <212> DNA
   <213> Artificial Sequence
<220>
   <223> Synthetic
<400> 598
   cagagtgttt ccggcagcta c 21
<210> 599
   <211> 7
   <212> PRT
   <213> Artificial Sequence
<220>
   <223> Synthetic
<400> 599
   Gln Ser Val Ser Gly Ser Tyr
    1               5
<210> 600
   <211> 9
   <212> DNA
   <213> Artificial Sequence
<220>
   <223> Synthetic
<400> 600
   ggtgcatcc 9
<210> 601
   <211> 3
   <212> PRT
   <213> Artificial Sequence
<220>
   <223> Synthetic
<400> 601
   Gly Ala Ser
   1
<210> 602
   <211> 27
   <212> DNA
   <213> Artificial Sequence
<220>
   <223> Synthetic
<400> 602
   cagcagagtg ctttctcacc gtggacg 27
<210> 603
   <211> 9
   <212> PRT
   <213> Artificial Sequence
<220>
   <223> Synthetic
<400> 603
   Gln Gln Ser Ala Phe Ser Pro Trp Thr
   1 5
<210> 604
   <211> 351
   <212> DNA
   <213> Artificial Sequence
<220>
   <223> Synthetic
<400> 604
   gaagtgcagc tggtgcagtc tgggggaggc ttggtacagc ctggggggtc cctgagactc 60
   tcctgtgcag cctctggatt caccttcagt atgtacgaca tgcactgggt ccgccaaact 120
   ataggaaaag gtctggagtg ggtctcagca attggtactg ctggtgacac atactatcca 180
   ggctccgtga agggccgatt caccatctcc agagaaaatg ccaagaactc cttgtttctt 240
   caaatgaaca gcctgagagc cggggacacg gctgtttatt actgtgtaag atccgggact 300
   acagagtggt tcgacccctg gggccaggga accccggtca ctgtctcctc a          351
<210> 605
   <211> 117
   <212> PRT
   <213> Artificial Sequence
<220>
   <223> Synthetic
<400> 605
   Glu Val Gln Leu Val Gln Ser Gly Gly Gly Leu Val Gln Pro Gly Gly
   1               5                   10                  15
   Ser Leu Arg Leu Ser Cys Ala Ala Ser Gly Phe Thr Phe Ser Met Tyr
               20                  25                  30
   Asp Met His Trp Val Arg Gln Thr Ile Gly Lys Gly Leu Glu Trp Val
           35                  40                  45
   Ser Ala Ile Gly Thr Ala Gly Asp Thr Tyr Tyr Pro Gly Ser Val Lys
       50                  55                  60
   Gly Arg Phe Thr Ile Ser Arg Glu Asn Ala Lys Asn Ser Leu Phe Leu
   65                  70                  75                  80
   Gln Met Asn Ser Leu Arg Ala Gly Asp Thr Ala Val Tyr Tyr Cys Val
                   85                  90                  95
   Arg Ser Gly Thr Thr Glu Trp Phe Asp Pro Trp Gly Gln Gly Thr Pro
               100                 105                 110
   Val Thr Val Ser Ser
           115
<210> 606
   <211> 24
   <212> DNA
   <213> Artificial Sequence
<220>
   <223> Synthetic
<400> 606
   ggattcacct tcagtatgta cgac 24
<210> 607
   <211> 8
   <212> PRT
   <213> Artificial Sequence
<220>
   <223> Synthetic
<400> 607
   Gly Phe Thr Phe Ser Met Tyr Asp
   1               5
<210> 608
   <211> 21
   <212> DNA
   <213> Artificial Sequence
<220>
   <223> Synthetic
<400> 608
   attggtactg ctggtgacac a 21
<210> 609
   <211> 7
   <212> PRT
   <213> Artificial Sequence
<220>
   <223> Synthetic
<400> 609
   Ile Gly Thr Ala Gly Asp Thr
   1 5
<210> 610
   <211> 33
   <212> DNA
   <213> Artificial Sequence
<220>
   <223> Synthetic
<400> 610
   gtaagatccg ggactacaga gtggttcgac ccc 33
<210> 611
   <211> 11
   <212> PRT
   <213> Artificial Sequence
<220>
   <223> Synthetic
<400> 611
   Val Arg Ser Gly Thr Thr Glu Trp Phe Asp Pro
   1 5 10
<210> 612
   <211> 321
   <212> DNA
   <213> Artificial Sequence
<220>
   <223> Synthetic
<400> 612
   gacatccggt tgacccagtc tccatcttcc gtgtctgcat ctgtgggaga cagagtcacc 60
   atcacttgtc ggacgagtca gggtattagt agctggttag cctggtatca gcagaaacca 120
   ggaaaagccc ctaacctcct gatctatgct gcatccagtt tacaaagtgg ggtcccatca 180
   aggttcagcg gcagtggatc tgggacagat ttcactctca ccatcagcag cctgcagcct 240
   gaagattttg caacttacta ttgtctacag gctaacagtt tcccgtacac ttttggccag 300
   gggaccaagg tggagatcaa a                                           321
<210> 613
   <211> 107
   <212> PRT
   <213> Artificial Sequence
<220>
   <223> Synthetic
<400> 613
   Asp Ile Arg Leu Thr Gln Ser Pro Ser Ser Val Ser Ala Ser Val Gly
    1               5                  10                  15
   Asp Arg Val Thr Ile Thr Cys Arg Thr Ser Gln Gly Ile Ser Ser Trp
               20                  25                  30
   Leu Ala Trp Tyr Gln Gln Lys Pro Gly Lys Ala Pro Asn Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Ala Asn Ser Phe Pro Tyr
                   85                  90                  95
   Thr Phe Gly Gln Gly Thr Lys Val Glu Ile Lys
               100                 105
<210> 614
   <211> 18
   <212> DNA
   <213> Artificial Sequence
<220>
   <223> Synthetic
<400> 614
   cagggtatta gtagctgg 18
<210> 615
   <211> 6
   <212> PRT
   <213> Artificial Sequence
<220>
   <223> Synthetic
<400> 615
   Gln Gly Ile Ser Ser Trp
    1               5
<210> 616
   <211> 9
   <212> DNA
   <213> Artificial Sequence
<220>
   <223> Synthetic
<400> 616
   gctgcatcc 9
<210> 617
   <211> 3
   <212> PRT
   <213> Artificial Sequence
<220>
   <223> Synthetic
<400> 617
   Ala Ala Ser
   1
<210> 618
   <211> 27
   <212> DNA
   <213> Artificial Sequence
<220>
   <223> Synthetic
<400> 618
   ctacaggcta acagtttccc gtacact 27
<210> 619
   <211> 9
   <212> PRT
   <213> Artificial Sequence
<220>
   <223> Synthetic
<400> 619
   Leu Gln Ala Asn Ser Phe Pro Tyr Thr
   1 5
<210> 620
   <211> 369
   <212> DNA
   <213> Artificial Sequence
<220>
   <223> Synthetic
<400> 620
   caggtccagc tggtgcagtc tgggggaggc gtggtccagc ctgggaggtc cctgagactc 60
   tcctgtgcag cgtctggatt caccttcagt agttatggca tgcactgggt ccgccaggct 120
   ccaggcaagg ggctggagtg ggtggcagtt ttatggtatg atggaagtaa taaaaactat 180
   gtagactccg tgaagggccg attcaccatc tcaagagaca attccaagaa cacgctttat 240
   ctgcaaatga acagcctgag agccgaggac acggctgtgt attattgtgc gagagatcat 300
   gattttagga gtggttatga ggggtggttc gacccctggg gccagggaac cctggtcacc 360
   gtctcctca 369
<210> 621
   <211> 123
   <212> PRT
   <213> Artificial Sequence
<220>
   <223> Synthetic
<400> 621
   Gln Val Gln Leu Val Gln Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Leu Trp Tyr Asp Gly Ser Asn Lys Asn Tyr Val Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp His Asp Phe Arg Ser Gly Tyr Glu Gly Trp Phe Asp Pro
   100 105 110
   Trp Gly Gln Gly Thr Leu Val Thr Val Ser Ser
   115 120
<210> 622
   <211> 24
   <212> DNA
   <213> Artificial Sequence
<220>
   <223> Synthetic
<400> 622
   ggattcacct tcagtagtta tggc 24
<210> 623
   <211> 8
   <212> PRT
   <213> Artificial Sequence
<220>
   <223> Synthetic
<400> 623
   Gly Phe Thr Phe Ser Ser Tyr Gly
   1 5
<210> 624
   <211> 24
   <212> DNA
   <213> Artificial Sequence
<220>
   <223> Synthetic
<400> 624
   ttatggtatg atggaagtaa taaa 24
<210> 625
   <211> 8
<212> PRT
   <213> Artificial Sequence
<220>
   <223> Synthetic
<400> 625
   Leu Trp Tyr Asp Gly Ser Asn Lys
   1 5
<210> 626
   <211> 48
   <212> DNA
   <213> Artificial Sequence
<220>
   <223> Synthetic
<400> 626
   gcgagagatc atgattttag gagtggttat gaggggtggt tcgacccc 48
<210> 627
   <211> 16
   <212> PRT
   <213> Artificial Sequence
<220>
   <223> Synthetic
<400> 627
   Ala Arg Asp His Asp Phe Arg Ser Gly Tyr Glu Gly Trp Phe Asp Pro
   1 5 10 15
<210> 628
   <211> 321
   <212> DNA
   <213> Artificial Sequence
<220>
   <223> Synthetic
<400> 628
   gaaattgtgc tgacgcagtc tccagccacc ctgtctttgt ctccagggga aagagccacc 60
   ctctcctgca gggccagtca gagtgttcgc agctacttag cctggtacca acagaaacct 120
   ggccaggctc ccaggctcct catctatgat gcatccaaca gggccactgg catcccagcc 180
   aggttcagtg gcagtgggtc tgggacagac ttcactctca ccatcagcag cctagagcct 240
   gaagattttg cagtttatta ctgtcaacac cgtagcaact ggcctcccac tttcggcgga 300
   gggaccaagg tggaaatcaa a                                           321
<210> 629
   <211> 107
   <212> PRT
   <213> Artificial Sequence
<220>
   <223> Synthetic
<400> 629
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Arg Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln His Arg Ser Asn Trp Pro Pro
                   85                  90                  95
   Thr Phe Gly Gly Gly Thr Lys Val Glu Ile Lys
               100                 105

<210> 630
   <211> 18
   <212> DNA
   <213> Artificial Sequence
<220>
   <223> Synthetic
<400> 630
   cagagtgttc gcagctac 18
<210> 631
   <211> 6
   <212> PRT
   <213> Artificial Sequence
<220>
   <223> Synthetic
<400> 631
   Gln Ser Val Arg Ser Tyr
   1 5
<210> 632
   <211> 9
   <212> DNA
   <213> Artificial Sequence
<220>
   <223> Synthetic
<400> 632
   gatgcatcc 9
<210> 633
   <211> 3
   <212> PRT
   <213> Artificial Sequence
<220>
   <223> Synthetic
<400> 633
   Asp Ala Ser
   1
<210> 634
   <211> 27
   <212> DNA
   <213> Artificial Sequence
<220>
   <223> Synthetic
<400> 634
   caacaccgta gcaactggcc tcccact 27
<210> 635
   <211> 9
   <212> PRT
   <213> Artificial Sequence
<220>
   <223> Synthetic
<400> 635
   Gln His Arg Ser Asn Trp Pro Pro Thr
   1 5
<210> 636
   <211> 354
   <212> DNA
   <213> Artificial Sequence
<220>
   <223> Synthetic
<400> 636
   caggtcacct tgaaggagtc gggcccagga ctggtgaagt cttcggagac cctgtccctc 60
   acttgcactg tctctactgg ctccatcagc agtagtagtt actactgggc ctggatccgc 120
   cagcccccag ggaagggact ggagtggatt gggagtatct attatagtgg gagtaaattc 180
   tacagcccgt ccctcaagag tcgagtcacc atatacgttg acacgtccaa gaatcagttc 240
   tccctgcaac tgagctcggt gaccgccgca gacacggctg tatattactg tgcgagacag 300
   gtcggtgcaa tctttgacta ctggggccag ggaaccctgg tcaccgtctc ctca       354
<210> 637
   <211> 118
   <212> PRT
   <213> Artificial Sequence
<220>
   <223> Synthetic
<400> 637
   Gln Val Thr Leu Lys Glu Ser Gly Pro Gly Leu Val Lys Ser Ser Glu
   1               5                   10                  15
   Thr Leu Ser Leu Thr Cys Thr Val Ser Thr Gly Ser Ile Ser Ser Ser
               20                  25                  30
   Ser Tyr Tyr Trp Ala Trp Ile Arg Gln Pro Pro Gly Lys Gly Leu Glu
           35                  40                  45
   Trp Ile Gly Ser Ile Tyr Tyr Ser Gly Ser Lys Phe Tyr Ser Pro Ser
       50                  55                  60
   Leu Lys Ser Arg Val Thr Ile Tyr Val Asp Thr Ser Lys Asn Gln Phe
   65                  70                  75                  80
   Ser Leu Gln Leu Ser Ser Val Thr Ala Ala Asp Thr Ala Val Tyr Tyr
                   85                  90                  95
   Cys Ala Arg Gln Val Gly Ala Ile Phe Asp Tyr Trp Gly Gln Gly Thr
               100                 105                 110
   Leu Val Thr Val Ser Ser
           115
<210> 638
   <211> 30
   <212> DNA
   <213> Artificial Sequence
<220>
   <223> Synthetic
<400> 638
   actggctcca tcagcagtag tagttactac 30
<210> 639
   <211> 10
   <212> PRT
   <213> Artificial Sequence
<220>
   <223> Synthetic
<400> 639
   Thr Gly Ser Ile Ser Ser Ser Ser Tyr Tyr
   1               5                   10
<210> 640
   <211> 21
   <212> DNA
   <213> Artificial Sequence
<220>
   <223> Synthetic
<400> 640
   atctattata gtgggagtaa a 21
<210> 641
   <211> 7
   <212> PRT
   <213> Artificial Sequence
<220>
   <223> Synthetic
<400> 641
   Ile Tyr Tyr Ser Gly Ser Lys
   1 5
<210> 642
   <211> 30
   <212> DNA
   <213> Artificial Sequence
<220>
   <223> Synthetic
<400> 642
   gcgagacagg tcggtgcaat ctttgactac 30
<210> 643
   <211> 10
   <212> PRT
   <213> Artificial Sequence
<220>
   <223> Synthetic
<400> 643
   Ala Arg Gln Val Gly Ala Ile Phe Asp Tyr
   1 5 10
<210> 644
   <211> 321
   <212> DNA
   <213> Artificial Sequence
<220>
   <223> Synthetic
<400> 644
   gccatccggt tgacccagtc tccttccacc ctgtctgcat ctgtaggaga cagagtcacc 60
   atctcttgcc gggccagtca gagtattagt agttggttgg cctggtatca gcacaaacca 120
   gggaaagccc ctaaactcct gctctataag gcgtctagtt tagaaagtgg ggtcccatca 180
   aggttcagcg gcagtggatc tgggacagag ttcactctca ccatcagcag cctgcagcct 240
   gatgattttg caacttatta ctgccaacag tataatagtt attctcggac gttcggccaa 300
   gggaccaagg tggagatcaa a                                           321
<210> 645
   <211> 107
   <212> PRT
   <213> Artificial Sequence
<220>
   <223> Synthetic
<400> 645
   Ala Ile Arg Leu Thr Gln Ser Pro Ser Thr Leu Ser Ala Ser Val Gly
    1               5                  10                  15
   Asp Arg Val Thr Ile Ser Cys Arg Ala Ser Gln Ser Ile Ser Ser Trp
               20                  25                  30
   Leu Ala Trp Tyr Gln His Lys Pro Gly Lys Ala Pro Lys Leu Leu Leu
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Ser Tyr Ser Arg
                   85                  90                  95
   Thr Phe Gly Gln Gly Thr Lys Val Glu Ile Lys
               100                 105
<210> 646
   <211> 18
   <212> DNA
   <213> Artificial Sequence
<220>
   <223> Synthetic
<400> 646
   cagagtatta gtagttgg 18
<210> 647
   <211> 6
   <212> PRT
   <213> Artificial Sequence
<220>
   <223> Synthetic
<400> 647
   Gln Ser Ile Ser Ser Trp
   1 5
<210> 648
   <211> 9
   <212> DNA
   <213> Artificial Sequence
<220>
   <223> Synthetic
<400> 648
   aaggcgtct 9
<210> 649
   <211> 3
   <212> PRT
   <213> Artificial Sequence
<220>
   <223> Synthetic
<400> 649
   Lys Ala Ser
   1
<210> 650
   <211> 27
   <212> DNA
   <213> Artificial Sequence
<220>
   <223> Synthetic
<400> 650
   caacagtata atagttattc tcggacg 27
<210> 651
   <211> 9
   <212> PRT
   <213> Artificial Sequence
<220>
   <223> Synthetic
<400> 651
   Gln Gln Tyr Asn Ser Tyr Ser Arg Thr
   1 5
<210> 652
   <211> 372
   <212> DNA
   <213> Artificial Sequence
<220>
   <223> Synthetic
<400> 652
   gaggtgcagc tggtgcagtc tggagctgag gtgaagaagc ctggggcctc agtgaaggtc 60
   tcctgcaagg cctctggtta cacctttaac atctatggta tcagctgggt acgacaggcc 120
   cctggacaag ggcttgagtg gatgggatgg atcagcgctt acaatggtaa cacaaactat 180
   gcacagaaac tccagggcag agtcaccatg accacagaaa catccacgac cacagcctac 240
   atggagttga ggagcctgag atctgacgac acggccgtgt attactgtgc gagagattct 300
   gattggggaa ctccctacca ctactacggt atggacgtct ggggccaagg gaccacggtc 360
   accgtctcct ca                                                     372
<210> 653
   <211> 124
   <212> PRT
   <213> Artificial Sequence
<220>
   <223> Synthetic
<400> 653
   Glu Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Asn Ile Tyr
               20                  25                  30
   Gly Ile Ser Trp Val Arg Gln Ala Pro Gly Gln Gly Leu Glu Trp Met
           35                  40                  45
   Gly Trp Ile Ser Ala Tyr Asn Gly Asn Thr Asn Tyr Ala Gln Lys Leu
       50                  55                  60
   Gln Gly Arg Val Thr Met Thr Thr Glu Thr Ser Thr Thr Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Ser Asp Trp Gly Thr Pro Tyr His Tyr Tyr Gly Met Asp
               100                 105                 110
   Val Trp Gly Gln Gly Thr Thr Val Thr Val Ser Ser
           115                 120
<210> 654
   <211> 24
   <212> DNA
   <213> Artificial Sequence
<220>
   <223> Synthetic
<400> 654
   ggttacacct ttaacatcta tggt 24
<210> 655
   <211> 8
   <212> PRT
   <213> Artificial Sequence
<220>
   <223> Synthetic
<400> 655
   Gly Tyr Thr Phe Asn Ile Tyr Gly
   1 5
<210> 656
   <211> 24
   <212> DNA
   <213> Artificial Sequence
<220>
   <223> Synthetic
<400> 656
   atcagcgctt acaatggtaa caca 24
<210> 657
   <211> 8
   <212> PRT
   <213> Artificial Sequence
<220>
   <223> Synthetic
<400> 657
   Ile Ser Ala Tyr Asn Gly Asn Thr
   1 5
<210> 658
   <211> 51
   <212> DNA
   <213> Artificial Sequence
<220>
   <223> Synthetic
<400> 658
   gcgagagatt ctgattgggg aactccctac cactactacg gtatggacgt c 51
<210> 659
   <211> 17
   <212> PRT
   <213> Artificial Sequence
<220>
   <223> Synthetic
<400> 659
   Ala Arg Asp Ser Asp Trp Gly Thr Pro Tyr His Tyr Tyr Gly Met Asp
   1 5 10 15
   Val
<210> 660
   <211> 339
   <212> DNA
   <213> Artificial Sequence
<220>
   <223> Synthetic
<400> 660
   gatattgtga tgacccagtc tccagactcc ctggctgtgt ctctgggcga gagggccacc 60
   atcaactgca agtccagcca gaatatttta tacacctcca acaataagaa ctacttagct 120
   tggtaccagc agaaaccagg acagcctcct aagctgctca tttactgggc atttacccgg 180
   aaatccgggg tccctgaccg attcagtggc agcgggtctg ggacagattt cactctcacc 240
   atcagcagcc tgcaggctga agatgtggca gtttattact gtcagcaata ttataatact 300
   cctcggacgt tcggccaagg gaccaaagtg gatatcaaa                        339
<210> 661
   <211> 113
   <212> PRT
   <213> Artificial Sequence
<220>
   <223> Synthetic
<400> 661
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Asn Ile Leu Tyr Thr
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Phe Thr Arg Lys Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Asn Thr Pro Arg Thr Phe Gly Gln Gly Thr Lys Val Asp Ile
               100                 105                 110
   Lys
<210> 662
   <211> 36
   <212> DNA
   <213> Artificial Sequence
<220>
   <223> Synthetic
<400> 662
   cagaatattt tatacacctc caacaataag aactac 36
<210> 663
   <211> 12
   <212> PRT
   <213> Artificial Sequence
<220>
   <223> Synthetic
<400> 663
   Gln Asn Ile Leu Tyr Thr Ser Asn Asn Lys Asn Tyr
   1 5 10
<210> 664
   <211> 9
   <212> DNA
   <213> Artificial Sequence
<220>
   <223> Synthetic
<400> 664
   tgggcattt 9
<210> 665
   <211> 3
   <212> PRT
   <213> Artificial Sequence
<220>
   <223> Synthetic
<400> 665 <210> 666
   <211> 27
   <212> DNA
   <213> Artificial Sequence
<220>
   <223> Synthetic
<400> 666
   cagcaatatt ataatactcc tcggacg 27
<210> 667
   <211> 9
   <212> PRT
   <213> Artificial Sequence
<220>
   <223> Synthetic
<400> 667
   Gln Gln Tyr Tyr Asn Thr Pro Arg Thr
   1 5
<210> 668
   <211> 357
   <212> DNA
   <213> Artificial Sequence
<220>
   <223> Synthetic
<400> 668
   cagatcacct tgaaggagtc gggcccagga ctggtgaagc cttcacagac cctgtccctc 60
   acctgcactg tctctgatgg ctccatcaac agtggtggtt cctactggag ctggatccgc 120
   cagcacccgg ggaagggcct ggagtggatt gggtacatca aatacagtgg gggcgtccac 180
   tataacccgt ccctcaagag tcgaatcacc atatcagtgg acacgtctaa gaaccatttc 240
   tccctgaaaa tgacctctgt gactgccgcg gacacggccg tgtatttctg tgcgagagca 300
   cctggaagtc acacttttga tatctggggc caggggacaa tggtcaccgt ctcttca    357
<210> 669
   <211> 119
   <212> PRT
   <213> Artificial Sequence
<220>
   <223> Synthetic
<400> 669
   Gln Ile Thr Leu Lys Glu Ser Gly Pro Gly Leu Val Lys Pro Ser Gln
    1               5                  10                  15
   Thr Leu Ser Leu Thr Cys Thr Val Ser Asp Gly Ser Ile Asn Ser Gly
               20                  25                  30
   Gly Ser Tyr Trp Ser Trp Ile Arg Gln His Pro Gly Lys Gly Leu Glu
           35                  40                  45
   Trp Ile Gly Tyr Ile Lys Tyr Ser Gly Gly Val His Tyr Asn Pro Ser
       50                  55                  60
   Leu Lys Ser Arg Ile Thr Ile Ser Val Asp Thr Ser Lys Asn His Phe
   65                  70                  75                  80
   Ser Leu Lys Met Thr Ser Val Thr Ala Ala Asp Thr Ala Val Tyr Phe
                   85                  90                  95
   Cys Ala Arg Ala Pro Gly Ser His Thr Phe Asp Ile Trp Gly Gln Gly
               100                 105                 110
   Thr Met Val Thr Val Ser Ser
           115
<210> 670
   <211> 30
   <212> DNA
   <213> Artificial Sequence
<220>
   <223> Synthetic
<400> 670
   gatggctcca tcaacagtgg tggttcctac 30
<210> 671
   <211> 10
   <212> PRT
   <213> Artificial Sequence
<220>
   <223> Synthetic
<400> 671
   Asp Gly Ser Ile Asn Ser Gly Gly Ser Tyr
   1 5 10
<210> 672
   <211> 21
   <212> DNA
   <213> Artificial Sequence
<220>
   <223> Synthetic
<400> 672
   atcaaataca gtgggggcgt c 21
<210> 673
   <211> 7
   <212> PRT
   <213> Artificial Sequence
<220>
   <223> Synthetic
<400> 673
   Ile Lys Tyr Ser Gly Gly Val
   1 5
<210> 674
   <211> 33
   <212> DNA
   <213> Artificial Sequence
<220>
   <223> Synthetic
<400> 674
   gcgagagcac ctggaagtca cacttttgat atc 33
<210> 675
   <211> 11
   <212> PRT
   <213> Artificial Sequence
<220>
   <223> Synthetic
<400> 675
   Ala Arg Ala Pro Gly Ser His Thr Phe Asp Ile
   1 5 10
<210> 676
   <211> 324
   <212> DNA
   <213> Artificial Sequence
<220>
   <223> Synthetic
<400> 676
   gatattgtga tgactcagtc tccaggcacc ctgtctttgt ctccagggga aagagccacc 60
   ctctcctgca gggccagtca gagtgttagc aacaactact tagcctggta ccagcagaaa 120
   cctggccagg ctcccagact cctcatctat ggtacatcca atagggtcag tggcatccca 180
   gacaggttca gtggcagtgg gtctgggaca gacttcactc tcaccatcag cagactggag 240
   cctgaagatt ttgaactata ttattgtcag cagtatagta ggtcaccgat caccttcggc 300
   caagggacac gactggagat taaa                                        324
<210> 677
   <211> 108
   <212> PRT
   <213> Artificial Sequence
<220>
   <223> Synthetic
<400> 677
   Asp Ile Val Met Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Asn Asn
               20                  25                  30
   Tyr Leu Ala Trp Tyr Gln Gln Lys Pro Gly Gln Ala Pro Arg Leu Leu
           35                  40                  45
   Ile Tyr Gly Thr Ser Asn Arg Val Ser Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Glu Leu Tyr Tyr Cys Gln Gln Tyr Ser Arg Ser Pro
                   85                  90                  95
   Ile Thr Phe Gly Gln Gly Thr Arg Leu Glu Ile Lys
               100                 105
<210> 678
   <211> 21
   <212> DNA
   <213> Artificial Sequence
<220>
   <223> Synthetic
<400> 678
   cagagtgtta gcaacaacta c 21
<210> 679
   <211> 7
   <212> PRT
   <213> Artificial Sequence
<220>
   <223> Synthetic
<400> 679
   Gln Ser Val Ser Asn Asn Tyr
   1 5
<210> 680
   <211> 9
   <212> DNA
   <213> Artificial Sequence
<220>
   <223> Synthetic
<400> 680
   ggtacatcc 9
<210> 681
   <211> 3
   <212> PRT
   <213> Artificial Sequence
<220>
   <223> Synthetic
<400> 681
   Gly Thr Ser
   1
<210> 682
   <211> 27
   <212> DNA
   <213> Artificial Sequence
<220>
   <223> Synthetic
<400> 682
   cagcagtata gtaggtcacc gatcacc 27
<210> 683
   <211> 9
   <212> PRT
   <213> Artificial Sequence
<220>
   <223> Synthetic
<400> 683
   Gln Gln Tyr Ser Arg Ser Pro Ile Thr
   1 5
<210> 684
   <211> 360
   <212> DNA
   <213> Artificial Sequence
<220>
   <223> Synthetic
<400> 684
   gaggtgcagc tggtgcagtc tgggggaggc ttggtacaac ctggggggtc cctgagactc 60
   tcctgtgcag cctctggatt cacctttaac aactttgcca tgacctgggt ccgccaggct 120
   ccagggaagg gcctggagtg ggtctcaact attagtggta gtggcgttga cacatactgc 180
   gcagactccg tgaagggccg gttcaccatc tccagagaca attccaagaa cacactgtat 240
   ctgcaaatga acagcctgag agccgaggac acggccgtat attactgtgc gaaagatggc 300
   gccttctata gtggctacga acactactgg ggccagggaa caatggtcac cgtctcctca 360
<210> 685
   <211> 120
   <212> PRT
   <213> Artificial Sequence
<220>
   <223> Synthetic
<400> 685
   Glu Val Gln Leu Val Gln Ser Gly Gly Gly Leu Val Gln Pro Gly Gly
   1               5                   10                  15
   Ser Leu Arg Leu Ser Cys Ala Ala Ser Gly Phe Thr Phe Asn Asn Phe
               20                  25                  30
   Ala Met Thr Trp Val Arg Gln Ala Pro Gly Lys Gly Leu Glu Trp Val
           35                  40                  45
   Ser Thr Ile Ser Gly Ser Gly Val Asp Thr Tyr Cys Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asp Gly Ala Phe Tyr Ser Gly Tyr Glu His Tyr Trp Gly Gln
               100                 105                 110
   Gly Thr Met Val Thr Val Ser Ser
           115                 120
<210> 686
   <211> 24
   <212> DNA
   <213> Artificial Sequence
<220>
   <223> Synthetic
<400> 686
   ggattcacct ttaacaactt tgcc 24
<210> 687
   <211> 8
   <212> PRT
   <213> Artificial Sequence
<220>
   <223> Synthetic
<400> 687
   Gly Phe Thr Phe Asn Asn Phe Ala
   1 5
<210> 688
   <211> 24
   <212> DNA
   <213> Artificial Sequence
<220>
   <223> Synthetic
<400> 688
   attagtggta gtggcgttga caca 24
<210> 689
   <211> 8
   <212> PRT
   <213> Artificial Sequence
<220>
   <223> Synthetic
<400> 689
   Ile Ser Gly Ser Gly Val Asp Thr
   1 5
<210> 690
   <211> 39
   <212> DNA
   <213> Artificial Sequence
<220>
   <223> Synthetic
<400> 690
   gcgaaagatg gcgccttcta tagtggctac gaacactac 39
<210> 691
   <211> 13
   <212> PRT
   <213> Artificial Sequence
<220>
   <223> Synthetic
<400> 691
   Ala Lys Asp Gly Ala Phe Tyr Ser Gly Tyr Glu His Tyr
   1 5 10
<210> 692
   <211> 324
   <212> DNA
   <213> Artificial Sequence
<220>
   <223> Synthetic
<400> 692
   gaaacgacac tcacgcagtc tccaggcacc ctgtctttgt ctccagggga aagagccacc 60
   ctctcctgca gggccagtca gagtgttagc agcagctact tagcctggta ccagcagaaa 120
   cctggccagg ctcccaggct cctcatctat ggtacatcca acagggcctc tggcatccca 180
   gacaggctca ttggcagtgg gtctgggaca gacttcactc tcaccatcag cagactggag 240
   tctgaagatt ttgcagtgta ttactgtcag cagtatggta gctcacctcg gacgttcggc 300
   caagggacca aagtggatat caaa                                        324
<210> 693
   <211> 108
   <212> PRT
   <213> Artificial Sequence
<220>
   <223> Synthetic
<400> 693
   Glu Thr Thr Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Thr Ser Asn Arg Ala Ser Gly Ile Pro Asp Arg Leu Ile
       50                  55                  60
   Gly Ser Gly Ser Gly Thr Asp Phe Thr Leu Thr Ile Ser Arg Leu Glu
   65                  70                  75                  80
   Ser Glu Asp Phe Ala Val Tyr Tyr Cys Gln Gln Tyr Gly Ser Ser Pro
                   85                  90                  95
   Arg Thr Phe Gly Gln Gly Thr Lys Val Asp Ile Lys
               100                 105
<210> 694
   <211> 21
   <212> DNA
   <213> Artificial Sequence
<220>
   <223> Synthetic
<400> 694
   cagagtgtta gcagcagcta c 21
<210> 695
   <211> 7
   <212> PRT
   <213> Artificial Sequence
<220>
   <223> Synthetic
<400> 695
   Gln Ser Val Ser Ser Ser Tyr
   1 5
<210> 696
   <211> 9
   <212> DNA
   <213> Artificial Sequence
<220>
   <223> Synthetic
<400> 696
   ggtacatcc 9
<210> 697
   <211> 3
   <212> PRT
   <213> Artificial Sequence
<220>
   <223> Synthetic
<400> 697
   Gly Thr Ser
   1
<210> 698
   <211> 27
   <212> DNA
   <213> Artificial Sequence
<220>
   <223> Synthetic
<400> 698
   cagcagtatg gtagctcacc tcggacg 27
<210> 699
   <211> 9
   <212> PRT
   <213> Artificial Sequence
<220>
   <223> Synthetic
<400> 699
   Gln Gln Tyr Gly Ser Ser Pro Arg Thr
   1 5
<210> 700
   <211> 363
   <212> DNA
   <213> Artificial Sequence
<220>
   <223> Synthetic
<400> 700
   gaggtgcagc tggtgcagtc tgggggaggc gtggtccagc ctgggaggtc cctgagactc 60
   tcctgtgcag cgtctggatt caccttcagt agctatggca tgcactgggt ccgccaggct 120
   ccaggcaagg ggctggagtg ggtggcagtt atatggtatg atggaagtaa taaatactat 180
   gcagactccg tgaagggccg attcaccatc tccagagaca attccaagaa catgctgtat 240
   ctgcaaatga acagcctgag agccgaggac acggctgtgt attactgtgc ggcttacgat 300
   attttgattg gttattcccc ggttgactac tggggccagg gaaccctggt caccgtctcc 360
   tca 363
<210> 701
   <211> 121
   <212> PRT
   <213> Artificial Sequence
<220>
   <223> Synthetic
<400> 701
   Glu Val Gln Leu Val Gln Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Trp Tyr Asp Gly Ser Asn Lys Tyr Tyr Ala Asp Ser Val
   50 55 60
   Lys Gly Arg Phe Thr Ile Ser Arg Asp Asn Ser Lys Asn Met Leu Tyr
   65 70 75 80
   Leu Gln Met Asn Ser Leu Arg Ala Glu Asp Thr Ala Val Tyr Tyr Cys
   85 90 95
   Ala Ala Tyr Asp Ile Leu Ile Gly Tyr Ser Pro Val Asp Tyr Trp Gly
   100 105 110
   Gln Gly Thr Leu Val Thr Val Ser Ser
   115 120
<210> 702
   <211> 24
   <212> DNA
   <213> Artificial Sequence
<220>
   <223> Synthetic
<400> 702
   ggattcacct tcagtagcta tggc 24
<210> 703
   <211> 8
   <212> PRT
   <213> Artificial Sequence
<220>
   <223> Synthetic
<400> 703
   Gly Phe Thr Phe Ser Ser Tyr Gly
   1 5
<210> 704
   <211> 24
   <212> DNA
   <213> Artificial Sequence
<220>
   <223> Synthetic
<400> 704
   atatggtatg atggaagtaa taaa 24
<210> 705
   <211> 8
   <212> PRT
   <213> Artificial Sequence
<220>
   <223> Synthetic
<400> 705
   Ile Trp Tyr Asp Gly Ser Asn Lys
   1 5
<210> 706
   <211> 42
   <212> DNA
   <213> Artificial Sequence
<220>
   <223> Synthetic
<400> 706
   gcggcttacg atattttgat tggttattcc ccggttgact ac 42
<210> 707
<211> 14
   <212> PRT
   <213> Artificial Sequence
<220>
   <223> Synthetic
<400> 707
   Ala Ala Tyr Asp Ile Leu Ile Gly Tyr Ser Pro Val Asp Tyr
   1 5 10
<210> 708
   <211> 318
   <212> DNA
   <213> Artificial Sequence
<220>
   <223> Synthetic
<400> 708
   gatattgtga tgacccagtc tccagccacc ctgtctgtgt ctccagggga aagagccacc 60
   ctctcctgca gggccagtca gactgttagt agcaacttag cctggttcca gcagaaacct 120
   ggccaggctc ccagactcct catctatgat gcatccacca gggccactgg tatcccagcc 180
   aggttcagtg gcagtgggtc tgggacagaa ttcactctca ccatcagcag cctgcagtct 240
   gaagattttg cagtttatta ctgtcagcag tataataact ggtacacttt tggccagggg 300
   accaagctgg agatcaaa                                               318
<210> 709
   <211> 106
   <212> PRT
   <213> Artificial Sequence
<220>
   <223> Synthetic
<400> 709
   Asp Ile Val Met Thr Gln Ser Pro Ala Thr Leu Ser Val Ser Pro Gly
   1               5                   10                  15
   Glu Arg Ala Thr Leu Ser Cys Arg Ala Ser Gln Thr Val Ser Ser Asn
               20                  25                  30
   Leu Ala Trp Phe Gln Gln Lys Pro Gly Gln Ala Pro Arg Leu Leu Ile
           35                  40                  45
   Tyr Asp Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Tyr Thr
                   85                  90                  95
   Phe Gly Gln Gly Thr Lys Leu Glu Ile Lys
               100                 105
<210> 710
   <211> 18
   <212> DNA
   <213> Artificial Sequence
<220>
   <223> Synthetic
<400> 710
   cagactgtta gtagcaac 18
<210> 711
   <211> 6
   <212> PRT
   <213> Artificial Sequence
<220>
   <223> Synthetic
<400> 711
   Gln Thr Val Ser Ser Asn
   1 5
<210> 712
   <211> 9
   <212> DNA
   <213> Artificial Sequence
<220>
   <223> Synthetic
<400> 712
   gatgcatcc 9
<210> 713
   <211> 3
   <212> PRT
   <213> Artificial Sequence
<220>
   <223> Synthetic
<400> 713
   Asp Ala Ser
   1
<210> 714
   <211> 24
   <212> DNA
   <213> Artificial Sequence
<220>
   <223> Synthetic
<400> 714
   cagcagtata ataactggta cact 24
<210> 715
   <211> 8
   <212> PRT
   <213> Artificial Sequence
<220>
   <223> Synthetic
<400> 715
   Gln Gln Tyr Asn Asn Trp Tyr Thr
   1 5
<210> 716
   <211> 357
   <212> DNA
   <213> Artificial Sequence
<220>
   <223> Synthetic
<400> 716
   gaggtgcagc tggtgcagtc gggcccagga ctggtgaagc cttcacagac cctgtccctc 60
   acctgcactg tctctggtgg ctccattacc agtggtggtt actactggac ctggatccgc 120
   cagcacccag ggaagggcct ggaatggatt ggatacatca aatttagtgg gaacacctac 180
   tacaacccgt ccctcaggag tcgagtcacc atatcacttg acacgtctaa gaatcagttc 240
   tccctgaata tgacctctgt gactgccgcg gacacggccg tgtattattg tgcgagagca 300
   cctggaagtc ataactttga catctggggc caagggacaa tggtcaccgt ctcttca    357
<210> 717
   <211> 119
   <212> PRT
   <213> Artificial Sequence
<220>
   <223> Synthetic
<400> 717
   Glu Val Gln Leu Val Gln Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Gly Tyr Tyr Trp Thr Trp Ile Arg Gln His Pro Gly Lys Gly Leu Glu
           35                  40                  45
   Trp Ile Gly Tyr Ile Lys Phe Ser Gly Asn Thr Tyr Tyr Asn Pro Ser
       50                  55                  60
   Leu Arg Ser Arg Val Thr Ile Ser Leu Asp Thr Ser Lys Asn Gln Phe
   65                  70                  75                  80
   Ser Leu Asn Met Thr Ser Val Thr Ala Ala Asp Thr Ala Val Tyr Tyr
                   85                  90                  95
   Cys Ala Arg Ala Pro Gly Ser His Asn Phe Asp Ile Trp Gly Gln Gly
               100                 105                 110
   Thr Met Val Thr Val Ser Ser
           115
<210> 718
   <211> 30
   <212> DNA
   <213> Artificial Sequence
<220>
   <223> Synthetic
<400> 718
   ggtggctcca ttaccagtgg tggttactac 30
<210> 719
   <211> 10
   <212> PRT
   <213> Artificial Sequence
<220>
   <223> Synthetic
<400> 719
   Gly Gly Ser Ile Thr Ser Gly Gly Tyr Tyr
   1 5 10
<210> 720
   <211> 21
   <212> DNA
   <213> Artificial Sequence
<220>
   <223> Synthetic

<400> 720
   atcaaattta gtgggaacac c 21
<210> 721
   <211> 7
   <212> PRT
   <213> Artificial Sequence
<220>
   <223> Synthetic
<400> 721
   Ile Lys Phe Ser Gly Asn Thr
   1 5
<210> 722
   <211> 33
   <212> DNA
   <213> Artificial Sequence
<220>
   <223> Synthetic
<400> 722
   gcgagagcac ctggaagtca taactttgac atc 33
<210> 723
   <211> 11
   <212> PRT
   <213> Artificial Sequence
<220>
   <223> Synthetic
<400> 723
   Ala Arg Ala Pro Gly Ser His Asn Phe Asp Ile
   1 5 10
<210> 724
   <211> 324
   <212> DNA
   <213> Artificial Sequence
<220>
   <223> Synthetic
<400> 724
   gccatccggt tgacccagtc tccagacacc ctgtctttgt ctccagggga aagagccacc 60
   ctctcctgca gggccagtgt gagtattagt aataactatt tagcctggta ccagcagaaa 120
   cctggccagg ctcccaggct cctcatctat ggtgcatcca gcagggccac tggcatccca 180
   gacaggttca gtggcagtgg gtctgggaca gacttcactc tcaccatcag aagactggag 240
   tctgcagatt ttgcaccgta ttactgtcag caatatagta ggtcaccgat caccttcggc 300
   caagggacac gactggagat taaa                                        324
<210> 725
   <211> 108
   <212> PRT
   <213> Artificial Sequence
<220>
   <223> Synthetic
<400> 725
   Ala Ile Arg Leu Thr Gln Ser Pro Asp Thr Leu Ser Leu Ser Pro Gly
   1               5                   10                  15
   Glu Arg Ala Thr Leu Ser Cys Arg Ala Ser Val Ser Ile Ser Asn Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Arg Arg Leu Glu
   65                  70                  75                  80
   Ser Ala Asp Phe Ala Pro Tyr Tyr Cys Gln Gln Tyr Ser Arg Ser Pro
                   85                  90                  95
   Ile Thr Phe Gly Gln Gly Thr Arg Leu Glu Ile Lys
               100                 105
<210> 726
   <211> 21
   <212> DNA
   <213> Artificial Sequence
<220>
   <223> Synthetic
<400> 726
   gtgagtatta gtaataacta t 21
<210> 727
   <211> 7
   <212> PRT
   <213> Artificial Sequence
<220>
   <223> Synthetic
<400> 727
   Val Ser Ile Ser Asn Asn Tyr
   1               5
<210> 728
   <211> 9
   <212> DNA
   <213> Artificial Sequence
<220>
   <223> Synthetic
<400> 728
   ggtgcatcc 9
<210> 729
   <211> 3
   <212> PRT
   <213> Artificial Sequence
<220>
   <223> Synthetic
<400> 729
   Gly Ala Ser
   1
<210> 730
   <211> 27
   <212> DNA
   <213> Artificial Sequence
<220>
   <223> Synthetic
<400> 730
   cagcaatata gtaggtcacc gatcacc 27
<210> 731
   <211> 9
   <212> PRT
   <213> Artificial Sequence
<220>
   <223> Synthetic
<400> 731
   Gln Gln Tyr Ser Arg Ser Pro Ile Thr
   1 5
<210> 732
   <211> 357
   <212> DNA
   <213> Artificial Sequence
<220>
   <223> Synthetic
<400> 732
   caggtgcagc tgcaggagtc gggcccagga ctggtgaagc cttcacagac cctgtccctc 60
   acctgcactg tctctggtgg ctccatcaac agtgttactt actactggac ctggatccgc 120
   cagcacccag ggaggggcct agagtggatt gggtacatca aattcagtgg gagcacctac 180
   tacaacccgt ccctcaaggg tcgagtcacc atatcagtgg acacgtctaa gaaccaattc 240
   tcccttaaaa ttaactctgt gactgccgcg gacacggccg tgttttactg tgcgagagct 300
   tctggaagtc atacttttga tatctggggc caagggacaa tggtcaccgt ctcctca    357
<210> 733
   <211> 119
   <212> PRT
   <213> Artificial Sequence
<220>
   <223> Synthetic
<400> 733
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Asn Ser Val
               20                  25                  30
   Thr Tyr Tyr Trp Thr Trp Ile Arg Gln His Pro Gly Arg Gly Leu Glu
           35                  40                  45
   Trp Ile Gly Tyr Ile Lys Phe Ser Gly Ser Thr Tyr Tyr Asn Pro Ser
       50                  55                  60
   Leu Lys Gly Arg Val Thr Ile Ser Val Asp Thr Ser Lys Asn Gln Phe
   65                  70                  75                  80
   Ser Leu Lys Ile Asn Ser Val Thr Ala Ala Asp Thr Ala Val Phe Tyr
                   85                  90                  95
   Cys Ala Arg Ala Ser Gly Ser His Thr Phe Asp Ile Trp Gly Gln Gly
               100                 105                 110
   Thr Met Val Thr Val Ser Ser
           115
<210> 734
   <211> 30
   <212> DNA
   <213> Artificial Sequence
<220>
   <223> Synthetic
<400> 734
   ggtggctcca tcaacagtgt tacttactac 30
<210> 735
   <211> 10
   <212> PRT
   <213> Artificial Sequence
<220>
   <223> Synthetic
<400> 735
   Gly Gly Ser Ile Asn Ser Val Thr Tyr Tyr
   1 5 10
<210> 736
   <211> 21
   <212> DNA
   <213> Artificial Sequence
<220>
   <223> Synthetic
<400> 736
   atcaaattca gtgggagcac c 21
<210> 737
   <211> 7
   <212> PRT
   <213> Artificial Sequence
<220>
   <223> Synthetic
<400> 737
   Ile Lys Phe Ser Gly Ser Thr
   1 5
<210> 738
   <211> 33
   <212> DNA
   <213> Artificial Sequence
<220>
   <223> Synthetic
<400> 738
   gcgagagctt ctggaagtca tacttttgat atc 33
   <210> 739
   <211> 11
   <212> PRT
   <213> Artificial Sequence
<220>
   <223> Synthetic
<400> 739
   Ala Arg Ala Ser Gly Ser His Thr Phe Asp Ile
   1 5 10
<210> 740
   <211> 324
   <212> DNA
   <213> Artificial Sequence
<220>
   <223> Synthetic
<400> 740
   gaaacgacac tcacgcagtc tccaggcacc ctgtctttgt ctccagggga aagagccacc 60
   ctctcctgca gggccagtca gagtgttagc aacagctact tagcctggta ccagcagaaa 120
   cctggccagg ctcccaggct cctcatctct ggtgcgtcca gcagggtcac tggcatccca 180
   gacaggttca gtggcagtgg gtctgggaca gacttcactc tcaccatcag cagactggag 240
   cctgaagatt ttggaatgta ttactgtcag cagtatagta ggtcaccgat caccttcggc 300
   caagggacca agctggagat caaa                                        324
<210> 741
   <211> 108
   <212> PRT
   <213> Artificial Sequence
<220>
   <223> Synthetic
<400> 741
   Glu Thr Thr Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Asn Ser
               20                  25                  30
   Tyr Leu Ala Trp Tyr Gln Gln Lys Pro Gly Gln Ala Pro Arg Leu Leu
           35                  40                  45
   Ile Ser Gly Ala Ser Ser Arg Val Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Gly Met Tyr Tyr Cys Gln Gln Tyr Ser Arg Ser Pro
                   85                  90                  95
   Ile Thr Phe Gly Gln Gly Thr Lys Leu Glu Ile Lys
               100                 105
<210> 742
   <211> 21
   <212> DNA
   <213> Artificial Sequence
<220>
   <223> Synthetic
<400> 742
   cagagtgtta gcaacagcta c 21
<210> 743
   <211> 7
   <212> PRT
   <213> Artificial Sequence
<220>
   <223> Synthetic
<400> 743
   Gln Ser Val Ser Asn Ser Tyr
    1               5
<210> 744
   <211> 9
   <212> DNA
   <213> Artificial Sequence
<220>
   <223> Synthetic
<400> 744
   ggtgcgtcc 9
<210> 745
   <211> 3
   <212> PRT
   <213> Artificial Sequence
<220>
   <223> Synthetic
<400> 745
   Gly Ala Ser
   1
<210> 746
   <211> 27
   <212> DNA
   <213> Artificial Sequence
<220>
   <223> Synthetic
<400> 746
   cagcagtata gtaggtcacc gatcacc 27
<210> 747
   <211> 9
   <212> PRT
   <213> Artificial Sequence
<220>
   <223> Synthetic
<400> 747
   Gln Gln Tyr Ser Arg Ser Pro Ile Thr
   1 5
<210> 748
   <211> 357
   <212> DNA
   <213> Artificial Sequence
<220>
   <223> Synthetic
<400> 748
   gaggtgcagc tggtgcagtc tgggggaggc ttggtacagc ctggcaggtc cctgagactc 60
   tcctgtgcag cctctggagt caccttggat gattatgcca tgcactgggt ccggcaagct 120
   ccagggaagg gcctggagtg ggtctcaagt attagttgga atagtggtag tataggctat 180
   gcggactctg tgaagggccg cttcaccatc tccagagaca acgccaagaa ctccctgtat 240
   ctgcaaatga acagtctgag agctgaggac acggccttgt attactgtgc aaaagatggg 300
   tggaacccgt actactttga ctattggggc cagggaataa cggtcaccgt ctcctca    357
<210> 749
   <211> 119
   <212> PRT
   <213> Artificial Sequence
<220>
   <223> Synthetic
<400> 749
   Glu Val Gln Leu Val Gln Ser Gly Gly Gly Leu Val Gln Pro Gly Arg
    1               5                  10                  15
   Ser Leu Arg Leu Ser Cys Ala Ala Ser Gly Val Thr Leu Asp Asp Tyr
               20                  25                  30
   Ala Met His Trp Val Arg Gln Ala Pro Gly Lys Gly Leu Glu Trp Val
           35                  40                  45
   Ser Ser Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Asp Gly Trp Asn Pro Tyr Tyr Phe Asp Tyr Trp Gly Gln Gly
               100                 105                 110
   Ile Thr Val Thr Val Ser Ser
           115
<210> 750
   <211> 24
   <212> DNA
   <213> Artificial Sequence
<220>
   <223> Synthetic
<400> 750
   ggagtcacct tggatgatta tgcc 24
<210> 751
   <211> 8
   <212> PRT
   <213> Artificial Sequence
<220>
   <223> Synthetic
<400> 751
   Gly Val Thr Leu Asp Asp Tyr Ala
    1               5
<210> 752
   <211> 24
   <212> DNA
   <213> Artificial Sequence
<220>
   <223> Synthetic
<400> 752
   attagttgga atagtggtag tata 24
<210> 753
   <211> 8
   <212> PRT
   <213> Artificial Sequence
<220>
   <223> Synthetic
<400> 753
   Ile Ser Trp Asn Ser Gly Ser Ile
   1 5
<210> 754
   <211> 36
   <212> DNA
   <213> Artificial Sequence
<220>
   <223> Synthetic
<400> 754
   gcaaaagatg ggtggaaccc gtactacttt gactat 36
<210> 755
   <211> 12
   <212> PRT
   <213> Artificial Sequence
<220>
   <223> Synthetic
<400> 755
   Ala Lys Asp Gly Trp Asn Pro Tyr Tyr Phe Asp Tyr
   1 5 10
<210> 756
   <211> 321
   <212> DNA
   <213> Artificial Sequence
<220>
   <223> Synthetic
<400> 756
   gacatccagt tgacccagtc tccatcctcc ctgtctgcat ctgtaggaga cagagtcacc 60
   atcacttgcc gggcaagtca gggcattaga agtgatttag gctggtatca gcagaaacca 120
   gggaaagccc ctaagcgcct gatctatgct gcatccagtt tgcaaagtgg ggtcccatca 180
   aggttcagcg gcagtggatc tgggacagaa ttcactctca caatcagcag cctgcagcct 240
   gaagattttg caacttatta ctgtctacag cataatagtt accctctcac tttcggcgga 300
   gggaccaagg tggaaatcaa a                                            321
<210> 757
   <211> 107
   <212> PRT
   <213> Artificial Sequence
<220>
   <223> Synthetic
<400> 757
   Asp Ile Gln Leu Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Ser Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Leu
                   85                  90                  95
   Thr Phe Gly Gly Gly Thr Lys Val Glu Ile Lys
               100                 105
<210> 758
   <211> 18
   <212> DNA
   <213> Artificial Sequence
<220>
   <223> Synthetic
<400> 758
   cagggcatta gaagtgat 18
<210> 759
   <211> 6
   <212> PRT
   <213> Artificial Sequence
<220>
   <223> Synthetic
<400> 759
   Gln Gly Ile Arg Ser Asp
   1               5
<210> 760
   <211> 9
   <212> DNA
   <213> Artificial Sequence
<220>
   <223> Synthetic
<400> 760
   gctgcatcc 9
<210> 761
   <211> 3
   <212> PRT
   <213> Artificial Sequence
<220>
   <223> Synthetic
<400> 761
   Ala Ala Ser
   1
<210> 762
   <211> 27
   <212> DNA
   <213> Artificial Sequence
<220>
   <223> Synthetic
<400> 762
   ctacagcata atagttaccc tctcact 27
<210> 763
   <211> 9
   <212> PRT
   <213> Artificial Sequence
<220>
   <223> Synthetic
<400> 763
   Leu Gln His Asn Ser Tyr Pro Leu Thr
   1 5
<210> 764
   <211> 357
   <212> DNA
   <213> Artificial Sequence
<220>
   <223> Synthetic
<400> 764
   cagatcacct tgaaggagtc gggcccagga ctggtgaagc cttcacagac cctgtccctc 60
   acctgcgccg tctctggtga ctccttcagc agtggtaatt actactggag ctggatccgc 120
   caacacccag ggaagggcct ggagtggatt gggtacatca agtacactgg gagcacctac 180
   tacaacccgt ccctcaagag tcgagttatt atattagtag acacgtctaa gacccagttc 240
   tccctgaagc tgagctctgt gaatgccgcg gacacggccg tgtattactg tgcgagagca 300
   cctggaactc atgcttttga tgtttggggc caagggacaa tggtcaccgt ctcctca    357
<210> 765
   <211> 119
   <212> PRT
   <213> Artificial Sequence
<220>
   <223> Synthetic
<400> 765
   Gln Ile Thr Leu Lys Glu Ser Gly Pro Gly Leu Val Lys Pro Ser Gln
    1               5                  10                  15
   Thr Leu Ser Leu Thr Cys Ala Val Ser Gly Asp Ser Phe Ser Ser Gly
               20                  25                  30
   Asn Tyr Tyr Trp Ser Trp Ile Arg Gln His Pro Gly Lys Gly Leu Glu
           35                  40                  45
   Trp Ile Gly Tyr Ile Lys Tyr Thr Gly Ser Thr Tyr Tyr Asn Pro Ser
       50                  55                  60
   Leu Lys Ser Arg Val Ile Ile Leu Val Asp Thr Ser Lys Thr Gln Phe
   65                  70                  75                  80
   Ser Leu Lys Leu Ser Ser Val Asn Ala Ala Asp Thr Ala Val Tyr Tyr
                   85                  90                  95
   Cys Ala Arg Ala Pro Gly Thr His Ala Phe Asp Val Trp Gly Gln Gly
               100                 105                 110
   Thr Met Val Thr Val Ser Ser
           115
<210> 766
   <211> 30
   <212> DNA
   <213> Artificial Sequence
<220>
   <223> Synthetic
<400> 766
   ggtgactcct tcagcagtgg taattactac 30
<210> 767
   <211> 10
   <212> PRT
   <213> Artificial Sequence
<220>
   <223> Synthetic
<400> 767
   Gly Asp Ser Phe Ser Ser Gly Asn Tyr Tyr
    1               5                  10
<210> 768
   <211> 21
   <212> DNA
   <213> Artificial Sequence
<220>
   <223> Synthetic
<400> 768
   atcaagtaca ctgggagcac c 21
<210> 769
   <211> 7
   <212> PRT
   <213> Artificial Sequence
<220>
   <223> Synthetic
<400> 769
   Ile Lys Tyr Thr Gly Ser Thr
   1 5
<210> 770
   <211> 33
   <212> DNA
   <213> Artificial Sequence
<220>
   <223> Synthetic
<400> 770
   gcgagagcac ctggaactca tgcttttgat gtt 33
<210> 771
   <211> 11
   <212> PRT
   <213> Artificial Sequence
<220>
   <223> Synthetic
<400> 771
   Ala Arg Ala Pro Gly Thr His Ala Phe Asp Val
   1 5 10
<210> 772
   <211> 324
   <212> DNA
   <213> Artificial Sequence
<220>
   <223> Synthetic
<400> 772
   gaaacgacac tcacgcagtc tccaggcacc ctgtctttgt ttccagggga aagagccacc 60
   ctctcctgca gggccagtca gagtgttagc agtagctact tagcctggta ccagcagaaa 120
   cctggccagg ctcccaggct cctcatctat ggtgcatcca gcagggccac tggcatccca 180
   gacaggttca gtggcagtgg gtctgtgaca gactccactc tcaccatcag cagcctggag 240
   cctgaagatt ttgcagtgta ttactgtcag cagtatagta ggtcaccgat caccttcggc 300
   caagggacca agctggagat caaa                                        324
<210> 773
   <211> 108
   <212> PRT
   <213> Artificial Sequence
<220>
   <223> Synthetic
<400> 773
   Glu Thr Thr Leu Thr Gln Ser Pro Gly Thr Leu Ser Leu Phe Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Val Thr Asp Ser Thr Leu Thr Ile Ser Ser Leu Glu
   65                  70                  75                  80
   Pro Glu Asp Phe Ala Val Tyr Tyr Cys Gln Gln Tyr Ser Arg Ser Pro
                   85                  90                  95
   Ile Thr Phe Gly Gln Gly Thr Lys Leu Glu Ile Lys
               100                 105
<210> 774
   <211> 21
   <212> DNA
   <213> Artificial Sequence
<220>
   <223> Synthetic
<400> 774
   cagagtgtta gcagtagcta c 21
<210> 775
   <211> 7
   <212> PRT
   <213> Artificial Sequence
<220>
   <223> Synthetic
<400> 775
   Gln Ser Val Ser Ser Ser Tyr
   1               5
<210> 776
   <211> 9
   <212> DNA
   <213> Artificial Sequence
<220>
   <223> Synthetic
<400> 776
   ggtgcatcc 9
<210> 777
   <211> 3
   <212> PRT
   <213> Artificial Sequence
<220>
   <223> Synthetic
<400> 777
   Gly Ala Ser
   1
<210> 778
   <211> 27
   <212> DNA
   <213> Artificial Sequence
<220>
   <223> Synthetic
<400> 778
   cagcagtata gtaggtcacc gatcacc 27
<210> 779
   <211> 9
   <212> PRT
   <213> Artificial Sequence
<220>
   <223> Synthetic
<400> 779
   Gln Gln Tyr Ser Arg Ser Pro Ile Thr
   1 5
<210> 780
   <211> 360
   <212> DNA
   <213> Artificial Sequence
<220>
   <223> Synthetic
<400> 780
   gaagtgcagc tggtgcagtc tgggggagcc ttggtacaac ctggggggtc cctgagactc 60
   tcctgtgcag cctctggatt cacctttaac aactttgcca tgacctgggt ccgccaggct 120
   ccagggaagg gcctggagtg ggtctcaact attagtggta gtggcgttga cacatactgc 180
   gcagactccg tgaagggccg gttcaccatc tccagagaca attccaagaa cacactgtat 240
   ctgcaaatga acagcctgag agccgaggac acggccgtat attactgttc gaaagatggc 300
   gccttctata gtggctacga acactactgg ggccagggaa ccacggtcac cgtctcctca 360
<210> 781
   <211> 120
   <212> PRT
   <213> Artificial Sequence
<220>
   <223> Synthetic
<400> 781
   Glu Val Gln Leu Val Gln Ser Gly Gly Ala Leu Val Gln Pro Gly Gly
   1               5                   10                  15
   Ser Leu Arg Leu Ser Cys Ala Ala Ser Gly Phe Thr Phe Asn Asn Phe
               20                  25                  30
   Ala Met Thr Trp Val Arg Gln Ala Pro Gly Lys Gly Leu Glu Trp Val
           35                  40                  45
   Ser Thr Ile Ser Gly Ser Gly Val Asp Thr Tyr Cys Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ser Lys Asp Gly Ala Phe Tyr Ser Gly Tyr Glu His Tyr Trp Gly Gln
               100                 105                 110
   Gly Thr Thr Val Thr Val Ser Ser
           115                 120
<210> 782
   <211> 24
   <212> DNA
   <213> Artificial Sequence
<220>
   <223> Synthetic
<400> 782
   ggattcacct ttaacaactt tgcc 24
<210> 783
   <211> 8
   <212> PRT
   <213> Artificial Sequence
<220>
   <223> Synthetic
<400> 783
   Gly Phe Thr Phe Asn Asn Phe Ala
   1               5
<210> 784
   <211> 24
   <212> DNA
   <213> Artificial Sequence
<220>
   <223> Synthetic
<400> 784
   attagtggta gtggcgttga caca 24
<210> 785
   <211> 8
   <212> PRT
   <213> Artificial Sequence
<220>
   <223> Synthetic
<400> 785
   Ile Ser Gly Ser Gly Val Asp Thr
   1 5
<210> 786
   <211> 39
   <212> DNA
   <213> Artificial Sequence
<220>
   <223> Synthetic
<400> 786
   tcgaaagatg gcgccttcta tagtggctac gaacactac 39
<210> 787
   <211> 13
   <212> PRT
   <213> Artificial Sequence
<220>
   <223> Synthetic
<400> 787
   Ser Lys Asp Gly Ala Phe Tyr Ser Gly Tyr Glu His Tyr
   1 5 10
<210> 788
   <211> 324
   <212> DNA
   <213> Artificial Sequence
<220>
   <223> Synthetic
<400> 788
   gaaacgacac tcacgcagtc tccaggcacc ctgtctttgt ctccagggga aagagccacc 60
   ctctcctgca gggccagtca gagtgttagc agcagctact tagcctggta ccagcagaaa 120
   cctggccagg ctcccaggct cctcatctat ggtacatcca acagggccac tggcatccca 180
   gacaggttca gtggcagtgg gtctgggaca gacttcactc tcaccatcag cagactggag 240
   tctgaagatt ttgcagtgta ttactgtcag cagtatggta gctcacctcg gacgttcggc 300
   caagggacca aggtggagat caaa                                        324
<210> 789
   <211> 108
   <212> PRT
   <213> Artificial Sequence
<220>
   <223> Synthetic
<400> 789
   Glu Thr Thr Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Thr Ser Asn Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Ser Glu Asp Phe Ala Val Tyr Tyr Cys Gln Gln Tyr Gly Ser Ser Pro
                   85                  90                  95
   Arg Thr Phe Gly Gln Gly Thr Lys Val Glu Ile Lys
               100                 105
<210> 790
   <211> 21
   <212> DNA
   <213> Artificial Sequence
<220>
   <223> Synthetic
<400> 790
   cagagtgtta gcagcagcta c 21
<210> 791
   <211> 7
   <212> PRT
   <213> Artificial Sequence
<220>
   <223> Synthetic
<400> 791
   Gln Ser Val Ser Ser Ser Tyr
    1               5
<210> 792
   <211> 9
   <212> DNA
   <213> Artificial Sequence
<220>
   <223> Synthetic
<400> 792
   ggtacatcc 9
<210> 793
   <211> 3
   <212> PRT
   <213> Artificial Sequence
<220>
   <223> Synthetic
<400> 793
   Gly Thr Ser
   1
<210> 794
   <211> 27
   <212> DNA
   <213> Artificial Sequence
<220>
   <223> Synthetic
<400> 794
   cagcagtatg gtagctcacc tcggacg 27
<210> 795
   <211> 9
   <212> PRT
   <213> Artificial Sequence
<220>
   <223> Synthetic
<400> 795
   Gln Gln Tyr Gly Ser Ser Pro Arg Thr
   1 5
<210> 796
   <211> 357
   <212> DNA
   <213> Artificial Sequence
<220>
   <223> Synthetic
<400> 796
   gaagtgcagc tggtgcagtc tggacctgag gtgaagaagc ctggggcctc agtgaaggtc 60
   tcctgcaagg cttctggtta cacctttacc tactatggta tcagttggat acgacagacc 120
   cctggacaag ggcttgagtg gatgggatgg atcagcgctt acgatggtaa cacagactat 180
   gcacagaagt tccaagacag aatcaccatg accacagaca catcctcgac cacagcctac 240
   atggaactga ggagcctgag atctgacgac acggccgtct attactgtgc gaggtatagt 300
   tggaacaagc actggttcga cccctggggc cagggaacca tggtcaccgt ctcttca 357
<210> 797
   <211> 119
   <212> PRT
   <213> Artificial Sequence
<220>
   <223> Synthetic
<400> 797
   Glu Val Gln Leu Val Gln Ser Gly Pro Glu Val Lys Lys Pro Gly Ala
   1 5 10 15
   Ser Val Lys Val Ser Cys Lys Ala Ser Gly Tyr Thr Phe Thr Tyr Tyr
   20 25 30
   Gly Ile Ser Trp Ile Arg Gln Thr Pro Gly Gln Gly Leu Glu Trp Met
   35 40 45
   Gly Trp Ile Ser Ala Tyr Asp Gly Asn Thr Asp Tyr Ala Gln Lys Phe
   50 55 60
   Gln Asp Arg Ile Thr Met Thr Thr Asp Thr Ser Ser Thr Thr Ala Tyr
   65 70 75 80
   Met Glu Leu Arg Ser Leu Arg Ser Asp Asp Thr Ala Val Tyr Tyr Cys
   85 90 95
   Ala Arg Tyr Ser Trp Asn Lys His Trp Phe Asp Pro Trp Gly Gln Gly
   100 105 110
   Thr Met Val Thr Val Ser Ser
   115
<210> 798
   <211> 24
   <212> DNA
   <213> Artificial Sequence
<220>
   <223> Synthetic
<400> 798
   ggttacacct ttacctacta tggt 24
<210> 799
   <211> 8
   <212> PRT
   <213> Artificial Sequence
<220>
   <223> Synthetic
<400> 799
   Gly Tyr Thr Phe Thr Tyr Tyr Gly
   1 5
<210> 800
   <211> 24
   <212> DNA
   <213> Artificial Sequence
<220>
   <223> Synthetic
<400> 800
   atcagcgctt acgatggtaa caca 24
<210> 801
   <211> 8
   <212> PRT
   <213> Artificial Sequence
<220>
   <223> Synthetic
<400> 801
   Ile Ser Ala Tyr Asp Gly Asn Thr
   1 5
<210> 802
   <211> 36
   <212> DNA
   <213> Artificial Sequence
<220>
   <223> Synthetic
<400> 802
   gcgaggtata gttggaacaa gcactggttc gacccc 36
<210> 803
   <211> 12
   <212> PRT
   <213> Artificial Sequence
<220>
   <223> Synthetic
<400> 803
   Ala Arg Tyr Ser Trp Asn Lys His Trp Phe Asp Pro
   1 5 10
<210> 804
   <211> 324
   <212> DNA
   <213> Artificial Sequence
<220>
   <223> Synthetic
<400> 804
   gaaattgtga tgacacagtc tccaggcacc ctgtctttgt ctccagggga cagagccacc 60
   ctctcctgca gggccagtca gagtgttacc ggcagctact tagcctggta ccagcagaaa 120
   cctggccagg ctcccagact cctcatctat ggtgcatcca acagggccac tggcatccca 180
   gacaggttca ctggcagtgg gtctgggaca gacttcactc tcaccatcag cagactggag 240
   cctgaagatt ttgcagtgta tttctgtcaa cagtctgctt tctcaccgtg gacgttcggc 300
   caggggacca aggtggaaat caaa                                        324
<210> 805
   <211> 108
   <212> PRT
   <213> Artificial Sequence
<220>
   <223> Synthetic
<400> 805
   Glu Ile Val Met Thr Gln Ser Pro Gly Thr Leu Ser Leu Ser Pro Gly
    1               5                  10                  15
   Asp Arg Ala Thr Leu Ser Cys Arg Ala Ser Gln Ser Val Thr Gly Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Asn Arg Ala Thr Gly Ile Pro Asp Arg Phe Thr
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Phe Cys Gln Gln Ser Ala Phe Ser Pro
                   85                  90                  95
   Trp Thr Phe Gly Gln Gly Thr Lys Val Glu Ile Lys
               100                 105
<210> 806
   <211> 21
   <212> DNA
   <213> Artificial Sequence
<220>
   <223> Synthetic
<400> 806
   cagagtgtta ccggcagcta c 21
<210> 807
   <211> 7
   <212> PRT
   <213> Artificial Sequence
<220>
   <223> Synthetic
<400> 807
   Gln Ser Val Thr Gly Ser Tyr
    1               5
<210> 808
   <211> 9
   <212> DNA
   <213> Artificial Sequence
<220>
   <223> Synthetic
<400> 808
   ggtgcatcc 9
<210> 809
   <211> 3
   <212> PRT
   <213> Artificial Sequence
<220>
   <223> Synthetic
<400> 809
   Gly Ala Ser
   1
<210> 810
   <211> 27
   <212> DNA
   <213> Artificial Sequence
<220>
   <223> Synthetic
<400> 810
   caacagtctg ctttctcacc gtggacg 27
<210> 811
   <211> 9
   <212> PRT
   <213> Artificial Sequence
<220>
   <223> Synthetic
<400> 811
   Gln Gln Ser Ala Phe Ser Pro Trp Thr
   1 5
<210> 812
   <211> 357
   <212> DNA
   <213> Artificial Sequence
<220>
   <223> Synthetic

<400> 812
   gaggtgcagc tggtgcagtc tgggggaggc ttggtacagc ctggcaggtc cctgagactc 60
   tcctgtgcag cctctggagt caccttggat gattatgcca tgcactgggt ccggcaagct 120
   ccagggaagg gcctggagtg ggtctcaagt attagttgga atagtggtag tataggctat 180
   gcggactctg tgaagggccg cttcaccatc tccagagaca acgccaagaa ctccctgtat 240
   ctgcaaatga acagtctgag agctgaggac acggccttgt attactgtgc aaaagatggg 300
   tggaacccgt actactttga ctattggggc cagggaatac cggtcaccgt ctcctca    357
<210> 813
   <211> 119
   <212> PRT
   <213> Artificial Sequence
<220>
   <223> Synthetic
<400> 813
   Glu Val Gln Leu Val Gln Ser Gly Gly Gly Leu Val Gln Pro Gly Arg
   1               5                   10                  15
   Ser Leu Arg Leu Ser Cys Ala Ala Ser Gly Val Thr Leu Asp Asp Tyr
               20                  25                  30
   Ala Met His Trp Val Arg Gln Ala Pro Gly Lys Gly Leu Glu Trp Val
           35                  40                  45
   Ser Ser Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Asp Gly Trp Asn Pro Tyr Tyr Phe Asp Tyr Trp Gly Gln Gly
               100                 105                 110
   Ile Pro Val Thr Val Ser Ser
           115
<210> 814
   <211> 24
   <212> DNA
   <213> Artificial Sequence
<220>
   <223> Synthetic
<400> 814
   ggagtcacct tggatgatta tgcc 24
<210> 815
   <211> 8
   <212> PRT
   <213> Artificial Sequence
<220>
   <223> Synthetic
<400> 815
   Gly Val Thr Leu Asp Asp Tyr Ala
   1               5
<210> 816
   <211> 24
   <212> DNA
   <213> Artificial Sequence
<220>
   <223> Synthetic
<400> 816
   attagttgga atagtggtag tata 24
<210> 817
   <211> 8
   <212> PRT
   <213> Artificial Sequence
<220>
   <223> Synthetic
<400> 817
   Ile Ser Trp Asn Ser Gly Ser Ile
   1 5
<210> 818
   <211> 36
   <212> DNA
   <213> Artificial Sequence
<220>
   <223> Synthetic
<400> 818
   gcaaaagatg ggtggaaccc gtactacttt gactat 36
<210> 819
   <211> 12
   <212> PRT
   <213> Artificial Sequence
<220>
   <223> Synthetic
<400> 819
   Ala Lys Asp Gly Trp Asn Pro Tyr Tyr Phe Asp Tyr
   1 5 10
<210> 820
   <211> 321
   <212> DNA
   <213> Artificial Sequence
<220>
   <223> Synthetic
<400> 820
   gacatccaga tgacccagtc tccatcctcc ctgtctgcat ctgtaggaga cagagtcacc 60
   atcacttgcc gggcaagtca gggcattaga agtgatttag gctggtatca gcagaaacca 120
   gggaaagccc ctaagcgcct gatctatgct gcatccagtt tgcaaagtgg ggtcccatca 180
   aggttcagcg gcagtggatc tgggacagaa ttcactctca caatcagcag cctgcagcct 240
   gaagattttg caacctatta ctgtctacag cataatagtt accctctcac tttcggcgga 300
   gggaccaagc tggagatcaa a                                           321
<210> 821
   <211> 107
   <212> PRT
   <213> Artificial Sequence
<220>
   <223> Synthetic
<400> 821
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Ser Asp
               20                  25                  30
   Leu Gly Trp Tyr Gln Gln Lys Pro Gly Lys Ala Pro Lys Arg Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Tyr Pro Leu
                   85                  90                  95
   Thr Phe Gly Gly Gly Thr Lys Leu Glu Ile Lys
               100                 105
<210> 822
   <211> 18
   <212> DNA
   <213> Artificial Sequence
<220>
   <223> Synthetic
<400> 822
   cagggcatta gaagtgat 18
<210> 823
   <211> 6
   <212> PRT
   <213> Artificial Sequence
<220>
   <223> Synthetic
<400> 823
   Gln Gly Ile Arg Ser Asp
    1               5
<210> 824
   <211> 9
   <212> DNA
   <213> Artificial Sequence
<220>
   <223> Synthetic
<400> 824
   gctgcatcc 9
<210> 825
   <211> 3
   <212> PRT
   <213> Artificial Sequence
<220>
   <223> Synthetic
<400> 825
   Ala Ala Ser
   1
<210> 826
   <211> 27
   <212> DNA
   <213> Artificial Sequence
<220>
   <223> Synthetic
<400> 826
   ctacagcata atagttaccc tctcact 27
<210> 827
   <211> 9
   <212> PRT
   <213> Artificial Sequence
<220>
   <223> Synthetic
<400> 827
   Leu Gln His Asn Ser Tyr Pro Leu Thr
   1 5
<210> 828
   <211> 357
   <212> DNA
   <213> Artificial Sequence
<220>
   <223> Synthetic
<400> 828
   caggtacagc tgcagcagtc gggcccagga ctggtgaagc cttcacagac cctgtccctc 60
   acctgcgctg tctctggtga ctccttcagc agtggtaatt actactggag ctggatccgc 120
   caacacccag ggaagggcct ggagtggatt gggtacatca agtacactgg gagcacctac 180
   tacaacccgt ccctcaagag tcgagttacc atattagtag acacgtctaa gacccagttc 240
   tccctgaagc tgagctctgt gactgccgcg gacacggccg tgtattactg tgcgagagca 300
   cctggaactc atgtttttga tgtctggggc caagggacaa tggtcaccgt ctcttca 357
<210> 829
   <211> 119
   <212> PRT
   <213> Artificial Sequence
<220>
   <223> Synthetic
<400> 829
   Gln Val Gln Leu Gln Gln Ser Gly Pro Gly Leu Val Lys Pro Ser Gln
   1 5 10 15
   Thr Leu Ser Leu Thr Cys Ala Val Ser Gly Asp Ser Phe Ser Ser Gly
   20 25 30
   Asn Tyr Tyr Trp Ser Trp Ile Arg Gln His Pro Gly Lys Gly Leu Glu
   35 40 45
   Trp Ile Gly Tyr Ile Lys Tyr Thr Gly Ser Thr Tyr Tyr Asn Pro Ser
   50 55 60
   Leu Lys Ser Arg Val Thr Ile Leu Val Asp Thr Ser Lys Thr Gln Phe
   65 70 75 80
   Ser Leu Lys Leu Ser Ser Val Thr Ala Ala Asp Thr Ala Val Tyr Tyr
   85 90 95
   Cys Ala Arg Ala Pro Gly Thr His Val Phe Asp Val Trp Gly Gln Gly
   100 105 110
   Thr Met Val Thr Val Ser Ser
   115
<210> 830
   <211> 30
   <212> DNA
   <213> Artificial Sequence
<220>
   <223> Synthetic
<400> 830
   ggtgactcct tcagcagtgg taattactac 30
<210> 831
   <211> 10
   <212> PRT
   <213> Artificial Sequence
<220>
   <223> Synthetic
<400> 831
   Gly Asp Ser Phe Ser Ser Gly Asn Tyr Tyr
   1 5 10
<210> 832
   <211> 21
   <212> DNA
   <213> Artificial Sequence
<220>
   <223> Synthetic
<400> 832
   atcaagtaca ctgggagcac c 21
<210> 833
   <211> 6
   <212> PRT
   <213> Artificial Sequence
<220>
   <223> Synthetic
<400> 833
   Lys Tyr Thr Gly Ser Thr
   1 5
<210> 834
   <211> 33
   <212> DNA
   <213> Artificial Sequence
<220>
   <223> Synthetic
<400> 834
   gcgagagcac ctggaactca tgtttttgat gtc 33
<210> 835
   <211> 11
   <212> PRT
   <213> Artificial Sequence
<220>
   <223> Synthetic
<400> 835
   Ala Arg Ala Pro Gly Thr His Val Phe Asp Val
   1 5 10
<210> 836
   <211> 324
   <212> DNA
   <213> Artificial Sequence
<220>
   <223> Synthetic
<400> 836
   gacatccagt tgacccagtc tccaggcacc ctgtctttgc ttccagggga aagagccacc 60
   ctctcctgca gggccagtca gagtgttagc agtagctatt tagcctggta ccagcagaaa 120
   cctggccagg ctcccaggct cctcatctat ggtgcatcca gcagggccac tggcatccca 180
   gacaggttca gtggcagtgg gtctgtgaca gacttcactc tcaccatcag cagcctggag 240
   cctgaagatt ttgcagtgta ttactgtcag cagtatagta ggtcaccgat caccttcggc 300
   caagggacca aggtggagat caaa                                        324
<210> 837
   <211> 108
   <212> PRT
   <213> Artificial Sequence
<220>
   <223> Synthetic
<400> 837
   Asp Ile Gln Leu Thr Gln Ser Pro Gly Thr Leu Ser Leu Leu Pro Gly
    1               5                  10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Val Thr Asp Phe Thr Leu Thr Ile Ser Ser Leu Glu
   65                  70                  75                  80
   Pro Glu Asp Phe Ala Val Tyr Tyr Cys Gln Gln Tyr Ser Arg Ser Pro
                   85                  90                  95
   Ile Thr Phe Gly Gln Gly Thr Lys Val Glu Ile Lys
               100                 105
<210> 838
   <211> 21
   <212> DNA
   <213> Artificial Sequence
<220>
   <223> Synthetic
<400> 838
   cagagtgtta gcagtagcta t 21
<210> 839
   <211> 7
   <212> PRT
   <213> Artificial Sequence
<220>
   <223> Synthetic
<400> 839
   Gln Ser Val Ser Ser Ser Tyr
    1               5
<210> 840
   <211> 9
   <212> DNA
   <213> Artificial Sequence
<220>
   <223> Synthetic
<400> 840
   ggtgcatcc 9
<210> 841
   <211> 3
   <212> PRT
   <213> Artificial Sequence
<220>
   <223> Synthetic
<400> 841
   Gly Ala Ser
   1
<210> 842
   <211> 27
   <212> DNA
   <213> Artificial Sequence
<220>
   <223> Synthetic
<400> 842
   cagcagtata gtaggtcacc gatcacc 27
<210> 843
   <211> 9
   <212> PRT
   <213> Artificial Sequence
<220>
   <223> Synthetic
<400> 843
   Gln Gln Tyr Ser Arg Ser Pro Ile Thr
   1 5
<210> 844
   <211> 363
   <212> DNA
   <213> Artificial Sequence
<220>
   <223> Synthetic
<400> 844
   caggtgcagc tgcaggagtc gggcccagga ctggtgaagc cttcacagac cctgtccctc 60
   acctgcactg tctctggtgg ctccatcagc agtggtggtt actactggag ttggatccgc 120
   cagcacccag ggaagggcct ggagtggatt gggtacatcc attatagtgg gaacacccac 180
   tacaatccga ccctcaagag tcgaattacc atatcagtag acacgtctaa gaaccagttc 240
   tcccttgagg tgaactctgt gactgccgcg gacacggccg tatactactg tgcgaggaat 300
   atggttcggg gagttcactg gttcgacccc tggggccagg gaaccacggt caccgtctcc 360
   tca                                                               363
<210> 845
   <211> 121
   <212> PRT
   <213> Artificial Sequence
<220>
   <223> Synthetic
<400> 845
   Gln Val Gln Leu Gln Glu Ser Gly Pro Gly Leu Val Lys Pro Ser Gln
    1               5                  10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile His Tyr Ser Gly Asn Thr His Tyr Asn Pro Thr
       50                  55                  60
   Leu Lys Ser Arg Ile Thr Ile Ser Val Asp Thr Ser Lys Asn Gln Phe
   65                  70                  75                  80
   Ser Leu Glu Val Asn Ser Val Thr Ala Ala Asp Thr Ala Val Tyr Tyr
                   85                  90                  95
   Cys Ala Arg Asn Met Val Arg Gly Val His Trp Phe Asp Pro Trp Gly
               100                 105                 110
   Gln Gly Thr Thr Val Thr Val Ser Ser
           115                 120
<210> 846
   <211> 30
   <212> DNA
   <213> Artificial Sequence
<220>
   <223> Synthetic
<400> 846
   ggtggctcca tcagcagtgg tggttactac 30
<210> 847
   <211> 10
   <212> PRT
   <213> Artificial Sequence
<220>
   <223> Synthetic
<400> 847
   Gly Gly Ser Ile Ser Ser Gly Gly Tyr Tyr
   1 5 10
<210> 848
   <211> 21
   <212> DNA
   <213> Artificial Sequence
<220>
   <223> Synthetic
<400> 848
   atccattata gtgggaacac c 21
<210> 849
   <211> 7
   <212> PRT
   <213> Artificial Sequence
<220>
   <223> Synthetic
<400> 849
   Ile His Tyr Ser Gly Asn Thr
   1 5
<210> 850
   <211> 39
   <212> DNA
   <213> Artificial Sequence
<220>
   <223> Synthetic
<400> 850
   gcgaggaata tggttcgggg agttcactgg ttcgacccc 39
<210> 851
   <211> 13
   <212> PRT
   <213> Artificial Sequence
<220>
   <223> Synthetic
<400> 851
   Ala Arg Asn Met Val Arg Gly Val His Trp Phe Asp Pro
   1 5 10
<210> 852
   <211> 324
   <212> DNA
   <213> Artificial Sequence
<220>
   <223> Synthetic
<400> 852
   gaaatagtgt tgacacagtc tccaggcacc ctgtctttgt ctccagggga gagagccacc 60
   ctcttctgtt gggccagtcg gagtgttagc agcagctact tagcctggta ccagcagaaa 120
   cctggccagg ctcccaggct cctcatctct ggtgcatcca gcagggccac tggcatccca 180
   gacaggttca gtggcagtgg gtctgggaca gacttcactc tcaccatcag cagactggag 240
   cctgaagatt ttgcagtata tttctgtcaa cagtatagta gttcaccgct cactttcggc 300
   ggagggacca agctggagat caaa                                        324
<210> 853
   <211> 108
   <212> PRT
   <213> Artificial Sequence
<220>
   <223> Synthetic
<400> 853
   Glu Ile Val Leu Thr Gln Ser Pro Gly Thr Leu Ser Leu Ser Pro Gly
    1               5                  10                  15
   Glu Arg Ala Thr Leu Phe Cys Trp Ala Ser Arg Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Ser Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Phe Cys Gln Gln Tyr Ser Ser Ser Pro
                   85                  90                  95
   Leu Thr Phe Gly Gly Gly Thr Lys Leu Glu Ile Lys
               100                 105
<210> 854
   <211> 21
   <212> DNA
   <213> Artificial Sequence
<220>
   <223> Synthetic
<400> 854
   cggagtgtta gcagcagcta c 21
<210> 855
   <211> 7
   <212> PRT
   <213> Artificial Sequence
<220>
   <223> Synthetic
<400> 855
   Arg Ser Val Ser Ser Ser Tyr
   1 5
<210> 856
   <211> 9
   <212> DNA
   <213> Artificial Sequence
<220>
   <223> Synthetic
<400> 856
   ggtgcatcc 9
<210> 857
   <211> 3
   <212> PRT
   <213> Artificial Sequence
<220>
   <223> Synthetic
<400> 857
   Gly Ala Ser
   1
<210> 858
   <211> 27
   <212> DNA
   <213> Artificial Sequence
<220>
   <223> Synthetic
<400> 858
   caacagtata gtagttcacc gctcact 27
<210> 859
   <211> 9
   <212> PRT
   <213> Artificial Sequence
<220>
   <223> Synthetic
<400> 859
   Gln Gln Tyr Ser Ser Ser Pro Leu Thr
   1 5

<210> 860
   <211> 357
   <212> DNA
   <213> Artificial Sequence
<220>
   <223> Synthetic
<400> 860
   caggtgcagc tgcaggagtc gggcccagga ctggtgaagc cttcacagac cctgtccctc 60
   acctgcactg tctctagtga ctccatcagc agtggtaata actactggac ctggatccgc 120
   cagcacccag ggaggggcct ggagtggatt gggtacatca aatacactgg gagcgcccac 180
   tacaacccgt ccctcaagag tcgagtcacc atgtcagtag acacgtctaa gaatcagttc 240
   tccctgaaaa tgacctctgt gactgacgcg gacacggccg tgtattattg tgcgagggca 300
   cctggaagcc attcttttga tatatggggc cgagggacaa tggtcaccgt ctcctca    357
<210> 861
   <211> 119
   <212> PRT
   <213> Artificial Sequence
   <220>
   <223> Synthetic
<400> 861
   Gln Val Gln Leu Gln Glu Ser Gly Pro Gly Leu Val Lys Pro Ser Gln
    1               5                  10                  15
   Thr Leu Ser Leu Thr Cys Thr Val Ser Ser Asp Ser Ile Ser Ser Gly
               20                  25                  30
   Asn Asn Tyr Trp Thr Trp Ile Arg Gln His Pro Gly Arg Gly Leu Glu
           35                  40                  45
   Trp Ile Gly Tyr Ile Lys Tyr Thr Gly Ser Ala His Tyr Asn Pro Ser
       50                  55                  60
   Leu Lys Ser Arg Val Thr Met Ser Val Asp Thr Ser Lys Asn Gln Phe
   65                  70                  75                  80
   Ser Leu Lys Met Thr Ser Val Thr Asp Ala Asp Thr Ala Val Tyr Tyr
                   85                  90                  95
   Cys Ala Arg Ala Pro Gly Ser His Ser Phe Asp Ile Trp Gly Arg Gly
               100                 105                 110
   Thr Met Val Thr Val Ser Ser
           115
<210> 862
   <211> 30
   <212> DNA
   <213> Artificial Sequence
<220>
   <223> Synthetic
<400> 862
   agtgactcca tcagcagtgg taataactac 30
<210> 863
   <211> 10
   <212> PRT
   <213> Artificial Sequence
<220>
   <223> Synthetic
<400> 863
   Ser Asp Ser Ile Ser Ser Gly Asn Asn Tyr
   1 5 10
<210> 864
   <211> 21
   <212> DNA
   <213> Artificial Sequence
<220>
   <223> Synthetic
<400> 864
   atcaaataca ctgggagcgc c 21
<210> 865
   <211> 7
   <212> PRT
   <213> Artificial Sequence
<220>
   <223> Synthetic
<400> 865
   Ile Lys Tyr Thr Gly Ser Ala
   1 5
<210> 866
   <211> 33
   <212> DNA
   <213> Artificial Sequence
<220>
   <223> Synthetic
<400> 866
   gcgagggcac ctggaagcca ttcttttgat ata 33
<210> 867
   <211> 11
   <212> PRT
   <213> Artificial Sequence
<220>
   <223> Synthetic
<400> 867
   Ala Arg Ala Pro Gly Ser His Ser Phe Asp Ile
   1 5 10
<210> 868
   <211> 324
   <212> DNA
   <213> Artificial Sequence
<220>
   <223> Synthetic
<400> 868
   gatgttgtga tgacccagtc tccaggcacc ctgtttttgt ctccagggga aagagccacc 60
   ctctcctgca gggccagtca gagtgttagc agcagctact tagcctggta ccagcagaaa 120
   cctggccagg ctcccaggct cctcatctat ggtgcatcca gcagggccac tggcatccca 180
   gacaggttca gtggcagtgg gtctgggaca gacttcactc tcaacatcag cagactggag 240
   cctgaagatt ttgcactgta ttactgtcag cagtatagta ggtcaccgat caccttcggc 300
   caagggacac gactggagat taaa                                        324
<210> 869
   <211> 108
   <212> PRT
   <213> Artificial Sequence
<220>
   <223> Synthetic
<400> 869
   Asp Val Val Met Thr Gln Ser Pro Gly Thr Leu Phe Leu Ser Pro Gly
    1               5                  10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Asn Ile Ser Arg Leu Glu
   65                  70                  75                  80
   Pro Glu Asp Phe Ala Leu Tyr Tyr Cys Gln Gln Tyr Ser Arg Ser Pro
                   85                  90                  95
   Ile Thr Phe Gly Gln Gly Thr Arg Leu Glu Ile Lys
               100                 105
<210> 870
   <211> 21
   <212> DNA
   <213> Artificial Sequence
<220>
   <223> Synthetic
<400> 870
   cagagtgtta gcagcagcta c 21
<210> 871
   <211> 7
   <212> PRT
   <213> Artificial Sequence
<220>
   <223> Synthetic
<400> 871
   Gln Ser Val Ser Ser Ser Tyr
   1 5
<210> 872
   <211> 9
   <212> DNA
   <213> Artificial Sequence
<220>
   <223> Synthetic
<400> 872
   ggtgcatcc 9
<210> 873
   <211> 3
   <212> PRT
   <213> Artificial Sequence
<220>
   <223> Synthetic
<400> 873
   Gly Ala Ser
   1
<210> 874
   <211> 27
   <212> DNA
   <213> Artificial Sequence
<220>
   <223> Synthetic
<400> 874
   cagcagtata gtaggtcacc gatcacc 27
<210> 875
   <211> 9
   <212> PRT
   <213> Artificial Sequence
<220>
   <223> Synthetic
<400> 875
   Gln Gln Tyr Ser Arg Ser Pro Ile Thr
   1 5
<210> 876
   <211> 360
   <212> DNA
   <213> Artificial Sequence
<220>
   <223> Synthetic
<400> 876
   caggtgcagc tggtgcagtc tgggggaggc ttggtacaac ctggggggtc cctaagactc 60
   tcctgtgcag cctctggatt cacctctaac aactttgcca tgacctgggt ccgccaggct 120
   ccagggaagg gcctggagtg ggtctcaact attagtggta gtggcgttga cacatactgc 180
   gcagactccg tgaagggccg gttcaccatc tccagagaca attccaagaa cacactgtat 240
   ctgcaaatga acagcctgag agtcgaggac acggccgtat attactgtgc taaagatggc 300
   gccttctata gtggctacga acactactgg ggccagggaa ccctggtcac cgtctcctca 360
<210> 877
   <211> 120
   <212> PRT
   <213> Artificial Sequence
<220>
   <223> Synthetic
<400> 877
   Gln Val Gln Leu Val Gln Ser Gly Gly Gly Leu Val Gln Pro Gly Gly
   1               5                   10                  15
   Ser Leu Arg Leu Ser Cys Ala Ala Ser Gly Phe Thr Ser Asn Asn Phe
               20                  25                  30
   Ala Met Thr Trp Val Arg Gln Ala Pro Gly Lys Gly Leu Glu Trp Val
           35                  40                  45
   Ser Thr Ile Ser Gly Ser Gly Val Asp Thr Tyr Cys Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Val Glu Asp Thr Ala Val Tyr Tyr Cys
                   85                  90                  95
   Ala Lys Asp Gly Ala Phe Tyr Ser Gly Tyr Glu His Tyr Trp Gly Gln
               100                 105                 110
   Gly Thr Leu Val Thr Val Ser Ser
           115                 120
<210> 878
   <211> 24
   <212> DNA
   <213> Artificial Sequence
<220>
   <223> Synthetic
<400> 878
   ggattcacct ctaacaactt tgcc 24
<210> 879
   <211> 8
   <212> PRT
   <213> Artificial Sequence
<220>
   <223> Synthetic
<400> 879
   Gly Phe Thr Ser Asn Asn Phe Ala
   1 5
<210> 880
   <211> 24
   <212> DNA
   <213> Artificial Sequence
<220>
   <223> Synthetic
<400> 880
   attagtggta gtggcgttga caca 24
<210> 881
   <211> 8
   <212> PRT
   <213> Artificial Sequence
<220>
   <223> Synthetic
<400> 881
   Ile Ser Gly Ser Gly Val Asp Thr
   1 5
<210> 882
   <211> 39
   <212> DNA
   <213> Artificial Sequence
<220>
   <223> Synthetic
<400> 882
   gctaaagatg gcgccttcta tagtggctac gaacactac 39
<210> 883
   <211> 13
   <212> PRT
   <213> Artificial Sequence
<220>
   <223> Synthetic
<400> 883
   Ala Lys Asp Gly Ala Phe Tyr Ser Gly Tyr Glu His Tyr
   1 5 10
<210> 884
   <211> 324
   <212> DNA
   <213> Artificial Sequence
<220>
   <223> Synthetic
<400> 884
   gatgttgtga tgacccagtc tccaggcacc ctgtctttgt ctccagggga aagagccacc 60
   ctctcctgca gggccagtca gagtgttagc agcagctact tagcctggta ccagcagaaa 120
   cctggccagg ctcccaggct cctcatctat ggtacatcca acagggcctc tggcatccca 180
   gacaagttca ttggcagtgg gtctgggaca gacttcactc tcaccatcag cagactggag 240
   tctgaagatt ttgcagtgta ttactgtcag cagtatggta gctcacctcg gacgttcggc 300
   caagggacca aagtggatat caaa                                         324
<210> 885
   <211> 108
   <212> PRT
   <213> Artificial Sequence
<220>
   <223> Synthetic
<400> 885
   Asp Val Val Met Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Thr Ser Asn Arg Ala Ser Gly Ile Pro Asp Lys Phe Ile
       50                  55                  60
   Gly Ser Gly Ser Gly Thr Asp Phe Thr Leu Thr Ile Ser Arg Leu Glu
   65                  70                  75                  80
   Ser Glu Asp Phe Ala Val Tyr Tyr Cys Gln Gln Tyr Gly Ser Ser Pro
                   85                  90                  95
   Arg Thr Phe Gly Gln Gly Thr Lys Val Asp Ile Lys
               100                 105
<210> 886
   <211> 21
   <212> DNA
   <213> Artificial Sequence
<220>
   <223> Synthetic
<400> 886
   cagagtgtta gcagcagcta c 21
<210> 887
   <211> 7
   <212> PRT
   <213> Artificial Sequence
<220>
   <223> Synthetic
<400> 887
   Gln Ser Val Ser Ser Ser Tyr
   1 5

<210> 888
   <211> 9
   <212> DNA
   <213> Artificial Sequence
<220>
   <223> Synthetic
<400> 888
   ggtacatcc 9
<210> 889
   <211> 3
   <212> PRT
   <213> Artificial Sequence
<220>
   <223> Synthetic
<400> 889
   Gly Thr Ser
   1
<210> 890
   <211> 27
   <212> DNA
   <213> Artificial Sequence
<220>
   <223> Synthetic
<400> 890
   cagcagtatg gtagctcacc tcggacg 27
<210> 891
   <211> 9
   <212> PRT
   <213> Artificial Sequence
<220>
   <223> Synthetic
<400> 891
   Gln Gln Tyr Gly Ser Ser Pro Arg Thr
   1 5
<210> 892
   <211> 360
   <212> DNA
   <213> Artificial Sequence
<220>
   <223> Synthetic
<400> 892
   caggtgcagc tgcaggagtc cggccccggc ctggtgaagc cctcccagac cctgtccctg 60
   acctgcaccg tgtccggcgg ctccatcggc tccggcggct actactggtc ctggatccgg 120
   cagcaccccg gcaagggcct ggagtggatc ggctacgtgc actactccgg caacacctac 180
   tacaacccct ccctgaagtc ccgggtgacc atctccgtgg acacctccaa gaaccagttc 240
   tccctgaagc tgtcctccgt gaccgccgcc gacaccgccg tgtactactg cgcccgggcc 300
   ccccggggct accactactt cgcctactgg ggccagggca ccctggtgac cgtgtcctcc 360
<210> 893
   <211> 120
   <212> PRT
   <213> Artificial Sequence
<220>
   <223> Synthetic
<400> 893
   Gln Val Gln Leu Gln Glu Ser Gly Pro Gly Leu Val Lys Pro Ser Gln
    1               5                  10                  15
   Thr Leu Ser Leu Thr Cys Thr Val Ser Gly Gly Ser Ile Gly Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Val His Tyr Ser Gly Asn Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Pro Arg Gly Tyr His Tyr Phe Ala Tyr Trp Gly Gln
               100                 105                 110
   Gly Thr Leu Val Thr Val Ser Ser
           115                 120
<210> 894
   <211> 324
   <212> DNA
   <213> Artificial Sequence
<220>
   <223> Synthetic
<400> 894
   gagatcgtgc tgacccagtc ccccggcacc ctgtccctgt cccccggcga gcgggccacc 60
   ctgtcctgcc gggcctccca gtccgtgtcc tcctcctacc tggcctggta ccagcagaag 120
   cccggccagg ccccccggct gctgatctac ggcgcctcct cccgggccac cggcatcccc 180
   gaccggttct ccggctccgg ctccggcacc gacttcaccc tgaccatctc ccggctggag 240
   cccgaggact tcgccgtgta ctactgccag cagtacggct cctcccccct gaccttcggc 300
   ggcggcacca aggtggagat caag                                       324
<210> 895
   <211> 108
   <212> PRT
   <213> Artificial Sequence
<220>
   <223> Synthetic
<400> 895
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gly Gly Thr Lys Val Glu Ile Lys
               100                 105
<210> 896
   <211> 369
   <212> DNA
   <213> Artificial Sequence
<220>
   <223> Synthetic
<400> 896
   caggtgcagc tggtggagtc cggcggcggc gtggtgcagc ccggccggtc cctgcggctg 60
   tcctgcgccg cctccggctt caccttctcc tcctacggca tgcactgggt gcggcaggcc 120
   cccggcaagg gcctggagtg ggtggccgtg ctgtggtacg acggcaccaa caagtactac 180
   gccgactccg tgaagggccg gttcaccatc tcccgggaca actccaagaa caccctgtac 240
   ctgcagatga actccctgcg ggccgaggac accgccgtgt actactgcgc ccgggaccac 300
   gacttccggt ccggctacga gggctggttc gacccctggg gccagggcac cctggtgacc 360
   gtgtcctcc                                                         369
<210> 897
   <211> 123
   <212> PRT
   <213> Artificial Sequence
<220>
   <223> Synthetic
<400> 897
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Leu Trp Tyr Asp Gly Thr Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp His Asp Phe Arg Ser Gly Tyr Glu Gly Trp Phe Asp Pro
               100                 105                 110
   Trp Gly Gln Gly Thr Leu Val Thr Val Ser Ser
           115                 120
<210> 898
   <211> 321
   <212> DNA
   <213> Artificial Sequence
<220>
   <223> Synthetic
<400> 898
   gagatcgtgc tgacccagtc ccccgccacc ctgtccctgt cccccggcga gcgggccacc 60
   ctgtcctgcc gggcctccca gtccgtgtcc tcctacctgg cctggtacca gcagaagccc 120
   ggccaggccc cccggctgct gatctacgac gcctccaacc gggccaccgg catccccgcc 180
   cggttctccg gctccggctc cggcaccgac ttcaccctga ccatctcctc cctggagccc 240
   gaggacttcg ccgtgtacta ctgccagcac cggtccaact ggccccccac cttcggcggc 300
   ggcaccaagg tggagatcaa g                                           321
<210> 899
   <211> 107
   <212> PRT
   <213> Artificial Sequence
<220>
   <223> Synthetic
<400> 899
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln His Arg Ser Asn Trp Pro Pro
                   85                  90                  95
   Thr Phe Gly Gly Gly Thr Lys Val Glu Ile Lys
               100                 105

<210> 900
   <211> 369
   <212> DNA
   <213> Artificial Sequence
<220>
   <223> Synthetic
<400> 900
   caggtgcagc tggtggagtc tgggggaggc gtggtccagc ctgggaggtc cctgagactc 60
   tcctgtgcag cgtctggatt caccttcagt agttatggca tgcactgggt ccgccaggct 120
   ccaggcaagg ggctggagtg ggtgtcattt ttatggtatg atggaactaa taaaaactat 180
   gtagagtccg tgaagggccg attcaccatc tcaagagaca attccaagaa tatgctgtat 240
   ctggaaatga acagcctgag agccgaggac acggctgtgt attactgtgc gagagatcac 300
   gattttagga gtggttatga ggggtggttc gacccctggg gccagggaac cctggtcacc 360
   gtctcctca                                                         369
<210> 901
   <211> 123
   <212> PRT
   <213> Artificial Sequence
<220>
   <223> Synthetic
<400> 901
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ser Phe Leu Trp Tyr Asp Gly Thr Asn Lys Asn Tyr Val Glu Ser Val
       50                  55                  60
   Lys Gly Arg Phe Thr Ile Ser Arg Asp Asn Ser Lys Asn Met Leu Tyr
   65                  70                  75                  80
   Leu Glu Met Asn Ser Leu Arg Ala Glu Asp Thr Ala Val Tyr Tyr Cys
                   85                  90                  95
   Ala Arg Asp His Asp Phe Arg Ser Gly Tyr Glu Gly Trp Phe Asp Pro
               100                 105                 110
   Trp Gly Gln Gly Thr Leu Val Thr Val Ser Ser
           115                 120
<210> 902
   <211> 321
   <212> DNA
   <213> Artificial Sequence
<220>
   <223> Synthetic
<400> 902
   gaaatagtgt tgacacagtc tccagccacc ctgtctttgt ctccagggga aagagccacc 60
   ctctcctgca gggccagtca gagtgttagc agctacttag cctggtacca acagaaacct 120
   ggccaggctc ccaggctcct catctatgat gcatccaaca gggccactgg catcccagcc 180
   aggttcagtg gcagtgggtc tgggacagac ttcactctca ccatcagcag cctagagcct 240
   gaagattttg cagtttatta ctgtcaacac cgtagcaact ggcctcccac tttcggcgga 300
   gggaccaagg tggaaatcaa a                                           321
<210> 903
   <211> 107
   <212> PRT
   <213> Artificial Sequence
<220>
   <223> Synthetic
<400> 903
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln His Arg Ser Asn Trp Pro Pro
                   85                  90                  95
   Thr Phe Gly Gly Gly Thr Lys Val Glu Ile Lys
               100                 105
<210> 904
   <211> 363
   <212> DNA
   <213> Artificial Sequence
<220>
   <223> Synthetic
<400> 904
   caggtgcagc tgcaggagtc gggcccagga ctggtgaagc cttcacagac cctgtccctc 60
   acctgcactg tctctggtgg ctccatcagc agtggtggtt actactggag ttggatccgc 120
   cagcacccag ggaagggcct ggagtggatt gggtacatcc attatagtgg gaacacccac 180
   tacaatccga ccctcaagag tcgaattacc atatcagtag acacgtctaa gaaccagttc 240
   tcccttgagg tgaactctgt gactgccgcg gacacggccg tatactactg tgcgaggaat 300
   atggttcggg gagttcactg gttcgacccc tggggccagg gaaccacggt caccgtctcc 360
   tca                                                               363
<210> 905
   <211> 121
   <212> PRT
   <213> Artificial Sequence
<220>
   <223> Synthetic
<400> 905
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile His Tyr Ser Gly Asn Thr His Tyr Asn Pro Thr
       50                  55                  60
   Leu Lys Ser Arg Ile Thr Ile Ser Val Asp Thr Ser Lys Asn Gln Phe
   65                  70                  75                  80
   Ser Leu Glu Val Asn Ser Val Thr Ala Ala Asp Thr Ala Val Tyr Tyr
                   85                  90                  95
   Cys Ala Arg Asn Met Val Arg Gly Val His Trp Phe Asp Pro Trp Gly
               100                 105                 110
   Gln Gly Thr Thr Val Thr Val Ser Ser
           115                 120
<210> 906
   <211> 324
   <212> DNA
   <213> Artificial Sequence
<220>
   <223> Synthetic
<400> 906
   gaaatagtgt tgacacagtc tccaggcacc ctgtctttgt ctccagggga gagagccacc 60
   ctcttctgtt gggccagtcg gagtgttagc agcagctact tagcctggta ccagcagaaa 120
   cctggccagg ctcccaggct cctcatctct ggtgcatcca gcagggccac tggcatccca 180
   gacaggttca gtggcagtgg gtctgggaca gacttcactc tcaccatcag cagactggag 240
   cctgaagatt ttgcagtata tttctgtcaa cagtatagta gttcaccgct cactttcggc 300
   ggagggacca agctggagat caaa 324
<210> 907
   <211> 108
   <212> PRT
   <213> Artificial Sequence
<220>
   <223> Synthetic
<400> 907
   Glu Ile Val Leu Thr Gln Ser Pro Gly Thr Leu Ser Leu Ser Pro Gly
    1               5                  10                  15
   Glu Arg Ala Thr Leu Phe Cys Trp Ala Ser Arg Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Ser Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Phe Cys Gln Gln Tyr Ser Ser Ser Pro
                   85                  90                  95
   Leu Thr Phe Gly Gly Gly Thr Lys Leu Glu Ile Lys
               100                 105
<210> 908
   <211> 363
   <212> DNA
   <213> Artificial Sequence
<220>
   <223> Synthetic
<400> 908
   caggtgcagc tgcaggagtc cggccccggc ctggtgaagc cctcccagac cctgtccctg 60
   acctgcaccg tgtccggcgg ctccatctcc tccggcggct actactggtc ctggatccgg 120
   cagcaccccg gcaagggcct ggagtggatc ggctacatcc actactccgg caacacctac 180
   tacaacccct ccctgaagtc ccgggtgacc atctccgtgg acacctccaa gaaccagttc 240
   tccctgaagc tgtcctccgt gaccgccgcc gacaccgccg tgtactactg cgcccggaac 300
   atggtgcggg gcgtgcactg gttcgacccc tggggccagg gcaccctggt gaccgtgtcc 360
   tcc                                                               363
<210> 909
   <211> 121
   <212> PRT
   <213> Artificial Sequence
<220>
   <223> Synthetic
<400> 909
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Ser Ser Gly
   20 25 30
   Gly Tyr Tyr Trp Ser Trp Ile Arg Gln His Pro Gly Lys Gly Leu Glu
   35 40 45
   Trp Ile Gly Tyr Ile His Tyr Ser Gly Asn Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Asn Met Val Arg Gly Val His Trp Phe Asp Pro Trp Gly
   100 105 110
   Gln Gly Thr Leu Val Thr Val Ser Ser
   115 120
<210> 910
   <211> 324
   <212> DNA
   <213> Artificial Sequence
<220>
   <223> Synthetic
<400> 910
   gagatcgtgc tgacccagtc ccccggcacc ctgtccctgt cccccggcga gcgggccacc 60
   ctgtcctgcc gggcctcccg gtccgtgtcc tcctcctacc tggcctggta ccagcagaag 120
   cccggccagg ccccccggct gctgatctac ggcgcctcct cccgggccac cggcatcccc 180
   gaccggttct ccggctccgg ctccggcacc gacttcaccc tgaccatctc ccggctggag 240
   cccgaggact tcgccgtgta ctactgccag cagtactcct cctcccccct gaccttcggc 300
   ggcggcacca aggtggagat caag 324
<210> 911
   <211> 108
   <212> PRT
   <213> Artificial Sequence
<220>
   <223> Synthetic
<400> 911
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Arg Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Ser Ser Ser Pro
   85 90 95
   Leu Thr Phe Gly Gly Gly Thr Lys Val Glu Ile Lys
   100 105
<210> 912
   <211> 363
   <212> DNA
   <213> Artificial Sequence
<220>
   <223> Synthetic
<400> 912
   caggtgcagc tgcaggagtc gggcctagga ctggtgaagc cttcacagac cctgtccctc 60
   acctgcactg tctctggtgg ctccatcagc agtggtggtt actactggag ttggatccgc 120
   cagcacccag ggaagggcct ggagtggatt gggtacatcc attatagtgg gaacacccac 180
   tacaatccga ccctcaagag tcgaattacc atatcagtag acacgtctaa gaaccagttc 240
   tcccttgagg tgaactctgt gactgccgcg gacacggccg tatactactg tgcgaggaat 300
   atggttcggg gagttcactg gttcgacccc tggggccagg gaaccctggt caccgtctcc 360
   tca                                                               363
<210> 913
   <211> 121
   <212> PRT
   <213> Artificial Sequence
<220>
   <223> Synthetic
<400> 913
   Gln Val Gln Leu Gln Glu Ser Gly Leu Gly Leu Val Lys Pro Ser Gln
    1               5                  10                  15
   Thr Leu Ser Leu Thr Cys Thr Val Ser Gly Gly Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile His Tyr Ser Gly Asn Thr His Tyr Asn Pro Thr
       50                  55                  60
   Leu Lys Ser Arg Ile Thr Ile Ser Val Asp Thr Ser Lys Asn Gln Phe
   65                  70                  75                  80
   Ser Leu Glu Val Asn Ser Val Thr Ala Ala Asp Thr Ala Val Tyr Tyr
                   85                  90                  95
   Cys Ala Arg Asn Met Val Arg Gly Val His Trp Phe Asp Pro Trp Gly
               100                 105                 110
   Gln Gly Thr Leu Val Thr Val Ser Ser
           115                 120
<210> 914
   <211> 324
   <212> DNA
   <213> Artificial Sequence
<220>
   <223> Synthetic
<400> 914
   gaaatagtgt tgacacagtc tccaggcacc ctgtctttgt ctccagggga gagagccacc 60
   ctcttctgtt gggccagtcg gagtgttagc agcagctact tagcctggta ccagcagaaa 120
   cctggccagg ctcccaggct cctcatctct ggtgcatcca gcagggccac tggcatccca 180
   gacaggttca gtggcagtgg gtctgggaca gacttcactc tcaccatcag cagactggag 240
   cctgaagatt ttgcagtata tttctgtcaa cagtatagta gttcaccgct cactttcggc 300
   ggagggacca aggtggaaat caaa                                        324
<210> 915
   <211> 108
   <212> PRT
   <213> Artificial Sequence
<220>
   <223> Synthetic
<400> 915
   Glu Ile Val Leu Thr Gln Ser Pro Gly Thr Leu Ser Leu Ser Pro Gly
    1               5                  10                  15
   Glu Arg Ala Thr Leu Phe Cys Trp Ala Ser Arg Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Ser Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Phe Cys Gln Gln Tyr Ser Ser Ser Pro
                   85                  90                  95
   Leu Thr Phe Gly Gly Gly Thr Lys Val Glu Ile Lys
               100                 105
<210> 916
   <211> 357
   <212> DNA
   <213> Artificial Sequence
<220>
   <223> Synthetic
<400> 916
   caggtgcagc tgcaggagtc cggccccggc ctggtgaagc cctcccagac cctgtccctg 60
   acctgcaccg tgtccggcgg ctccatcacc tccggcggct actactggtc ctggatccgg 120
   cagcaccccg gcaagggcct ggagtggatc ggctacatca agttctccgg caacacctac 180
   tacaacccct ccctgaagtc ccgggtgacc atctccgtgg acacctccaa gaaccagttc 240
   tccctgaagc tgtcctccgt gaccgccgcc gacaccgccg tgtactactg cgcccgggcc 300
   cccggctccc acaacttcga catctggggc cagggcacca tggtgaccgt gtcctcc    357
<210> 917
   <211> 119
   <212> PRT
   <213> Artificial Sequence
<220>
   <223> Synthetic
<400> 917
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Ile Thr Ser Gly
   20 25 30
   Gly Tyr Tyr Trp Ser Trp Ile Arg Gln His Pro Gly Lys Gly Leu Glu
   35 40 45
   Trp Ile Gly Tyr Ile Lys Phe Ser Gly Asn Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Ala Pro Gly Ser His Asn Phe Asp Ile Trp Gly Gln Gly
   100 105 110
   Thr Met Val Thr Val Ser Ser
   115
<210> 918
   <211> 324
   <212> DNA
   <213> Artificial Sequence
<220>
   <223> Synthetic
<400> 918
   gagatcgtgc tgacccagtc ccccggcacc ctgtccctgt cccccggcga gcgggccacc 60
   ctgtcctgcc gggcctccgt gtccatctcc aacaactacc tggcctggta ccagcagaag 120
   cccggccagg ccccccggct gctgatctac ggcgcctcct cccgggccac cggcatcccc 180
   gaccggttct ccggctccgg ctccggcacc gacttcaccc tgaccatccg gcggctggag 240
   cccgaggact tcgcccccta ctactgccag cagtactccc ggtcccccat caccttcggc 300
   cagggcaccc ggctggagat caag 324
<210> 919
   <211> 108
   <212> PRT
   <213> Artificial Sequence
<220>
   <223> Synthetic
<400> 919
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Val Ser Ile Ser Asn Asn
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Arg Arg Leu Glu
   65 70 75 80
   Pro Glu Asp Phe Ala Pro Tyr Tyr Cys Gln Gln Tyr Ser Arg Ser Pro
   85 90 95
   Ile Thr Phe Gly Gln Gly Thr Arg Leu Glu Ile Lys
   100 105
<210> 920
   <211> 360
   <212> DNA
   <213> Artificial Sequence
<220>
   <223> Synthetic
<400> 920
   gaggtgcagc tggtggagtc cggcggcggc ctggtgcagc ccggcggctc cctgcggctg 60
   tcctgcgccg cctccggctt caccttcaac aacttcgcca tgtcctgggt gcggcaggcc 120
   cccggcaagg gcctggagtg ggtgtccgcc atctccggct ccggcgtgga cacctactac 180
   gccgactccg tgaagggccg gttcaccatc tcccgggaca actccaagaa caccctgtac 240
   ctgcagatga actccctgcg ggccgaggac accgccgtgt actactgctc caaggacggc 300
   gccttctact ccggctacga gcactactgg ggccagggca ccctggtgac cgtgtcctcc 360
<210> 921
   <211> 120
   <212> PRT
   <213> Artificial Sequence
<220>
   <223> Synthetic
<400> 921
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Asn Asn Phe
   20 25 30
   Ala Met Ser Trp Val Arg Gln Ala Pro Gly Lys Gly Leu Glu Trp Val
   35 40 45
   Ser Ala Ile Ser Gly Ser Gly Val Asp Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ser Lys Asp Gly Ala Phe Tyr Ser Gly Tyr Glu His Tyr Trp Gly Gln
   100 105 110
   Gly Thr Leu Val Thr Val Ser Ser
   115 120
<210> 922
   <211> 324
   <212> DNA
   <213> Artificial Sequence
<220>
   <223> Synthetic
<400> 922
   gagatcgtgc tgacccagtc ccccggcacc ctgtccctgt cccccggcga gcgggccacc 60
   ctgtcctgcc gggcctccca gtccgtgtcc tcctcctacc tggcctggta ccagcagaag 120
   cccggccagg ccccccggct gctgatctac ggcacctcct cccgggccac cggcatcccc 180
   gaccggttct ccggctccgg ctccggcacc gacttcaccc tgaccatctc ccggctggag 240
   cccgaggact tcgccgtgta ctactgccag cagtacggct cctccccccg gaccttcggc 300
   cagggcacca aggtggagat caag                                        324
<210> 923
   <211> 108
   <212> PRT
   <213> Artificial Sequence
<220>
   <223> Synthetic
<400> 923
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Thr Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Arg Thr Phe Gly Gln Gly Thr Lys Val Glu Ile Lys
               100                 105
<210> 924
   <211> 357
   <212> DNA
   <213> Artificial Sequence
<220>
   <223> Synthetic
<400> 924
   caggtgcagc tggtgcagtc cggcgccgag gtgaagaagc ccggcgcctc cgtgaaggtg 60
   tcctgcaagg cctccggcta caccttcacc tactacggca tctcctgggt gcggcaggcc 120
   cccggccagg gcctggagtg gatgggctgg atctccgcct acgacggcaa caccaactac 180
   gcccagaagc tgcagggccg ggtgaccatg accaccgaca cctccacctc caccgcctac 240
   atggagctgc ggtccctgcg gtccgacgac accgccgtgt actactgcgc ccggtactcc 300
   tggaacaagc actggttcga cccctggggc cagggcaccc tggtgaccgt gtcctcc    357
<210> 925
   <211> 119
   <212> PRT
   <213> Artificial Sequence
<220>
   <223> Synthetic
<400> 925
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Tyr Tyr
               20                  25                  30
   Gly Ile Ser Trp Val Arg Gln Ala Pro Gly Gln Gly Leu Glu Trp Met
           35                  40                  45
   Gly Trp Ile Ser Ala Tyr Asp Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Tyr Ser Trp Asn Lys His Trp Phe Asp Pro Trp Gly Gln Gly
               100                 105                 110
   Thr Leu Val Thr Val Ser Ser
           115
<210> 926
   <211> 324
   <212> DNA
   <213> Artificial Sequence
<220>
   <223> Synthetic
<400> 926
   gagatcgtgc tgacccagtc ccccggcacc ctgtccctgt cccccggcga gcgggccacc 60
   ctgtcctgcc gggcctccca gtccgtgacc ggctcctacc tggcctggta ccagcagaag 120
   cccggccagg ccccccggct gctgatctac ggcgcctcct cccgggccac cggcatcccc 180
   gaccggttct ccggctccgg ctccggcacc gacttcaccc tgaccatctc ccggctggag 240
   cccgaggact tcgccgtgta ctactgccag cagtccgcct tctccccctg gaccttcggc 300
   cagggcacca aggtggagat caag                                        324
<210> 927
   <211> 108
   <212> PRT
   <213> Artificial Sequence
<220>
   <223> Synthetic
<400> 927
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Thr Gly Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Ser Ala Phe Ser Pro
   85 90 95
   Trp Thr Phe Gly Gln Gly Thr Lys Val Glu Ile Lys
   100 105
<210> 928
   <211> 8
   <212> PRT
   <213> Artificial Sequence
<220>
   <223> Synthetic
<220>
   <221> VARIANT
   <222> (1) ... (8)
   <223> Xaa = Any amino acid or absent
<400> 928
   Xaa Xaa Xaa Xaa Xaa Xaa Xaa Xaa
   1 5
<210> 929
   <211> 8
   <212> PRT
   <213> Artificial Sequence
<220>
   <223> Synthetic
<220>
   <221> VARIANT
   <222> (1) ... (8)
   <223> Xaa = Any amino acid or absent
<400> 929
   Xaa Xaa Xaa Xaa Xaa Xaa Xaa Xaa
   1 5
<210> 930
   <211> 16
   <212> PRT
   <213> Artificial Sequence
<220>
   <223> Synthetic
<220>
   <221> VARIANT
   <222> (1)...(16)
   <223> Xaa = Any amino acid or absent
<400> 930
   Xaa Xaa Xaa Xaa Xaa Xaa Xaa Xaa Xaa Xaa Xaa Xaa Xaa Xaa Xaa Xaa
    1               5                   10                  15
<210> 931
   <211> 7
   <212> PRT
   <213> Artificial Sequence
<220>
   <223> Synthetic
<220>
   <221> VARIANT
   <222> (1) ... (7)
   <223> Xaa = Any amino acid or absent
<400> 931
   Xaa Xaa Xaa Xaa Xaa Xaa Xaa
    1               5
<210> 932
   <211> 3
   <212> PRT
   <213> Artificial Sequence
<220>
   <223> Synthetic
<220>
   <221> VARIANT
   <222> (1)...(3)
   <223> Xaa = Any amino acid or absent
<400> 932
   Xaa Xaa Xaa
    1
<210> 933
   <211> 9
   <212> PRT
   <213> Artificial Sequence
<220>
   <223> Synthetic
<220>
   <221> VARIANT
   <222> (1)...(9)
   <223> Xaa = Any amino acid or absent
<400> 933
   Xaa Xaa Xaa Xaa Xaa Xaa Xaa Xaa Xaa
   1 5
<210> 934
   <211> 357
   <212> DNA
   <213> Artificial Sequence
<220>
   <223> Synthetic
<400> 934
   caggtgcagc tgcaggagtc gggcccagga ctggtgaagc cttcacagac cctgtccctc 60
   acctgcactg tctctggtgg ctccattacc agtggtggtt actactggac ctggatccgc 120
   cagcacccag ggaagggcct ggaatggatt ggatacatca aatttagtgg gaacacctac 180
   tacaacccgt ccctcaggag tcgagtcacc atatcacttg acacgtctaa gaatcagttc 240
   tccctgaata tgacctctgt gactgccgcg gacacggccg tgtattattg tgcgagagca 300
   cctggaagtc ataactttga catctggggc caagggacaa tggtcaccgt ctcttca    357
<210> 935
   <211> 119
   <212> PRT
   <213> Artificial Sequence
<220>
   <223> Synthetic
<400> 935
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Gly Tyr Tyr Trp Thr Trp Ile Arg Gln His Pro Gly Lys Gly Leu Glu
           35                  40                  45
   Trp Ile Gly Tyr Ile Lys Phe Ser Gly Asn Thr Tyr Tyr Asn Pro Ser
       50                  55                  60
   Leu Arg Ser Arg Val Thr Ile Ser Leu Asp Thr Ser Lys Asn Gln Phe
   65                  70                  75                  80
   Ser Leu Asn Met Thr Ser Val Thr Ala Ala Asp Thr Ala Val Tyr Tyr
                   85                  90                  95
   Cys Ala Arg Ala Pro Gly Ser His Asn Phe Asp Ile Trp Gly Gln Gly
               100                 105                 110
   Thr Met Val Thr Val Ser Ser
           115
<210> 936
   <211> 324
   <212> DNA
   <213> Artificial Sequence
<220>
   <223> Synthetic
<400> 936
   gaaatcgtgt tgacccagtc tccagacacc ctgtctttgt ctccagggga aagagccacc 60
   ctctcctgca gggccagtgt gagtattagt aataactatt tagcctggta ccagcagaaa 120
   cctggccagg ctcccaggct cctcatctat ggtgcatcca gcagggccac tggcatccca 180
   gacaggttca gtggcagtgg gtctgggaca gacttcactc tcaccatcag aagactggag 240
   tctgcagatt ttgcaccgta ttactgtcag caatatagta ggtcaccgat caccttcggc 300
   caagggacac gactggagat taaa                                        324
<210> 937
   <211> 108
   <212> PRT
   <213> Artificial Sequence
<220>
   <223> Synthetic
<400> 937
   Glu Ile Val Leu Thr Gln Ser Pro Asp Thr Leu Ser Leu Ser Pro Gly
    1               5                  10                  15
   Glu Arg Ala Thr Leu Ser Cys Arg Ala Ser Val Ser Ile Ser Asn Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Arg Arg Leu Glu
   65                  70                  75                  80
   Ser Ala Asp Phe Ala Pro Tyr Tyr Cys Gln Gln Tyr Ser Arg Ser Pro
                   85                  90                  95
   Ile Thr Phe Gly Gln Gly Thr Arg Leu Glu Ile Lys
               100                 105
<210> 938
   <211> 360
   <212> DNA
   <213> Artificial Sequence
<220>
   <223> Synthetic
<400> 938
   gaagtgcagc tggtggagtc tgggggagcc ttggtacaac ctggggggtc cctgagactc 60
   tcctgtgcag cctctggatt cacctttaac aactttgcca tgacctgggt ccgccaggct 120
   ccagggaagg gcctggagtg ggtctcaact attagtggta gtggcgttga cacatactgc 180
   gcagactccg tgaagggccg gttcaccatc tccagagaca attccaagaa cacactgtat 240
   ctgcaaatga acagcctgag agccgaggac acggccgtat attactgttc gaaagatggc 300
   gccttctata gtggctacga acactactgg ggccagggaa ccctggtcac cgtctcctca 360
<210> 939
   <211> 120
   <212> PRT
   <213> Artificial Sequence
<220>
   <223> Synthetic
<400> 939
   Glu Val Gln Leu Val Glu Ser Gly Gly Ala Leu Val Gln Pro Gly Gly
   1               5                   10                  15
   Ser Leu Arg Leu Ser Cys Ala Ala Ser Gly Phe Thr Phe Asn Asn Phe
               20                  25                  30
   Ala Met Thr Trp Val Arg Gln Ala Pro Gly Lys Gly Leu Glu Trp Val
           35                  40                  45
   Ser Thr Ile Ser Gly Ser Gly Val Asp Thr Tyr Cys Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ser Lys Asp Gly Ala Phe Tyr Ser Gly Tyr Glu His Tyr Trp Gly Gln
               100                 105                 110
   Gly Thr Leu Val Thr Val Ser Ser
           115                 120
<210> 940
   <211> 324
   <212> DNA
   <213> Artificial Sequence
<220>
   <223> Synthetic
<400> 940
   gaaattgtgc tcacgcagtc tccaggcacc ctgtctttgt ctccagggga aagagccacc 60
   ctctcctgca gggccagtca gagtgttagc agcagctact tagcctggta ccagcagaaa 120
   cctggccagg ctcccaggct cctcatctat ggtacatcca acagggccac tggcatccca 180
   gacaggttca gtggcagtgg gtctgggaca gacttcactc tcaccatcag cagactggag 240
   tctgaagatt ttgcagtgta ttactgtcag cagtatggta gctcacctcg gacgttcggc 300
   caagggacca aggtggagat caaa                                        324
<210> 941
   <211> 108
   <212> PRT
   <213> Artificial Sequence
<220>
   <223> Synthetic
<400> 941
   Glu Ile Val Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Thr Ser Asn Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Ser Glu Asp Phe Ala Val Tyr Tyr Cys Gln Gln Tyr Gly Ser Ser Pro
                   85                  90                  95
   Arg Thr Phe Gly Gln Gly Thr Lys Val Glu Ile Lys
               100                 105
<210> 942
   <211> 357
   <212> DNA
   <213> Artificial Sequence
<220>
   <223> Synthetic
<400> 942
   caggtgcagc tggtgcagtc tggacctgag gtgaagaagc ctggggcctc agtgaaggtc 60
   tcctgcaagg cttctggtta cacctttacc tactatggta tcagttggat acgacagacc 120
   cctggacaag ggcttgagtg gatgggatgg atcagcgctt acgatggtaa cacagactat 180
   gcacagaagt tccaagacag aatcaccatg accacagaca catcctcgac cacagcctac 240
   atggaactga ggagcctgag atctgacgac acggccgtct attactgtgc gaggtatagt 300
   tggaacaagc actggttcga cccctggggc cagggaaccc tggtcaccgt ctcttca    357
<210> 943
   <211> 119
   <212> PRT
   <213> Artificial Sequence
<220>
   <223> Synthetic
<400> 943
   Gln Val Gln Leu Val Gln Ser Gly Pro Glu Val Lys Lys Pro Gly Ala
    1               5                  10                  15
   Ser Val Lys Val Ser Cys Lys Ala Ser Gly Tyr Thr Phe Thr Tyr Tyr
               20                  25                  30
   Gly Ile Ser Trp Ile Arg Gln Thr Pro Gly Gln Gly Leu Glu Trp Met
           35                  40                  45
   Gly Trp Ile Ser Ala Tyr Asp Gly Asn Thr Asp Tyr Ala Gln Lys Phe
       50                  55                  60
   Gln Asp Arg Ile Thr Met Thr Thr Asp Thr Ser Ser Thr Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Tyr Ser Trp Asn Lys His Trp Phe Asp Pro Trp Gly Gln Gly
               100                 105                 110
   Thr Leu Val Thr Val Ser Ser
           115
<210> 944
   <211> 324
   <212> DNA
   <213> Artificial Sequence
<220>
   <223> Synthetic
<400> 944
   gaaattgtgt tgacacagtc tccaggcacc ctgtctttgt ctccagggga cagagccacc 60
   ctctcctgca gggccagtca gagtgttacc ggcagctact tagcctggta ccagcagaaa 120
   cctggccagg ctcccagact cctcatctat ggtgcatcca acagggccac tggcatccca 180
   gacaggttca ctggcagtgg gtctgggaca gacttcactc tcaccatcag cagactggag 240
   cctgaagatt ttgcagtgta tttctgtcaa cagtctgctt tctcaccgtg gacgttcggc 300
   caggggacca aggtggaaat caaa                                        324
<210> 945
   <211> 108
   <212> PRT
   <213> Artificial Sequence
<220>
   <223> Synthetic
<400> 945
   Glu Ile Val Leu Thr Gln Ser Pro Gly Thr Leu Ser Leu Ser Pro Gly
    1               5                  10                  15
   Asp Arg Ala Thr Leu Ser Cys Arg Ala Ser Gln Ser Val Thr Gly Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Asn Arg Ala Thr Gly Ile Pro Asp Arg Phe Thr
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Phe Cys Gln Gln Ser Ala Phe Ser Pro
                   85                  90                  95
   Trp Thr Phe Gly Gln Gly Thr Lys Val Glu Ile Lys
               100                 105
<210> 946
   <211> 361
   <212> DNA
   <213> Artificial Sequence
<220>
   <223> Synthetic
<400> 946
   caggtgcagt tgcaggagtc gggcccagga ctggtgaagc cttcacagac cctgtccctc 60
   acctgcactg tctctggtgg ctccatcggc agtggtggtt actactggag ctggatccgc 120
   cagcacccag ggaagggcct ggagtggatt gggtacgtcc attacagtgg gaacacccac 180
   tacaacccgt ccctcaagag tcgactttcc atatcaatag acacgtctaa gatccagttc 240
   tccctgaagc tgagctctgt gactgccgcg gacacggccg tgtattactg tgcgagagcc 300
   ccccgtggat accattactt tgcctactgg ggccagggaa ccctggtcac cgtctcctca 360
   g                                                                 361
<210> 947
   <211> 120
   <212> PRT
   <213> Artificial Sequence
<220>
   <223> Synthetic
<400> 947
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Gly Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Val His Tyr Ser Gly Asn Thr His Tyr Asn Pro Ser
       50                  55                  60
   Leu Lys Ser Arg Leu Ser Ile Ser Ile Asp Thr Ser Lys Ile Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Ala Pro Arg Gly Tyr His Tyr Phe Ala Tyr Trp Gly Gln
               100                 105                 110
   Gly Thr Leu Val Thr Val Ser Ser
           115                 120
<210> 948
   <211> 325
   <212> DNA
   <213> Artificial Sequence
<220>
   <223> Synthetic
<400> 948
   gaaattgtgt tgacacaatc tccaggcacc ctgtctttgt ctccagggga aagagccacc 60
   ctctcctgca gggccagtca gagtgttagc agcagctact tagcctggta ccagcagaaa 120
   cctggccagg ctcccaggct cctcatcttt ggtgcatcca gcagggccac tggcatccca 180
   gacaggttca gtggcagtgg gtctgggaca gacttcactc tcaccatcag cagactggag 240
   cctgaagatt ttgcagtgta ttactgtcag cagtatggta gctcaccgct cactttcggc 300
   ggagggacca aggtggagat caaac                                       325
<210> 949
   <211> 108
   <212> PRT
   <213> Artificial Sequence
<220>
   <223> Synthetic
<400> 949
   Glu Ile Val Leu Thr Gln Ser Pro Gly Thr Leu Ser Leu Ser Pro Gly
    1              5                  10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Phe Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gly Gly Thr Lys Val Glu Ile Lys
               100                 105
<210> 950
   <211> 330
   <212> PRT
   <213> Artificial Sequence
<220>
   <223> Synthetic
<400> 950
<210> 951
   <211> 327
<212> PRT
   <213> Artificial Sequence
<220>
   <223> Synthetic
<400> 951
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Lys Thr
   65 70 75 80
   Tyr Thr Cys Asn Val Asp His Lys Pro Ser Asn Thr Lys Val Asp Lys
   85 90 95
   Arg Val Glu Ser Lys Tyr Gly Pro Pro Cys Pro Ser Cys Pro Ala Pro
   100 105 110
   Glu Phe Leu Gly Gly Pro Ser Val Phe Leu Phe Pro Pro Lys Pro Lys
   115 120 125
   Asp Thr Leu Met Ile Ser Arg Thr Pro Glu Val Thr Cys Val Val Val
   130 135 140
   Asp Val Ser Gln Glu Asp Pro Glu Val Gln Phe Asn Trp Tyr Val Asp
   145 150 155 160
   Gly Val Glu Val His Asn Ala Lys Thr Lys Pro Arg Glu Glu Gln Phe
   165 170 175
   Asn Ser Thr Tyr Arg Val Val Ser Val Leu Thr Val Leu His Gln Asp
   180 185 190
   Trp Leu Asn Gly Lys Glu Tyr Lys Cys Lys Val Ser Asn Lys Gly Leu
   195 200 205
   Pro Ser Ser Ile Glu Lys Thr Ile Ser Lys Ala Lys Gly Gln Pro Arg
   210 215 220
   Glu Pro Gln Val Tyr Thr Leu Pro Pro Ser Gln Glu Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Arg Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu Gly Lys
   325
<210> 952
   <211> 327
   <212> PRT
   <213> Artificial Sequence
<220>
   <223> Synthetic
<400> 952
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
               35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 953
   <211> 544
   <212> PRT
   <213> Homo sapiens
<400> 953
   Met Gly Ser Arg Cys Ala Leu Ala Leu Ala Val Leu Ser Ala Leu Leu
    1              5                   10                  15
   Cys Gln Val Trp Ser Ser Gly Val Phe Glu Leu Lys Leu Gln Glu Phe
               20                  25                  30
   Val Asn Lys Lys Gly Leu Leu Gly Asn Arg Asn Cys Cys Arg Gly Gly
           35                  40                  45
   Ala Gly Pro Pro Pro Cys Ala Cys Arg Thr Phe Phe Arg Val Cys Leu
       50                  55                  60
   Lys His Tyr Gln Ala Ser Val Ser Pro Glu Pro Pro Cys Thr Tyr Gly
   65                  70                  75                  80
   Ser Ala Val Thr Pro Val Leu Gly Val Asp Ser Phe Ser Leu Pro Asp
                   85                  90                  95
   Gly Gly Gly Ala Asp Ser Ala Phe Ser Asn Pro Ile Arg Phe Pro Phe
               100                 105                 110
   Gly Phe Thr Trp Pro Gly Thr Phe Ser Leu Ile Ile Glu Ala Leu His
           115                 120                 125
   Thr Asp Ser Pro Asp Asp Leu Ala Thr Glu Asn Pro Glu Arg Leu Ile
       130                 135                 140
   Ser Arg Leu Ala Thr Gln Arg His Leu Thr Val Gly Glu Glu Trp Ser
   145                 150                 155                 160
   Gln Asp Leu His Ser Ser Gly Arg Thr Asp Leu Lys Tyr Ser Tyr Arg
   165 170 175
   Phe Val Cys Asp Glu His Tyr Tyr Gly Glu Gly Cys Ser Val Phe Cys
   180 185 190
   Arg Pro Arg Asp Asp Ala Phe Gly His Phe Thr Cys Gly Glu Arg Gly
   195 200 205
   Glu Lys Val Cys Asn Pro Gly Trp Lys Gly Pro Tyr Cys Thr Glu Pro
   210 215 220
   Ile Cys Leu Pro Gly Cys Asp Glu Gln His Gly Phe Cys Asp Lys Pro
   225 230 235 240
   Gly Glu Cys Lys Cys Arg Val Gly Trp Gln Gly Arg Tyr Cys Asp Glu
   245 250 255
   Cys Ile Arg Tyr Pro Gly Cys Leu His Gly Thr Cys Gln Gln Pro Trp
   260 265 270
   Gln Cys Asn Cys Gln Glu Gly Trp Gly Gly Leu Phe Cys Asn Gln Asp
   275 280 285
   Leu Asn Tyr Cys Thr His His Lys Pro Cys Lys Asn Gly Ala Thr Cys
   290 295 300
   Thr Asn Thr Gly Gln Gly Ser Tyr Thr Cys Ser Cys Arg Pro Gly Tyr
   305 310 315 320
   Thr Gly Ala Thr Cys Glu Leu Gly Ile Asp Glu Cys Asp Pro Ser Pro
   325 330 335
   Cys Lys Asn Gly Gly Ser Cys Thr Asp Leu Glu Asn Ser Tyr Ser Cys
   340 345 350
   Thr Cys Pro Pro Gly Phe Tyr Gly Lys Ile Cys Glu Leu Ser Ala Met
   355 360 365
   Thr Cys Ala Asp Gly Pro Cys Phe Asn Gly Gly Arg Cys Ser Asp Ser
   370 375 380
   Pro Asp Gly Gly Tyr Ser Cys Arg Cys Pro Val Gly Tyr Ser Gly Phe
   385 390 395 400
   Asn Cys Glu Lys Lys Ile Asp Tyr Cys Ser Ser Ser Pro Cys Ser Asn
   405 410 415
   Gly Ala Lys Cys Val Asp Leu Gly Asp Ala Tyr Leu Cys Arg Cys Gln
   420 425 430
   Ala Gly Phe Ser Gly Arg His Cys Asp Asp Asn Val Asp Asp Cys Ala
   435 440 445
   Ser Ser Pro Cys Ala Asn Gly Gly Thr Cys Arg Asp Gly Val Asn Asp
   450 455 460
   Phe Ser Cys Thr Cys Pro Pro Gly Tyr Thr Gly Arg Asn Cys Ser Ala
   465 470 475 480
   Pro Val Ser Arg Cys Glu His Ala Pro Cys His Asn Gly Ala Thr Cys
   485 490 495
   His Glu Arg Gly His Arg Tyr Val Cys Glu Cys Ala Arg Gly Tyr Gly
   500 505 510
   Gly Pro Asn Cys Gln Phe Leu Leu Pro Glu Leu Pro Pro Gly Pro Ala
   515 520 525
   Val Val Asp Leu Thr Glu Lys Leu Glu Gly Gln Gly Gly Pro Phe Pro
   530 535 540
<210> 954
   <211> 490
   <212> PRT
   <213> Homo sapiens
<400> 954
   Met Val Ser Pro Arg Met Ser Gly Leu Leu Ser Gln Thr Val Ile Leu
    1               5                  10                  15
   Ala Leu Ile Phe Leu Pro Gln Thr Arg Pro Ala Gly Val Phe Glu Leu
               20                  25                  30
   Gln Ile His Ser Phe Gly Pro Gly Pro Gly Pro Gly Ala Pro Arg Ser
           35                  40                  45
   Pro Cys Ser Ala Arg Leu Pro Cys Arg Leu Phe Phe Arg Val Cys Leu
       50                  55                  60
   Lys Pro Gly Leu Ser Glu Glu Ala Ala Glu Ser Pro Cys Ala Leu Gly
   65                  70                  75                  80
   Ala Ala Leu Ser Ala Arg Gly Pro Val Tyr Thr Glu Gln Pro Gly Ala
                   85                  90                  95
   Pro Ala Pro Asp Leu Pro Leu Pro Asp Gly Leu Leu Gln Val Pro Phe
               100                 105                 110
   Arg Asp Ala Trp Pro Gly Thr Phe Ser Phe Ile Ile Glu Thr Trp Arg
           115                 120                 125
   Glu Glu Leu Gly Asp Gln Ile Gly Gly Pro Ala Trp Ser Leu Leu Ala
       130                 135                 140
   Arg Val Ala Gly Arg Arg Arg Leu Ala Ala Gly Gly Pro Trp Ala Arg
   145                 150                 155                 160
   Asp Ile Gln Arg Ala Gly Ala Trp Glu Leu Arg Phe Ser Tyr Arg Ala
                   165                 170                 175
   Arg Cys Glu Pro Pro Ala Val Gly Thr Ala Cys Thr Arg Leu Cys Arg
               180                 185                 190
   Pro Arg Ser Ala Pro Ser Arg Cys Gly Pro Gly Leu Arg Pro Cys Ala
           195                 200                 205
   Pro Leu Glu Asp Glu Cys Glu Ala Pro Pro Val Cys Arg Ala Gly Cys
       210                 215                 220
   Ser Pro Glu His Gly Phe Cys Glu Gln Pro Gly Glu Cys Arg Cys Leu
   225                 230                 235                 240
   Glu Gly Trp Thr Gly Pro Leu Cys Thr Val Pro Val Ser Thr Ser Ser
                   245                 250                 255
   Cys Leu Ser Pro Arg Gly Pro Ser Ser Ala Thr Thr Gly Cys Leu Val
               260                 265                 270
   Pro Gly Pro Gly Pro Cys Asp Gly Asn Pro Cys Ala Asn Gly Gly Ser
           275                 280                 285
   Cys Ser Glu Thr Pro Arg Ser Phe Glu Cys Thr Cys Pro Arg Gly Phe
       290                 295                 300
   Tyr Gly Leu Arg Cys Glu Val Ser Gly Val Thr Cys Ala Asp Gly Pro
   305                 310                 315                 320
   Cys Phe Asn Gly Gly Leu Cys Val Gly Gly Ala Asp Pro Asp Ser Ala
                   325                 330                 335
   Tyr Ile Cys His Cys Pro Pro Gly Phe Gln Gly Ser Asn Cys Glu Lys
               340                 345                 350
   Arg Val Asp Arg Cys Ser Leu Gln Pro Cys Arg Asn Gly Gly Leu Cys
           355                 360                 365
   Leu Asp Leu Gly His Ala Leu Arg Cys Arg Cys Arg Ala Gly Phe Ala
       370                 375                 380
   Gly Pro Arg Cys Glu His Asp Leu Asp Asp Cys Ala Gly Arg Ala Cys
   385                 390                 395                 400
   Ala Asn Gly Gly Thr Cys Val Glu Gly Gly Gly Ala His Arg Cys Ser
                   405                 410                 415
   Cys Ala Leu Gly Phe Gly Gly Arg Asp Cys Arg Glu Arg Ala Asp Pro
               420                 425                 430
   Cys Ala Ala Arg Pro Cys Ala His Gly Gly Arg Cys Tyr Ala His Phe
           435                 440                 445
   Ser Gly Leu Val Cys Ala Cys Ala Pro Gly Tyr Met Gly Ala Arg Cys
       450                 455                 460
   Glu Phe Pro Val His Pro Asp Gly Ala Ser Ala Leu Pro Ala Ala Pro
   465                 470                 475                 480
   Pro Gly Leu Arg Pro Gly Asp Pro Gln Arg
                   485                 490

<210> 955
   <211> 527
   <212> PRT
   <213> Mus musculus
<400> 955
   Met Thr Pro Ala Ser Arg Ser Ala Cys Arg Trp Ala Leu Leu Leu Leu
    1               5                  10                  15
   Ala Val Leu Trp Pro Gln Gln Arg Ala Ala Gly Ser Gly Ile Phe Gln
               20                  25                  30
   Leu Arg Leu Gln Glu Phe Val Asn Gln Arg Gly Met Leu Ala Asn Gly
           35                  40                  45
   Gln Ser Cys Glu Pro Gly Cys Arg Thr Phe Phe Arg Ile Cys Leu Lys
       50                  55                  60
   His Phe Gln Ala Thr Phe Ser Glu Gly Pro Cys Thr Phe Gly Asn Val
   65                  70                  75                  80
   Ser Thr Pro Val Leu Gly Thr Asn Ser Phe Val Val Arg Asp Lys Asn
                   85                  90                  95
   Ser Gly Ser Gly Arg Asn Pro Leu Gln Leu Pro Phe Asn Phe Thr Trp
               100                 105                 110
   Pro Gly Thr Phe Ser Leu Asn Ile Gln Ala Trp His Thr Pro Gly Asp
           115                 120                 125
   Asp Leu Arg Pro Glu Thr Ser Pro Gly Asn Ser Leu Ile Ser Gln Ile
       130                 135                 140
   Ile Ile Gln Gly Ser Leu Ala Val Gly Lys Ile Trp Arg Thr Asp Glu
   145                 150                 155                 160
   Gln Asn Asp Thr Leu Thr Arg Leu Ser Tyr Ser Tyr Arg Val Ile Cys
                   165                 170                 175
   Ser Asp Asn Tyr Tyr Gly Glu Ser Cys Ser Arg Leu Cys Lys Lys Arg
               180                 185                 190
   Asp Asp His Phe Gly His Tyr Glu Cys Gln Pro Asp Gly Ser Leu Ser
           195                 200                 205
   Cys Leu Pro Gly Trp Thr Gly Lys Tyr Cys Asp Gln Pro Ile Cys Leu
       210                 215                 220
   Ser Gly Cys His Glu Gln Asn Gly Tyr Cys Ser Lys Pro Asp Glu Cys
   225                 230                 235                 240
   Ile Cys Arg Pro Gly Trp Gln Gly Arg Leu Cys Asn Glu Cys Ile Pro
                   245                 250                 255
   His Asn Gly Cys Arg His Gly Thr Cys Ser Ile Pro Trp Gln Cys Ala
               260                 265                 270
   Cys Asp Glu Gly Trp Gly Gly Leu Phe Cys Asp Gln Asp Leu Asn Tyr
           275                 280                 285
   Cys Thr His His Ser Pro Cys Lys Asn Gly Ser Thr Cys Ser Asn Ser
       290                 295                 300
   Gly Pro Lys Gly Tyr Thr Cys Thr Cys Leu Pro Gly Tyr Thr Gly Glu
   305                 310                 315                 320
   His Cys Glu Leu Gly Leu Ser Lys Cys Ala Ser Asn Pro Cys Arg Asn
                   325                 330                 335
   Gly Gly Ser Cys Lys Asp Gln Glu Asn Ser Tyr His Cys Leu Cys Pro
               340                 345                 350
   Pro Gly Tyr Tyr Gly Gln His Cys Glu His Ser Thr Leu Thr Cys Ala
           355                 360                 365
   Asp Ser Pro Cys Phe Asn Gly Gly Ser Cys Arg Glu Arg Asn Gln Gly
       370                 375                 380
   Ser Ser Tyr Ala Cys Glu Cys Pro Pro Asn Phe Thr Gly Ser Asn Cys
   385                 390                 395                 400
   Glu Lys Lys Val Asp Arg Cys Thr Ser Asn Pro Cys Ala Asn Gly Gly
                   405                 410                 415
   Gln Cys Gln Asn Arg Gly Pro Ser Arg Thr Cys Arg Cys Arg Pro Gly
               420                 425                 430
   Phe Thr Gly Thr His Cys Glu Leu His Ile Ser Asp Cys Ala Arg Ser
           435                 440                 445
   Pro Cys Ala His Gly Gly Thr Cys His Asp Leu Glu Asn Gly Pro Val
       450                 455                 460
   Cys Thr Cys Pro Ala Gly Phe Ser Gly Arg Arg Cys Glu Val Arg Ile
   465                 470                 475                 480
   Thr His Asp Ala Cys Ala Ser Gly Pro Cys Phe Asn Gly Ala Thr Cys
                   485                 490                 495
   Tyr Thr Gly Leu Ser Pro Asn Asn Phe Val Cys Asn Cys Pro Tyr Gly
               500                 505                 510
   Phe Val Gly Ser Arg Cys Glu Phe Pro Val Gly Leu Pro Pro Ser
           515                 520                 525
<210> 956
   <211> 498
   <212> PRT
   <213> Macaca fascicularis
<400> 956
   Ser Gly Val Phe Gln Leu Gln Leu Gln Glu Phe Val Asn Glu Arg Gly
   1 5 10 15
   Val Leu Ala Ser Gly Arg Pro Cys Glu Pro Gly Cys Arg Thr Phe Phe
   20 25 30
   Arg Val Cys Leu Lys His Phe Gln Ala Val Val Ser Pro Gly Pro Cys
   35 40 45
   Thr Phe Gly Ser Val Ser Thr Pro Val Leu Gly Thr Asn Ser Phe Ala
   50 55 60
   Val Arg Asp Asp Ser Ser Gly Gly Gly Arg Asn Pro Leu Gln Leu Pro
   65 70 75 80
   Phe Asn Phe Thr Trp Pro Gly Thr Phe Ser Leu Ile Ile Glu Ala Trp
   85 90 95
   His Ala Pro Gly Asp Asp Leu Arg Pro Glu Ala Leu Pro Pro Asp Ala
   100 105 110
   Leu Ile Ser Lys Ile Ala Ile Gln Gly Ser Leu Ala Val Gly Gln Asn
   115 120 125
   Trp Leu Leu Asp Glu Gln Thr Ser Thr Leu Thr Arg Leu Arg Tyr Ser
   130 135 140
   Tyr Arg Val Ile Cys Ser Asp Asn Tyr Tyr Gly Asp Asn Cys Ser Arg
   145 150 155 160
   Leu Cys Lys Lys Arg Asn Asp His Phe Gly His Tyr Val Cys Gln Pro
   165 170 175
   Asp Gly Asn Leu Ser Cys Leu Pro Gly Trp Thr Gly Glu Tyr Cys Gln
   180 185 190
   Gln Pro Ile Cys Leu Ser Gly Cys His Glu Gln Asn Gly Tyr Cys Ser
   195 200 205
   Lys Pro Ala Glu Cys Leu Cys Arg Pro Gly Trp Gln Gly Arg Leu Cys
   210 215 220
   Asn Glu Cys Ile Pro His Asn Gly Cys Arg His Gly Thr Cys Ser Thr
   225 230 235 240
   Pro Trp Gln Cys Thr Cys Asp Glu Gly Trp Gly Gly Leu Phe Cys Asp
   245 250 255
   Gln Asp Leu Asn Tyr Cys Thr His His Ser Pro Cys Lys Asn Gly Ala
   260 265 270
   Thr Cys Ser Asn Ser Gly Gln Arg Ser Tyr Thr Cys Thr Cys Arg Pro
   275 280 285
   Gly Tyr Thr Gly Val Asp Cys Glu Leu Glu Leu Ser Glu Cys Asp Ser
   290 295 300
   Asn Pro Cys Arg Asn Gly Gly Ser Cys Lys Asp Gln Glu Asp Gly Tyr
   305 310 315 320
   His Cys Leu Cys Pro Pro Gly Tyr Tyr Gly Leu His Cys Glu His Ser
   325 330 335
   Thr Leu Ser Cys Ala Asp Ser Pro Cys Phe Asn Gly Gly Ser Cys Arg
   340 345 350
   Glu Arg Asn Gln Gly Ala Ser Tyr Ala Cys Glu Cys Pro Pro Asn Phe
   355 360 365
   Thr Gly Ser Asn Cys Glu Lys Lys Val Asp Arg Cys Thr Ser Asn Pro
   370 375 380
   Cys Ala Asn Gly Gly Gln Cys Leu Asn Arg Gly Pro Ser Arg Met Cys
   385 390 395 400
   Arg Cys Arg Pro Gly Phe Thr Gly Thr Tyr Cys Glu Arg His Val Ser
   405 410 415
   Asp Cys Ala Arg Asn Pro Cys Ala His Gly Gly Thr Cys His Asp Leu
   420 425 430
   Glu Ser Gly Leu Met Cys Thr Cys Pro Ala Gly Phe Ser Gly Arg Arg
   435 440 445
   Cys Glu Val Arg Thr Ser Ile Asp Ala Cys Ala Ser Ser Pro Cys Phe
   450 455 460
   Asn Arg Ala Thr Cys Tyr Thr Asp Leu Ser Thr Asp Thr Phe Val Cys
   465 470 475 480
   Asn Cys Pro Tyr Gly Phe Val Gly Ser Arg Cys Glu Phe Pro Met Gly
   485 490 495
   Leu Pro
<210> 957
   <211> 498
   <212> PRT
   <213> Macaca mulatta
<400> 957
   Ser Gly Val Phe Gln Leu Gln Leu Gln Glu Phe Val Asn Glu Arg Gly
   1               5                   10                  15
   Val Leu Ala Ser Gly Arg Pro Cys Glu Pro Gly Cys Arg Thr Phe Phe
               20                  25                  30
   Arg Val Cys Leu Lys His Phe Gln Ala Val Val Ser Pro Gly Pro Cys
           35                  40                  45
   Thr Phe Gly Ser Val Ser Thr Pro Val Leu Gly Thr Asn Ser Phe Ala
       50                  55                  60
   Val Arg Asp Asp Ser Ser Gly Gly Gly Arg Asn Pro Leu Gln Leu Pro
   65                  70                  75                  80
   Phe Asn Phe Thr Trp Pro Gly Thr Phe Ser Leu Ile Ile Glu Ala Trp
                   85                  90                  95
   His Ala Pro Gly Asp Asp Leu Arg Pro Glu Ala Leu Pro Pro Asp Ala
               100                 105                 110
   Leu Ile Ser Lys Ile Ala Ile Gln Gly Ser Leu Ala Val Gly Gln Asn
           115                 120                 125
   Trp Leu Leu Asp Glu Gln Thr Ser Thr Leu Thr Arg Leu Arg Tyr Ser
       130                 135                 140
   Tyr Arg Val Ile Cys Ser Asp Asn Tyr Tyr Gly Asp Asn Cys Ser Arg
   145                 150                 155                 160
   Leu Cys Lys Lys Arg Asn Asp His Phe Gly His Tyr Val Cys Gln Pro
                   165                 170                 175
   Asp Gly Asn Leu Ser Cys Leu Pro Gly Trp Thr Gly Glu Tyr Cys Gln
               180                 185                 190
   Gln Pro Ile Cys Leu Ser Gly Cys His Glu Gln Asn Gly Tyr Cys Ser
           195                 200                 205
   Lys Pro Ala Glu Cys Leu Cys Arg Pro Gly Trp Gln Gly Arg Leu Cys
       210                 215                 220
   Asn Glu Cys Ile Pro His Asn Gly Cys Arg His Gly Thr Cys Ser Thr
   225                 230                 235                 240
   Pro Trp Gln Cys Thr Cys Asp Glu Gly Trp Gly Gly Leu Phe Cys Asp
                   245                 250                 255
   Gln Asp Leu Asn Tyr Cys Thr His His Ser Pro Cys Lys Asn Gly Ala
               260                 265                 270
   Thr Cys Ser Asn Ser Gly Gln Arg Ser Tyr Thr Cys Thr Cys Arg Pro
           275                 280                 285
   Gly Tyr Thr Gly Val Asp Cys Glu Leu Glu Leu Ser Glu Cys Asp Ser
       290                 295                 300
   Asn Pro Cys Arg Asn Gly Gly Ser Cys Lys Asp Gln Glu Asp Gly Tyr
   305                 310                 315                 320
   His Cys Leu Cys Pro Pro Gly Tyr Tyr Gly Leu His Cys Glu His Ser
                   325                 330                 335
   Thr Leu Ser Cys Ala Asp Ser Pro Cys Phe Asn Gly Gly Ser Cys Arg
               340                 345                 350
   Glu Arg Asn Gln Gly Ala Ser Tyr Ala Cys Glu Cys Pro Pro Asn Phe
           355                 360                 365
   Thr Gly Ser Asn Cys Glu Lys Lys Val Asp Arg Cys Thr Ser Asn Pro
       370                 375                 380
   Cys Ala Asn Gly Gly Gln Cys Leu Asn Arg Gly Pro Ser Arg Met Cys
   385                 390                 395                 400
   Arg Cys Arg Pro Gly Phe Thr Gly Thr Tyr Cys Glu Arg His Val Ser
                   405                 410                 415
   Asp Cys Ala Arg Asn Pro Cys Ala His Gly Gly Thr Cys His Asp Leu
                   420                     425                     430
   Glu Ser Gly Leu Met Cys Thr Cys Pro Ala Gly Phe Ser Gly Arg Arg
               435                     440                     445
   Cys Glu Val Arg Thr Ser Ile Asp Ala Cys Ala Ser Ser Pro Cys Phe
           450                     455                     460
   Asn Arg Ala Thr Cys Tyr Thr Asp Leu Ser Thr Asp Thr Phe Val Cys
   465                     470                     475                     480
   Asn Cys Pro Tyr Gly Phe Val Gly Ser Arg Cys Glu Phe Pro Val Gly
                       485                     490                     495
   Leu Pro

## Description

### BACKGROUND

The Notch-signaling pathway is a system for cell-to-cell communication used by a wide range of eukaryotes for many biological processes, such as differentiation, proliferation, and homeostasis. Delta like 4 (DI4) or delta-like ligand 4 (DII4) (hereinafter "DII4") is a member of the Delta family of Notch ligands which exhibits highly selective expression by vascular endothelium (Shutter et al. (2000) Genes Develop. 14:1313-1318). DII4 is a ligand for Notch receptors, including Notch1 and Notch 4. The nucleic acid and amino acid sequences for human DII4 are shown in SEQ ID NO:1-2, respectively.

Methods to produce antibodies useful as human therapeutics include generation of chimeric antibodies and humanized antibodies (see, for example, U.S. 6,949,245). See, for example, WO 94/02602 (Abgenix) and U.S. 6,596,541 (Regeneron Pharmaceuticals) describing methods of generating nonhuman transgenic mice capable of producing human antibodies. Hainaud et al (Cancer Research 2006 66 : (17)) disclose the role of DLL4 in tumor vessel remodeling.

Japanese patent application 2003/047470A2 (Asahi Kasei Kogyo) describes antibodies to the extracellular portion of human Notch ligand protein.

### BRIEF SUMMARY OF THE INVENTION

In a first aspect, the invention provides human antibodies, preferably recombinant human antibodies, that specifically bind human delta-like ligand 4 (hDII4). The antibody of the invention is a human antibody or antibody fragment which specifically binds human delta-like ligand 4 (hDII4), said antibody or antibody fragment comprising a heavy chain variable region/light chain variable region (HCVR/LCVR) selected from the amino acid sequence pairs of SEQ ID NO: 429/437 and 901/903. These antibodies are characterized by binding to hDII4 with high affinity and by the ability to neutralize DII4 activity. The antibodies of the invention are capable of blocking DII4 binding to its Notch receptor(s), and thus inhibit signaling by DII4. The antibodies can be full-length (for example, an IgG1 or IgG4 antibody) or may comprise only an antigen-binding portion (for example, a Fab, F(ab')₂ or scFv fragment), and may be modified to effect functionality, e.g., to eliminate residual effector functions (Glu which eliminates residual effector functions (Reddy et al. (2000) J. Immunol. 164:1925-1933).
The invention further provides an isolated nucleic acid molecule encoding an antibody or antigen-binding fragment of the invention and a vector comprising said nucleic acid molecule.
The invention additionally provides a host cell comprising a vector of the invention. In a related aspect, the invention also provides a method for producing an anti-human DII4 antibody or antigen-binding fragment thereof, comprising growing host cells of the invention under conditions permitting production of the antibody or fragment thereof and recovering the antibody or fragment so produced.
The invention further provides use of an antibody or antigen-binding fragment of an antibody of the invention in the manufacture of a medicament for treating cancer in a human.
The invention also provides an antibody or antibody fragment of the invention for use in a method of treating cancer in a human patient.

Also described herein is an antibody comprising a heavy chain variable region (HCVR) selected from the group consisting of SEQ ID NO:4, 20, 36, 52, 68, 84, 100, 116, 132, 148, 164, 180, 196, 212, 228, 244, 260, 276, 292, 308, 324, 340, 356, 372, 397, 413, 429, 445, 461, 477, 493, 509, 525, 541, 557, 573, 589, 605, 621, 637, 653, 669, 685, 701, 717, 733, 749, 765, 781, 797, 813, 893, 897, 901, 905, 909, 913, 917, 921, 925, 935, 939, 943, and 947 or a substantially identical sequence thereof. In the invention, the HCVR is the amino acid sequence of SEQ ID NO:429 or 901.

Additionally described herein is an antibody comprising a light chain variable region (LCVR) selected from the group consisting of SEQ ID NO:12, 28, 44, 60, 76, 92, 108, 124, 140, 156, 172, 188, 204, 220, 236, 252, 268, 284, 300, 316, 332, 348, 364, 380, 405, 421, 437, 453, 469, 485, 501, 517, 533, 549, 565, 581, 597, 613, 629, 645, 661, 677, 693, 709, 725, 741, 757, 773, 789, 805, 821, 895, 899, 903, 907, 911, 915, 919, 923, 927, 937, 941, 945, and 949 or a substantially identical sequence thereof. In the invention, the LCVR is the amino acid sequence of SEQ ID NO:437 or 903.

Further described herein is an antibody comprising a HCVR selected from the group consisting of SEQ ID NO: 4, 20, 36, 52, 68, 84, 100, 116, 132, 148, 164, 180, 196, 212, 228, 244, 260, 276, 292, 308, 324, 340, 356, 372, 397, 413, 429, 445, 461, 477, 493, 509, 525, 541, 557, 573, 589, 605, 621, 637, 653, 669, 685, 701, 717, 733, 749, 765, 781, 797, 813, 893, 897, 901, 905, 909, 913, 917, 921, 925, 935, 939, 943, and 947 or a substantially identical sequence thereof, and a LCVR selected from the group consisting of SEQ ID NO:12, 28, 44, 60, 76, 92, 108, 124, 140, 156, 172, 188, 204, 220, 236, 252, 268, 284, 300, 316, 332, 348, 364, 380, 405, 421, 437, 453, 469, 485, 501, 517, 533, 549, 565, 581, 597, 613, 629, 645, 661, 677, 693, 709, 725, 741, 757, 773, 789, 805, 821, 895, 899, 903, 907, 911, 915, 919, 923, 927, 937, 941, 945, and 949 or a substantially identical sequence thereof. In a preferred embodiment of the invention, the HCVR/LCVR are the amino acid sequence pairs SEQ ID NO:429/437or 901/903.

Also described herein is a human antibody or antibody fragment comprising a heavy chain complementary determining region 1 (CDR1) selected from the group consisting of SEQ ID NO:6, 22, 38, 54, 70, 86, 102, 118, 134, 150, 166, 182, 198, 214, 230, 246, 262, 278 294, 310, 326, 342, 358, 374, 399, 415, 431, 447, 463, 479, 495, 511, 527, 543, 559, 575, 591, 607, 623, 639, 655, 671, 687, 703, 711119, 735, 751, 767, 783, 799, 815, 831, 847, 863 and 879, or a substantially identical sequence thereof.

Additionally described herein is a human antibody or antibody fragment comprising a heavy chain CDR2 selected from the group consisting of SEQ ID NO:8, 24, 40, 56, 72, 88, 104, 120, 136, 152, 168, 184, 200, 216, 232, 248, 264, 280, 296, 312, 328, 344, 360, 376, 401, 417, 433, 449, 465, 481, 497, 513, 529, 545, 561, 577, 593, 609, 625, 641, 657, 673, 689, 705, 721, 737, 753, 769, 785, 801, 817, 833, 849, 865 and 881, or a substantially identical sequence thereof.

Further described herein is a human antibody or antibody fragment comprising a heavy chain CDR3 selected from the group consisting of SEQ ID NO:10, 26, 42, 58, 74, 90, 106, 122, 138, 154, 170, 186, 202, 218, 234, 250, 266, 282, 298, 314, 330, 346, 362, 378, 403, 419, 435, 451, 467, 483, 499, 515, 531, 547, 563, 579, 595, 611, 627, 643, 659, 675, 691, 707, 723, 739, 755, 771, 787, 803, 819, 835, 851, 867 and 883, or a substantially identical sequence thereof.

Also described herein is a human antibody or antibody fragment comprising a heavy chain CDR1 selected from the group consisting of SEQ ID NO: 6, 22, 38, 54, 70, 86. 102, 118, 134, 150, 166, 182, 198, 214, 230, 246, 262, 278 294, 310, 326, 342, 358, 374, 399, 415, 431, 447, 463, 479, 495, 511, 527, 543, 559, 575, 591, 607, 623, 639, 655, 671, 687, 703, 711119, 735, 751, 767, 783, 799, 815, 831, 847, 863 and 879, or a substantially identical sequence thereof; a heavy chain CDR2 selected from the group consisting of SEQ ID NO: 8, 24, 40, 56, 72, 88, 104, 120, 136, 152, 168, 184, 200, 216, 232, 248, 264, 280, 296, 312, 328, 344, 360, 376, 401, 417, 433, 449, 465, 481, 497, 513, 529, 545, 561, 577, 593, 609, 625, 641, 657, 673, 689, 705, 721, 737, 753, 769, 785, 801, 817, 833, 849, 865 and 881, or a substantially identical sequence thereof; and a heavy chain CDR3 selected from the group consisting of SEQ ID NO: 10, 26, 42, 58, 74, 90, 106, 122, 138, 154, 170, 186, 202, 218, 234, 250, 266, 282, 298, 314, 330, 346, 362, 378, 403, 419, 435, 451, 467, 483, 499, 515, 531, 547, 563, 579, 595, 611, 627, 643, 659, 675, 691, 707, 723, 739, 755, 771, 787, 803, 819, 835, 851, 867 and 883, or a substantially identical sequence thereof. The antibody or antibody fragment may comprise heavy chain CDR1, CDR2 and CDR3 selected from the group consisting of SEQ ID NO:431/433/435; 374/376/378; 783/785/787; and 799/801/803.

Additionally described herein is a human antibody or antibody fragment comprising a light chain CDR1 selected from the group consisting of SEQ ID NO: 14, 30, 46, 62, 78, 94, 110, 126, 142, 158, 174, 190, 206, 222, 238, 254, 270, 286, 302, 318, 334, 350, 366, 382, 407, 423, 439, 455, 471, 487, 503, 519, 535, 551, 567, 583, 599, 615, 631, 647, 663, 679, 695, 711, 727, 743, 759, 775, 791, 807, 823, 839, 855, 871 and 887, or a substantially identical sequence thereof.

Also described herein is a human antibody or antibody fragment comprising a light chain CDR2 selected from the group consisting of SEQ ID N0:16, 32, 48, 64, 80, 96, 112, 128, 144, 160, 176, 192, 208, 224, 240, 256, 272, 288, 304, 320, 336, 352, 368, 384, 409. 425, 441, 457, 473, 489, 505, 521, 537, 553, 569, 585, 601, 617, 633, 649, 665, 681, 697, 713, 729, 745, 761, 777, 793, 809, 825, 841, 857, 873 and 889, or a substantially identical sequence thereof.

Further described herein is a human antibody or antibody fragment comprising a light chain CDR3 selected from the group consisting of SEQ ID NO:18, 34, 50, 66, 82, 98, 11, 130, 146, 162, 178, 194, 210, 226, 242, 258, 274, 290, 306, 322, 338, 354, 370, 386, 411, 427, 443, 459, 475, 491, 507, 523, 539, 555, 571, 587, 603, 619, 635, 651, 667, 683, 699, 715, 731, 747, 763, 779, 795, 811, 827, 843, 859, 875 and 891, or a substantially identical sequence thereof.

Additionally described herein is a human antibody or antibody fragment comprising a light chain CDR1 selected from the group consisting of SEQ ID NO: 14, 30, 46, 62, 78, 94, 110, 126, 142, 158, 174, 190, 206, 222, 238, 254, 270, 286, 302, 318, 334, 350, 366, 382, 407, 423, 439, 455, 471, 487, 503, 519, 535, 551, 567, 583, 599, 615, 631, 647, 663, 679, 695, 711, 727, 743, 759, 775, 791, 807, 823, 839, 855, 871 and 887, or a substantially identical sequence thereof; a light chain CDR2 selected from the group consisting of SEQ ID NO: 16, 32, 48, 64, 80, 96, 112, 128, 144, 160, 176, 192, 208, 224, 240, 256, 272, 288, 304, 320, 336, 352, 368, 384, 409. 425, 441, 457, 473, 489, 505, 521, 537, 553, 569, 585, 601, 617, 633, 649, 665, 681, 697, 713, 729, 745, 761, 777, 793, 809, 825, 841, 857, 873 and 889, or a substantially identical sequence thereof; and a light chain CDR3 selected from the group consisting of SEQ ID NO: 18, 34, 50, 66, 82, 98, 11, 130, 146, 162, 178, 194, 210, 226, 242, 258, 274, 290, 306, 322, 338, 354, 370, 386, 411, 427, 443, 459, 475, 491, 507, 523, 539, 555, 571, 587, 603, 619, 635, 651, 667, 683, 699, 715, 731, 747, 763, 779, 795, 811, 827, 843, 859, 875 and 891, or a substantially identical sequence thereof. The antibody or antibody fragment may comprise the light chain CDR1, CDR2 and CDR3 selected from the group consisting of SEQ ID NO:439/441/443; 382/384/386; 791/793/795; and 807/809/811.

The invention provides nucleic acid molecules encoding the antibodies, or antigen-binding portions, of the invention. Recombinant expression vectors carrying the antibody-encoding nucleic acids of the invention, and host cells into which such vectors have been introduced, are also described herein, as are methods of making the antibodies of the invention by culturing the host cells of the invention.

Also described herein is an antibody which comprises a HCVR encoded by a nucleotide sequence selected from the group consisting of SEQ ID NO:3, 19, 35, 51, 67, 83, 99, 115, 131, 147, 163, 179, 195, 211, 227, 243, 259, 275, 291, 307, 323, 339, 355, 371, 396, 412, 428, 444, 460, 476, 492, 508, 524, 540, 556, 572, 588, 604, 620, 636, 652, 668, 684, 700, 716, 732, 748, 764, 780, 796, 812, 892, 896, 900, 904, 908, 912, 916, 920, 924, 934, 938, 942, and 946 or a substantially similar sequence having at least 95% homology thereof.

Further described herein is an antibody which comprises a LCVR encoded by a nucleotide sequence selected from the group consisting of SEQ ID NO:11, 27, 43, 59, 75, 91, 107, 123, 139, 155, 171, 187, 203, 219, 235, 251, 267, 283, 299, 315, 331, 347, 363, 379, 404, 420, 436, 452, 468, 484, 500, 516, 532, 548, 564, 580, 596, 612, 628, 644, 660, 676, 692, 708, 724, 740, 756, 772, 788, 804, 820, 894, 898, 902, 906, 910, 914, 918, 922, 926, 936, 940, 944, and 948 or a substantially similar sequence having at least 95% homology thereof.

Additionally described herein is an antibody which comprises a HCVR encoded by a nucleotide sequence selected from the group consisting of SEQ ID NO: 3, 19, 35, 51, 67, 83, 99, 115, 131, 147, 163, 179, 195, 211, 227, 243, 259, 275, 291, 307, 323, 339, 355, 371, 396, 412, 428, 444, 460, 476, 492, 508, 524, 540, 556, 572, 588, 604, 620, 636, 652, 668, 684, 700, 716, 732, 748, 764, 780, 796, 812, 892, 896, 900, 904, 908, 912, 916, 920, 924, 934, 938, 942, and 946 or a substantially similar sequence having at least 95% homology thereof, and a LCVR encoded by a nucleotide sequence selected from the group consisting of SEQ ID NO: 11, 27, 43, 59, 75, 91, 107, 123, 139, 155, 171, 187, 203, 219, 235, 251, 267, 283, 299, 315, 331, 347, 363, 379, 404, 420, 436, 452, 468, 484, 500, 516, 532, 548, 564, 580, 596, 612, 628, 644, 660, 676, 692, 708, 724, 740, 756, 772, 788, 804, 820, 894, 898, 902, 906, 910, 914, 918, 922, 926, 936, 940, 944, and 948 or a substantially similar sequence having at least 95% homology thereof.

Further described herein is a human antibody or antibody fragment comprising a heavy chain CDR1 encoded by a nucleotide sequence selected from the group consisting of SEQ ID NO:5, 21, 37, 53, 69, 85, 101, 117, 133, 149, 165, 181, 197, 213, 229, 245, 261, 277, 293, 309, 325, 341, 357, 373, 398, 414, 430, 446, 462, 478, 494, 510, 526, 542, 558, 574, 590, 606, 622, 638, 654, 670, 686, 702, 718, 734, 750, 766, 782, 798, 814, 830, 846, 862 and 878, or a substantially similar sequence having at least 95% homology thereof.

Also described herein is a human antibody or antibody fragment comprising a heavy chain CDR2 encoded by a nucleotide sequence selected from the group consisting of SEQ ID NO:7, 23, 39, 55, 71, 87, 103, 119, 135, 151, 167, 183, 100, 215, 231, 247, 263, 279, 295, 311, 327, 343, 359, 375, 400, 416, 432, 448, 464, 480, 496, 512, 528, 544, 560, 576, 592, 608, 624, 640, 656, 672, 688, 704, 720, 736, 752, 768, 784, 800, 816, 832, 848, 864 and 880, or a substantially similar sequence having at least 95% homology thereof.

Additionally described herein is a human antibody or antibody fragment comprising a heavy chain CDR3 encoded by a nucleotide sequence selected from the group consisting of SEQ ID NO:9, 25, 41, 57, 73, 89, 105, 121, 137, 153, 169, 185, 201, 217, 233, 249, 265, 281, 297, 313, 329, 345, 361, 377, 402, 418, 434, 450, 466, 482, 498, 514, 530, 546, 562, 578, 594, 610, 626, 642, 658, 674, 690, 706, 722, 738, 754, 770, 786, 802, 818, 834, 850, 866 and 882, or a substantially similar sequence having at least 95% homology thereof.

Further described herein is a human antibody or antibody fragment comprising a heavy chain CDR1 encoded by a nucleotide sequence selected from the group consisting of SEQ ID NO:5, 21, 37, 53, 69, 85, 101, 117, 133, 149, 165, 181, 197, 213, 229, 245, 261, 277, 293, 309, 325, 341, 357, 373, 398, 414, 430, 446, 462, 478, 494, 510, 526, 542, 558, 574, 590, 606, 622, 638, 654, 670, 686, 702, 718, 734, 750, 766, 782, 798, 814, 830, 846, 862 and 878, or a substantially similar sequence having at least 95% homology thereof; a heavy chain CDR2 encoded by a nucleotide sequence selected from the group consisting of SEQ ID NO:7, 23, 39, 55, 71, 87, 103, 119, 135, 151, 167, 183, 100, 215, 231, 247, 263, 279, 295, 311, 327, 343, 359, 375, 400, 416, 432, 448, 464, 480, 496, 512, 528, 544, 560, 576, 592, 608, 624, 640, 656, 672, 688, 704, 720, 736, 752, 768, 784, 800, 816, 832, 848, 864 and 880, or a substantially similar sequence having at least 95% homology thereof; and a heavy chain CDR3 encoded by a nucleotide sequence selected from the group consisting of SEQ ID NO: 9, 25, 41, 57, 73, 89, 105, 121, 137, 153, 169, 185, 201, 217, 233, 249, 265, 281, 297, 313, 329, 345, 361, 377, 402, 418, 434, 450, 466, 482, 498, 514, 530, 546, 562, 578, 594, 610, 626, 642, 658, 674, 690, 706, 722, 738, 754, 770, 786, 802, 818, 834, 850, 866 and 882, or a substantially similar sequence having at least 95% homology thereof. The antibody or antibody fragment may comprise heavy chain CDR1, CDR2 and CDR3 encoded by a nucleic acid sequence selected from the group consisting of SEQ ID NO:430/432/434; 373/375/377; 782/784/786; and 798/800/802.

Also described herein is a human antibody or antibody fragment comprising a light chain CDR1 encoded by a nucleotide sequence selected from the group consisting of SEQ ID NO:13, 29, 45, 61, 77, 93, 109, 125, 141, 157, 173, 189, 205, 221, 237, 253, 269, 285, 301, 317, 333, 349, 365, 381, 406, 422, 438, 454, 470, 486, 502, 518, 534, 550, 566, 582, 598, 614, 630, 646, 662, 678, 694, 710, 726, 742, 758, 774, 790, 806, 822, 838, 854, 870 and 886, or a substantially similar sequence having at least 95% homology thereof.

Additionally described herein is a human antibody or antibody fragment comprising a light chain CDR2 encoded by a nucleotide sequence selected from the group consisting of SEQ ID NO:15, 31, 47, 63, 79, 95, 111, 127, 143, 159, 175, 191, 207, 223, 239, 255, 271, 287, 303, 319, 335, 351, 367, 383, 408, 424, 440, 456, 472, 488, 504, 520, 536, 552, 568, 584, 600, 616, 632, 648, 664, 680, 696, 712, 728, 744, 760, 776, 792, 808, 824, 840, 856, 872, and 888, or a substantially similar sequence having at least 95% homology thereof.

Further described herein is a human antibody or antibody fragment comprising a light chain CDR3 encoded by a nucleotide sequence selected from the group consisting of SEQ ID NO:17, 33, 49, 65, 81, 97, 113, 129, 145, 161, 177, 193, 209, 225, 241, 257, 273, 289, 305, 321, 337, 353, 369, 385, 410, 426, 442, 458, 474, 490, 506, 522, 538, 554, 570, 586, 602, 618, 634, 650, 666, 682, 698, 714, 730, 746, 762, 778, 794, 810, 826, 842, 858, 874 and 890, or a substantially similar sequence having at least 95% homology thereof.

Also described herein is a human antibody or antibody fragment comprising a light chain CDR1 encoded by a nucleotide sequence selected from the group consisting of SEQ ID NO:13, 29, 45, 61, 77, 93, 109, 125, 141, 157, 173, 189, 205, 221, 237, 253, 269, 285, 301, 317, 333, 349, 365, 381, 406, 422, 438, 454, 470, 486, 502, 518, 534, 550, 566, 582, 598, 614, 630, 646, 662, 678, 694, 710, 726, 742, 758, 774, 790, 806, 822, 838, 854, 870 and 886, or a substantially similar sequence having at least 95% homology thereof; a light chain CDR2 encoded by a nucleotide sequence selected from the group consisting of SEQ ID NO: 15, 31, 47, 63, 79, 95, 111, 127, 143, 159, 175, 191, 207, 223, 239, 255, 271, 287, 303, 319, 335, 351, 367, 383, 408, 424, 440, 456, 472, 488, 504, 520, 536, 552, 568, 584, 600, 616, 632, 648, 664, 680, 696, 712, 728, 744, 760, 776, 792, 808, 824, 840, 856, 872, and 888, or a substantially similar sequence having at least 95% homology thereof; and a light chain CDR3 encoded by a nucleotide sequence selected from the group consisting of SEQ ID NO: 17, 33, 49, 65, 81, 97, 113, 129, 145, 161, 177, 193, 209, 225, 241, 257, 273, 289, 305, 321, 337, 353, 369, 385, 410, 426, 442, 458, 474, 490, 506, 522, 538, 554, 570, 586, 602, 618, 634, 650, 666, 682, 698, 714, 730, 746, 762, 778, 794, 810, 826, 842, 858, 874 and 890, or a substantially similar sequence having at least 95% homology thereof. The antibody or antibody fragment may comprise the light chain CDR1, CDR2 and CDR3 encoded by a nucleic acid sequence selected from the group consisting of SEQ ID NO:438/440/442; 381/383/385; 790/792/794; and 806/808/810.

Additionally described herein is an isolated antibody or antibody fragment that specifically binds hDII4, comprising a CDR 1, 2 and 3 selected from the group consisting of (a) a heavy chain CDR1 region comprising an amino acid sequence of the formula X¹ - X² - X³ - X⁴ - X⁵ - X⁶ - X⁷ - X⁸ (SEQ ID NO:928), wherein X¹ is Gly; X² is Phe or Tyr; X³ is Thr; X⁴ is Phe; X⁵ is Ser, Thr or Asn; X⁶ is Ser, Asn or Tyr; X⁷ is Tyr or Phe; and X⁸ is Gly or Ala; (b) a heavy chain CDR2 region comprising an amino acid sequence of the formula X¹ - X² - X³ - X⁴ - X⁵ - X⁶ - X⁷ - X⁸ (SEQ ID NO:929), wherein X¹ is Ile or Leu; X² is Trp or Ser; X³ is Tyr, Ala or Gly; X⁴ is Asp, Ser or Tyr; X⁵ is Gly or Asp; X⁶ is Ser, Gly, Thr or Val; X⁷ is Asn or Asp; and X⁸ is Lys or Arg; (c) a heavy chain CDR3 region comprising an amino acid sequence of the formula X¹ - X² - X³ - X⁴ - X⁵ - X⁶ - X⁷ - X⁸ - X⁹ - X¹⁰ - X¹¹ - X¹² - X¹³ - X¹⁴ - X¹⁵ - X¹⁶ (SEQ ID NO:930), wherein X¹ is Ala or Ser; X² is Arg or Lys; X³ is Asp or Tyr; X⁴ is Ser, Gly or His; X⁵ is Asp, Ala or Trp; X⁶ is Asn, or Phe; X⁷ is Tyr, Arg or Lys; X⁸ is His or Ser; X⁹ is Gly or Trp; X¹⁰ is Tyr or Phe; X¹¹ is Glu or Asp; X¹² is Gly, His or Pro; X¹³ is Tyr, Trp or absent; X¹⁴ is Phe or absent; X¹⁵ is Asp or absent; and X¹⁶ is Pro or absent.

An antibody or antibody fragment described herein may comprise heavy chain CDR 1, 2 and 3 selected from the group consisting of (a) a heavy chain CDR1 region comprising an amino acid sequence of the formula X¹ - X² - X³ - X⁴ - X⁵ - X⁶ - X⁷ - X⁸ (SEQ ID NO:928), wherein X¹ is Gly; X² is Phe; X³ is Thr; X⁴ is Phe; X⁵ is Ser or Asn; X⁶ is Ser or Asn; X⁷ is Tyr or Phe; and X⁸ is Gly or Ala; (b) a heavy chain CDR2 region comprising an amino acid sequence of the formula X¹ - X² - X³ - X⁴ - X⁵ - X⁶ - X⁷ - X⁸ (SEQ ID NO:929), wherein X¹ is Ile or Leu; X² is Trp or Ser; X³ is Tyr or Gly; X⁴ is Asp or Ser; X⁵ is Gly; X⁶ is Ser, Thr or Val; X⁷ is Asn or Asp; and X⁸ is Lys or Arg; (c) a heavy chain CDR3 region comprising an amino acid sequence of the formula X¹ - X² - X³ - X⁴ - X⁵ - X⁶ - X⁷ - X⁸ - X⁹ - X¹⁰ - X¹¹ - X¹² - X¹³ - X¹⁴ - X¹⁵ - X¹⁶ (SEQ ID NO:930), wherein X¹ is Ala or Ser; X² is Arg or Lys; X³ is Asp; X⁴ is Gly or His; X⁵ is Asp or Ala; X⁶ is Phe; X⁷ is Tyr or Arg; X⁸ is Ser; X⁹ is Gly; X¹⁰ is Tyr; X¹¹ is Glu; X¹² is Gly or His; X¹³ is Tyr or Trp; X¹⁴ is Phe or absent; X¹⁵ is Asp or absent; and X¹⁶ is Pro or absent.

The isolated antibody or antibody fragment described herein may further comprise (d) a light chain CDR1 region comprising an amino acid sequence of the formula X¹ - X² - X³ - X⁴ - X⁵ - X⁶ - X⁷ (SEQ ID NO:931), wherein X¹ is Gln; X² is Ser; X³ is Val; X⁴ is Arg, Ser or Thr; X⁵ is Ser or Gly; X⁶ is Ser or Tyr; and X⁷ is Tyr or absent; (e) a light chain CDR2 region comprising an amino acid sequence of the formula X¹ - X² - X³ (SEQ ID NO:932), wherein X¹ is Gly or Asp; X² is Ala or Thr; and X³ is Ser; and (f) a light chain CDR3 region comprising an amino acid sequence of the formula X¹ - X² - X³ - X⁴ - X⁵ - X⁶ - X⁷ - X⁸ - X⁹ (SEQ ID NO:933), wherein X¹ is Gln; X² is Gln or His; X³ is Tyr, Arg or Ser; X⁴ is Gly, Ser or Ala; X⁵ is Ser, Asn or Phe; X⁶ is Trp or Ser; X⁷ is Pro; X⁸ is Trp, Pro or Arg; and X⁹ is Thr.

The isolated antibody or antibody fragment described herein may further comprise (d) a light chain CDR1 region comprising an amino acid sequence of the formula X¹ - X² - X³ - X⁴ - X⁵ - X⁶ - X⁷ (SEQ ID NO:931), wherein X¹ is Gln; X² is Ser; X³ is Val; X⁴ is Arg or Ser; X⁵ is Ser; X⁶ is Ser or Tyr; and X⁷ is Tyr or absent; (e) a light chain CDR2 region comprising an amino acid sequence of the formula X¹ - X² - X³ (SEQ ID NO:932), wherein X¹ is Gly or Asp; X² is Ala or Thr; and X³ is Ser; and (f) a light chain CDR3 region comprising an amino acid sequence of the formula X¹ - X² - X³ - X⁴ - X⁵ - X⁶ - X⁷ - X⁸ - X⁹ (SEQ ID NO:933), wherein X¹ is Gln; X² is Gln or His; X³ is Tyr or Arg; X⁴ is Gly or Ser; X⁵ is Ser or Asn; X⁶ is Trp or Ser; X⁷ is Pro; X⁸ is Pro or Arg; and X⁹ is Thr.

Also described herein is a fully human antibody or antibody fragment which binds hDII4 with an IC₅₀ of less than about 10 nM, as measured in *in vitro* assay or ELISA-based DII4 blocking assay (described below). Such an antibody may exhibit an IC₅₀ of about 500 pM or less. Such an antibody may preferably exhibit an IC₅₀ of about 100 pM or less.

Further described herein is a fully human monoclonal antibody which specifically binds and inhibits human DII4 and exhibits an IC₅₀ of less than or equal to about 150 pM, 100 pM, 75 pM, or 50 pM, as measured by Notch-inducible luciferase bioassay with hDII4-Fc. As shown in the experimental section below, the anti-hDII4 antibodies of the invention do not cross-react with closely related delta proteins, such as hDII1 and hDII3.

Additionally described herein is an isolated human antibody, or an antigen-binding portion thereof, that binds hDII4 with a K_{D} of less than about 500 pM, preferably less than about 300 pM, even more preferably less than about 100 pM, less than about 50 pM, less than about 10 pM, as determined by surface plasmon resonance (BIACORE™), for example, using dimeric hDII4 (Table 2).

Described herein are anti-hDII4 antibodies having a modified glycosylation pattern. In some applications, modification to remove undesirable glycosylation sites may be useful, or an antibody lacking a fucose moiety present on the oligosaccharide chain, for example, to increase antibody dependent cellular cytotoxicity (ADCC) function (see Shield et al. (2002) JBC 277:26733). In other applications, modification of a galactosylation can be made in order to modify complement dependent cytotoxicity (CDC).

Also described herein are anti-hDII4 antibodies which bind specific epitopes of hDII4 and are capable of blocking the biological activity of hDII4. The extracellular domain of DII4 is composed of an N-terminal domain, a Delta/Serrate/Lag-2 (DSL) domain, and a tandem of eight epidermal growth factor (EGF)-like repeats. Generally, the EGF domains are recognized as occurring at about amino acid residues 218-251 (domain 1), 252-282 (domain 2), 284-322 (domain 3), 324-360 (domain 4), and 362-400 (domain 5), with the DSL domain at about amino acid residues 173-217 and the N-terminal domain at about amino acid residues 27-172 of hDII4 (SEQ ID NO:2).

A blocking antibody described herein may bind within amino acids residues 27 to 524 of SEQ ID NO:2. In a more specific embodiment, a blocking antibody described herein binds an epitope within the N-terminus-DSL domains 27-217 of SEQ ID NO:2; in an even more specific embodiment, the blocking antibody binds an epitope within about amino acid residues 27-172 (N-terminal domain) or 173-217 (DSL domain). In another embodiment, a blocking antibody described herein binds the EGF-2 epitope within about amino acids residues 252-282 of SEQ ID NO:2.

The invention features a composition comprising a recombinant human anti-human DII4 antibody of the invention and an acceptable carrier. Further described herein are vectors and host cells comprising vectors which contain nucleic acid molecules encoding the human anti-hDII4 antibody of the invention, as well as methods of producing these novel antibodies, comprising growing a host cell comprising nucleic acid encoding the anti-hDII4 antibody of the invention or an antibody fragment, under conditions permitting production of the protein and recovering the protein so produced.
Additionally described herein are methods for inhibiting hDII4 activity using an antibody, or antigen-binding portion thereof, of the invention. In one embodiment, the method described herein may comprise contacting hDII4 with the instant antibody or antigen-binding portion thereof, such that hDII4 is inhibited from binding to Notch receptor, for example Notch-1. In another embodiment, the method described herein comprises administering an antibody or antibody fragment of the invention, to a human subject suffering from a disorder which is ameliorated by inhibition of DII4 activity. The disorder treated is a disease or condition which is improved, ameliorated, inhibited or prevented by removal, inhibition or reduction of DII4 activity, for example, pathological vascularization associated with tumor angiogenesis and cancer, immunodeficiency diseases, transplant rejection, or inflammation; and neurodegenerative conditions, e.g., associated with prion disease. Also described herein is the use of an antibody or antigen-binding fragment of an antibody, as described above, in the manufacture of a medicament for use to attenuate or inhibit a DII4-mediated disease or disorder in a human.

Other objects and advantages will become apparent from a review of the ensuing detailed description.

### DETAILED DESCRIPTION

Before the present methods are described, it is to be understood that this invention is not limited to particular methods, and experimental conditions described, as such methods and conditions may vary. It is also to be understood that the terminology used herein is for the purpose of describing particular embodiments only, and is not intended to be limiting, since the scope of the present invention will be limited only by the appended claims.

As used in this specification and the appended claims, the singular forms "a", "an", and "the" include plural references unless the context clearly dictates otherwise. Thus for example, a reference to "a method" includes one or more methods, and/or steps of the type described herein and/or which will become apparent to those persons skilled in the art upon reading this disclosure.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

### Definitions

"Delta-like ligand 4", "DII4", "hDII4" are used interchangeably to refer to the protein encoded by the nucleic acid sequence of SEQ ID NO:1 and the protein having the amino acid sequence of SEQ ID NO:2.

The term "antibody", as used herein, is intended to refer to immunoglobulin molecules comprised of four polypeptide chains, two heavy (H) chains and two light (L) chains interconnected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementary determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4.

The term "high affinity" antibody refers to those antibodies having a binding affinity to hDII4 of at least 10⁻⁸ M; preferably 10⁻⁹ M; even more preferably 10⁻¹⁰ M, as measured by surface plasmon resonance, e.g., BIACORE™ or solution-affinity ELISA.

By the term "slow off rate" or "Koff" is meant an antibody that dissociates from hDII4 with a rate constant of 1 x 10⁻³ s⁻¹ or less, preferably 1 x 10⁻⁴ s⁻¹ or less, as determined by surface plasmon resonance, e.g., BIACORE™.

A "neutralizing" or "blocking" antibody, is intended to refer to an antibody whose binding to DII4 results in inhibition of the biological activity of DII4. This inhibition of the biological activity of DII4 can be assessed by measuring one or more indicators of DII4 biological activity. These indicators of DII4 biological activity can be assessed by one or more of several standard *in vitro* or *in vivo* assays known in the art (see examples below). Preferably, the ability of an antibody to neutralize DII4 activity is assessed by inhibition of DII4 binding to a Notch receptor.

The term "antigen-binding portion" of an antibody (or simply "antibody portion" or "antibody fragment"), as used herein, refers to one or more fragments of an antibody that retain the ability to specifically bind to an antigen (e.g., hDII4). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al. (1989) Nature 241:544-546), which consists of a VH domain; and (vi) an isolated CDR. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see e.g., Holliger et al. (1993) Proc. Natl. Acad Sci. USA 90:6444-6448; Poljak et al. (1994) Structure 2:1121-1123).

Still further, an antibody or antigen-binding portion thereof may be part of a larger immunoadhesion molecule, formed by covalent or noncovalent association of the antibody or antibody portion with one or more other proteins or peptides. Examples of such immunoadhesion molecules include use of the streptavidin core region to make a tetrameric scFv molecule (Kipriyanov et al. (1995) Human Antibodies and Hybridomas 6:93-101) and use of a cysteine residue, a marker peptide and a C-terminal polyhistidine tag to make bivalent and biotinylated scFv molecules (Kipriyanov et al. (1994) Mol. Immunol. 31:1047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

The term "human antibody", as used herein, is intended to include antibodies having variable and constant regions derived from human germline immunoglobulin sequences. The human antibodies of the invention may include amino acid residues not encoded by human germline immunoglobulin sequences (e.g., mutations introduced by random or site-specific mutagenesis in vitro or by somatic mutation in vivo), for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

An "isolated antibody", as used herein, is intended to refer to an antibody that is substantially free of other antibodies having different antigenic specificities (e.g., an isolated antibody that specifically binds hDII4 is substantially free of antibodies that specifically bind antigens other than hDII4). An isolated antibody that specifically binds hDII4 may, however, have cross-reactivity to other antigens, such as hDII4 molecules from other species. Moreover, an isolated antibody may be substantially free of other cellular material and/or chemicals.

The term "surface plasmon resonance", as used herein, refers to an optical phenomenon that allows for the analysis of real-time biospecific interactions by detection of alterations in protein concentrations within a biosensor matrix, for example using the BIACORE™ system (Pharmacia Biosensor AB, Uppsala, Sweden and Piscataway, N.J.).

The term "K_{D}", as used herein, is intended to refer to the dissociation constant of a particular antibody-antigen interaction.

The term "epitope" includes any determinant, preferably a polypeptide determinant, capable of specific binding to an immunoglobulin or T-cell receptor. In certain embodiments, epitope determinants include chemically active surface groupings of molecules such as amino acids, sugar side chains, phosphoryl groups, or sulfonyl groups, and, in certain embodiments, may have specific three-dimensional structural characteristics, and/or specific charge characteristics. An epitope is a region of an antigen that is bound by an antibody. In certain embodiments, an antibody is said to specifically bind an antigen when it preferentially recognizes its target antigen in a complex mixture of proteins and/or macromolecules. In preferred embodiments, an antibody is said to specifically bind an antigen when the equilibrium dissociation constant is less than or equal to 10⁻⁸ M, more preferably when the equilibrium dissociation constant is less than or equal to 10⁻⁹ M, and most preferably when the dissociation constant is less than or equal to 10⁻¹⁰ M.

A protein or polypeptide is "substantially pure," "substantially homogeneous" or "substantially purified" when at least about 60 to 75% of a sample exhibits a single species of polypeptide. The polypeptide or protein may be monomeric or multimeric. A substantially pure polypeptide or protein will typically comprise about 50%, 60, 70%, 80% or 90% W/W of a protein sample, more usually about 95%, and preferably will be over 99% pure. Protein purity or homogeneity may be indicated by a number of means well known in the art, such as polyacrylamide gel electrophoresis of a protein sample, followed by visualizing a single polypeptide band upon staining the gel with a stain well known in the art. For certain purposes, higher resolution may be provided by using HPLC or other means well known in the art for purification.

The term "polypeptide analog or variant" as used herein refers to a polypeptide that is comprised of a segment of at least 25 amino acids that has substantial identity to a portion of an amino acid sequence and that has at least one of the following properties: (1) specific binding to hDII4 under suitable binding conditions, or (2) ability to block DII4 binding to a Notch receptor. Typically, polypeptide analogs or variants comprise a conservative amino acid substitution (or insertion or deletion) with respect to the naturally-occurring sequence. Analogs typically are at least 20 amino acids long, preferably at least 50, 60, 70, 80, 90, 100, 150 or 200 amino acids long or longer, and can often be as long as a full-length naturally-occurring polypeptide.

Preferred amino acid substitutions are those which: (1) reduce susceptibility to proteolysis, (2) reduce susceptibility to oxidation, (3) alter binding affinity for forming protein complexes, (4) alter binding affinities, and (4) confer or modify other physicochemical or functional properties of such analogs. Analogs can include various mutations of a sequence other than the naturally-occurring peptide sequence. For example, single or multiple amino acid substitutions (preferably conservative amino acid substitutions) may be made in the naturally-occurring sequence (preferably in the portion of the polypeptide outside the domain(s) forming intermolecular contacts. A conservative amino acid substitution should not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton 1984 W. H. Freeman and Company, New York; Introduction to Protein Structure (Branden & Tooze, eds., 1991, Garland Publishing, NY); and Thornton et at. 1991 Nature 354:105.

Non-peptide analogs are commonly used in the pharmaceutical industry as drugs with properties analogous to those of the template peptide. These types of non-peptide compound are termed "peptide mimetics" or "peptidomimetics" (see, for example, Fauchere (1986) J. Adv. Drug Res. 15:29; and Evans et al. (1987) J. Med. Chem. 30:1229. Systematic substitution of one or more amino acids of a consensus sequence with a D-amino acid of the same type (e.g., D-lysine in place of L-lysine) may also be used to generate more stable peptides. In addition, constrained peptides comprising a consensus sequence or a substantially identical consensus sequence variation may be generated by methods known in the art (Rizo et al. (1992) Ann. Rev. Biochem. 61:387), for example, by adding internal cysteine residues capable of forming intramolecular disulfide bridges which cyclize the peptide.

The term "percent sequence identity" in the context of nucleic acid sequences refers to the residues in two sequences which are the same when aligned for maximum correspondence. The length of sequence identity comparison may be over a stretch of at least about nine nucleotides or more, usually at least about 18 nucleotides, more usually at least about 24 nucleotides, typically at least about 28 nucleotides, more typically at least about 32 nucleotides, and preferably at least about 36, 48 or more nucleotides. There are a number of different algorithms known in the art which can be used to measure nucleotide sequence identity. For instance, polynucleotide sequences can be compared using FASTA, Gap or Bestfit, which are programs in Wisconsin Package Version 10.0, Genetics Computer Group (GCG), Madison, Wis. FASTA, which includes, e.g., the programs FASTA2 and FASTA3, provides alignments and percent sequence identity of the regions of the best overlap between the query and search sequences (Pearson (1990) Methods Enzymol. 183:63-98 and (2000) Methods Mol. Biol. 132:185-219). Unless otherwise specified, default parameters for a particular program or algorithm are used. For instance, percent sequence identity between nucleic acid sequences can be determined using FASTA with its default parameters (a word size of 6 and the NOPAM factor for the scoring matrix) or using Gap with its default parameters as provided in GCG Version 6.1.

A reference to a nucleic acid sequence encompasses its complement unless otherwise specified. Thus, a reference to a nucleic acid molecule having a particular sequence should be understood to encompass its complementary strand, with its complementary sequence. Generally, the art uses the terms "percent sequence identity", "percent sequence similarity" and "percent sequence homology" interchangeably. In this application, these terms shall have the same meaning with respect to nucleic acid sequences.

The term "substantial similarity", or "substantial sequence similarity," when referring to a nucleic acid or fragment thereof, indicates that, when optimally aligned with appropriate nucleotide insertions or deletions with another nucleic acid (or its complementary strand), there is nucleotide sequence identity in at least about 90%, preferably at least about 95%, and more preferably at least about 96%, 97%, 98% or 99% of the nucleotide bases, as measured by any well-known algorithm of sequence identity, such as FASTA, BLAST or Gap, as discussed above.

As applied to polypeptides, the term "substantial identity" or "substantially identical" means that two peptide sequences, when optimally aligned, such as by the programs GAP or BESTFIT using default gap weights, share at least 80% sequence identity, preferably at least 90% or 95% sequence identity, even more preferably at least 98% or 99% sequence identity. Preferably, residue positions which are not identical differ by conservative amino acid substitutions. A "conservative amino acid substitution" is one in which an amino acid residue is substituted by another amino acid residue having a side chain (R group) with similar chemical properties (e.g., charge or hydrophobicity). In general, a conservative amino acid substitution will not substantially change the functional properties of a protein. In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. See, e.g., Pearson (1994) Methods Mol. Biol. 24: 307-331. Examples of groups of amino acids that have side chains with similar chemical properties include 1) aliphatic side chains: glycine, alanine, valine, leucine and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; and 6) sulfur-containing side chains are cysteine and methionine. Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al. (1992) Science 256: 1443 45. A "moderately conservative" replacement is any change having a nonnegative value in the PAM250 log-likelihood matrix.

Sequence similarity for polypeptides, which is also referred to as sequence identity, is typically measured using sequence analysis software. Protein analysis software matches similar sequences using measures of similarity assigned to various substitutions, deletions and other modifications, including conservative amino acid substitutions. For instance, GCG contains programs such as "Gap" and "Bestfit" which can be used with default parameters to determine sequence homology or sequence identity between closely related polypeptides, such as homologous polypeptides from different species of organisms or between a wild type protein and a mutein thereof. See, e.g., GCG Version 6.1. Polypeptide sequences also can be compared using FASTA using default or recommended parameters, a program in GCG Version 6.1. FASTA (e.g., FASTA2 and FASTA3) provides alignments and percent sequence identity of the regions of the best overlap between the query and search sequences (Pearson (2000) *supra*). Another preferred algorithm when comparing a sequence of the invention to a database containing a large number of sequences from different organisms is the computer program BLAST, especially blastp or tblastn, using default parameters. See, e.g., Altschul et al. (1990) J. Mol. Biol. 215: 403 410 and Altschul et al. (1997) Nucleic Acids Res. 25:3389 402.

The length of polypeptide sequences compared for homology will generally be at least about 16 amino acid residues, usually at least about 20 residues, more usually at least about 24 residues, typically at least about 28 residues, and preferably more than about 35 residues. When searching a database containing sequences from a large number of different organisms, it is preferable to compare amino acid sequences.

### Preparation of Human Antibodies

Methods for generating human antibodies include, for example, VELOCIMMUNE^{®} (Regeneron Pharmaceuticals), XENOMOUSE™ technology (Abgenix), the "minilocus" approach, and phage display. The VELOCIMMUNE^{®} technology (U.S. 6,596,541) encompasses a method of generating a high specificity fully human antibody to a select antigen. This technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody. In specific embodiment, the cell is a CHO cell.

The XENOMOUSE™ technology (Green et al. (1994) Nature Genetics 7:13-21) generates a mouse having both human variable and constant regions from both the heavy chain and kappa light chain loci. In an alternative approach, others have utilized a 'minilocus" approach in which an exogenous Ig locus is mimicked through inclusion of individual genes from the Ig locus (see, for example, U.S. 5,545,807). The DNA encoding the variable regions can be isolated with or without being operably linked to the DNA encoding the human heavy and light chain constant region.

Other methods of generating human antibodies, including isolation from a human donor, are known. See, for example, U.S. 6,787,637.

Antibodies may be therapeutically useful in blocking a ligand-receptor interaction or inhibiting receptor component interaction, rather than by killing cells through fixation of complement and participation in CDC. The constant region of an antibody is important in the ability of an antibody to fix complement and participate in CDC or direct cell killing through antibody-dependent cellular cytoxicity (ADCC). Thus, the isotype of an antibody may be selected on the basis of the desirability for the antibody to fix complement.

Human immunoglobulins can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-heavy chain disulfide bonds and a molecule of about 75-80 kDa is formed composed of a single light and heavy chain. These forms have been difficult to separate, even after affinity purification.

The frequency of appearance of the second form in various intact IgG isotypes is due to, but not limited to, structural differences associated with the hinge region isotype of the antibody. In fact, a single amino acid substitution in the hinge region of the human IgG4 hinge can significantly reduce the appearance of the second form (Angal et al. 1993 Molecular Immunology 30:105) to levels typically observed using a human IgG1 hinge. The instant invention encompasses antibodies having one or more mutations in the hinge, CH2 or CH3 region which may be desirable, for example, in production to improve the yield, or modulate effector functions.

Antibodies of the invention are preferably prepared with the use of VELOCIMMUNE^{®} technology. A transgenic mouse in which the endogenous immunoglobulin heavy and light chain variable regions are replaced with the corresponding human variable regions is challenged with the antigen of interest, and lymphatic cells (such as B-cells) recovered from the mice that express antibodies. The lymphatic cells may be fused with a myeloid-type cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies may be isolated directly from antigen-specific lymphocytes. In various embodiments, the transgenic mouse comprises 12 functional human variable heavy chain genes and 11 functional human variable kappa light chain genes; 25 to 30 human variable heavy chain genes and from 18 to 20 human variable kappa light chain genes; 43 to 48 human variable heavy chain genes and 20 to 22 human variable kappa light chain genes; or about 80 human variable heavy chain genes and about 40 human variable kappa light chain genes.

In general, the antibodies of the instant invention possess very high affinities, typically possessing K_{D} of from about 10⁻⁹ through about 10⁻¹¹ M, when measured by binding to antigen either immobilized on solid phase or in solution phase. The mouse constant regions are replaced with desired human constant regions to generate the fully human antibodies of the invention, for example wild-type or modified IgG1 or IgG4 (for example, SEQ ID NO:950, 951, or 952). While the constant region selected may vary according to specific use, high affinity antigen-binding and target specificity characteristics reside in the variable region.

Cancer, infectious diseases, autoimmunity, immunodeficiency, transplants, inflammation, injury and degenerative conditions can be treated by modulation of the immune system. In cases of disease due to inappropriate function or hyperactivity of the immune system, such as autoimmunity or inflammation, can be ameliorated through inhibition of immune cell function or reduction of immune cell numbers. This can be accomplished by blockade of positive signals or stimulation of negative signals on immune cell populations critical to the disease process, such as T, B or NK cells, neutrophils, macrophages, antigen presenting cells, mast cells or other cell types. Overactivity can also be inhibited through elimination of various immune cell populations by stimulation of apoptosis, targeting of specific surface receptors with depleting antibodies or antibody-drug conjugates, or the blockade or alteration of the differentiation of immune cell lineages or specific cell types. Inefficient or reduced immune function can cause or exacerbate disorders such as cancer, infectious disease, and other immunodeficiencies. Hypoactivity of the immune system can be improved through activation of immune cells by stimulation of positive signals by crosslinking or agonistic antibodies or blockade of negative signals. Immune cell populations can be increased by stimulation of development of some or all immune cell lineages, prevention of apoptosis, or elimination of inhibitory signals. In a specific application, the antibodies of the invention are useful for treatment, inhibition or amelioration of a condition or disease such as, for example, cancer immunodeficiency, transplant rejection, or inflammation.

### Epitope Mapping and Related Technologies

To screen for antibodies which bind to a particular epitope (e.g., those which block binding of IgE to its high affinity receptor), a routine cross-blocking assay such as that described in Harlow and Lane (1990) *supra* can be performed. Other methods include alanine scanning mutants, peptide blots (Reineke (2004) Methods Mol Biol 248:443-63), or peptide cleavage analysis. In addition, methods such as epitope excision, epitope extraction and chemical modification of antigens can be employed (Tomer (2000) Protein Science 9: 487-496).

The term "epitope" refers to a site on an antigen to which B and/or T cells respond. B-cell epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, and more usually, at least 5 or 8-10 amino acids in a unique spatial conformation.

Modification Assisted Profiling (MAP), also known as Antigen Structure-based Antibody Profiling (ASAP) is a method that categorizes large numbers of monoclonal antibodies (mAbs) directed against the same antigen according to the similarities of the binding profile of each antibody to chemically or enzymatically modified antigen surfaces (U.S. patent Publication No. 2004/0101920). Each category may reflect a unique epitope either distinctly different from or partially overlapping with epitope represented by another category. This technology allows rapid filtering of genetically identical antibodies, such that characterization can be focused on genetically distinct antibodies. When applied to hybridoma screening, MAP may facilitate identification of rare hybridoma clones that produce mAbs having the desired characteristics. MAP may be used to sort the hDII4 antibodies of the invention into groups of antibodies binding different epitopes.

Agents useful for altering the structure of the immobilized antigen are enzymes, such as, for example proteolytic enzymes, for example, trypsin, endoproteinase Glu-C, endoproteinase Asp-N, chymotrypsin, etc. Agents useful for altering the structure of the immobilized antigen may also be chemical agents, such as, succinimidyl esters and their derivatives, primary amine-containing compounds, hydrazines and carbohydrazines, free amino acids, etc.

The antigen protein may be immobilized on either biosensor chip surfaces or polystyrene beads. The latter can be processed with, for example, an assay such as multiplex LUMINEX™ detection assay (Luminex Corp., Austin, TX). Because of the capacity of LUMINEX™ to handle multiplex analysis with up to 100 different types of beads, LUMINEX™ provides almost unlimited antigen surfaces with various modifications, resulting in improved resolution in antibody epitope profiling over a biosensor assay.

### Therapeutic Administration and Formulations

The administration of therapeutic entities in accordance with the invention will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences (15th ed, Mack Publishing Company, Easton, PA). These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing mixtures may be appropriate in treatments and therapies in accordance with the present invention, provided that the active ingredient in the formulation is not inactivated by the formulation and the formulation is physiologically compatible and tolerable with the route of administration. See also Powell et al. "Compendium of excipients for parenteral formulations" PDA (1998) J Pharm Sci Technol. 52:238-311 and the citations therein for additional information related to excipients and carriers well known to pharmaceutical chemists.

### EXAMPLES

### Example 1. Generation of Human Antibodies to Human DII4.

Mice may be immunized by any method known in the art (see, for example, Harlow and Lane *supra*). In one embodiment, hDII4 antigen is administered directly to VELOCIMMUNE^{®} mice comprising DNA loci encoding human Ig heavy chain variable regions and kappa light chain variable regions, with an adjuvant to stimulate the immune response. Such an adjuvant includes complete and incomplete Freund's adjuvant, MPL+TDM adjuvant system (Sigma), or RIBI (muramyl dipeptides) (see O'Hagan 2000 Vaccine Adjuvant, by Human Press, Totawa, NJ). The antibody immune response is monitored by standard antigen specific immunoassay. When a desired immune response is achieved, antibody-expressing B cells were harvested and fused with mouse myeloma cells to preserve their viability, forming hybridoma cell lines. Such hybridoma cell lines are screened and selected to identify cell lines that produce antigen-specific antibodies using assays as described below.

Alternatively, antigen-specific hybridoma cells may be isolated by flow cytometry. Briefly, after fusion to myeloma cells, pooled hybridoma cells were grown for 10 days in HAT medium. The cells were then harvested and stained with biotin-labeled DII4 at 2 mg/ml for one hour, followed by addition of phycoerythrin-streptavidin. The fluorescence-labeled cells were sorted by flow cytometry (single cell per well into 96 well plates containing hybridoma growth medium), cultured for 8-10 days, and conditioned media screened for the presence of functionally desirable monoclonal antibodies, as described below.

Anti-hDII4 antibodies generated via direct isolation of splenocytes. Antigen-specific antibodies may also be isolated directly from antigen-immunized B cells without fusion to myeloma cells, as described in U.S. Patent Publication 2007/0280945A1. Stable recombinant antibody-expressing CHO cell lines were established from the isolated proper recombinants.

### Example 2. Antigen Binding Affinity Determination.

Equilibrium dissociation constants (K_{D} values) for antigen binding to the selected antibodies described above were determined by surface kinetics on a real-time biosensor surface plasmon resonance assay (BIACORE™ 2000). The antibody was captured on a goat anti-mouse IgG polyclonal antibody surface created through direct chemical coupling to a BIACORE™ chip to form a captured antibody surface. Varying concentrations of monomeric hDII4 or dimeric hDII4-hFc were injected over the captured antibody surfaces, and antigen-antibody binding and dissociation monitored in real time. Kinetic analysis was performed to calculate K_{D}, dissociation rate constants, and half-life of antigen/antibody complex dissociation (Table 1). A similar method was applied to measure single B cell-derived monoclonal antibodies modified to contain a human IgG constant domain. Antibodies were presented by goat anti-hFc polyclonal antibody reagent (Jackson Immuno Research Lab) immobilized on BIACORE™ chip, and exposed to either dimeric DII4-mFc or monomeric DII4 protein (Table 2).

Antibody-antigen binding affinity may also be assessed using an ELISA based solution competition assay. Briefly, on a 96-well microtiter plate, antibodies (purified proteins or in conditioned medium) were premixed with serial dilutions of antigen protein (monomeric or dimeric) ranging from 0 to 10 µg/ml with a constant concentration of antibody. After a 2 hr incubation of antigen with antibody, the solutions were transferred to a microtiter plate precoated with antigen for measurement of free antibody (MAXISORB™, VWR, West Chester, PA). The plate was coated with 1 µg/ml hDII4-hFc protein in PBS solution overnight at 4° C and nonspecific binding sites blocked with BSA for 2 hrs. After a 1 hr incubation following transfer, the plate was washed and the plate-bound antibodies were detected with an HRP-conjugated goat anti-mouse IgG polyclonal antibody reagent (Jackson Immuno Laboratory) and developed using colorimetric substrates (OPTEIA^{™}; BD Biosciences Pharmingen, San Diego, CA). The enzymatic reaction was stopped with 1 M phosphoric acid, optical absorptions at 450 nm were recorded and the data were analyzed using a sigmoidal dose-response model and an IC₅₀ values were reported (Table 1).

**TABLE 1**

| **Antibody** | **K_{D} DII4 (nM)** | **K_{D} DII4-Fc (nM)** | **IC₅₀ DII4-Fc (nM)** |
|---|---|---|---|
| 13B6 | 2.79 | 0.188 | 0.06 |
| 15E10 | 0.55 | 0.023 | 0.58 |
| 22G12 | 1.29 | 0.076 | 0.03 |
| 24C8 | 0.52 | 0.047 | 0.01 |
| VAV 2H4-19 | 1.51 | 0.611 | 0.10 |
| VAV 4H10-9 | 13.70 | 0.662 | 0.30 |
| VAV 7B9-9 | 0.88 | 0.021 | 0.27 |
| VAW 10E4-9 | 89.00 | 0.468 | 0.06 |
| VAW 10G11-2 | 31.30 | 1.430 | 1.66 |
| VAW 1 C6-1 | 45.80 | 0.092 | 0.25 |
| VAW 1G2-4 | 83.80 | 0.035 | 0.40 |
| VAW 1 H2-2 | 67.00 | 0.148 | 0.30 |
| VAW 2H3-2 | 0.30 | 0.150 | 0.26 |
| VAW 3A7-2 | 1.64 | 0.162 | 0.02 |
| VAW 3A9-5 | NA | 2.510 | 16.00 |
| VAW 3F12-8 | 8.12 | 0.648 | 0.07 |
| VAW 6B8-12 | 0.89 | 0.060 | 0.43 |
| VAW 6C6-2 | 91.70 | 0.092 | 0.50 |
| VAW 6G12-10 | 3.74 | 0.527 | 0.19 |
| VAW 7C10-11 | 17.10 | 0.853 | 0.28 |
| VAW 8A10-14 | 1.41 | 0.648 | 0.08 |
| VAW 8G1-12 | 6.09 | 8.300 | 8.60 |
| VAW 9B11-2 | 62.20 | 0.048 | 0.00 |
| VAW 9F12-6 | 16.00 | 1.350 | 0.02 |
| VAW 9G10-1 | 56.10 | 0.555 | 0.10 |

**TABLE 2**

| **Antibody** | **K_{D} DII4 (nM)** | **K_{D} DII4-Fc (nM)** |
|---|---|---|
| 314266-06F12-B7 | 2.17 | 0.075 |
| 318518-01A04-D5 | 0.237 | 0.244 |
| 318518-01A10-D8 | 0.399 | 0.018 |
| 318518-01 B09-C3 | 0.833 | 0.180 |
| 318518-01 B11-D4 | 0.382 | 0.088 |
| 318518-01 E07-H2 | 0.165 | 0.238 |
| 318518-01G04-F3 | 0.501 | 0.107 |
| 318518-01 G05-B5 | 1.06 | 0.196 |
| 318518-02A07-B3 | 0.208 | 0.148 |
| 318518-02B06-E2 | 2.15 | 0.193 |
| 318518-02B08-F7 | N/A | N/A |
| 318518-02C04-D1 | 0.478 | 0.331 |
| 318518-02 F05-D10 | 1.28 | 0.035 |
| 318518-02G03-F2 | 1.31 | 0.042 |
| 318518-02G04-B11 | 0.813 | 0.048 |
| 318518-02G08-F11 | N/A | N/A |
| 318518-03A03-B2 | 0.136 | 0.124 |
| 318518-03C10-F2 | 1.18 | 0.131 |
| 318518-03D04-B5 | 0.904 | 0.136 |
| 318518-03D07-G11 | 3.74 | 0.163 |
| 318518-03F04-A6 | 0.501 | 0.088 |
| 318518-03F06-A3 | 0.556 | 0.037 |
| 318518-03H03-F3 | 8.89 | 0.084 |
| 318518-14A06-E7 | 4.54 | 0.282 |
| 318518-14A07-C4 | 0.235 | 0.035 |
| 318518-14D08-G1 | 0.541 | 0.046 |
| 318518-14H08-A2 | 6.67 | 0.128 |
| 318518-1 H08-E9 | 0.225 | 0.050 |

### Example 3. Inhibition of DII4 and Notch Interaction

The ability of the antibodies to block D114 binding to Notch was evaluated with an ELISA-based immunoassay. Briefly, Notch-hFc recombinant protein was coated on a 96-well plate in PBS buffer overnight at 4°C at 1 mg/ml, and the nonspecific binding sites were blocked with BSA. This plate was used to measure free biotin-DII4-hFc from antibody titration sample solutions. To make the antibody titration samples, a constant amount of biotin-DII4-hFc at 25 pM was pre-mixed with varied amounts of antibody, either in crude hybridoma condition medium or as purified antibody protein, ranging from 0 to -50 nM in serial dilutions, followed by 2 hr incubation at room temperature to allow antibody-antigen binding to reach equilibrium. The equilibrated sample solutions were then transferred to the Notch-hFc coated plates for the measurement of free biotin-DII4-hFc. After 1 hour binding, the plate was washed and bound biotin-DII4-hFc was detected using HRP conjugated streptavidin (Poly HRP streptavidin, Pierce Endogen), and developed using TMB substrate (BD Pharmigen). Data was analyzed using GraphPad Prism software and IC₅₀ values were determined as the amount of antibody required to achieve 50% reduction of biotin-DII4-hFc bound to the plate-coated Notch-Fc (Table 3)

**TABLE 3**

| **Antibody** | **IC₅₀ (nM)** |
|---|---|
| VAV 2H4-19 | 0.01 |
| VAW 3A7-2 | 0.017 |
| VAW 9G10-1 | 0.019 |
| VAW 10E4-9 | 0.032 |
| VAW 8A10-11 | 0.04 |
| VAW 9F12-6 | 0.059 |
| VAW 3F12-8 | 0.066 |
| VAW1C6-1 | 0.086 |
| VAW 1 G2-4 | 0.11 |
| VAW 6C6-2 | 0.119 |
| VAV 7B9-4 | 0.123 |
| VAW 1 H2-2 | 0.154 |
| VAW2H3-2 | 0.168 |
| VAW 6B8-12 | 0.255 |
| VAW 6G12-10 | 0.257 |
| VAW 7C10-11 | 0.273 |
| VAV 4 H 10-9 | 0.599 |
| VAW 10G11-2 | 0.931 |
| VAW 3A9-5 | 3.8 |
| VAW8G1-12 | 10.7 |
| VAW 9B11-2 | 0.069 |
| 15E10* | 0.04 |
| 22G12 | 0.10 |
| 13B6 | 0.11 |
| 24C8 | 0.031 |
| 314266-06F12-B7 | 0.07 |
| 318518-01A04-D5 | 0.11 |
| 318518-01A10-D8 | 0.05 |
| 318518-01 B09-C3 | 0.03 |
| 318518-01811-D4 | 0.03 |
| 318518-01 E07-H2 | 1.17 |
| 318518-01 G04-F3 | 0.02 |
| 318518-01 G05-B5 | 1.08 |
| 318518-02A07-B3 | 0.03 |
| 318518-02B06-E2 | 0.09 |
| 318518-02B08-F7 | N/A |
| 318518-02C04-D1 | 0.60 |
| 318518-02F05-D10 | 0.16 |
| 318518-02G03-F2 | 0.07 |
| 318518-02G04-B11 | 1.09 |
| 318518-02G08-F11 | N/A |
| 318518-03A03-B2 | 0.57 |
| 318518-03C10-F2 | 0.12 |
| 318518-03D04-B5 | 0.04 |
| 318518-03D07-G11 | 0.45 |
| 318518-03F04-A6 | 0.01 |
| 318518-03F06-A3 | 0.02 |
| 318518-03H03-F3 | 0.17 |
| 318518-14A06-E7 | 0.04 |
| 318518-14A07-C4 | 0.02 |
| 318518-14D08-G1 | 0.14 |
| 318518-14H08-A2 | 0.25 |
| 318518-1 H08-E9 | 0.03 |

The ability of selected purified anti-hDll4 antibodies to block DII4 binding to Notch was also evaluated with the ELISA-based immunoassay described above, modified by replacing 25 pM of biotin-DII4-hFc with 30 pM of biotin-DII4-hFc, and reducing antibody-antigen incubation duration from 2 hrs to 1 hr. For convenience, antibody 318518-01A10-D8 was renamed "REGN281" (HCVR/LCVR SEQ ID NOs:429/437 and hlgG1 SEQ ID NO:950). Derived antibodies tested included REGN421 (HCVR/LCVR SEQ ID NO:901/903, hlgG1 SEQ ID NO:950); and REGN422 (HCVR/LCVR SEQ ID NO:901/903, with modified hlgG4 SEQ ID NO:952). Results are shown in Table 4.

**TABLE 4**

| **Antibody** | **IC₅₀ (nM)** |
|---|---|
| REGN281 | 0.042 |
| REGN421 | 0.045 |
| REGN422 | 0.039 |

Ability of antibody to neutralize DII4 mediated cellular function was also tested *in vitro* using DII4 expressing human umbilical vein endothelial cells (HUVEC). Inhibition of Notch mediated hHes1 and EphB2 gene expression in HUVEC with the derived antibodies was monitored as follows: low passage HUVEC were cultured in MCDB-131 media (Vec Technologies). One day prior to analysis, HUVEC cells were seeded at a density of 2 x 10⁵ cells/well in 24-well plates in 1 ml total media volume. The test antibody or other inhibitor was added directly to the individual sample wells in triplicate followed by 5-hour culture at 37°C. At the end of the culture period, media was removed and total RNA was isolated using QIAZOL™ and the RNEASY™ lipid tissue kit (Qiagen). mRNA level quantification was performed by the use of PCR and the fluorogenic 5' nuclease assay (TAQMAN® assay, Appied Biosystems). For each sample, cDNA was synthesized from 1-2 mg of total RNA. cDNA generated from an equivalent amount of starting RNA (typically 25 ng) was loaded in triplicate on ABI PRISM™ optical reaction plates. For each RNA sample a "no RT" control was also run in which no reverse transcriptase was added to allow for subtraction of any potential genomic DNA contributions to the signal. 2x Mastermix (TAQMAN® 2x PCR Mastermix; ABI) was added to each reaction to a final concentration of 1x. Additionally, TASMAN® probe and primers for the gene of interest were added to each reaction. Each primer was used at a final concentration of 900 nM and the probe was added at a final concentration of 200 nM. Human genomic DNA was used as a standard. The assays were performed under standard TASMAN® conditions on a ABI 7900HT instrument. Levels of Hes1 and EphrinB2 were measured and normalized to an endogenous control gene (cydophilin) (Table 5). Probes and primers: human Hes1 probe (SEQ ID NO:387); Oligos: hHes1-869F (SEQ ID NO:388); hHes1-940R (SEQ ID NO:389), human ephrinB2 probe: hEphB2-773T (SEQ ID NO:390); Oligos: hEphB2-752F (SEQ ID NO:391) hEphB2-812R (SEQ ID NO:392); human cyclophilin: probe: hCyclophilin-343T (SEQ ID NO:393); Oligos: hCyclophilin-323F (SEQ ID NO:394); hCyclophilin-389R (SEQ ID NO:395).

**TABLE 5**

| **Antibody** | **EphrinB2 Expression IC₅₀ (nM)** | **Hes1 Expression IC₅₀ (nM)** |
|---|---|---|
| 22G12 | 0.379 | 0.381 |
| 15E10 | 2.56 | 4.49 |
| VAW 3A7-2 | 0.409 | 0.533 |
| 314266-06F12-B7 | 0.239 | 0.405 |
| 318518-01A10-D8 | 0.305 | 0.329 |
| 318518-01 G04-F3 | 0.088 | 0.172 |
| 318518-01 H08-E9 | 0.413 | 0.548 |
| 318518-02A07-B3 | 0.398 | 0.128 |
| 318518-03F04-A6 | 0.158 | 0.115 |
| 318518-03F06-A3 | 0.304 | 0.692 |
| 318518-014A07-C4 | 0.175 | 0.312 |
| 318518-014 D08-G1 | 0.510 | 0.568 |
| hDII4-hFc | 0.843 | 0.974 |

HUVEC Proliferation Assay. The ability of antibody to block DII4 mediated growth inhibition of Human umbilical vein endothelial cells (HUVEC) cells was tested in an *in vitro* cell proliferation assay. Low passage HUVEC cells were obtained and cultured in MCDB-131 media (Vec Technologies). One day prior to analysis 12-well tissue culture plates were coated with hDII4-hFc in PBS at 4°C overnight (0.2 µg/ml; 0.5 ml PBS/well). Plates were washed 1x with PBS and HUVEC cells were seeded at a density of 4x10³ cells/well in 1.0 ml total media volume. Immediately following addition of cells anti-hDII4 antibodies were added in 0.5 ml total volume over a range of concentrations to generate an inhibition curve. Cells were grown for 96-hours at 37°C. Cell number was quantitated using the CCK-8 reagent (Dojindo). All assays were run in triplicate. (Table 6, NB, not blocking).

**TABLE 6**

| **Antibody** | **IC₅₀ (nM)** |
|---|---|
| 15E10 | 0.284 |
| VAW9B11-2 | 1.868 |
| VAW8D8-12 | NB |
| 13B6 | 5.01 |
| VAW2H4-19 | NB |
| VAW3A7-2 | 0.198 |
| VAW8A10-14 | 0.214 |
| 22G12 | 0.888 |
| 318518-06F12-B7 | 2.067 |
| 318518-01A10-D8 | 0.096 |
| 318518-01 G04-F3 | 0.106 |
| 318518-01 H08-E9 | 0.188 |
| 318518-02A07-B3 | 0.200 |
| 318518-03F04-A6 | 0.184 |
| 318518-03F06-A3 | 0.188 |
| 318518-014A07-C4 | 0.159 |
| 318518-014D08-G1 | 0.165 |

Notch-Inducible Luciferase Assay. A bioassay was developed to determine the ability of selected purified antibodies to neutralize DII4-mediated cellular function *in vitro* using an engineered HEK293 cell line (ATCC) that constitutively expresses human Notch 1 and contains a Notch-responsive promoter driving luciferase. Inhibition of Notch-inducible luciferase activity was determined as follows: 1 day prior to assay, each well of an opaque 96 well tissue culture plate was coated with 100 µl of either 1 nM or 1.5 nM hDII4-hFc in PBS overnight at 4°C. Cells were seeded onto the coated plates at 2 x10⁴ cells/well in media. Purified antibody protein, in serial dilutions starting from 2 nM in cell media, was incubated with the cells at 37°C for 24 hrs. Luciferase activity was determined by adding an equal well volume of STEADY-GLO^{®} Substrate (Promega) (Table 7).

**TABLE 7**

| **Antibody** | **IC₅₀ (pM)** | |
|---|---|---|
| | **1 nM hDII4-hFc** | **1.5 nM hDII4-hFc** |
| REGN281 | 50.5 | 78.7 |
| REGN421 | 54.4 | 87.3 |
| REGN422 | 88.2 | 131.1 |

### Example 4. Inhibition of Notch1 Cleavage

The ability of selected anti-hDII4 antibodies to inhibit Notch1 cleavage was tested by examination of total cleaved Notch1 protein by SDS-Page/Western blotting. Low passage HUVEC cells were cultured as described above. One day prior to analysis 6-well plates were coated with hDII4-hFc in PBS at 4°C overnight (0.2 µg/ml; 1.0 ml PBS/well). Plates were washed 1x with PBS and HUVEC cells were seeded at 7.5 ×10⁵ cells/well in 2.0 ml total media volume. Immediately following cell seeding, anti-hDII4 antibody was added to each well at 10nM final concentration. Cells were grown for 24 hours at 37°C following which whole cell extracts were prepared and analyzed by SDS-PAGE. Levels of cleaved Notch1 were determined using an anti-cleaved Notch1 (Va11744) antibody (Cell Signaling) and standard western blotting techniques. The anti-hDII4 antibodies were able to entirely block the Notch1 cleavage induced by plate coated hDII4-hFc (data not shown).

### Example 5. ADCC and CDC Assays

Antibody-dependent cell-mediated cytotoxicity (ADCC) induced by two test antibodies (REGN421, REGN422) was assessed using a panel of eight target cell lines with varying hDll4 expression levels. The eight target cell lines were (1) HUVECs; (2) HUVECs stimulated with 10 nM VEGF for 24 hours; (3) Colo205; (4) engineered C6 rat glioma cells expressing eGFP; (5) engineered C6 rat glioma cells expressing hDll4; (6) engineered HT1080 cells expressing eGFP; (7) engineered HT1080 cells expressing hDll4; and (8) HT29. Human DII4 or eGFP was integrated into the C6 cell or HT1080 genome through retroviral transfection. Briefly, cells from each target cell line (10,000 cells/well in 50 µl) were first mixed with an equal volume of serially diluted REGN421 or REGN422, resulting in a final antibody concentration ranging from 0.169 pM to 10 nM, and incubated for 10 min at room temperature in a 96-well plate format (control = wells without antibody). Separately, human peripheral blood mononuclear cells (PBMCs, effector cells) were prepared following a conventional Ficoll-Hypaque gradient centrifugation enrichment procedure. Approximately 300,000 PBMCs were added to each mixture of antibody and target cells to give a final ratio of effector to target cells of approximately 30:1. The 96-well plates were then incubated for 4 h at 37°C, 5% CO₂ followed by centrifugation at 250 x g. Supernatants were harvested and assayed for lactate dehydrogenase (LDH) activity using the CYTOTOX 96^{®} Non-Radioactive Cytotoxicity Assay system (Promega). Results are shown in Table 8. REGN421-induced dose dependent cell lysis was only observed in C6 cells expressing hDll4 (col. 5), which exhibited the highest hDll4 expression among all cell lines (as determined by immunoprecipitation/Western blot and flow cytometry). The maximum cell cytotoxicity in the C6-hDll4 cell line ranged from 20% to 60%. No REGN421-induced cell lysis was observed in the remaining seven target cell lines. REGN422 did not induce cell lysis in any of the target cell lines.

**TABLE 8**

| **Ab** | **% Maximum Cytotoxicity** | | | | | | | |
|---|---|---|---|---|---|---|---|---|
| | **1** | **2** | **3** | **4** | **5** | **6** | **7** | **8** |
| REGN421 | 0 | 0 | 0 | 0 | 20-60 | 0 | 0 | 0 |
| REGN422 | 0 | 0 | 0 | 0 | 0 | 0 | 0 | 0 |

Complement-dependent cytotoxicity (CDC) induced by REGN421 was assessed using the same panel of cells lines described above. Briefly, cells from each of the target cell lines (50,000 cells/well in 50 µl) were first mixed with an equal volume of serially diluted REGN421, resulting in a final antibody concentration ranging from 0.169 pM to 10 nM, and incubated for 10 min at room temperature in a 96-well plate format. Normal human serum, with complement components (Quidel Corp., San Diego, CA) was added to each well to give a final serum concentration of 5%. The plates were then incubated at 37°C, 5% CO₂ for 2 hours followed by addition of CELLTITER-BLUE^{®} reagent (Promega) (controls = wells without antibody and wells with antibody but no serum). The plates were incubated overnight and cell survival (CDC levels) assayed. As a positive control, Daudi cells were treated with rituximab. REGN421 exhibited no CDC toward any of the target cell lines tested (data not shown).

### Example 6. Epitope Mapping and Specificity

In order to determine epitope binding specificity, a series of seven chimeric D114 proteins were generated in which specific human D114 domains were substituted into a mouse D114 protein as follows: #1 contained the human N-terminal and DSL domains (S27-Q218); #2 contained human N-terminal, DSL and EGF-1 domains (S27-N252); #3 contained human N-terminal, DSL, EGF-1 and EGF-2 domains (S27-Q283); #4 contained human N-terminal, DSL, EGF-1, EGF-2, EGF-3, EGF-4 and EGF-5;.#5 contained human N-terminal domain (S27-R172); #6 contained human DSL domain (V173-Q218); and #7 contained human EGF-2 domain (E252-D282). The chimeric proteins were fused to a mouse IgG2a-Fc fragment and expressed in CHO-K1 cell. The conditioned media were harvested and protein expression confirmed by western blot.

Binding specificity of test antibodies to hDII4, mDII4, and chimeric proteins #1, #2, #3, and #4 were tested as follows: purified antibodies 22G12, VAW3A7-2, and 15E10 were amine coupled between 5000- 6000 RU on CM5 chip. Conditioned media from CHO K1 cells containing the chimeric DII4 proteins, hDII4-mFc, and mDII4-mFc were injected sequentially followed by surface regeneration over antibody-coupled surfaces. A blank amine coupled flowcell surface was used as a control for nonspecific binding of the conditioned media. Results are summarized in Table 9. 22G12 bound an epitope between S27-Q218 of hDll4; VAW3A7-2 bound an epitope between Q283-E400 hDll4; and 15E10 bound an epitope between E252-D282 of hDII4.

**TABLE 9**

| **Antibody** | **hDII4-mFc** | **mDII4-mFc** | **Chimeric Proteins** | | | |
|---|---|---|---|---|---|---|
| | | | **#1** | **#2** | **#3** | **#4** |
| 22G12 | + | - | + | + | + | + |
| VAW3A7-2 | + | - | - | - | - | + |
| 15E10 | + | - | - | - | + | + |

Binding specificity of purified test mAb to hDII4, mDII4 and the chimeric proteins (described above) was determined (REGN279 = 314266-6F12-B7; REGN287= 318518-1 G04-F3; REGN289= 318518-1H08-E9; REGN290=318518-2A07-B3; REGN306= 318518-3F06-A3). Briefly, each DII4 protein was captured (70-130 RU) on goat anti-mouse IgG antibody surfaces, followed by injection of test mAb at a concentration of 100 µg/ml. An antibody that bound mDII4-mFc was used as a positive control (positive control= 6C10). The results (Table 10) show that REGN279 bound an epitope between S27-Q218 of hDII4; REGN287 bound between Q283-E400 of hDII4; REGN289, REGN290 and REGN306 bound between S27-E400 of hDII4.

**TABLE 10**

| **Antibody** | **hDII4-mFc** | **mDII4-mFc** | **Chimeric human-mouse DII4 Fusion Proteins** | | | | | | |
|---|---|---|---|---|---|---|---|---|---|
| | | | **#1** | **#2** | **#3** | **#4** | **#5** | **#6** | **#7** |
| REGN279 | + | - | + | + | + | + | + | - | - |
| REGN287 | + | - | - | - | - | + | - | - | - |
| REGN289 | + | - | + | + | + | + | - | + | - |
| REGN290 | + | - | + | + | + | + | - | + | - |
| REGN306 | + | - | + | + | + | + | - | + | - |
| Control | + | + | + | + | + | + | + | + | + |

Further epitope binding specificity determinations were conducted as described above with the following purified test antibodies: REGN281=318518-1A10-D8; REGN305=318518-3F04-A6; REGN309=318518-14A07-C4; REGN310=318518-14D08-G1; REGN421, and REGN422. Briefly, each of the DII4 proteins was captured (240-470 RU) on goat anti-mouse IgG antibody surfaces, followed by injection of test antibody at a concentration of 100 µg/ml (Table 11).

**TABLE 11**

| **Antibody** | **hDII4-mFc** | **mDII4-mFc** | **Chimeric human-mouse DII4 Fusion Proteins** | | | | | | |
|---|---|---|---|---|---|---|---|---|---|
| | | | **#1** | **#2** | **#3** | **#4** | **#5** | **#6** | **#7** |
| REGN281 | + | - | + | + | + | + | + | + | - |
| REGN305 | + | - | - | - | - | + | - | - | - |
| REGN309 | + | - | + | + | + | - | + | + | - |
| REGN310 | + | - | - | - | + | + | - | + | + |
| REGN421 | + | - | + | + | + | + | + | + | - |
| REGN422 | + | - | + | + | + | + | + | + | - |

Western blot analysis. Binding specificity of selected antibodies to chimeric, mouse and human DII4 was determined by Western blot. Briefly, hDII4-mFc (200 ng per lane), mDII4-mFc (200 ng per lane), and chimeric proteins #1 - #7 (approximate 150 ng per lane) were subjected to electrophoresis on duplicate SDS-PAGE gels using non-reducing sample buffer. Each gel was then transferred to a PVDF membrane. Blots were first exposed to REGN421 at 0.2 µg/ml and then to HRP-conjugated anti-hlgG antibody (Pierce). Control blots were exposed to HRP-conjugated anti-mFc antibody (Pierce). Results: REGN421 recognized hDII4-mFc and chimeric proteins containing the human N-terminal domain (#5), a human DSL domain (#6), or both (#1, #2, #3, and #4). REGN421 did not recognize a chimeric protein containing a human EGF-2 domain (#7).

Protease digestion analysis. Binding between REGN281 and hDll4 was further assessed by protective protease digestion and liquid chromatography/mass spectrometry (LC/MS) using an HPLC1100 (Agilent) and LCQ Classical Ion Trap Mass Spectrometer (Thermo). Briefly, a mixture of hDII4 and REGN281, in a molar ratio of 1:5, or hDII4 alone, was incubated with protease overnight at either 25°C (for GluC protease) or 37°C (for trypsin). Each of the resulting proteolytic digest mixtures was then subjected to LC/MS. Unique peptide peaks present in proteolytic digests performed in the absence of REGN281, which either diminished or disappeared in proteolytic digests performed in the presence of REGN281, indicate potential REGN281 binding sites on hDII4 that were protected from protease digestion by the binding of REGN281 to hDII4. These unique peptide peaks were analyzed by mass spectrometry. The observed mass, predicted mass, and the N-terminal sequences of the peptides are shown in Table 12.

**TABLE 12**

| **Peak** | **Observed Mass** | **Predicted Mass** | **hDII4 Peptide (SEQ ID NO:2)** | **Protease** | **Domain** |
|---|---|---|---|---|---|
| G1 | 521 | 521.5 | Phe37-Glu40 | GluC | N-terminal |
| G2 | 1362.8 | 1363.4 | Ala121-Glu132 | Gluc | N-terminal |
| T1 | 758 | 760.8 | Pro49-Arg55 | Trypsin | N-terminal |
| T2 | 587.1 | 587.2 | Tyr169-Arg172 | Trypsin | N-terminal |
| T3 | 1607.4 | 1607.6 | Va1173-Arg186 | Trypsin | DSL |
| T4 | 1399 | 1400.7 | Gly42-Arg55 | Trypsin | N-terminal |
| T5 | 569.2 | 569.3 | Thr56-Arg59 | Trypsin | N-terminal |
| T7 | 2615 | 2613.4 | Ile143-Arg166 | Trypsin | N-terminal |
| T8 | 1807.2 | 1806.9 | Ser27-Arg41 | Trypsin | N-terminal |

### Example 7. Binding Affinity of Purified Antibodies to Human and Monkey DII4.

The binding affinities of selected purified antibodies to hDII4, *M. facscicularis* DII4 (mfDll4, SEQ IN NO:956), and *M*. *mulatta* DII4 (mmDII4, SEQ ID NO:957) monomers were determined using a BIACORE™ 2000 & 3000. Goat anti-hFc polyclonal antibody reagent immobilized on a BIACORE™ chip was used to present REGN281, REGN421, and REGN422. Varying concentrations of each proteins, hDII4 (from 12.5 nM to 100 nM), mfDII4 (from 3.13. nM to 100 nM), or mmDII4 (from 12.5 nM to 100 nM) were used as analyte, and injected over the antibody surfaces. Antigen-antibody binding and dissociation of bound complex were monitored in real time (Table 13).

**TABLE 13**

| | **ka (M-1s-1)** | | | **kd (s-1)** | | | **K_{D} (nM)** | | |
|---|---|---|---|---|---|---|---|---|---|
| | hDII4 | mfDII4 | mmDII4 | hDII4 | mfDII4 | mmDII4 | hDII4 | mfDII4 | mmDII4 |
| REGN281 | 1.56 x 10⁵ | 6.64 x10⁴ | 9.27 x 10⁴ | 2.30 x 10⁻⁵ | 2.04 x 10⁻⁵ | 3.05 x 10⁻⁵ | 0.148 | 0.307 | 0.329 |
| REGN421 | 1.63 x 10⁵ | 7.28 x 10⁴ | 9.70x 10⁴ | 2.17 x 10⁻⁵ | 2.02 x 10⁻⁵ | 3.23 x -10⁻⁵ | 0.133 | 0.278 | 0.333 |
| REGN422 | 1.64 x 10⁵ | 8.01 x 10⁴ | 9.27 x 10⁴ | 2.36 x 10⁻⁵ | 2.88 x 10⁻⁵ | 3.41 x 10⁻⁵ | 0.144 | 0.360 | 0.375 |

The binding affinities of anti-hDII4 antibodies toward hDll4 and mmDll4 dimers were also determined using a BIACORE^{™} 2000 and the method describe above, except that hDll4 was replaced with hDI14-mFc (from 3.13 nM to 100 nM), or mmDII4 with mmDII4-mFc (from 0.78 nM to 25 nM) as analyte (Table 14).

**TABLE 14**

| | **ka (M-1 s-1)** | | **kd (s-1)** | | **K_{D} (nM)** | |
|---|---|---|---|---|---|---|
| | hDII4-mFc | mmDII4-mFc | hDII4-mFc | mmDII4-mFc | hDII4-mFc | mmDII4-mFc |
| REGN281 | 3.02 x 10⁵ | 3.16 x 10⁵ | 4.96 x 10⁻⁶ | 4.64 x 10⁻⁶ | 0.0163 | 0.0147 |
| REGN421 | 3.43 x 10⁵ | 3.35 x 10⁵ | 4.70 x 10⁻⁶ | 3.80 x 10⁻⁶ | 0.0137 | 0.013 |
| REGN422 | 3.46 x 10⁵ | 4.23 x 10⁶ | 4.60 x 10⁻⁶ | 4.15 x 10⁻⁶ | 0.0133 | 0.0098 |

### Example 8. Cross-Reactivity of Antibodies With hDII1, hDII3, mDI14, or mfDII4.

Cross-reactivity of the antibodies to human delta-like ligand1 (SEQ ID NO:953) and human delta-like ligand 3 (SEQ ID NO:954) proteins was determined. REGN281, REGN421 and REGN422 were presented by a goat anti-human kappa (hK) polyclonal antibody reagent (Southern Biotech) immobilized on a BIACORE™ chip, and either hDII4-hFc or hDII1-hFc protein at 100 µg/ml were used as analyte injected over the antibody surfaces. All three anti-hDll4 antibodies only bound to hDII4-hFc, and did not bind hDII1-hFc.

An alternative BIACORE™ format was used to assess cross-reactivity between anti-hDII4 antibody and either hDII1-hFc or hDII3-hFc. Briefly, ligands hDII4-hFc, hDII1-hFc, and hDII3-hFc were each covalently linked to a CM-5 chip, through amine coupling, to an RU range of about 8,000 to 10,000. REGN421, at 300 µg/ml; was injected over the surface of each chip. REGN421 bound only to hDII4-hFc; no binding was observed to either hDII1-hFc or hDII3-hFc. The same result was observed for REGN422 instead of REGN421.

OCTET™-based binding assays were employed to determine the binding between selected purified anti-hDll4 antibodies and hDI1I4-hFc, hDII3-hFc, hDII1-hFc, mfDII4-mmh, or mDII4-mFc. Briefly, strepavidin high binding FA biosensors (ForteBio, Inc., Menlo Park, CA) were first incubated with biotin-anti-hK at 5 µg/ml for 10 min at 30°C, to achieve saturation. Biotin-anti-hK-bound biosensors were then incubated with antibodies REGN281, REGN421 or REGN422, at 20 µg/ml for 10 min at 30°C, to achieve saturation. The antibody-bound biosensors were then incubated with either hDII4-hFc or hDII3-hFc, hDII1-hFc, or mDII4-mFc, at 200 nM, or mfDll4-mmh at 100 nM, for 10 min at 30°C. Changes in the thickness of the biological layer after each incubation were measured. Human DII4-hFc and mfDII4-mmh bound to anti-hDII4-antibody-bound-biosensors, whereas hDII3-hFc, hDII1-hFc, and mDII4-mFc did not bind to anti-hDll4 antibody-bound biosensors.

### Example 9. Effect of Anti-hDII4 Antibody on Tumor Growth

The effect of REGN421 on tumor growth was evaluated on tumors implanted in Severe Combined Immunodeficiency (SCID) mice expressing a humanized DII4 protein (SCIDxhDII4). Briefly, the humanized DII4 mouse was made by replacing the entire extracellular domain of the mouse DII4 gene with the corresponding extracellular region of the human DII4 gene (7kb) in embryonic stem (ES) cells. Homozygous hDII4 mice were generated and bred into SCID background. Each mouse was then implanted subcutaneously (SC) with 2.5 x 10⁶ human HT1080 tumor cells. After the tumors were established in the mice (~ 100-150 mm³ 18 days after implantation), mice were measured and treated with hFc, hDII4-Fc or REGN421. A total of 7 mice were divided into three groups. The first group (n=3) was treated subcutaneously with hFc at 25 mg/kg; the second group (n=1) was treated with hDll4-Fc at 25 mg/kg; and the third group (n=3) was treated with REGN421 at 10 mg/kg. The treatments were repeated every 48 hours starting at day 18. *In vivo* tumor measurements were obtained three days before the initial treatment (day 15), on the same day of each treatment (days 18, 20, and 22), and on day 25. Tumor size was calculated using the formula I x w²/2. Results are shown in Table 15. On day 25, mice were euthanized and each tumor was removed and measured *ex vivo* and calculated (length x width x depth) (Table 16).

In addition, a group of SCID mice expressing endogenous mDII4 (n=2) was implanted with tumor cells and treated with hDII4-Fc (25 mg/kg) following the same dosing schedule.

**TABLE 15**

| **Mouse** | **Treatment** | **Tumor Size (mm³)** | | | | |
|---|---|---|---|---|---|---|
| | | **Day 15** | **Day 18** | **Day 20** | **Day 22** | **Day 25** |
| SCID | hDII4-hFc | 162.0 | 232.8 | 320.0 | 336.0 | 253.1 |
| SCID | hDII4-hFc | 22.5 | 117.0 | 117.0 | 108.0 | 68.8 |
| SCIDxhDI14 | hDII4-h Fc | 288.0 | 320.0 | 352.0 | 446.0 | 320.0 |
| SCIDxhDII4 | hFc | 162.0 | 288.0 | 320.0 | 500.0 | 550.0 |
| SCIDxhDII4 | hFc | 162.0 | 220.5 | 352.0 | 662:0 | 661.5 |
| SCIDxhDII4 | hFc | 93.8 | 135.0 | 179.6 | 352.0 | 726.0 |
| SCIDxhDII4 | REGN421 | 144.0 | 245.0 | 320.0 | 162.0 | 144.0 |
| SCIDxhDII4 | REGN421 | 87.5 | 162.0 | 153.0 | 225.0 | 135.0 |
| SCIDxhDII4 | REGN421 | 144.0 | 196.0 | 272.0 | 162.0 | 152.5 |

**TABLE 16**

| **Mouse** | **Treatment** | **Tumor Size (mm³)** |
|---|---|---|
| SCID | hDII4-hFc | 308.0 |
| SCID | hDII4-hFc | 105.0 |
| SCIDxhDII4 | hDII4-hFc | 480.0 |
| SCIDxhDII4 | hFc | 924.0 |
| SCIDxhDII4 | hFc | 1020.0 |
| SCIDxhDII4 | hFc | 792.0 |
| SCIDxhDII4 | REGN421 | 168.0 |
| SCIDxhDII4 | REGN421 | 84.0 |
| SCIDxhDII4 | REGN421 | 189.0 |

## Claims

1. A human antibody or antibody fragment which specifically binds human delta-like ligand 4 (hD114), said antibody or antibody fragment comprising a heavy chain variable region/light chain variable region (HCVR/LCVR) selected from the amino acid sequence pairs of SEQ ID NO:429/437 and 901/903.

2. A human antibody or antibody fragment of claim 1, further comprising a constant region selected from the group consisting of SEQ ID NO:950, 951 and 952.

3. An isolated nucleic acid molecule encoding an antibody or antigen-binding fragment according to claim 1 or 2.

4. A vector comprising the nucleic acid molecule of claim 3.

5. A host cell comprising a vector according to claim 4.

6. A host cell according to claim 5, which is a prokaryotic or eukaryotic cell selected from one of an *E*. *coli* or a CHO cell.

7. A method for producing an anti-human D114 antibody or antigen-binding fragment thereof, comprising growing host cells according to claim 5 or 6 under conditions permitting production of the antibody or fragment thereof and recovering the antibody or fragment so produced.

8. Use of an antibody or antigen-binding fragment of an antibody according to claim 1 or 2 in the manufacture of a medicament for treating cancer in a human.

9. An antibody or antibody fragment according to claim 1 or 2 for use in a method of treating cancer in a human patient.

10. A composition comprising an antibody or antibody fragment according to claim 1 or 2 and an acceptable carrier.

## Patentansprüche

1. Humaner Antikörper oder Antikörper-Fragment, der/das spezifisch humanen delta-ähnlichen Liganden 4 (hDll4) bindet, wobei der Antikörper oder das Antikörper-Fragment eine variable Region einer schweren Kette/eine variable Region einer leichten Kette (HCVR/LCVR) umfasst, ausgewählt aus den Aminsäure-Sequenzpaaren von SEQ ID NO:429/437 und 901/903.

2. Humaner Antikörper oder Antikörper-Fragment gemäß Anspruch 1, der/das weiter eine konstante Region umfasst, ausgewählt aus der Gruppe, bestehend aus SEQ ID NO:950, 951 und 952.

3. Isoliertes Nucleinsäuremolekül, das einen Antikörper oder ein Antigen-bindendes Fragment gemäß Anspruch 1 oder 2 codiert.

4. Vektor, der das Nucleinsäuremolekül gemäß Anspruch 3 umfasst.

5. Wirtszelle, die einen Vektor gemäß Anspruch 4 umfasst.

6. Wirtszelle gemäß Anspruch 5, die eine prokaryontische oder eukaryontische Zelle ist, ausgewählt aus einer *E*. *coli-*oder einer CHO-Zelle.

7. Verfahren zur Herstellung eines anti-humanen Dll4-Antikörpers oder Antigen-bindenden Fragments davon, umfassend das Anzüchten von Wirtszellen gemäß Anspruch 5 oder 6 unter Bedingungen, die die Herstellung des Antikörpers oder des Fragments davon gestatten, und Gewinnen des Antikörpers, oder Fragments, der/das so hergestellt wurde.

8. Verwendung eines Antikörpers oder Antigen-bindenden Fragments eines Antikörpers gemäß Anspruch 1 oder 2 zur Herstellung eines Medikaments zur Behandlung von Krebs bei einem Menschen.

9. Antikörper oder Antikörper-Fragment gemäß Anspruch 1 oder 2 zur Verwendung in einem Verfahren zur Behandlung von Krebs bei einem humanen Patienten.

10. Zusammensetzung, die einen Antikörper oder ein Antikörper-Fragment gemäß Anspruch 1 oder 2, und einen verträglichen Träger umfasst.

## Revendications

1. Anticorps humain ou fragment d'anticorps humain, qui se lie spécifiquement au ligand humain hD114 (human delta-like ligand 4), lequel anticorps ou fragment d'anticorps comprend une paire de régions HCVR/LCVR (région variable de chaîne lourde / région variable de chaîne légère) choisie parmi les paires de séquences d'acides aminés formées par les Séquences N° 429/437 et par les Séquences N° 901/903.

2. Anticorps humain ou fragment d'anticorps humain, conforme à la revendication 1, qui comprend en outre une région constante choisie dans l'ensemble formé par les Séquences N° 950, 951 et 952.

3. Molécule d'acide nucléique isolée codant un anticorps, ou un fragment se liant à l'antigène, conforme à la revendication 1 ou 2.

4. Vecteur comprenant une molécule d'acide nucléique conforme à la revendication 3.

5. Cellule hôte comportant un vecteur conforme à la revendication 4.

6. Cellule hôte conforme à la revendication 5, qui est une cellule procaryote ou eucaryote, choisie parmi une cellule d'E. *coli* et une cellule CHO.

7. Procédé de production d'un anticorps anti-ligand D114 humain ou d'un fragment se liant à l'antigène d'un tel anticorps, lequel procédé comporte le fait de faire croître des cellules hôtes, conformes à la revendication 5 ou 6, dans des conditions permettant la production de l'anticorps ou de son fragment, et le fait de récupérer l'anticorps ou le fragment ainsi produit.

8. Utilisation d'un anticorps, ou d'un fragment se liant à l'antigène d'un anticorps, conforme à la revendication 1 ou 2, dans la fabrication d'un médicament destiné au traitement d'un cancer chez un humain.

9. Anticorps ou fragment d'anticorps conforme à la revendication 1 ou 2 pour utilisation dans un procédé de traitement d'un cancer chez un humain.

10. Composition comprenant un anticorps ou un fragment d'anticorps, conforme à la revendication 1 ou 2, et un véhicule admissible.
